(19) Europäisches Patentamt
European Patent Office
Office européen des brevets

(11) EP 3 599 853 B1

(12) **EUROPEAN PATENT SPECIFICATION**

(45) Date of publication and mention of the grant of the patent:
**30.07.2025 Bulletin 2025/31**

(21) Application number: **18771880.4**

(22) Date of filing: **21.03.2018**

(51) International Patent Classification (IPC):
***A01N 43/90*** *(2006.01)* ***A01P 13/00*** *(2006.01)*
***C12N 15/82*** *(2006.01)* ***A01H 5/10*** *(2018.01)*
***C07D 307/93*** *(2006.01)* ***C07D 303/38*** *(2006.01)*
***A01N 63/34*** *(2020.01)* ***C07K 14/38*** *(2006.01)*

(52) Cooperative Patent Classification (CPC):
**A01N 43/90; A01N 63/32; A01N 63/34; C07D 303/38; C07D 307/93; C07K 14/38; C12N 15/8274**

(86) International application number:
**PCT/US2018/023630**

(87) International publication number:
**WO 2018/175635 (27.09.2018 Gazette 2018/39)**

(54) **HERBICIDAL COMPOSITIONS AND METHODS OF USE THEREOF**
HERBIZIDE ZUSAMMENSETZUNG UND VERFAHREN ZUR VERWENDUNG DAVON
COMPOSITIONS HERBICIDES ET LEURS PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.03.2017 US 201762474528 P**

(43) Date of publication of application:
**05.02.2020 Bulletin 2020/06**

(73) Proprietor: **The Regents of the University of California**
**Oakland, CA 94607-5200 (US)**

(72) Inventors:
- **YAN, Yan**
  **Los Angeles, California 90095-1406 (US)**
- **TANG, Yi**
  **San Gabriel**
  **California 91775 (US)**
- **JACOBSEN, Steve E.**
  **Agoura Hills**
  **California 91301 (US)**
- **LIU, Qikun**
  **Walnut**
  **California 91789 (US)**

(74) Representative: **J A Kemp LLP**
**80 Turnmill Street**
**London EC1M 5QU (GB)**

(56) References cited:
**US-A- 5 185 023**

- **ATSUMI SHIMADA ET AL: "Aspterric Acid and 6-Hydroxymellein, Inhibitors of Pollen Development in Arabidopsis thaliana, Produced by Aspergillus terreus", ZEITSCHRIFT FUER NATURFORSCHUNG. C, A JOURNAL OF BIOSCIENCES., vol. 57, no. 5-6, 1 June 2002 (2002-06-01), DE, pages 459 - 464, XP055543311, ISSN: 0939-5075, DOI: 10.1515/znc-2002-5-610**
- **ATSUMI SHIMADA ET AL: "Interaction between Aspterric Acid and Indole-3-acetic Acid on Reproductive Growth in Arabidopsis thaliana", ZEITSCHRIFT FUER NATURFORSCHUNG. C, A JOURNAL OF BIOSCIENCES., vol. 60, no. 7-8, 1 August 2005 (2005-08-01), DE, pages 572 - 576, XP055723992, ISSN: 0939-5075, DOI: 10.1515/znc-2005-7-810**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **OXTOBY E ET AL: "Engineering herbicide tolerance into crops", TRENDS IN BIOTECHNOLOGY, ELSEVIER PUBLICATIONS, CAMBRIDGE, GB, vol. 8, 1 January 1990 (1990-01-01), pages 61 - 65, XP023471329, ISSN: 0167-7799, [retrieved on 19900101], DOI: 10.1016/ 0167-7799(90)90137-M**
- **B. T. BROWN: "A new screening procedure for detecting plant growth regulating compounds", PESTICIDE SCIENCE., vol. 3, no. 2, 1 April 1972 (1972-04-01), GB, pages 161 - 168, XP055724159, ISSN: 0031-613X, DOI: 10.1002/ps.2780030208**
- **YAN YAN ET AL: "Resistance-gene-directed discovery of a natural-product herbicide with a new mode of action", NATURE, MACMILLAN JOURNALS LTD., ETC, LONDON, vol. 559, no. 7714, 11 July 2018 (2018-07-11), pages 415 - 418, XP036553228, ISSN: 0028-0836, [retrieved on 20180711], DOI: 10.1038/S41586-018-0319-4**
- **SHIMADA ET AL.: "Aspterric Acid and 6-Hydroxymellein, Inhibitors of Pollen Development in Arabidopsis thaliana, Produced by Aspergillus terreus", Z NATURFORSCH C, vol. 57, no. 5-6, May 2002 (2002-05-01), pages 459 - 464, XP055543311**

Remarks:

The complete document including Reference Table(s) and the Sequence Listing(s) can be downloaded from the EPO website

## Description

### FIELD

The present disclosure relates generally to herbicidal compositions and methods of use thereof, and more specifically to herbicidal compositions containing aspterric acid or a derivative thereof for use in inhibiting vegetative growth in plants.

### BACKGROUND

As herbicides are increasingly applied in crop production worldwide, the demand for herbicides with novel modes of action becomes ever more urgent, mainly due to continuously emerging weed resistance. According to an international survey of herbicide resistant weeds, there are currently 478 unique cases of herbicide resistant weeds globally within 252 species including 147 dicots and 105 monocots. Weeds have evolved resistance to 23 of the 26 known herbicide sites of action and to 161 different herbicides. Accordingly, there exists a need for the development of new herbicidal compositions.

Shimada, A., et al. (2005), Zeitschrift für Naturforschung, Vol. 60, No. 7-8, pages 572-576, , discloses the interaction between aspterric acid and indole-3-acetic acid on reproductive growth in *Arabidopsis thaliana.*

### BRIEF SUMMARY OF THE INVENTION

The invention provides a method of reducing growth of a vegetative tissue in a plant, the method comprising:

spraying a vegetative tissue in the plant one or more times with a herbicidally active amount of a composition comprising (a) aspterric acid and (b) glufosinate;
wherein growth of the vegetative tissue in the plant is reduced as compared to a corresponding control plant not receiving the composition.

Further aspects and preferred embodiments are defined in the dependent claims. Any aspects, embodiments and examples of the present disclosure which do not fall under the scope of the appended claims do not form part of the invention and are merely provided for illustrative purposes.

### BRIEF SUMMARY OF TECHNICAL TEACHINGS

In one aspect, the present disclosure provides a method of reducing growth of a vegetative tissue in a plant, the method including: a) contacting the plant with a composition including aspterric acid or derivative thereof; and b) maintaining the plant under conditions such that growth of the vegetative tissue in the plant is reduced as compared to a corresponding control plant. In some aspects of the disclosure, the composition further includes an ingredient selected from the group of silwet L-77, DMSO, ethanol, corn oil, tween 80, and glufosinate. In some aspects of the disclosure that may be combined with any of the preceding aspects of the disclosure, the concentration of aspterric acid or derivative thereof in the composition is in the range of about 25 $\mu$M to about 75 $\mu$M. In some aspects of the disclosure that may be combined with any of the preceding aspects of the disclosure, the concentration of aspterric acid or derivative thereof in the composition is in the range of about 50 $\mu$M to about 300 $\mu$M. In some aspects of the disclosure that may be combined with any of the preceding aspects of the disclosure, the concentration of aspterric acid or derivative thereof in the composition is in the range of about 0.5 mM to about 1.5 mM. In some aspects of the disclosure that may be combined with any of the preceding aspects of the disclosure, the plant is grown in a growth medium including soil or agar. In some aspects of the disclosure that may be combined with any of the preceding aspects of the disclosure, the contacting occurs on multiple occasions over a time interval. In some aspects of the disclosure that may be combined with any of the preceding aspects of the disclosure, the contacting occurs for a total duration of about one week to about one month. In some aspects of the disclosure that may be combined with any of the preceding aspects of the disclosure, the growth rate of the vegetative tissue in the plant is reduced by at least about 50% as compared to a corresponding control plant.

In another aspect, the present disclosure provides a method of generating an aspterric acid-resistant plant, the method including: a) providing a plant that is susceptible to aspterric acid; b) contacting the plant with a nucleic acid encoding an AstD polypeptide; and c) maintaining the plant under conditions such that the nucleic acid is expressed and produces an AstD protein, thereby generating a plant having increased resistance to aspterric acid as compared to a corresponding control. In some aspects of the disclosure, the AstD polypeptide includes an amino acid sequence having at least 80% sequence identity to SEQ ID NO: 10. In some aspects of the disclosure, the AstD polypeptide includes an amino acid sequence having at least 95% sequence identity to SEQ ID NO: 10. In some aspects of the disclosure that may be

combined with any of the previous aspects of the disclosure, the AstD polypeptide further includes a chloroplast localization sequence. In some aspects of the disclosure that may be combined with any of the previous aspects of the disclosure, the plant having increased resistance to aspterric acid exhibits a rate of development of one or more herbicidal symptoms when contacted with aspterric acid that is at least about 50% reduced as compared to a corresponding control.

In another aspect, the present disclosure provides an aspterric acid-resistant plant, the plant including a nucleic acid encoding an AstD polypeptide. In some aspects of the disclosure, the AstD polypeptide includes an amino acid sequence having at least 80% sequence identity to SEQ ID NO: 10. In some aspects of the disclosure, the AstD polypeptide includes an amino acid sequence having at least 95% sequence identity to SEQ ID NO: 10. In some aspects of the disclosure that may be combined with any of the previous aspects of the disclosure, the AstD polypeptide further includes a chloroplast localization sequence. In some aspects of the disclosure that may be combined with any of the previous aspects of the disclosure, the plant exhibits a rate of development of one or more herbicidal symptoms when contacted with aspterric acid that is at least about 50% reduced as compared to a corresponding control.

In another aspect, the present disclosure provides a method of producing hybrid seed, the method including: a) obtaining a first parent plant and a second parent plant; b) treating a flower from the first parent plant with aspterric acid or derivative thereof in a quantity sufficient to inhibit pollen development in said flower; and c) crossing the first parent plant treated with aspterric acid or derivative thereof with the second parent plant to create progeny seed, wherein all progeny seed are hybrids of the first parent plant and the second parent plant.

In another aspect, the present disclosure provides a method of reducing growth of a vegetative tissue in a plant, the method including: a) contacting the plant with a composition including a compound that is a DHAD polypeptide inhibitor; and b) maintaining the plant under conditions such that growth of the vegetative tissue in the plant is reduced as compared to a corresponding control plant. In some aspects of the disclosure, the compound that is a DHAD polypeptide inhibitor is aspterric acid or a derivative thereof. In some aspects of the disclosure that may be combined with any of the preceding aspects of the disclosure, the composition further includes an ingredient selected from the group of silwet L-77, DMSO, ethanol, corn oil, tween 80, and glufosinate. In some aspects of the disclosure that may be combined with any of the preceding aspects of the disclosure, the plant is grown in a growth medium including soil or agar. In some aspects of the disclosure that may be combined with any of the preceding aspects of the disclosure, the contacting occurs on multiple occasions over a time interval. In some aspects of the disclosure that may be combined with any of the preceding aspects of the disclosure, the contacting occurs for a total duration of about one week to about one month. In some aspects of the disclosure that may be combined with any of the preceding aspects of the disclosure, the growth rate of the vegetative tissue in the plant is reduced by at least about 50% as compared to a corresponding control plant.

In another aspect, the present disclosure provides a method of generating an aspterric acid-resistant plant, the method including: a) providing a plant that contains a nucleic acid which encodes a DHAD polypeptide that is susceptible to inhibition by aspterric acid or a derivative thereof; and b) modifying the DHAD polypeptide-encoding nucleic acid in the plant such that the resulting DHAD polypeptide activity has reduced susceptibility to inhibition by aspterric acid or a derivative thereof to generate a plant having reduced susceptibility to one or more herbicidal symptoms that are induced by aspterric acid or a derivative thereof as compared to a corresponding control plant.

In another aspect, the present disclosure provides a plant having reduced susceptibility to one or more herbicidal symptoms that are induced by aspterric acid as compared to a corresponding control plant.

## DESCRIPTION OF THE FIGURES

**FIG. 1** illustrates the branched-chain amino acid (valine, leucine and isoleucine) biosynthetic pathway.

**FIG. 2A** illustrates biological gene clusters (BGCs) identified through a target-guided genome mining approach. **FIG. 2B** illustrates the biochemical reaction that is catalyzed by DHAD.

**FIG. 3** illustrates the expression of AstA, AstB, and AstC in *Saccharomyces cerevisiae. astA, astB, and astC* were cloned into expression vectors and transformed into *Saccharomyces cerevisiae,* either independently or in combination. Synthesized products that were identified with 1D and 2D NMR spectroscopy are shown on the right side of the figure.

**FIG. 4** illustrates a proposed biosynthetic pathway for the production of aspterric acid.

**FIG. 5A-FIG. 5E** illustrates the results of enzymatic activity and aspterric acid inhibition assays for the *Arabidopsis thaliana* housekeeping DHAD enzyme. **FIG. 5A** illustrates the DHAD enzymatic reaction and phenylhydrazine

derivatization reaction used for enzymatic activity detection. **FIG. 5B** illustrates the results of the phenylhydrazine derivatization control reaction. **FIG. 5C** and **FIG. 5D** illustrate the results of DHAD enzymatic activity assays in the presence or absence of DMSO and show that DHAD is enzymatically functional. **FIG. 5E** illustrates the results of aspterric acid inhibition assays and shows that aspterric acid inhibits DHAD enzymatic activity.

**FIG. 6A** and **FIG. 6B** illustrate the $IC_{50}$ of aspterric acid on the *Aspergillus terreus* housekeeping DHAD and the *Arabidopsis thaliana* housekeeping DHAD, respectively. **FIG. 6C** illustrates the inhibition kinetics of aspterric acid on the *Arabidopsis thaliana* housekeeping DHAD. **FIG. 6D** illustrates linear fitting of inhibition kinetics data to obtain the $K_i$ of aspterric acid on the *Arabidopsis thaliana* housekeeping DHAD.

**FIG. 7** illustrates a proposed model for inhibition of the DHAD active site by aspterric acid.

**FIG. 8** illustrates a proposed model for inhibition of the DHAD active site by derivatives of aspterric acid.

**FIG. 9A** and **FIG. 9B** illustrate cytotoxicity data of aspterric acid compared to glyphosate on two human tumor cell lines, as determined by MTT cytotoxicity assays.

**FIG. 10A** illustrates growth of different phototrophic *Saccharomyces cerevisiae* strains (DHY210, DHY211, and DHY212) when plated on media that contains aspterric acid and lacks isoleucine, leucine, and valine (bottom row), as compared to the growth of *Saccharomyces cerevisiae* when plated on media that does not contain aspterric acid and lacks isoleucine, leucine, and valine (top row). **FIG. 10B** illustrates growth of *Streptomyces* when plated on MS media that contains aspterric acid (bottom row), as compared to the growth of *Streptomyces* when plated on MS media that does not contain aspterric acid (top row).

**FIG. 11** illustrates growth and development *Arabidopsis thaliana* seedlings that were plated on MS media and grown for 4 days, and then transferred to MS media containing 50 μM aspterric acid (right panel), as compared to *Arabidopsis thaliana* seedlings that were plated on DMSO control plates that lacked aspterric acid (left panel) when observed on day 8 and day 12.

**FIG. 12** illustrates growth and development of green bean seedlings that were grown on MS media containing 50 μM aspterric acid (right panel), as compared to green bean seedlings that were plated on DMSO control media that lacked aspterric acid (left panel) when observed on day 3 and day 7.

**FIG. 13** illustrates growth and development of tomato seedlings that were grown on MS media containing 50 μM aspterric acid (middle panel), as compared to tomato seedlings that were plated on DMSO control media that lacked aspterric acid (left panel) or that were plated on media containing glyphosate (right panel) when observed on day 3 and day 7.

**FIG. 14** illustrates an herbicidal spray experiment where aspterric acid dissolved in formulation (1) was sprayed on soil-grown *Arabidopsis thaliana* Col-0 ecotype plants every two days. Plants were compared to other plants treated with various other formulations.

**FIG. 15** illustrates an herbicidal spray experiment where aspterric acid dissolved in formulation (2) was sprayed on soil-grown *Arabidopsis thaliana* Col-0 ecotype plants every two days. Plants were compared to other plants treated with various other formulations.

**FIG. 16** illustrates an herbicidal spray experiment where aspterric acid dissolved in formulation (3) was sprayed on soil-grown glufosinate-resistant *Arabidopsis thaliana* Col-0 ecotype plants every two days. Plants were compared to other plants treated with various other formulations.

**FIG. 17** illustrates an exemplary transformation and selection scheme for introducing a heterologous *astD* gene into plants.

**FIG.18A-FIG. 18C** illustrates the function and evolution of DHAD. **FIG. 18A** illustrates parallel pathways of BCAA biosynthesis. Valine, leucine and isoleucine are produced by two parallel pathways using three enzymatic steps: ALS, KARI and DHAD. **FIG. 18B** illustrates a phylogenetic tree of DHAD among bacteria, fungi and plants. **FIG. 18C** illustrates representatives of inhibitors that inhibit DHAD *in vitro,* but fail to inhibit plant growth.

**FIG. 19A** and **FIG. 19B** illustrates an alignment of amino acid sequences of DHADs from different plant species. The identity of DHAD among flowering plant is around 80%. The lack of identity at the N-terminal of these DHAD results from the differences in chloroplast localization signals from different species. Chlamydomonas_reinhardtii (SEQ ID NO: 22), Physcomitrella_patens (SEQ ID NO: 23), Zea_mays (SEQ ID NO: 6), Solanum_lycopersicum (SEQ ID NO: 7), Glycine_max (SEQ ID NO: 5), Arabidopsis_thiliana (SEQ ID NO: 4), Populus_euphratica (SEQ ID NO: 24).

**FIG. 20** illustrates examples of co-localization of biosynthetic gene clusters (BGCs) and targets. The biosynthetic core genes are shown in blue and the self-resistance enzymes (SREs) are shown in red. Upper panel: the blockbuster cholesterol-lowering lovastatin drug targets HMG-CoA reductase (HMGR) in eukaryotes. In the fungus *Aspergillus terreus* that produces lovastatin, a second copy of HMGR encoded by ORF8 is present in the gene cluster. Lower panel: BGC of the immunosuppressant mycophenolic acid from *Penicillium sp.* contains a second copy of inosine monophosphate dehydrogenase (IMPDH), which represents the SRE to this cluster.

**FIG. 21A-FIG. 21C** illustrate genome mining of a DHAD inhibitor and biosynthesis of aspterric acid **(AA). FIG. 21A** illustrates a 17 kb gene cluster from *A. terreus* containing four ORFs, which are also conserved among several fungal species. AstA has sequence homology to sesquiterpene cyclase; AstB and AstC are predicted to be P450 monooxygenases; AstD is predicted to encode a DHAD, and is proposed to confer self-resistance in the presence of the NP produced in the cluster. **FIG. 21B** illustrates HPLC-MS traces of metabolites produced from *S. cerevisiae* RC01 expressing different *ast* genes under $P_{ADH2}$ promoter control. i: *S. cerevisiae* without expression plasmids. The negative ion peak at 10 minutes (pink) represents a yeast metabolite. ii: *S. cerevisiae* transformed with plasmids expressing *astA* and *astB* produces **2**. iii: *S. cerevisiae* transformed with plasmids expressing *astA-C* produces **AA** at a titer of 20 mg/L. **FIG. 21C** illustrates a proposed biosynthetic pathway of **AA.** AstA cyclizes farnesyl diphosphate (FPP) into (-)-daucane **1,** while the P450 enzymes AstB and AstC sequentially transform **1** into **2** and **3 (AA),** respectively.

**FIG. 22A** and **FIG. 22B** illustrates an alignment of amino acid sequences of AstD and housekeeping DHAD from different strains. The identity of AstD and housekeeping DHAD is around 70% in each strain. DHAD_A. terreus (SEQ ID NO: 1), DHAD_A. fischeri (SEQ ID NO: 2), DHAD_P.brasilianum (SEQ ID NO: 3), AstD_A. terreus (SEQ ID NO: 10), AstD_A. fischeri (SEQ ID NO: 11), AstD_P. brasilianum (SEQ ID NO: 12).

**FIG. 23A-FIG. 23L** illustrates NMR analyses of compounds. Numbered compounds are those identified in **FIG. 21B** and **FIG. 21C. FIG. 23A** illustrates $^1H$ NMR of compound **1** (500 MHz, $CDCl_3$). **FIG. 23B** illustrates $^{13}C$ NMR of compound **1** (125 MHz, $CDCl_3$). **FIG. 23C** illustrates HSQC of compound **1** (500 MHz, $CDCl_3$). **FIG. 23D** illustrates HMBC of compound **1** (500 MHz, $CDCl_3$). **FIG. 23E** illustrates $^1H$ NMR of compound **2** (500 MHz, $CDCl_3$). **FIG. 23F** illustrates $^{13}C$ NMR of compound 2 (125 MHz, $CDCl_3$). **FIG. 23G** illustrates HSQC of compound **2** (500 MHz, $CDCl_3$). **FIG. 23H** illustrates HMBC of compound **2** (500 MHz, $CDCl_3$). **FIG. 23I** illustrates $^1H$ NMR of **AA** (500 MHz, $CDCl_3$). **FIG. 23J** illustrates $^{13}C$ NMR of **AA** (125 MHz, $CDCl_3$). **FIG. 23K** illustrates HSQC of **AA** (500 MHz, $CDCl_3$). **FIG. 23L** illustrates HMBC of **AA** (500 MHz, $CDCl_3$). **FIG. 23M** illustrates EI-MS of compound **1** by GC-MS analysis. The structure of compound **1** (top right) and its known enantiomer (+)-Dauca-4(11),8-diene (top left). The EI-MS of compound **1** (bottom). The EI-MS spectrum of (+)-Dauca-4(11),8-diene is reported as m/z (rel.int): 204 $[M]^+$ (22), 189 $[M-Me]^+$ (2), 161 (18), 148 (3), 136 (100), 133 (10), 121 (60), 119 (10), 107 (17) 105 (15), 93 (19), 91 (18), 79 (12), 77 (11), 55 (10), 41 (22). The EI-MS of both compound **1** and (+)-Dauca-4(11),8-diene are identical (Cool et al., 2001).

**FIG. 24A-FIG. 24D** illustrates that aspterric acid **(AA)** is a plant growth inhibitor. **FIG. 24A** illustrates 2-week old *Arabidopsis thaliana* growing on MS media containing no **AA** (left) or 50 μM **AA** (right). **FIG. 24B** illustrates 2-week old dicot *Solanum lycopersicum* and monocot *Zea mays* growing on MS media containing no **AA** (left) or 50 μM **AA** (right). The picture shown is representative of two replicates. The same assays were repeated twice. **FIG. 24C** illustrates verification of the self-resistance function of AstD. Growth inhibition curve of **AA** on *S. cerevisiae ΔILV3* strains expressing fungal (*Aspergillus terreus*) housekeeping DHAD (fDHAD) (blue) and AstD (orange) in isoleucine, leucine and valine (ILV) dropout media. This yeast strain is unable to grow in this media without complementation with either ILV or a functional DHAD. Percent inhibition is calculated by dividing the cell density ($OD_{600}$) of the **AA**-treated strain to the corresponding untreated strains when $OD_{600}$ reaches ~ 0.8 (center values are averages, errors bars are s.d., n = 3). **AA** is able to inhibit the growth of fDHAD-complemented yeast with $IC_{50}$ ~ 2 μM, while an $IC_{50}$ ~ 200 μM is required to inhibit growth of AstD-complemented yeast. **FIG. 24D** illustrates root length of **AA** treated Arabidopsis. Wild type *A. thaliana* was grown on MS media with and without 250 μM **AA.** The lengths of roots were measured at four different time points after seed germination. Each group contains 23 individual replicates.

**FIG. 25A-FIG. 25C** illustrates SDS-PAGE analysis of purified proteins. **FIG. 25A** illustrates SDS-PAGE analysis of

purified *Arabidopsis thaliana* DHAD (pDHAD) (~62 kD) from *E. coli* BL21 (DE3). **FIG. 25B** illustrates SDS-PAGE analysis of purified *Aspergillus terrerus* DHAD (fDHAD) (~62 kD) from *E. coli* BL21 (DE3). **FIG. 25C** illustrates SDS-PAGE analysis of purified AstD (~62kD) from *E. coli* BL21 (DE3).

**FIG. 26A-FIG. 26B** illustrates biochemical assays of DHAD functions. **FIG. 26A** illustrates assaying DHAD activities in converting the dihydroxyacid **4** into the $\alpha$-ketoacid **5.** Formation of **5** can be detected on HPLC by chemical derivatization using phenylhydrazine (PHH) to yield **6. FIG. 26B** illustrates LC-MS traces of the biochemical assays of *A. thaliana* DHAD (pDHAD). Extracted ion chromatogram (EIC) of positive ion mass of $[M+H]^+=207$ is shown in red. **i.** The derivatization reaction was validated the using authentic **5. ii.** The bioactivity of pDHAD in converting **4** into **5** was validated. **iii.** Addition of DMSO to pDHAD enzymatic reaction mixture has no effect. **iv.** Addition of 10 $\mu$M **AA** to the reaction mixture abolished pDHAD activity.

**FIG. 27A-FIG. 27C** illustrates inhibition assays of DHADs using **AA.** Three DHAD enzymes were assayed, including pDHAD (plant DHAD from *A. thaliana*), fDHAD (fungal housekeeping DHAD from *A. terreus*) and AstD (DHAD homolog within *ast* cluster). $IC_{50}$ and $K_i$ values of **AA** were measured based on inhibition percentage at different **AA** concentrations. Center values are averages, errors bars are s.d., n = 3. **FIG. 27A** illustrates a plot of the inhibition percentage of 0.5 $\mu$M fDHAD as a function of **AA** concentration. **FIG. 27B** illustrates a plot of the inhibition percentage of 0.5 $\mu$M pDHAD as a function of **AA** concentration. **FIG. 27C** illustrates analysis of inhibitory kinetics of **AA** on pDHAD using the Lineweaver-Burke method at different concentrations of **AA** (left). Linear fitting of apparent Michaelis constant ($K_{m,app}$) as a function of **AA** concentration yields the inhibition constant ($K_i$) of **AA** on pDHAD (right). **FIG. 27D** illustrates a plot of the inhibition percentage of 0.5 $\mu$M AstD as a function of **AA** concentration.

**FIG. 28** illustrates cytotoxicity assays of **AA.** Percent growth inhibition of melanoma cell line A375 (left) and SK-MEL-1 (right) indicate **AA** has no significant cytotoxicity on these cell lines. Treatments of **AA** were initiated at 24 h postseeding for 72 h, cell viability was measured by CellTiter-GLO Luminescence (Promega) following the manufacturer's recommendations. Results are representative data in duplicate from three independent experiments (center values are averages, errors bars are s.d., n = 5).

**FIG. 29A-FIG. 29D** illustrates growth curves of *S. cerevisiae ΔILV3* expressing AstD and fDHAD. The genome copy of DHAD encoded by *ILV3* was first deleted from *Saccharomyces cerevisiae* strain DHY Δ*URA3* to give UB02. UB02 was then either chemically complemented by growth on ILV (leucine, isoleucine and valine)-containing media or genetically by expressing of fDHAD or AstD episomally (TY06 or TY07, respectively). The empty vector pXP318 was also transformed into UB02 to generate a control strain TY05. The optical density of cell growth under different conditions were plotted as a function of time. Center values are averages, errors bars are s.d., n = 3. **FIG. 29A** illustrates the growth curve in ILV dropout media with no **AA. FIG. 29B** illustrates the growth curve in ILV dropout media with 125 $\mu$M **AA. FIG. 29C** illustrates the growth curve in ILV supplemented media. **FIG. 29D** illustrates the growth curve in ILV supplemented media with 250 $\mu$M **AA.**

**FIG. 30A-FIG. 30E** illustrates X-ray structure of *holo*-pDHAD and homology model of AstD. **FIG. 30A** illustrates superimpositions monomer of *holo*-pDHAD (PDB: 5ZE4, 2.11 Å) and RlArDHT (PDB: 5J84). The *holo* structure containing the 2Fe-2S cofactor and $Mg^{2+}$ ion in the active site. The structure of *holo*-pDHAD is in white; the crystal structure of RlArDHT is in cyan. **FIG. 30A** illustrates superimpositions of *holo*-pDHAD and homology modeled AstD. The structure of AstD was constructed by homology modeling based on the structure *holo*-pDHAD. The structure of *holo*-pDHAD is in white; the crystal structure of AstD is in green. **FIG. 30C** illustrates the electron density map of cofactors in the *holo* structure of pDHAD. White grid: 2Fo-Fc map at 1.2 $\sigma$ level. Green grid: Fo-Fc positive map at 3.2$\sigma$ level. Cyan sticks: acetic acid molecule. **FIG. 30D** illustrates a comparison of the active sites in the crystal structure of pDHAD and the modeled structure of AstD. The cartoon represents superimposed binding sites of pDHAD (white) and AstD (green). The shift of a loop in AstD, where L518 (correspond to V496 in pDHAD) is located, coupled with a larger L198 residue (correspond to 1177 in pDHAD) lead to a smaller hydrophobic pocket of AstD than that in pDHAD. **FIG. 30E** illustrates the surface of binding sites of AstD (left) and pDHAD (right). The smaller hydrophobic channel in modeled AstD cannot accommodate the **AA** molecule (yellow balls-and-sticks).

**FIG. 31A-FIG. 31B** illustrate structural features of DHAD. **FIG. 31A** illustrates a crystal structure of the *holo A. thaliana* DHAD (pDHAD) with the docked **AA** in the active site. The *holo* structure containing the cofactor 2Fe-2S cluster and a $Mg^{2+}$ ion. i: The overall structure of the dimeric pDHAD and the active site located at the dimer interface. One of the pDHAD monomers is show in cyan, whereas the other one is shown in electrostatic surface representation. The docked **AA** is shown in the inset in spaced-filled model. The hydrophobic portions of **AA** are surrounded by several hydrophobic residues (white spheres) from both monomers. **FIG. 31B** illustrates a cross-section electrostatic map of

modeled *holo*-pDHAD in the binding site. Red map: the normalized negatively charged regions; blue map: the normalized positively charged regions; white map: the hydrophobic regions. The docked **AA** in the active site of pDHAD is shown on the left, while the docked native substrate dihydroxyisovalerate is shown on the right. The docking studies suggest the hydrophobic entrance to the reaction chamber preferentially binds the bulkier, tricyclic **AA.**

**FIG. 32** illustrates a spray assay of AA on *A. thaliana.* Glufosinate resistant A. *thaliana* was treated with (right) or without (left) **AA** in the solvent, which is a commercial glufosinate based herbicide marketed as Finale®. To improve the wetting and penetration, **AA** was firstly dissolved in ethanol and then added to solvent (0.06 g/L Finale® Bayer Inc. + 20 g/L ethanol) to make 250 μM **AA** spray solution. The control plants were treated with solvent containing ethanol only. Spraying treatments began when the seeds germinated, and was repeated once every two days with approximately 0.4 mL **AA** solution per time per pot for 4 weeks. The picture shown below is taken after one month with treatment. The application rate of **AA** is approximately 1.79336 kg/ha (1.6 lb/acre), which is comparable to the commonly used herbicide glyphosate (0.84064 kg/ha~1.68128 kg/ha (0.75~1.5 lb/acre)).

**FIG. 33A-FIG. 33B** illustrates plant treatment assays with **AA. FIG. 33A** illustrates specific inhibition of anther development of *A. thaliana.* Comparison of flower organs between the **AA** treated (panels a-c) and non-treated (panels d-f) *Arabidopsis.* Panel a compared to panel d, the **AA** treated flower shows abnormal pistil elongation due to the lack of pollination. Panel b compared to panel e, the AA treated flower is missing one stamen. Panel c compared to panel f, the **AA** treated anther is depleted of healthy and mature pollen. **FIG. 33B** and **FIG. 33C** provide a schematic illustration of results from a cross experiment. **FIG. 33B** shows wild type *A. thaliana* treated with 250 μM **AA** was pollinated with pollen from the un-treated plant that carries the glufosinate resistant gene. Offspring was obtained, and inherited the glufosinate resistance from the pollen donor. **FIG. 33C** is similar to **FIG. 33B,** except that the pollen donor was also treated with 250 μM **AA.** No offspring was obtained from this cross. Similar results were obtained with the treatment of **AA** at 100 μM. Results from the cross are presented in **Table 9H. FIG. 33D** illustrates the impact of AA on wheat inflorescence. The treatment of 250 μM **AA** begins when spikelet is fully emerged. The center floret was removed from each spikelet. Lemma and palea were dissected to reveal the anther and stigma. 250 μM **AA** were added directly upon the intact stigma and anther for both the control and the treatment plant. Each floret was treated by AA for three times within one week. After **AA** treatment, the treatment plant was covered with a transparent plastic bag to prevent wind pollination, whereas the control plant was left uncovered allowing wind pollination. Grains were removed and displayed by the side of each spikelet to allow counting. The grains developing at the bottom of the treated plant were likely due to improper bagging at the bottom of the spikelet.

**FIG. 34A-FIG. 34D** illustrates **AA** resistance of *Arabidopsis* plants expressing *astD* transgenes. **FIG. 34A** illustrates the growth phenotype of *Arabidopsis* with (lower) and without (upper) *astD* transgene growing on media containing 100 μM **AA.** Control plants were transformed with a vector that carries the glufosinate ammonium selection marker but no *astD* transgene. Pictures were taken 10 days after germination. **FIG. 34B** illustrates the fresh weight of 3-week old Arabidopsis seedlings growing on media with (grey bar) and without (yellow bar) 100 μM **AA.** The bar plot shows mean values + SE (error bars); n > 20 plants each. **FIG. 34C** illustrates glufosinate-resistant *Arabidopsis* with (lower) and without (upper) *astD* transgene growing in soil were sprayed with 250 μM **AA** + glufosinate ammonium (left), or glufosinate ammonium only (right). Control plants only carry the selection marker, but no *astD* transgene. i. control sprayed with 250 μM **AA** + glufosinate ammonium. ii. Control sprayed with glufosinate ammonium. iii. *Arabidopsis* with *astD* transgene sprayed with 250 μM **AA** + glufosinate ammonium. iv. *Arabidopsis* with *astD* transgene sprayed with glufosinate ammonium. **FIG. 34D** illustrates the plant height of *Arabidopsis* with (dots) and without (square) *astD* transgene growing in soil. Plants were sprayed with 250 μM **AA** with glufosinate ammonium (red), or glufosinate ammonium (no treatment, blue) only.

**FIG. 35** illustrates verification of AstD expression in *A. thaliana* using western blot. Western blot verification of AstD expression in *A. thaliana.* Ponceau staining shows equal loading (bottom) and AstD detection with anti-FLAG antibody (top).

**FIG. 36** illustrates a sequence alignment between pDHAD (SEQ ID NO: 4) and AstD (SEQ ID NO: 10). The sequence identity between pDHAD and AstD is 56.8%, whereas the similarity between them is 75.0%. Residues were colored according to their property and similarity.

## DETAILED DESCRIPTION

**Overview**

The invention is defined in the appended claims. Any aspects, embodiments and examples of the present disclosure which do not fall under the scope of the appended claims do not form part of the invention and are merely provided for illustrative purposes.

The following description is presented to enable a person of ordinary skill in the art to make and use the various aspects of the disclosure. Descriptions of specific devices, techniques, and applications are provided only as examples.

The present disclosure relates generally to herbicidal compositions and methods of use thereof, and more specifically to herbicidal compositions containing aspterric acid or a derivative thereof for use in inhibiting vegetative growth in plants.

The present disclosure is based, at least in part, on Applicant's discovery of a biosynthetic gene cluster in *Aspergillus terreus* that encodes proteins involved in the production of the compound aspterric acid. This gene cluster also encodes an AstD protein, which shares ~70% amino acid sequence homology with the housekeeping DHAD protein (involved in primary metabolism) in this same organism. DHAD is a component of a branched-chain amino acid biosynthetic pathway found in bacteria, archaea, fungi, and plants. It was demonstrated that aspterric acid has herbicidal activity against plants. Further, while the activity of various DHAD proteins was found to be inhibited by aspterric acid, AstD was not inhibited by aspterric acid. AstD may thus be used to develop aspterric acid-resistant plants that contain heterologous AstD proteins.

Accordingly, the present disclosure provides compositions and methods for reducing growth of a vegetative tissue in a plant involving contacting the plant with a composition containing aspterric acid. The present disclosure further provides aspterric acid-resistant plants containing heterologous AstD proteins, as well as methods of generating said plants. Further provided are methods of producing hybrid seed by using aspterric acid to inhibit pollen development in the flower of the female parent of the hybrid.

The use of the terms "a," "an," and "the," and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (*i.e.,* meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. For example, if the range 10-15 is disclosed, then 11, 12, 13, and 14 are also disclosed. All methods described herein can be performed in any suitable order unless otherwise indicated herein or otherwise clearly contradicted by context. The use of any and all examples, or exemplary language (e.g., "such as") provided herein, is intended merely to better illuminate the aspects of the disclosure. No language in the specification should be construed as indicating any non-claimed element as essential to the practice of the aspects of the disclosure.

Reference to "about" a value or parameter herein refers to the usual error range for the respective value readily known to the skilled person in this technical field. Reference to "about" a value or parameter herein includes (and describes) aspects that are directed to that value or parameter *per se.* For example, description referring to "about X" includes description of "X."

It is understood that aspects and aspects of the present disclosure described herein include "comprising," "consisting," and "consisting essentially of" aspects of the disclosure.

It is to be understood that one, some, or all of the properties of the various aspects of the disclosure may be combined to form other aspects of the present disclosure. These and other aspects of the present disclosure will become apparent to one of skill in the art. These and other aspects of the present disclosure are further described by the detailed description that follows.

The terms "isolated" and "purified" as used herein refers to a material that is removed from at least one component with which it is naturally associated (e.g., removed from its original environment). The term "isolated," when used in reference to an isolated protein, refers to a protein that has been removed from the culture medium of the host cell that expressed the protein. As such an isolated protein is free of extraneous or unwanted compounds (e.g., nucleic acids, native bacterial or other proteins, etc.).

**Aspterric Acid and Derivatives Thereof**

Certain aspects of the present disclosure relate to inhibitors of DHAD (dihydroxy acid dehydratase) proteins. In some aspects of the disclosure, the DHAD protein inhibitor is aspterric acid or a derivative thereof. Compositions are provided herein that include a DHAD protein inhibitor (e.g. aspterric acid or a derivative thereof), as well as methods of using such compositions to modulate plant growth.

In some variations, the compositions described herein contain aspterric acid or a derivative thereof, wherein the aspterric acid or a derivative thereof is a compound of Formula (X), or a salt thereof:

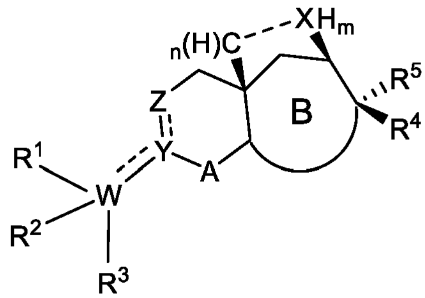

(X),

wherein:

A is a bond, $CH_2$, or absent;

W, Y and Z are CH, or $CH_2$;

--- is a single bond or a double bond,
wherein

when W---Y and Y---Z are both single bonds, $R^1$ and $R^2$ are independently H or alkyl, and $R^3$ is H or alkyl, or $R^3$ and W-Y are taken together to form a $C_3$-$C_7$ cycloalkyl;

when W---Y is a double bond and Y---Z is a single bond, $R^3$ is absent, and $R^1$ and $R^2$ are each independently H or alkyl, or $R^1$ and $R^2$ are taken together with W to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, $-NH_2$, or $C_1$-$C_6$ alkyl;

when W---Y is a single bond and Y---Z is a double bond, $R^2$ and $R^3$ are absent, and $R^1$ is $C_6$-$C_{12}$ aryl or 5-10 membered heteroaryl, wherein the $C_6$-$C_{12}$ aryl or the 5-10 membered heteroaryl is optionally substituted, one, two or three times, independently from each other, with -OH, $-NH_2$, or $C_1$-$C_6$ alkyl;

B is a 6- or 7-membered saturated or unsaturated carbocycle;

--- is absent, a single bond, or a double bond;

$R^4$ is -COOH or $-PO_3^{2-}$;

$R^5$ is -OH or $-NH_2$;

X is selected from the group consisting of O, N, and S;

n is 1, 2, or 3; and

m is 0, 1, or 2.

In some aspects of the disclosure, W---Y and Y---Z are both single bonds, $R^1$ and $R^2$ are independently H or alkyl, and $R^3$ and W-Y are taken together to form a $C_3$-$C_7$ cycloalkyl. For example, in certain aspects of the disclosure, the compound of Formula (X) is::

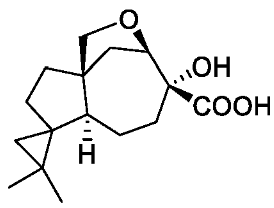

.

In other variations, the compositions described herein contain aspterric acid or a derivative thereof, wherein the aspterric acid or a derivative thereof is a compound of Formula (Y), or a salt thereof:

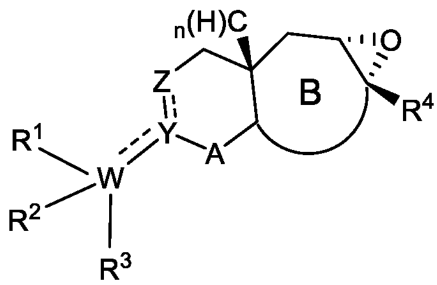

(Y),

wherein:

A is a bond, $CH_2$, or absent;

W, Y and Z are CH, or $CH_2$;

$\overline{---}$ is a single bond or a double bond,
wherein

when W$\overline{---}$Y and Y$\overline{---}$Z are both single bonds, $R^1$ and $R^2$ are independently H or alkyl, and $R^3$ is H or alkyl, or $R^3$ and W-Y are taken together to form a $C_3$-$C_7$ cycloalkyl;

when W$\overline{---}$Y is a double bond and Y$\overline{---}$Z is a single bond, $R^3$ is absent, and $R^1$ and $R^2$ are each independently H or alkyl, or $R^1$ and $R^2$ are taken together with W to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, $-NH_2$, or $C_1$-$C_6$ alkyl;

when W$\overline{---}$Y is a single bond and Y$\overline{---}$Z is a double bond, $R^2$ and $R^3$ are absent, and $R^1$ is $C_6$-$C_{12}$ aryl or 5-10 membered heteroaryl, wherein the $C_6$-$C_{12}$ aryl or the 5-10 membered heteroaryl is optionally substituted, one, two or three times, independently from each other, with -OH, $-NH_2$, or $C_1$-$C_6$ alkyl;

B is a 6- or 7-membered saturated or unsaturated carbocycle;

--- is absent, a single bond, or a double bond;

$R^4$ is -COOH or $-PO_3^{2-}$; and

n is 1, 2, or 3.

In some variations, the compound of Formula (X) or salt thereof is a compound of Formula (X-A), or a salt thereof:

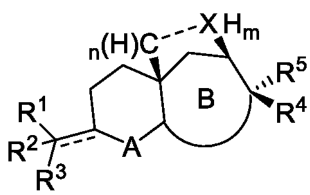

(X-A),

wherein:

A is a bond, $CH_2$, or absent;

$\overline{---}$ is a single bond or a double bond, wherein $R^3$ is absent when $\overline{---}$ is a double bond;

$R^1$ and $R^2$ are independently H or alkyl; or $R^1$ and $R^2$ are taken together with the carbon atom to which they attached to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, -$NH_2$, or $C_1$-$C_6$ alkyl;

$R^3$, if present, is H or alkyl;

B is a 6- or 7-membered saturated or unsaturated carbocycle;

--- is absent, a single bond, or a double bond;

$R^4$ is -COOH or -$PO_3^{2-}$;

$R^5$ is -OH or -$NH_2$;

X is selected from the group consisting of O, N, and S;

n is 1, 2, or 3; and

m is 0, 1, or 2.

In some variations of Formula (X-A), A is absent and B is a seven-membered unsaturated carbocycle. For example, in certain variations, the compound of Formula (X-A) is:

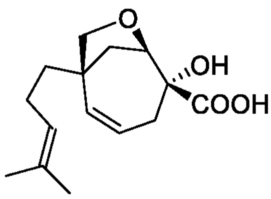

.

In other variations, A is $CH_2$ and B is a six-membered saturated carbocycle. For example, in certain variations, the compound of Formula (X-A) is:

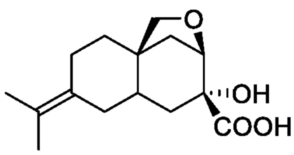

.

In some variations, the compound of Formula (X) or salt thereof is a compound of Formula (X-B), or a salt thereof:

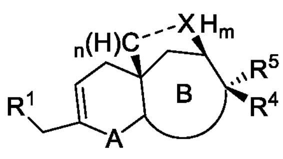

(X-B),

wherein:

A is a bond, $CH_2$, or absent;

$R^1$ is $C_6$-$C_{12}$ aryl or 5-10 membered heteroaryl, wherein the $C_6$-$C_{12}$ aryl or the 5-10 membered heteroaryl is optionally substituted, one, two or three times, independently from each other, with -OH, -$NH_2$, or $C_1$-$C_6$ alkyl;

B is a 6- or 7-membered saturated or unsaturated carbocycle;

--- is absent, a single bond, or a double bond;

$R^4$ is -COOH or -$PO_3^{2-}$;

$R^5$ is -OH or -$NH_2$;

X is selected from the group consisting of O, N, and S;

n is 1, 2, or 3; and

m is 0, 1, or 2.

In some variations, the compound of Formula (Y) or salt thereof is a compound of Formula (Y-A), or a salt thereof:

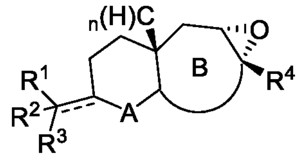

(Y-A),

wherein:

A is a bond, $CH_2$, or absent;

B is a 6- or 7-membered saturated or unsaturated carbocycle;

$\overline{- - -}$ is a single bond or a double bond, wherein $R^3$ is absent when $\overline{- - -}$ is a double bond;

$R^1$ and $R^2$ are independently H or alkyl; or $R^1$ and $R^2$ are taken together with the carbon atom to which they attached to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, -$NH_2$, or $C_1$-$C_6$ alkyl;

$R^3$, if present, is H or alkyl;

$R^4$ is -COOH or -$PO_3^{2-}$; and

n is 1, 2, or 3.

In some variations of Formula (Y-A), A is a bond and B is a seven-membered saturated carbocycle. For example, in some variations, the compound of Formula (Y-A) is:

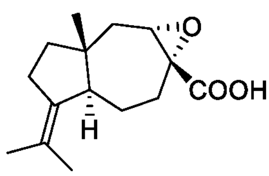

.

## *Compounds of Formula (I)*

In some variations, the compound of Formula (X-A) or salt thereof is a compound of Formula (I), or a salt thereof:

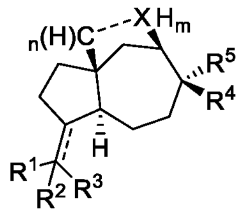

(I),

wherein:

--- is absent, a single bond, or a double bond;

$\overline{---}$ is a single bond or a double bond, wherein $R^3$ is absent when $\overline{---}$ is a double bond;

$R^1$ and $R^2$ are independently H or alkyl; or $R^1$ and $R^2$ are taken together with the carbon atom to which they attached to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, $-NH_2$, or $C_1$-$C_6$ alkyl;

$R^3$, if present, is H or alkyl;

$R^4$ is -COOH or $-PO_3^{2-}$;

$R^5$ is -OH or $-NH_2$;

X is selected from the group consisting of O, N, and S;

n is 1, 2, or 3; and

m is 0, 1, or 2.

In some variations, $R^3$, if present, is H, and $R^1$ and $R^2$ are independently alkyl. In some variations, $R^1$ and $R^2$ are independently methyl or ethyl. In some variations, $R^1$ and $R^2$ are taken together with the carbon atom to which they attached to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, $-NH_2$, or $C_1$-$C_6$ alkyl. In certain variations, m is 0 and X is O, N, or S. In other variations, m is 1 and X is O, N, or S. In still other variations, m is 2 and X is N. In some variations, $R^4$ is -COOH. In other variations, $R^5$ is -OH.

## *Compounds of Formula (I-A)*

In some variations, --- is absent, n is 3, and the compound of Formula (I) or salt thereof is a compound of Formula (I-A) or a salt thereof:

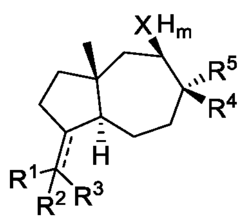

(I-A),

wherein m is 1 or 2; and $\overline{---}$, $R^1$, $R^2$, $R^3$, $R^4$, $R^5$ and X are as described for Formula (I) above.

In some variations, $R^3$, if present, is H, and $R^1$ and $R^2$ are independently alkyl. In certain variations, $R^1$ and $R^2$ are independently methyl or ethyl. In certain variations, or $R^1$ and $R^2$ are taken together with the carbon atom to which they attached to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, $-NH_2$, or $C_1$-$C_6$ alkyl. In other variations, $R^5$ is -OH. In certain variations, $R^4$ is - COOH.

In certain variations, X is S, m is 1, and the compound of Formula (I-A) is:

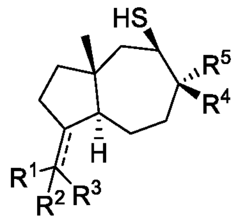

.

In one variation, the compound of Formula (I-A) is:

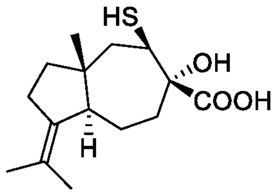

.

*Compounds of Formula (I-B)*

In some variations, --- is a single bond, n is 2, and the compound of Formula (I) or salt thereof is a compound of Formula (I-B), or a salt thereof:

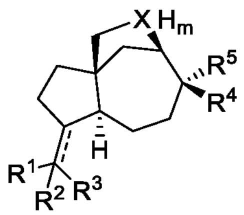

(I-B),

wherein m is 0 or 1; and - - -, $R^1$, $R^2$, $R^3$, $R^4$, $R^5$ and X are as described for Formula (I) above.

In some variations, $R^3$, if present, is H, and $R^1$ and $R^2$ are independently alkyl. In certain variations, $R^1$ and $R^2$ are methyl or ethyl. In certain variations, or $R^1$ and $R^2$ are taken together with the carbon atom to which they attached to form a $C_3$-$C_7$ cycloalkyl or 3-7 membered heterocyclyl, wherein the $C_3$-$C_7$ cycloalkyl or the 3-7 membered heterocyclyl is optionally substituted, one, two or three times, independently from each other, with -OH, -$NH_2$, or $C_1$-$C_6$ alkyl. In other variations, $R^5$ is -OH. In certain variations, $R^4$ is - COOH.

In some variations, X is O, m is 0, and the compound of Formula (I-B) is:

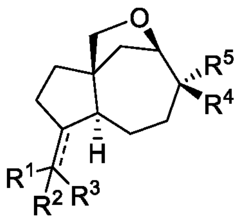

.

In certain variations, X is O, m is 0, $R^5$ is -OH, $R^4$ is -COOH, and the compound of Formula (I-B) is:

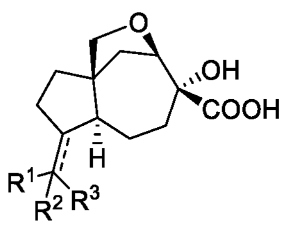

.

In certain variations, the compound of Formula (I-B) is:

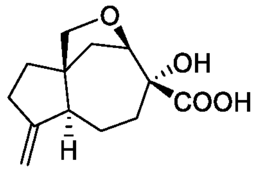, 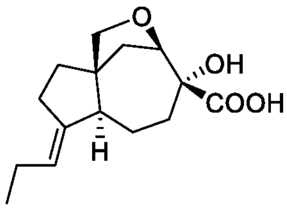, 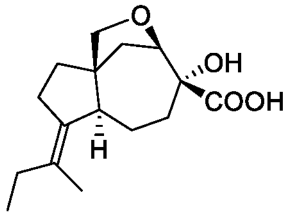, 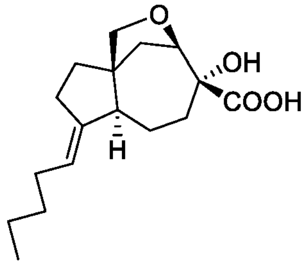,

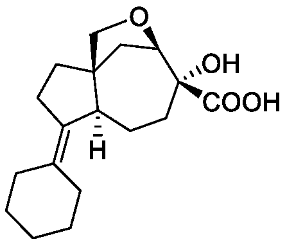, 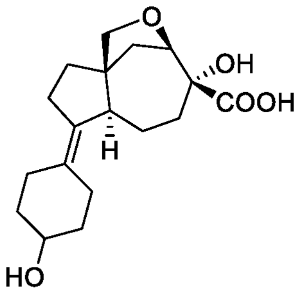, or 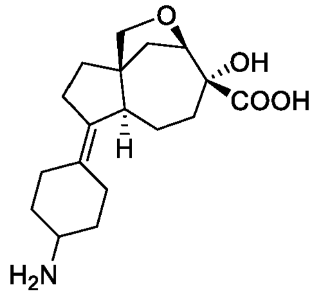

In certain variations, the compound of Formula (I-B) is:

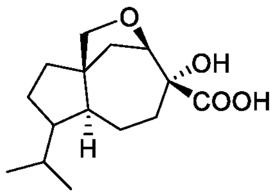 or 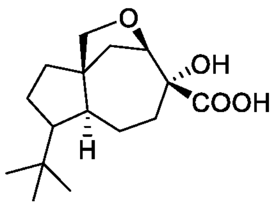.

In one variation, the compound of Formula (I-B) is aspterric acid:

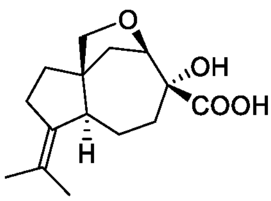.

In other variations, the compound of Formula (I-B) is:

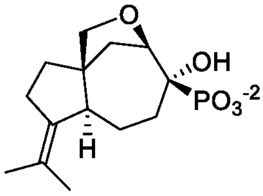 or 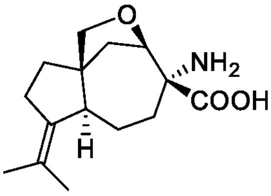.

In other variations, X is S, m is 0, $R^5$ is -OH, $R^4$ is -COOH, and the compound of Formula (I-B) is:

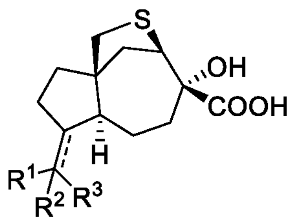

.

For example, in certain variations, the compound of Formula (I-B) is:

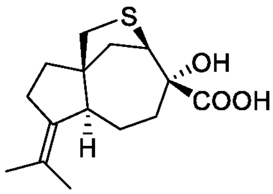

.

## *Compounds of Formula (I-C)*

In certain variations, --- is a double bond, n is 1, m is 0, X is N, and the compound of Formula (I) or salt thereof is a compound of Formula (I-C), or a salt thereof:

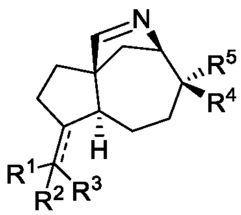

(I-C),

wherein $\overline{- - -}$, $R^1$, $R^2$, $R^3$, $R^4$ and $R^5$ are as described for Formula (I) above.

In one variation, the compound of Formula (I-C) is:

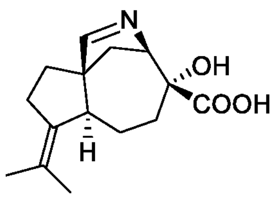

.

## *Compounds of Formula (II)*

In some variations, the compound of Formula (X-B) or salt thereof is a compound of Formula (II), or a salt thereof:

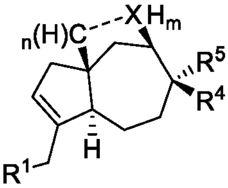

(II),

wherein:

--- is absent, a single bond, or a double bond;

$R^1$ is $C_6$-$C_{12}$ aryl or 5-10 membered heteroaryl, wherein the $C_6$-$C_{12}$ aryl or the 5-10 membered heteroaryl is optionally substituted, one, two or three times, independently from each other, with -OH, $-NH_2$, or $C_1$-$C_6$ alkyl;

$R^4$ is -COOH or $-PO_3^{2-}$;

$R^5$ is -OH or $-NH_2$;

X is selected from the group consisting of O, N, and S;

n is 1, 2, or 3; and

m is 0, 1, or 2.

In some variations, $R^1$ is $C_6$ aryl. In some variations, $R^1$ is 9-10 membered heteroaryl. In certain variations, m is 0 and X is O, N, or S. In other variations, m is 1 and X is O, N, or S. In still other variations, m is 2 and X is N. In some variations, $R^4$ is -COOH. In other variations, $R^5$ is -OH.

*Compounds of Formula (II-A)*

In some variations, --- is a single bond, n is 2, and the compound of Formula (II) or salt thereof is a compound of Formula (II-A), or a salt thereof:

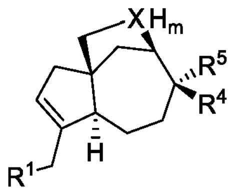

(II-A),

wherein m is 0 or 1; and $R^1$, $R^4$, $R^5$ and X are as described for Formula (II) above.

In some aspects of a compound of Formulae (X), (Y), (X-B), (II) and (II-A), $R^1$ is a 5-10 membered heteroaryl, wherein the 5-10 membered heteroaryl is selected from the group consisting of:

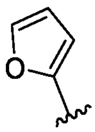, 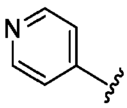, 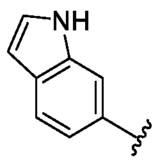, 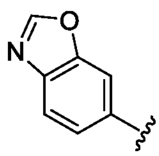, 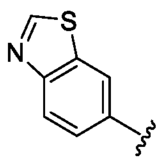, 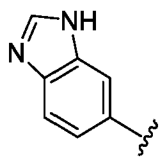, 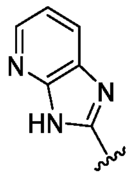,

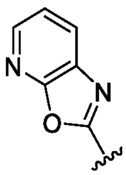, 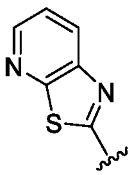, 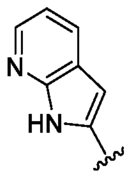, 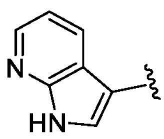, 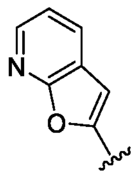, 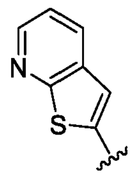, 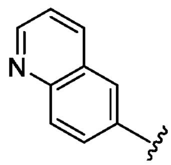,

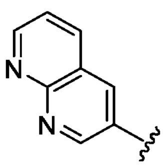, 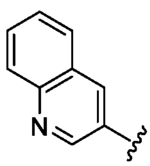, 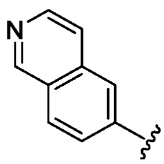, 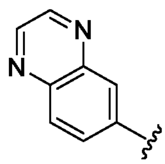, 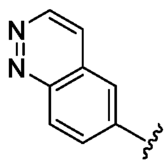, and 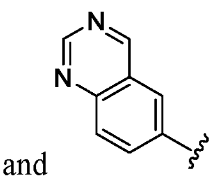,

each optionally substituted.

In some variations, $R^1$ is $C_6$ aryl. In some variations, $R^1$ is 9-10 membered heteroaryl. In other variations, $R^5$ is -OH. In certain variations, $R^4$ is -COOH.

In some variations, X is O, m is 0, $R^5$ is -OH, $R^4$ is -COOH, and the compound of Formula (II-A) is:

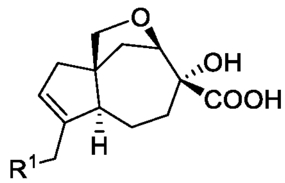

.

In certain variations, the compound of Formula (II-A) is:

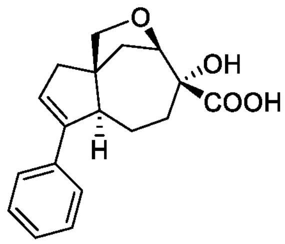

or

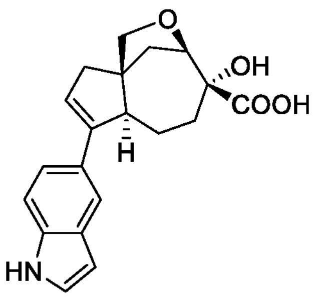

.

In one aspect, the present disclosure provides a compound of Formula (X) or salt thereof or Formula (Y) or salt thereof, including compounds of Formulae (X-A), (X-B), (Y-A), (I), (I-A), (I-B), (I-C), and (II-A), or salts thereof. In some aspects, the compounds described herein are derivatives of aspterric acid, which exclude aspterric acid.

*Synthesis of Compounds of Formula (X) or (Y)*

The compound of Formula (X) or salt thereof or Formula (Y) or salt thereof used in the methods described herein, including compounds of Formulae (X-A), (X-B), (Y-A), (I), (I-A), (I-B), (I-C), and (II-A), or salts thereof, may be obtained from any source (including any commercially available sources) or be produced by any methods known in the art. In some variations, the compound of Formula (X) or salt thereof or Formula (Y) or salt thereof is produced through one or more chemical synthesis steps. In other variations, the compound of Formula (X) or salt thereof or Formula (Y) or salt thereof is produced through one or more biosynthesis steps. In still other variations, the compound of Formula (X) or salt thereof or Formula (Y) or salt thereof is produced through a combination of chemical and biosynthetic steps.

The compounds disclosed herein may be prepared by a number of processes as generally described below. In the following process descriptions, the symbols when used in the formulae depicted are to be understood to represent those groups described above in relation to the formulae herein.

Chemical synthesis steps may include, for example, epoxide ring opening, ether ring cleavage, sulphurisation, hydrogenation of a C-C double bond, or olefin metathesis, or any combinations thereof. In certain variations, a reactant compound of Formula (X) or Formula (Y), such as a compound of Formula (I), undergoes one or more chemical synthesis steps to produce a different compound of Formula (X) or Formula (Y), such as a different compound of Formula (I), for use in the methods described herein.

In some variations, a compound of Formula (I-B) wherein X is S and m is 0, is produced from a compound of Formula (I-B) wherein X is O and m is 0, using ether ring cleavage and sulphurisation:

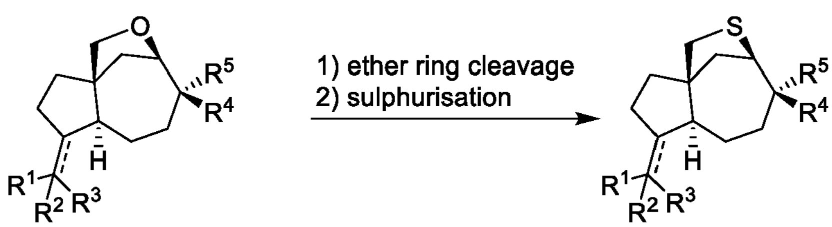


In some variations, the sulphurisation is performed with a bisulfide agent. In one variation, the bisulfide agent is sodium sulfide. In one variation, aspterric acid undergoes ether ring cleavage and sulphurisation with a bisulfide agent to produce a compound of Formula (I-B) of the structure:

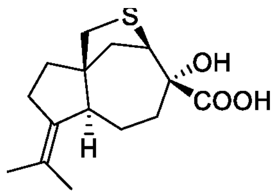

In still other variations, a compound of Formula (I) wherein $\overline{---}$ is a single bond and $R^3$ is H, is produced using hydrogenation of a compound of Formula (I) wherein $\overline{---}$ is a double bond:

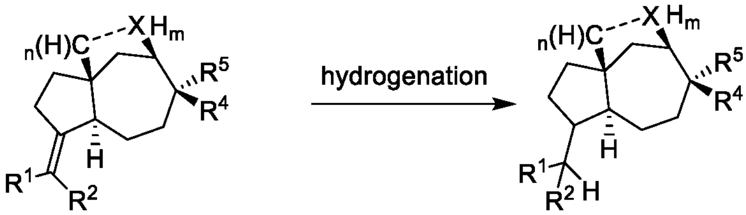


In some variations, hydrogenation occurs in the presence of $H_2$ and a hydrogenation catalyst. In one variation, aspterric acid undergoes hydrogenation to produce a compound of Formula (I) of the structure:

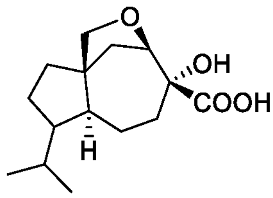

In yet other variations, a compound of Formula (I) wherein $\overline{---}$ is a double bond, is produced using olefin metathesis of a reactant compound of Formula (I) wherein $\overline{---}$ is a double bond, and wherein at least one of $R^1$ or $R^2$ of the produced compound of Formula (I) is different than the $R^1$ or $R^2$ of the reactant compound of Formula (I):

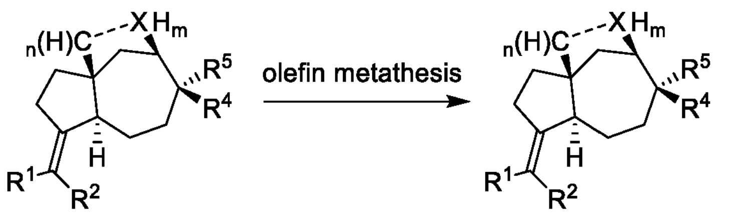


reactant product ,

wherein $\overline{---}$, ---, $R^4$, $R^5$, X, n, and m are the same for the reactant and the product; $R^1$ of the product is different than the $R^1$ of the reactant, or the $R^2$ of the product is different than the $R^2$ of the reactant, or both $R^1$ and $R^2$ of the reactant are different than the $R^1$ and $R^2$ of the product. In other variations, $R^5$ is -OH. In certain variations, $R^4$ is -COOH.

The olefin metathesis may occur in the presence of an organometallic catalyst, such as a Grubbs catalyst. In one variation, aspterric acid undergoes olefin metathesis in the presence of an organometallic catalyst to produce a compound of Formula (I) of the structure:

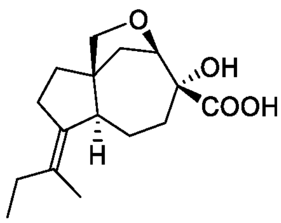

In some variations, a compound of Formula (I-A) is produced from a compound of Formula (i) via a ring opening reaction:

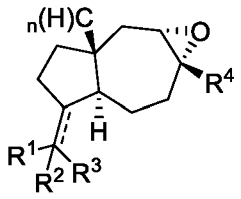

(i),

wherein $R^1$, $R^2$, $R^3$, $R^4$, $\overline{- - -}$ and n are as defined for the compound of Formula (I-A) above.

In one variation, the ring opening reaction is performed in the presence of a bisulfide agent, and a compound of Formula (I-A) is produced wherein X is S and m is 1:

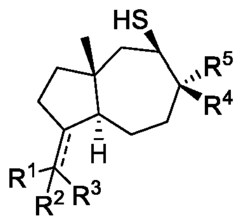

In some variations, the reactant compound of Formula (I) or compound of Formula (i) is produced through one or more biosynthetic steps, and then undergoes one or more chemical synthesis steps as described above to produce the compound of Formula (I) used in the methods described herein.

For example, in some variations, the reactant compound of Formula (I) is produced by a cell expressing the gene *astA, astB,* or *ast*C, or any combinations thereof. In some variations, the cells are *Saccharomyces cerevisiae* cells. In one variation, the reactant compound of Formula (I) is aspterric acid, and is produced from farnesyl diphosphate by cells expressing the genes *astA, astB,* and *ast*C, and then the reactant aspterric acid undergoes one or more of the chemical synthesis steps described above to produce the compound of Formula (I) used in the methods described herein.

In certain variations, the compound of Formula (i) described above is produced biosynthetically. For example, in some aspects of the disclosure, the compound of Formula (i) is produced by a cell expressing the genes *ast*A and *ast*B. In certain aspects of the disclosure, the cells are *Saccharomyces cerevisiae* cells.

In one aspect of the disclosure, farnesyl diphosphate is converted to a compound of Formula (i) through one or more biosynthetic steps, and the compound of Formula (i) is converted to a compound of Formula (I-A) by a ring opening reaction in the presence of a bisulfide agent:

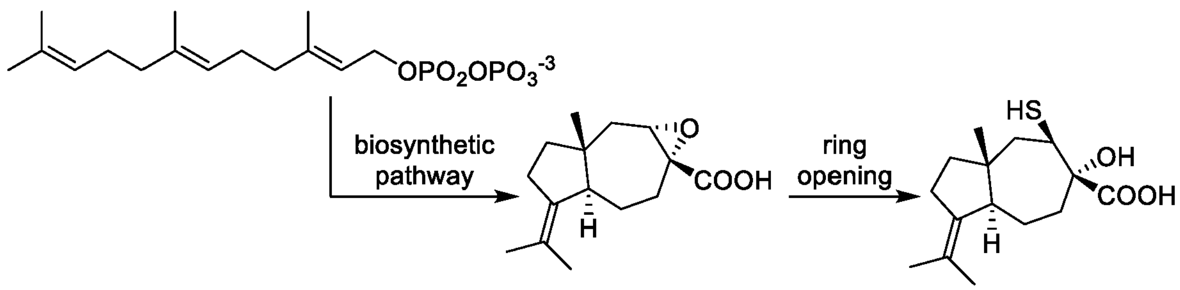


In some variations, farnesyl diphosphate is converted to a compound of Formula (i) by cells expressing the genes *ast*A and *ast*B.

In some variations, the compound of Formula (I) used in the methods described herein is produced biosynthetically. For example, in one variation, the compound of Formula (I) is produced by a cell expressing the gene *astA, astB,* or *ast*C, or any combinations thereof. In some variations, farnesyl diphosphate undergoes one or more biosynthetic steps to produce the compound of Formula (I).

In certain variations, one or more of the following compounds undergo one or more biosynthetic steps to produce a compound of Formula (I), or a salt thereof:

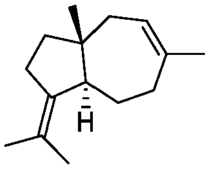, 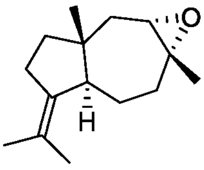, or 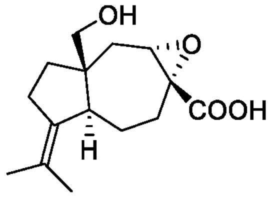

or a combination thereof.

For example, in one aspect of the disclosure, aspterric acid (an example of a compound of Formula (I)) is produced from farnesyl diphosphate by cells expressing the genes *astA, astB,* and *ast*C. In one aspect of the disclosure, the cells are *Saccharomyces cerevisiae* cells.

As used herein, "alkyl" refers to a linear or branched saturated hydrocarbon chain. Examples of alkyl groups include methyl, ethyl, *n*-propyl, *iso*-propyl, *n*-butyl, *sec*-butyl, *tert*-butyl, *n*-pentyl, 2-pentyl, *iso*-pentyl, *neo*-pentyl, hexyl, 2-hexyl, 3-hexyl, and 3-methylpentyl. When an alkyl group having a specific number of carbons is named, all geometric isomers having that number of carbons may be encompassed; thus, for example, "butyl" can include *n*-butyl, *sec*-butyl, *iso*-butyl and *tert*-butyl; "propyl" can include *n*-propyl and *iso*-propyl. In some aspects of the disclosure, alkyl as used herein, such as in compounds of Formula (X) or (Y), has 1 to 30 carbon atoms (*i.e.,* $C_{1-30}$ alkyl), 1 to 20 carbon atoms (*i.e.,* $C_{1-20}$ alkyl), 1 to 15 carbon atoms (*i.e.,* $C_{1-15}$ alkyl), 1 to 9 carbon atoms (*i.e.,* $C_{1-9}$ alkyl), 1 to 8 carbon atoms (*i.e.,* $C_{1-8}$ alkyl), 1 to 7 carbon atoms (*i.e.,* $C_{1-7}$ alkyl), 1 to 6 carbon atoms (*i.e.,* $C_{1-6}$ alkyl), 1 to 5 carbon atoms (*i.e.,* $C_{1-5}$ alkyl), 1 to 4 carbon atoms (*i.e.,* $C_{1-4}$ alkyl), 1 to 3 carbon atoms (*i.e.,* $C_{1-3}$ alkyl), 1 to 2 carbon atoms (*i.e.,* $C_{1-2}$ alkyl), or 1 carbon atom (*i.e.,* $C_1$ alkyl).

The term "aryl" refers to and includes polyunsaturated aromatic hydrocarbon groups. Aryl may contain additional fused rings (*e.g.*, from 1 to 3 rings), including additionally fused aryl, heteroaryl, cycloalkyl, and/or heterocyclyl rings. In some aspects of the disclosure, aryl as used herein contains from 6 to 12 annular carbon atoms. Examples of aryl groups include, but are not limited to, phenyl, naphthyl, and biphenyl.

The term "cycloalkyl" refers to and includes cyclic univalent hydrocarbon structures, which may be fully saturated, mono- or polyunsaturated, but which are nonaromatic, having the number of carbon atoms designated (*e. g.*, $C_1$-$C_{10}$ means one to ten carbons). Cycloalkyl can consist of one ring, such as cyclohexyl, or multiple rings, such as adamantly, but excludes aryl groups. A cycloalkyl comprising more than one ring may be fused, spiro or bridged, or combinations thereof. In some aspects of the disclosure, cycloalkyl as used herein is a cyclic hydrocarbon having from 3 to 7 annular carbon atoms (a "$C_3$-$C_7$ cycloalkyl"). Examples of cycloalkyl include, but are not limited to, cyclopropyl, cyclobutyl, cyclopentyl, cyclohexyl, 1-cyclohexenyl, 3-cyclohexenyl, cycloheptyl, and norbornyl.

The term "heteroaryl" refers to and includes unsaturated aromatic cyclic groups having carbon atoms and at least one annular heteroatom, including but not limited to heteroatoms such as nitrogen, oxygen and sulfur, wherein the nitrogen and sulfur atoms are optionally oxidized, and the nitrogen atom(s) are optionally quaternized. A heteroaryl group can be attached to the remainder of the molecule at an annular carbon or at an annular heteroatom. Heteroaryl may contain additional fused rings (*e.g.,* from 1 to 3 rings), including additionally fused aryl, heteroaryl, cycloalkyl, and/or heterocyclyl rings. Examples of 5-10 membered heteroaryl include, but are not limited to,

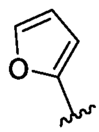, 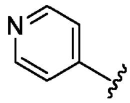, 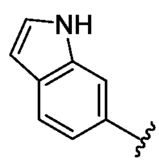,

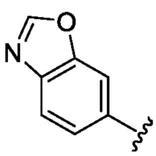, 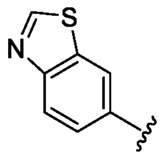, 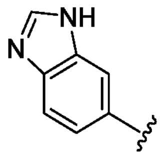, 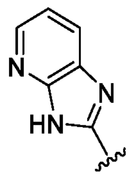, 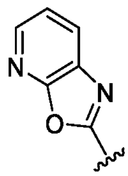, 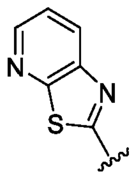, 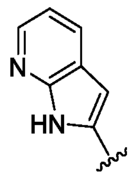,

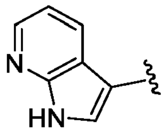, 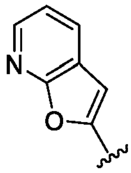, 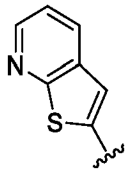, 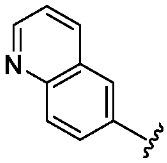, 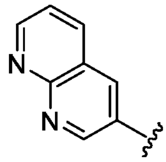, 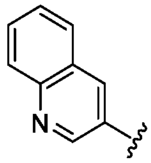, 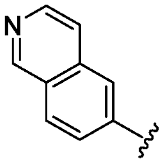

, 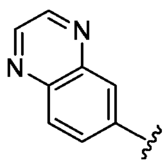, 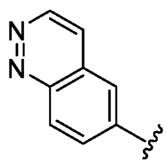, and 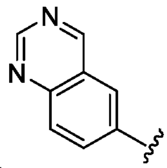.

The term "heterocyclyl" refers to and includes a saturated or an unsaturated nonaromatic group having carbon atoms and at least one annular heteroatom, such as nitrogen, sulfur or oxygen, wherein the nitrogen and sulfur atoms are optionally oxidized, and the nitrogen atom(s) are optionally quaternized. A heterocyclyl group may have a single ring or multiple condensed rings, but excludes heteroaryl groups. A heterocycle comprising more than one ring may be fused, spiro or bridged, or any combination thereof. In fused ring systems, one or more of the fused rings can be aryl or heteroaryl.

"Optionally substituted" unless otherwise specified means that a group may be unsubstituted or substituted by one or more (e.g., 1, 2, 3, 4 or 5) of the substituents listed for that group in which the substituents may be the same of different. In one aspect of the disclosure, an optionally substituted group has one substituent. In another aspect of the disclosure, an optionally substituted group has two substituents. In another aspect of the disclosure, an optionally substituted group has three substituents. In another aspect of the disclosure, an optionally substituted group has four substituents. In some aspects of the disclosure, an optionally substituted group has 1 to 2, 2 to 5, 3 to 5, 2 to 3, 2 to 4, 3 to 4, 1 to 3, 1 to 4 or 1 to 5 substituents.

**Compositions Containing Aspterric Acid or Derivatives Thereof**

Certain aspects of the present disclosure relate to compositions containing aspterric acid or a derivative thereof. In some aspects of the disclosure, these compositions may be used as herbicidal compositions. Compositions containing aspterric acid or a derivative thereof may include one or more additional compounds or ingredients. Exemplary additional compounds or ingredients may include, for example, compounds that enhance the herbicidal activity of the composition, compounds that increase the solubility of aspterric acid or a derivative thereof in the composition, etc. One of skill in the art would readily recognize suitable compounds or ingredients for inclusion in the compositions of the present disclosure.

Various quantities of aspterric acid or a derivative thereof may be used in the compositions of the present disclosure. Exemplary concentrations of aspterric acid or a derivative thereof in compositions of the present disclosure may include, for example, at least 1 μM, at least 2.5 μM, at least 5 μM, at least 7.5 μM, at least 10 μM, at least 20 μM, at least 30 μM, at least 40 μM, at least 50 μM, at least 60 μM, at least 70 μM, at least 80 μM, at least 90 μM, at least 100 μM, 125 μM, at least 150 μM, at least 175 μM, at least 200 μM, at least 225 μM, at least 250 μM, at least 275 μM, at least 300 μM, at least 325 μM, at least 350 μM, at least 375 μM, at least 400 μM, at least 500 μM, at least 600 μM, 700 μM, at least 800 μM, at least 900 μM, at least 1 mM, at least 2 mM, at least 3 mM, at least 4 mM, at least 5 mM, at least 6 mM, at least 7 mM, at least 8 mM, at least 9 mM, or at least 10 mM or more.

Compositions of the present disclosure containing aspterric acid or a derivative thereof may further contain one or more surfactants, detergents, solubilizing agents, alcohols, or oils such as, for example, Silwet L-77, Tween 80, corn oil, ethanol, DMSO, etc. Various quantities of such ingredients may be used in these compositions. For example, such ingredients may be present as at least 0.05%, at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5% of the total weight of the composition.

Compositions of the present disclosure containing aspterric acid or a derivative thereof may further contain one or more compounds having herbicidal activity (*e.g.* glufosinate, etc.). Various quantities of such ingredients may be used in these compositions. Concentrations of such compounds in the composition may be, for example, at least 1 μM, at least 2.5 μM, at least 5 μM, at least 7.5 μM, at least 10 μM, at least 20 μM, at least 30 μM, at least 40 μM, at least 50 μM, at least 60 μM, at least 70 μM, at least 80 μM, at least 90 μM, at least 100 μM, 125 μM, at least 150 μM, at least 175 μM, at least 200 μM, at least 225 μM, at least 250 μM, at least 275 μM, at least 300 μM, at least 325 μM, at least 350 μM, at least 375 μM, at least 400 μM, at least 500 μM, at least 600 μM, 700 μM, at least 800 μM, at least 900 μM, at least 1 mM, at least 2 mM, at least 3 mM, at least 4 mM, at least 5 mM, at least 6 mM, at least 7 mM, at least 8 mM, at least 9 mM, or at least 10 mM or more.

## Polypeptides and Recombinant Polypeptides

Certain aspects of the present disclosure relate to polypeptides (*e.g.* DHAD) that are targeted and inhibited by certain compounds (*e.g.* aspterric acid). Accordingly, in certain aspects, the present disclosure provides compounds that are inhibitors of DHAD polypeptides.

Certain aspects of the present disclosure relate to expressing recombinant polypeptides (*e.g.* AstD polypeptides) in a host organism (*e.g.* plant or plant cell). In some aspects of the disclosure, a recombinant AstD polypeptide is expressed in a host plant in order to generate a plant that is resistant to inhibition of vegetative growth induced by aspterric acid.

As used herein, a "polypeptide" is an amino acid sequence including a plurality of consecutive polymerized amino acid residues (*e.g.*, at least about 15 consecutive polymerized amino acid residues). "Polypeptide" refers to an amino acid sequence, oligopeptide, peptide, protein, or portions thereof, and the terms "polypeptide" and "protein" are used interchangeably.

Polypeptides as described herein also include polypeptides having various amino acid additions, deletions, or substitutions relative to the native amino acid sequence of a polypeptide of the present disclosure. In some aspects of the disclosure, polypeptides that are homologs of a polypeptide of the present disclosure contain non-conservative changes of certain amino acids relative to the native sequence of a polypeptide of the present disclosure. In some aspects of the disclosure, polypeptides that are homologs of a polypeptide of the present disclosure contain conservative changes of certain amino acids relative to the native sequence of a polypeptide of the present disclosure, and thus may be referred to as conservatively modified variants. A conservatively modified variant may include individual substitutions, deletions or additions to a polypeptide sequence which result in the substitution of an amino acid with a chemically similar amino acid. Conservative substitution tables providing functionally similar amino acids are well-known in the art. Such conservatively modified variants are in addition to and do not exclude polymorphic variants, interspecies homologs, and alleles of the disclosure. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). A modification of an amino acid to produce a chemically similar amino acid may be referred to as an analogous amino acid.

Recombinant polypeptides of the present disclosure that are composed of individual polypeptide domains may be described based on the individual polypeptide domains of the overall recombinant polypeptide. A domain in such a recombinant polypeptide refers to the particular stretches of contiguous amino acid sequences with a particular function or activity. For example, in a recombinant polypeptide that is a fusion of a chloroplast localization signal and an AstD polypeptide, the contiguous amino acids that encode the chloroplast localization signal may be described as the *e.g.* chloroplast localization domain in the overall recombinant polypeptide, and the contiguous amino acids that encode the AstD polypeptide may be described as the AstD domain in the overall recombinant polypeptide. Individual domains in an overall recombinant protein may also be referred to as units of the recombinant protein. Recombinant polypeptides that are composed of individual polypeptide domains may also be referred to as fusion polypeptides.

Certain aspects of the present disclosure relate to fusion polypeptides (*e.g.* AstD polypeptides containing a chloroplast localization sequence). In fusion polypeptides, the individual polypeptide domains may be in various N-terminal or C-terminal orientations relative to other polypeptide domains in the overall recombinant polypeptide. The fusion of various polypeptide domains into an overall fusion polypeptide may also be a direct fusion or an indirect fusion (*e.g.* separated by additional amino acid sequences between two polypeptide domains). In aspects of the disclosure where the fusion is indirect, a linker domain or other contiguous amino acid sequence may separate the various polypeptide domains.

### *DHAD Polypeptides*

Certain aspects of the present disclosure relate to DHAD polypeptides. DHAD (dihydroxy acid dehydratase) is an enzyme present in the branched-chain amino acid (valine, leucine, and isoleucine) biosynthetic pathway that is present in bacteria, archaea, fungi, and plants. In this pathway, DHAD is involved in the conversion of dihydroxymethylvalerate to ketomethylvalerate. However, the more general reaction that is catalyzed by DHAD is outlined in **FIG. 2B.** As outlined in the present disclosure, the compound aspterric acid is an inhibitor of DHAD.

In some aspects, a DHAD protein of the present disclosure includes a functional fragment of a full-length DHAD protein where the fragment maintains the ability to catalyze the reaction outlined in **FIG. 2B.** In some aspects of the disclosure, a DHAD protein fragment contains at least 20 consecutive amino acids, at least 30 consecutive amino acids, at least 40 consecutive amino acids, at least 50 consecutive amino acids, at least 60 consecutive amino acids, at least 70 consecutive amino acids, at least 80 consecutive amino acids, at least 90 consecutive amino acids, at least 100 consecutive amino acids, at least 120 consecutive amino acids, at least 140 consecutive amino acids, at least 160 consecutive amino acids, at least 180 consecutive amino acids, at least 200 consecutive amino acids, at least 220 consecutive amino acids, at least 240 consecutive amino acids, or 241 or more consecutive amino acids of a full-length

DHAD protein. In some aspects of the disclosure, DHAD protein fragments may include sequences with one or more amino acids removed from the consecutive amino acid sequence of a full-length DHAD protein. In some aspects of the disclosure, DHAD protein fragments may include sequences with one or more amino acids replaced/substituted with an amino acid different from the endogenous amino acid present at a given amino acid position in a consecutive amino acid sequence of a full-length DHAD protein. In some aspects of the disclosure, DHAD protein fragments may include sequences with one or more amino acids added to an otherwise consecutive amino acid sequence of a full-length DHAD protein.

Suitable DHAD proteins may be identified and isolated from various organisms. Examples of such organisms may include, for example, *Aspergillus terreus, Aspergillus fischeri, Penicillium brasilianum, Arabidopsis thaliana, Glycine max, Zea mays, Solanum lycopersicum, Oryza sativa Japonica Group,* and *Sorghum bicolor.* Examples of suitable DHAD proteins may include, for example, those listed in **Table 1,** homologs thereof, and orthologs thereof.

**Table 1: DHAD Proteins**

| Organism | Gene Name | SED ID NO. | % Identity to SEQ ID NO: 1 |
|---|---|---|---|
| *Aspergillus terreus* NIH2624 | XP_001208445.1 | 1 | - |
| *Aspergillus fischeri* NRRL 181 | XP_001260877.1 | 2 | 91 |
| *Penicillium brasilianum* | CEJ62287.1 | 3 | 85 |
| *Arabidopsis thaliana* | NP_189036.1 | 4 | 62 |
| *Glycine max* | KRH20898.1 | 5 | 64 |
| *Zea mays* | NP_001170508.1 | 6 | 63 |
| *Solanum lycopersicum* | NP_001311413.1 | 7 | 61 |
| *Oryza sativa Japonica Group* | XP_015649747.1 | 8 | 65 |
| *Sorghum bicolor* | XP_002445678.1 | 9 | 65 |

In some aspects, a DHAD protein or fragment thereof of the present disclosure has an amino acid sequence with at least about 20%, at least about 25%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% amino acid identity to the amino acid sequence of the *Aspergillus terreus* NIH2624 DHAD protein (SEQ ID NO: 1).

A DHAD protein may include the amino acid sequence or a fragment thereof of any DHAD homolog or ortholog, such as any one of those listed in **Table 1.** One of skill would readily recognize that additional DHAD homologs and/or orthologs may exist and may be used herein.

*AstD Polypeptides*

Certain aspects of the present disclosure relate to AstD polypeptides. As outlined in the present disclosure, AstD proteins share a degree of sequence homology with housekeeping DHAD proteins. However, AstD proteins of the present disclosure are not inhibited or have substantially reduced inhibition by aspterric acid in comparison to the housekeeping DHAD proteins described herein, which are inhibited by aspterric acid.

In some aspects, an AstD protein of the present disclosure includes a fragment of a full-length AstD protein where the fragment is not inhibited by aspterric acid. In some aspects of the disclosure, an AstD protein fragment contains at least 20 consecutive amino acids, at least 30 consecutive amino acids, at least 40 consecutive amino acids, at least 50 consecutive amino acids, at least 60 consecutive amino acids, at least 70 consecutive amino acids, at least 80 consecutive amino acids, at least 90 consecutive amino acids, at least 100 consecutive amino acids, at least 120 consecutive amino acids, at least 140 consecutive amino acids, at least 160 consecutive amino acids, at least 180 consecutive amino acids, at least 200 consecutive amino acids, at least 220 consecutive amino acids, at least 240 consecutive amino acids, or 241 or more consecutive amino acids of a full-length AstD protein. In some aspects of the disclosure, AstD protein fragments may include sequences with one or more amino acids removed from the consecutive amino acid sequence of a full-length AstD protein. In some aspects of the disclosure, AstD protein fragments may include sequences with one or more amino acids replaced/substituted with an amino acid different from the endogenous amino acid present at a given amino acid position in a consecutive amino acid sequence of a full-length AstD protein. In some aspects of the disclosure, AstD protein fragments may include sequences with one or more amino acids added to an otherwise consecutive amino acid sequence of a full-length AstD protein.

Suitable AstD proteins may be identified and isolated from various organisms. Examples of such organisms may include, for example, *Aspergillus terreus, Aspergillus fischeri, Penicillium brasilianum, Aspergillus brasiliensis, Aspergillus niger, Penicillium expansum,* and *Aspergillus oryzae.* Examples of suitable AstD proteins may include, for example, those listed in **Table 2,** homologs thereof, and orthologs thereof.

**Table 2: AstD Proteins**

| Organism | Gene Name | SED ID NO. | % Identity to SEQ ID NO: 10 |
|---|---|---|---|
| *Aspergillus terreus* NIH2624 | XP_001213593.1 | 10 | - |
| *Aspergillus, fischeri* NRRL 181 | XP_001266525.1 | 11 | 94 |
| *Penicillium brasilianum* | CEJ61173.1 | 12 | 95 |
| *Aspergillus brasiliensis* CBS 101740 | OJJ72940.1 | 13 | 98 |
| *Aspergillus niger* | CAK43184.1 | 14 | 97 |
| *Penicillium expansum* | KG043050.1 | 15 | 95 |
| *Aspergillus oryzae RIB40* | XP_001727833.2 | 16 | 94 |

In some aspects, an AstD protein or fragment thereof of the present disclosure has an amino acid sequence with at least about 20%, at least about 25%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% amino acid identity to the amino acid sequence of the *Aspergillus terreus* NIH2624 AstD protein (SEQ ID NO: 10).

An AstD protein may include the amino acid sequence or a fragment thereof of any AstD homolog or ortholog, such as any one of those listed in **Table 2.** One of skill would readily recognize that additional AstD homologs and/or orthologs may exist and may be used herein.

In some aspects, AstD polypeptides of the present disclosure have reduced ability to catalyze the reaction outlined in **FIG. 2B** as compared to a housekeeping DHAD polypeptide (*e.g.* SEQ ID NO: 1). The rate at which an AstD polypeptide catalyzes the reaction outlined in **FIG. 2B** may be decreased by, for example, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% or more (*e.g.* 100%) as compared to a housekeeping DHAD polypeptide (*e.g.* SEQ ID NO: 1).

In some aspects, AstD polypeptides of the present disclosure have substantially reduced potential to have their activity inhibited by aspterric acid as compared to a housekeeping DHAD polypeptide (*e.g.* SEQ ID NO: 1). The concentration of aspterric acid needed to inhibit the activity of an AstD polypeptide may be, for example, at least 2-fold greater, at least 3-fold greater, at least 4-fold greater, at least 5-fold greater, at least 7.5-fold greater, at least 10-fold greater, at least 12.5-fold greater, at least 15-fold greater, at least 17.5-fold greater, at least 20-fold greater, at least 22.5-fold greater, at least 25-fold greater, at least 27.5-fold greater, at least 30-fold greater, at least 35-fold greater, at least 40-fold greater, at least 45-fold greater, at least 50-fold greater, at least 55-fold greater, at least 60-fold greater, at least 70-fold greater, at least 80-fold greater, at least 90-fold greater, at least 100-fold greater, at least 125-fold greater, or at least 150-fold greater or more as compared to the concentration of aspterric acid needed to inhibit the activity of a housekeeping DHAD polypeptide (*e.g.* SEQ ID NO: 1). Inhibition of protein activity may be based on $IC_{50}$ values of aspterric acid's ability to inhibit a reaction as outlined in **FIG. 2B.** In this instance, the $IC_{50}$ value indicates the quantity of aspterric acid needed to inhibit the ability of a polypeptide (*e.g.* AstD, DHAD) to catalyze a reaction as outlined in **FIG. 2B** by half (50%). In some aspects of the disclosure, aspterric acid may have no detectable ability to inhibit the activity of an AstD polypeptide.

An amino acid sequence alignment between the DHAD protein from *A. thaliana* (SEQ ID NO: 4) and the AstD protein from *Aspergillus terreus* NIH2624 (SEQ ID NO: 10) is presented in **FIG. 36.** The sequence identity between pDHAD and AstD is 56.8%. In some aspects, an AstD polypeptide of the present disclosure has at least 80% or greater (*e.g.* at least 85%, at least 90%, at least 95%, at least 99%, or 100%) sequence identity to any one of SEQ ID NOs: 10-16, while also having less than about 75% (*e.g.* less than 70%, less than 65%, less than 60%, less than 55%, less than 50%) sequence identity to a housekeeping DHAD polypeptide (*e.g.* any one of SEQ ID NOs: 1-9). In some aspects, an AstD polypeptide of the present disclosure has at least 80% or greater sequence identity to SEQ ID NO: 10, while also having less than about 60% sequence identity to a housekeeping DHAD polypeptide (*e.g.* SEQ ID NO: 4).

As discussed herein, without wishing to be bound by theory, it is thought that the resistance of AstD polypeptides to aspterric acid is derived from the smaller hydrophobic pocket in AstD polypeptides than in DHAD polypeptides, such that

the smaller hydrophobic pocket cannot accommodate the aspterric acid molecule. In some aspects, an AstD polypeptide of the present disclosure: 1) has at least 80% or greater *(e.g.* at least 85%, at least 90%, at least 95%, at least 99%, or 100%) sequence identity to any one of SEQ ID NOs: 10-16, and 2) also contains a leucine (or a chemically similar amino acid) at an amino acid position that corresponds to amino acid 518 of SEQ ID NO: 10, and/or also contains a leucine (or a chemically similar amino acid) at an amino acid position that corresponds to amino acid 198 of SEQ ID NO: 10.

In some aspects of the disclosure, a plant's endogenous DHAD protein (which is susceptible to inhibition by aspterric acid) may be modified such that it adopts the features of an AstD protein which result in reduced susceptibility to inhibition by aspterric acid. As discussed above, it is thought that the resistance of AstD polypeptides to aspterric acid is derived from the smaller hydrophobic pocket in AstD polypeptides than in DHAD polypeptides, such that the smaller hydrophobic pocket cannot accommodate the aspterric acid molecule, while natural substrates of DHAD can still bind. Certain aspects of the present disclosure therefore relate to structural modification of a DHAD polypeptide such that the hydrophobic pocket that would normally accommodate the aspterric acid molecule is no longer able to do so (and thus the modified DHAD polypeptide will have reduced or eliminated susceptibility to inhibition of its function or activity by aspterric acid).

In some aspects, a modified DHAD polypeptide of the present disclosure: 1) has at least 80% or greater *(e.g.* at least 85%, at least 90%, at least 95%, at least 99%, or 100%) sequence identity to any one of SEQ ID NOs: 1-9, and 2) also contains an amino acid substitution at an amino acid position that corresponds to amino acid 496 and/or 177 of SEQ ID NO: 4, such that the amino acid substitution results in a hydrophobic pocket in the polypeptide that would normally accommodate the aspterric acid molecule is no longer able to do so (and thus the modified DHAD polypeptide will have reduced or eliminated susceptibility to inhibition of its function or activity by aspterric acid). In some aspects of the disclosure, a leucine (or a chemically similar amino acid) is substituted for the endogenous amino acid at an amino acid position that corresponds to amino acid 496 of SEQ ID NO: 4 (normally V496), and/or a leucine (or a chemically similar amino acid) is substituted for the endogenous amino acid at an amino acid position that corresponds to amino acid 177 of SEQ ID NO: 4 (normally I177).

### Recombinant Nucleic Acids Encoding Recombinant Proteins

Certain aspects of the present disclosure relate to recombinant nucleic acids encoding recombinant proteins of the present disclosure (*e.g*. AstD proteins).

As used herein, the terms "polynucleotide," "nucleic acid," and variations thereof shall be generic to polydeoxyribonucleotides (containing 2-deoxy-D-ribose), to polyribonucleotides (containing D-ribose), to any other type of polynucleotide that is an N-glycoside of a purine or pyrimidine base, and to other polymers containing non-nucleotidic backbones, provided that the polymers contain nucleobases in a configuration that allows for base pairing and base stacking, as found in DNA and RNA. Thus, these terms include known types of nucleic acid sequence modifications, for example, substitution of one or more of the naturally occurring nucleotides with an analog, and inter-nucleotide modifications. As used herein, the symbols for nucleotides and polynucleotides are those recommended by the IUPAC-IUB Commission of Biochemical Nomenclature.

In one aspect, the present disclosure provides a recombinant nucleic acid encoding an AstD protein. In some aspects of the disclosure, the recombinant nucleic acid encodes an AstD polypeptide or fragment thereof that has an amino acid sequence that is at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identical to any one of SEQ ID NOs: 10, 11, 12, 13, 14, 15, and 16.

Sequences of the polynucleotides of the present disclosure may be prepared by various suitable methods known in the art, including, for example, direct chemical synthesis or cloning. For direct chemical synthesis, formation of a polymer of nucleic acids typically involves sequential addition of 3'-blocked and 5'-blocked nucleotide monomers to the terminal 5'-hydroxyl group of a growing nucleotide chain, wherein each addition is effected by nucleophilic attack of the terminal 5'-hydroxyl group of the growing chain on the 3'-position of the added monomer, which is typically a phosphorus derivative, such as a phosphotriester, or phosphoramidite. Such methodology is known to those of ordinary skill in the art and is described in the pertinent texts and literature (*e.g.,* in Matteucci et al., (1980) Tetrahedron Lett 21:719-722; U.S. Pat. Nos. 4,500,707; 5,436,327; and 5,700,637). In addition, the desired sequences may be isolated from natural sources by splitting DNA using appropriate restriction enzymes, separating the fragments using gel electrophoresis, and thereafter, recovering the desired polynucleotide sequence from the gel via techniques known to those of ordinary skill in the art, such as utilization of polymerase chain reactions (PCR; *e.g.,* U.S. Pat. No. 4,683,195).

The nucleic acids employed in the methods and compositions described herein may be codon optimized relative to a parental template for expression in a particular host cell. Cells differ in their usage of particular codons, and codon bias corresponds to relative abundance of particular tRNAs in a given cell type. By altering codons in a sequence so that they are tailored to match with the relative abundance of corresponding tRNAs, it is possible to increase expression of a product *(e.g.* a polypeptide) from a nucleic acid. Similarly, it is possible to decrease expression by deliberately choosing codons

corresponding to rare tRNAs. Thus, codon optimization/deoptimization can provide control over nucleic acid expression in a particular cell type (*e.g.* bacterial cell, plant cell, mammalian cell, etc.). Methods of codon optimizing a nucleic acid for tailored expression in a particular cell type are well-known to those of skill in the art.

**Methods of Identifying Sequence Similarity**

Various methods are known to those of skill in the art for identifying similar (*e.g.* homologs, orthologs, paralogs, etc.) polypeptide and/or polynucleotide sequences, including phylogenetic methods, sequence similarity analysis, and hybridization methods.

Phylogenetic trees may be created for a gene family by using a program such as CLUSTAL (Thompson et al. Nucleic Acids Res. 22: 4673-4680 (1994); Higgins et al. Methods Enzymol 266: 383-402 (1996)) or MEGA (Tamura et al. Mol. Biol. & Evo. 24:1596-1599 (2007)). Once an initial tree for genes from one species is created, potential orthologous sequences can be placed in the phylogenetic tree and their relationships to genes from the species of interest can be determined. Evolutionary relationships may also be inferred using the Neighbor-Joining method (Saitou and Nei, Mol. Biol. & Evo. 4:406-425 (1987)). Homologous sequences may also be identified by a reciprocal BLAST strategy. Evolutionary distances may be computed using the Poisson correction method (Zuckerkandl and Pauling, pp. 97-166 in Evolving Genes and Proteins, edited by V. Bryson and H.J. Vogel. Academic Press, New York (1965)).

In addition, evolutionary information may be used to predict gene function. Functional predictions of genes can be greatly improved by focusing on how genes became similar in sequence (i.e. by evolutionary processes) rather than on the sequence similarity itself (Eisen, Genome Res. 8: 163-167 (1998)). Many specific examples exist in which gene function has been shown to correlate well with gene phylogeny (Eisen, Genome Res. 8: 163-167 (1998)). By using a phylogenetic analysis, one skilled in the art would recognize that the ability to deduce similar functions conferred by closely-related polypeptides is predictable.

When a group of related sequences are analyzed using a phylogenetic program such as CLUSTAL, closely related sequences typically cluster together or in the same clade (a group of similar genes). Groups of similar genes can also be identified with pair-wise BLAST analysis (Feng and Doolittle, J. Mol. Evol. 25: 351-360 (1987)). Analysis of groups of similar genes with similar function that fall within one clade can yield sub-sequences that are particular to the clade. These sub-sequences, known as consensus sequences, can not only be used to define the sequences within each clade, but define the functions of these genes; genes within a clade may contain paralogous sequences, or orthologous sequences that share the same function (see also, for example, Mount, Bioinformatics: Sequence and Genome Analysis Cold Spring Harbor Laboratory Press, Cold Spring Harbor, N.Y., page 543 (2001)).

To find sequences that are homologous to a reference sequence, BLAST nucleotide searches can be performed with the BLASTN program, score=100, wordlength=12, to obtain nucleotide sequences homologous to a nucleotide sequence encoding a protein of the disclosure. BLAST protein searches can be performed with the BLASTX program, score=50, wordlength=3, to obtain amino acid sequences homologous to a protein or polypeptide of the disclosure. To obtain gapped alignments for comparison purposes, Gapped BLAST (in BLAST 2.0) can be utilized as described in Altschul et al. (1997) Nucleic Acids Res. 25:3389. Alternatively, PSI-BLAST (in BLAST 2.0) can be used to perform an iterated search that detects distant relationships between molecules. See Altschul et al. (1997) supra. When utilizing BLAST, Gapped BLAST, or PSI-BLAST, the default parameters of the respective programs (*e.g.*, BLASTN for nucleotide sequences, BLASTX for proteins) can be used.

Methods for the alignment of sequences and for the analysis of similarity and identity of polypeptide and polynucleotide sequences are well-known in the art.

As used herein "sequence identity" refers to the percentage of residues that are identical in the same positions in the sequences being analyzed. As used herein "sequence similarity" refers to the percentage of residues that have similar biophysical / biochemical characteristics in the same positions (e.g. charge, size, hydrophobicity) in the sequences being analyzed.

Methods of alignment of sequences for comparison are well-known in the art, including manual alignment and computer assisted sequence alignment and analysis. This latter approach is a preferred approach in the present disclosure, due to the increased throughput afforded by computer assisted methods. As noted below, a variety of computer programs for performing sequence alignment are available, or can be produced by one of skill.

The determination of percent sequence identity and/or similarity between any two sequences can be accomplished using a mathematical algorithm. Examples of such mathematical algorithms are the algorithm of Myers and Miller, CABIOS 4:11-17 (1988); the local homology algorithm of Smith et al., Adv. Appl. Math. 2:482 (1981); the homology alignment algorithm of Needleman and Wunsch, J. Mol. Biol. 48:443-453 (1970); the search-for-similarity-method of Pearson and Lipman, Proc. Natl. Acad. Sci. 85:2444-2448 (1988); the algorithm of Karlin and Altschul, Proc. Natl. Acad. Sci. USA 87:2264-2268 (1990), modified as in Karlin and Altschul, Proc. Natl. Acad. Sci. USA 90:5873-5877 (1993).

Computer implementations of these mathematical algorithms can be utilized for comparison of sequences to determine sequence identity and/or similarity. Such implementations include, for example: CLUSTAL in the PC/Gene

program (available from Intelligenetics, Mountain View, Calif.); the AlignX program, version10.3.0 (Invitrogen, Carlsbad, CA) and GAP, BESTFIT, BLAST, FASTA, and TFASTA in the Wisconsin Genetics Software Package, Version 8 (available from Genetics Computer Group (GCG), 575 Science Drive, Madison, Wis., USA). Alignments using these programs can be performed using the default parameters. The CLUSTAL program is well described by Higgins et al. Gene 73:237-244 (1988); Higgins et al. CABIOS 5:151-153 (1989); Corpet et al., Nucleic Acids Res. 16:10881-90 (1988); Huang et al. CABIOS 8:155-65 (1992); and Pearson et al., Meth. Mol. Biol. 24:307-331 (1994). The BLAST programs of Altschul et al. J. Mol. Biol. 215:403-410 (1990) are based on the algorithm of Karlin and Altschul (1990) supra.

Polynucleotides homologous to a reference sequence can be identified by hybridization to each other under stringent or under highly stringent conditions. Single stranded polynucleotides hybridize when they associate based on a variety of well characterized physical-chemical forces, such as hydrogen bonding, solvent exclusion, and base stacking. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number thereof), as described in more detail in references cited below (e.g., Sambrook et al., Molecular Cloning: A Laboratory Manual, 2nd Ed., Vol. 1-3, Cold Spring Harbor Laboratory, Cold Spring Harbor, N.Y. ("Sambrook") (1989); Berger and Kimmel, Guide to Molecular Cloning Techniques, Methods in Enzymology, vol. 152 Academic Press, Inc., San Diego, Calif. ("Berger and Kimmel") (1987); and Anderson and Young, "Quantitative Filter Hybridisation." In: Hames and Higgins, ed., Nucleic Acid Hybridisation, A Practical Approach. Oxford, TRL Press, 73-111 (1985)).

Encompassed by the disclosure are polynucleotide sequences that are capable of hybridizing to the disclosed polynucleotide sequences and fragments thereof under various conditions of stringency (see, for example, Wahl and Berger, Methods Enzymol. 152: 399-407 (1987); and Kimmel, Methods Enzymo. 152: 507-511, (1987)). Full length cDNA, homologs, orthologs, and paralogs of polynucleotides of the present disclosure may be identified and isolated using well-known polynucleotide hybridization methods.

With regard to hybridization, conditions that are highly stringent, and means for achieving them, are well known in the art. See, for example, Sambrook et al. (1989) (supra); Berger and Kimmel (1987) pp. 467-469 (supra); and Anderson and Young (1985)(supra).

Hybridization experiments are generally conducted in a buffer of pH between 6.8 to 7.4, although the rate of hybridization is nearly independent of pH at ionic strengths likely to be used in the hybridization buffer (Anderson and Young (1985)(supra)). In addition, one or more of the following may be used to reduce non-specific hybridization: sonicated salmon sperm DNA or another non-complementary DNA, bovine serum albumin, sodium pyrophosphate, sodium dodecylsulfate (SDS), polyvinyl-pyrrolidone, ficoll and Denhardt's solution. Dextran sulfate and polyethylene glycol 6000 act to exclude DNA from solution, thus raising the effective probe DNA concentration and the hybridization signal within a given unit of time. In some instances, conditions of even greater stringency may be desirable or required to reduce non-specific and/or background hybridization. These conditions may be created with the use of higher temperature, lower ionic strength and higher concentration of a denaturing agent such as formamide.

Stringency conditions can be adjusted to screen for moderately similar fragments such as homologous sequences from distantly related organisms, or to highly similar fragments such as genes that duplicate functional enzymes from closely related organisms. The stringency can be adjusted either during the hybridization step or in the post-hybridization washes. Salt concentration, formamide concentration, hybridization temperature and probe lengths are variables that can be used to alter stringency. As a general guideline, high stringency is typically performed at $T_m$-5°C to $T_m$-20°C, moderate stringency at $T_m$-20°C to $T_m$-35°C and low stringency at $T_m$-35°C to $T_m$-50° C for duplex >150 base pairs. Hybridization may be performed at low to moderate stringency (25-50°C below $T_m$), followed by post-hybridization washes at increasing stringencies. Maximum rates of hybridization in solution are determined empirically to occur at $T_m$-25°C for DNA-DNA duplex and $T_m$-15°C for RNA-DNA duplex. Optionally, the degree of dissociation may be assessed after each wash step to determine the need for subsequent, higher stringency wash steps.

High stringency conditions may be used to select for nucleic acid sequences with high degrees of identity to the disclosed sequences. An example of stringent hybridization conditions obtained in a filter-based method such as a Southern or northern blot for hybridization of complementary nucleic acids that have more than 100 complementary residues is about 5°C to 20°C lower than the thermal melting point ($T_m$) for the specific sequence at a defined ionic strength and pH.

Hybridization and wash conditions that may be used to bind and remove polynucleotides with less than the desired homology to the nucleic acid sequences or their complements of the present disclosure include, for example: 6X SSC and 1% SDS at 65°C; 50% formamide, 4X SSC at 42°C; 0.5X SSC to 2.0 X SSC, 0.1% SDS at 50°C to 65°C; or 0.1X SSC to 2X SSC, 0.1% SDS at 50°C - 65°C; with a first wash step of, for example, 10 minutes at about 42°C with about 20% (v/v) formamide in 0.1X SSC, and with, for example, a subsequent wash step with 0.2 X SSC and 0.1% SDS at 65°C for 10, 20 or 30 minutes.

For identification of less closely related homologs, wash steps may be performed at a lower temperature, e.g., 50°

C. An example of a low stringency wash step employs a solution and conditions of at least 25°C in 30 mM NaCl, 3 mM trisodium citrate, and 0.1% SDS over 30 min. Greater stringency may be obtained at 42°C in 15 mM NaCl, with 1.5 mM trisodium citrate, and 0.1% SDS over 30 min. Wash procedures will generally employ at least two final wash steps. Additional variations on these conditions will be readily apparent to those skilled in the art (see, for example, US Patent Application No. 20010010913).

If desired, one may employ wash steps of even greater stringency, including conditions of 65°C -68°C in a solution of 15 mM NaCl, 1.5 mM trisodium citrate, and 0.1% SDS, or about 0.2X SSC, 0.1% SDS at 65° C and washing twice, each wash step of 10, 20 or 30 min in duration, or about 0.1 X SSC, 0.1% SDS at 65° C and washing twice for 10, 20 or 30 min. Hybridization stringency may be increased further by using the same conditions as in the hybridization steps, with the wash temperature raised about 3°C to about 5°C, and stringency may be increased even further by using the same conditions except the wash temperature is raised about 6°C to about 9°C.

**Plants of the Present Disclosure**

Certain aspects of the present disclosure relate to methods of reducing growth of a vegetative tissue in a plant by contacting the plant with aspterric acid or a derivative thereof. Certain aspects of the present disclosure relate to plants containing AstD proteins. In some aspects of the disclosure, plants containing AstD proteins have substantially increased resistance to inhibition of vegetative growth induced by aspterric acid or a derivative thereof as compared to plants that do not contain an AstD protein. Certain aspects of the present disclosure relate to methods of producing hybrid seed in plants. These methods involve use of aspterric acid or a derivative thereof as a hybridization agent. Certain aspects of the present disclosure relate to plants (and methods of producing such plants) that have reduced susceptibility to one or more herbicidal symptoms that are induced by aspterric acid as compared to a corresponding control plant. In some aspects of the disclosure, such plants have modified DHAD polypeptides whose activity has reduced susceptibility to inhibition by aspterric acid.

As used herein, a "plant" refers to any of various photosynthetic, eukaryotic multicellular organisms of the kingdom Plantae, characteristically producing embryos, containing chloroplasts, having cellulose cell walls and lacking locomotion. As used herein, a "plant" includes any plant or part of a plant at any stage of development, including seeds, suspension cultures, plant cells, embryos, meristematic regions, callus tissue, leaves, roots, shoots, gametophytes, sporophytes, pollen, microspores, and progeny thereof. Also included are cuttings, and cell or tissue cultures. As used in conjunction with the present disclosure, plant tissue includes, for example, whole plants, plant cells, plant organs, e.g., leafs, stems, roots, meristems, plant seeds, protoplasts, callus, cell cultures, and any groups of plant cells organized into structural and/or functional units.

In some aspects, a plant of the present disclosure is contacted with a composition containing aspterric acid or a derivative thereof. Plants that are contacted with aspeterric acid or a derivative thereof may have reduced growth of one or more vegetative tissues in the plant as compared to a corresponding control plant (*e.g.* a plant not contacted with aspterric acid). In some aspects of the disclosure, a vegetative tissue of a plant is contacted with a composition containing aspterric acid or a derivative thereof.

Vegetative tissues of the present disclosure generally refer to those tissues and/or organs associated with vegetative growth and development in plants. Vegetative tissues may include, for example, roots, leaves, and vegetative shoots. In some aspects of the disclosure, the vegetative tissue is a diploid tissue. Vegetative tissues in a plant would be readily apparent to one of skill in the art. Vegetative tissues are in contrast to reproductive tissues, which are associated with reproductive growth and development in plants. Reproductive tissues may include, for example, reproductive or floral shoots, flowers and parts thereof (*e.g.* stamen, pistil), fruits, and seeds. In some aspects of the disclosure, the reproductive tissue is a haploid tissue. Reproductive tissues in a plant would be readily apparent to one of skill in the art.

Various plants may be used in the methods of the present disclosure. Suitable plants include both monocotyledonous (monocot) plants and dicotyledonous (dicot) plants. Examples of suitable plants may include, for example, species of the Family Gramineae, including Sorghum bicolor and Zea mays; species of the genera: Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Avena, Hordeum, Secale, and Triticum.

In some aspects of the disclosure, plants and plant cells may include, for example, those from corn (Zea mays), canola (Brassica napus, Brassica rapa ssp.), Brassica species useful as sources of seed oil, alfalfa (Medicago sativa), rice (Oryza sativa), rye (Secale cereale), sorghum (Sorghum bicolor, Sorghum vulgare), millet (e.g., pearl millet (Pennisetum glaucum), proso millet (Panicum miliaceum), foxtail millet (Setaria italica), finger millet (Eleusine coracana)), sunflower (Helianthus annuus), safflower (Carthamus tinctorius), wheat (Triticum aestivum), duckweed (Lemna), soybean (Glycine max), tobacco (Nicotiana tabacum), potato (Solanum tuberosum), peanuts (Arachis hypogaea), cotton (Gossypium

barbadense, Gossypium hirsutum), sweet potato (Ipomoea batatus), cassava (Manihot esculenta), coffee (Coffea spp.), coconut (Cocos nucijra), pineapple (Ananas comosus), citrus trees (Citrus spp.), cocoa (Theobroma cacao), tea (Camellia sinensis), banana (Musa spp.), avocado (Persea americana), fig (Ficus casica), guava (Psidium guajava), mango (Mangifera indica), olive (Olea europaea), papaya (Carica papaya), cashew (Anacardium occidentale), macadamia (Macadamia spp.), almond (Prunus amygdalus), sugar beets (Beta vulgaris), sugarcane (Saccharum spp.), oats, barley, vegetables, ornamentals, and conifers.

Examples of suitable vegetables plants may include, for example, tomatoes (Lycopersicon esculentum), lettuce (e.g., Lactuca sativa), green beans (Phaseolus vulgaris), lima beans (Phaseolus limensis), peas (Lathyrus spp.), and members of the genus Cucumis such as cucumber (C. sativus), cantaloupe (C. cantalupensis), and musk melon (C. melo).

Examples of suitable ornamental plants may include, for example, azalea (Rhododendron spp.), hydrangea (Macrophylla hydrangea), hibiscus (Hibiscus rosasanensis), roses (Rosa spp.), tulips (Tulipa spp.), daffodils (Narcissus spp.), petunias (Petunia hybrida), carnation (Dianthus caryophyllus), poinsettia (Euphorbiapulcherrima), and chrysanthemum.

Examples of suitable conifer plants may include, for example, loblolly pine (Pinus taeda), slash pine (Pinus elliotii), ponderosa pine (Pinus ponderosa), lodgepole pine (Pinus contorta), Monterey pine (Pinus radiata), Douglas-fir (Pseudotsuga menziesii), Western hemlock (Isuga canadensis), Sitka spruce (Picea glauca), redwood (Sequoia sempervirens), silver fir (Abies amabilis), balsam fir (Abies balsamea), Western red cedar (Thuja plicata), and Alaska yellow-cedar (Chamaecyparis nootkatensis).

Examples of suitable leguminous plants may include, for example, guar, locust bean, fenugreek, soybean, garden beans, cowpea, mungbean, lima bean, fava bean, lentils, chickpea, peanuts (Arachis sp.), crown vetch (Vicia sp.), hairy vetch, adzuki bean, lupine (Lupinus sp.), trifolium, common bean (Phaseolus sp.), field bean (Pisum sp.), clover (Melilotus sp.) Lotus, trefoil, lens, and false indigo.

Examples of suitable forage and turf grass may include, for example, alfalfa (Medicago s sp.), orchard grass, tall fescue, perennial ryegrass, creeping bent grass, and redtop.

Examples of suitable crop plants and model plants may include, for example, Arabidopsis, corn, rice, alfalfa, sunflower, canola, soybean, cotton, peanut, sorghum, wheat, tobacco, and lemna.

Certain aspects of the present disclosure relate to expression of heterologous nucleic acids in a plant cell. Various plant cells may be used in the present disclosure so long as it remains viable after being transformed with a sequence of nucleic acids. Preferably, the plant cell is not adversely affected by the transduction of the necessary nucleic acid sequences, the subsequent expression of the proteins or the resulting intermediates.

The plants of the present disclosure may be genetically modified in that recombinant nucleic acids have been introduced into the plants, and as such the genetically modified plants do not occur in nature. In such aspects, a suitable plant of the present disclosure is one capable of expressing one or more nucleic acid constructs encoding one or more recombinant proteins. The recombinant proteins encoded by the nucleic acids may be *e.g.* AstD proteins.

As used herein, the terms "transgenic plant" and "genetically modified plant" are used interchangeably and refer to a plant which contains within its genome a recombinant nucleic acid. Generally, the recombinant nucleic acid is stably integrated within the genome such that the polynucleotide is passed on to successive generations. However, in certain aspects of the disclosure, the recombinant nucleic acid is transiently expressed in the plant. The recombinant nucleic acid may be integrated into the genome alone or as part of a recombinant expression cassette. "Transgenic" is used herein to include any cell, cell line, callus, tissue, plant part or plant, the genotype of which has been altered by the presence of exogenous nucleic acid including those transgenics initially so altered as well as those created by sexual crosses or asexual propagation from the initial transgenic.

"Recombinant nucleic acid" or "heterologous nucleic acid" or "recombinant polynucleotide" as used herein refers to a polymer of nucleic acids wherein at least one of the following is true: (a) the sequence of nucleic acids is foreign to (i.e., not naturally found in) a given host cell; (b) the sequence may be naturally found in a given host cell, but in an unnatural (e.g., greater than expected) amount; or (c) the sequence of nucleic acids contains two or more subsequences that are not found in the same relationship to each other in nature. For example, regarding instance (c), a recombinant nucleic acid sequence will have two or more sequences from unrelated genes arranged to make a new functional nucleic acid. Specifically, the present disclosure describes the introduction of an expression vector into a plant cell, where the expression vector contains a nucleic acid sequence coding for a protein that is not normally found in a plant cell or contains a nucleic acid coding for a protein that is normally found in a plant cell but is under the control of different regulatory sequences. With reference to the plant cell's genome, then, the nucleic acid sequence that codes for the protein is recombinant. A protein that is referred to as recombinant generally implies that it is encoded by a recombinant nucleic acid sequence which may be present in the plant cell. Recombinant proteins of the present disclosure may also be exogenously supplied directly to host cells (*e.g.* plant cells).

A "recombinant" polypeptide, protein, or enzyme of the present disclosure, is a polypeptide, protein, or enzyme that is encoded by a "recombinant nucleic acid" or "heterologous nucleic acid" or "recombinant polynucleotide."

In some aspects of the disclosure, the genes encoding the recombinant proteins in the plant cell may be

heterologous to the plant cell. In certain aspects of the disclosure, the plant cell does not naturally produce the recombinant proteins, and contains heterologous nucleic acid constructs capable of expressing one or more genes necessary for producing those molecules. In certain aspects of the disclosure, the plant cell does not naturally produce one or more polypeptides of the present disclosure, and is provided the one or more polypeptides through exogenous delivery of the polypeptides directly to the plant cell without the need to express a recombinant nucleic acid encoding the recombinant polypeptide in the plant cell.

Recombinant nucleic acids and/or recombinant proteins of the present disclosure may be present in host cells (*e.g.* plant cells). In some aspects of the disclosure, recombinant nucleic acids are present in an expression vector, and the expression vector may be present in host cells (*e.g.* plant cells).

**Expression of Recombinant Proteins in Host Cells**

Certain aspects of the present disclosure relate to expression of recombinant proteins in host cells (*e.g.* plant cells). A host cell of the present disclosure may include, for example, bacterial cells, fungal cells (*e.g.* yeast), and plant cells. Recombinant proteins of the present disclosure may be introduced into host cells via suitable methods known in the art.

In some aspects, a host cell of the present disclosure is a plant cell. Various methods for expressing proteins in plant cells are known in the art. For example, a recombinant protein (*e.g.* an AstD protein) can be exogenously added to plant cells. Alternatively, a recombinant nucleic acid encoding a recombinant protein of the present disclosure (*e.g.* an AstD protein) can be expressed in plant cells. Additionally, in some aspects, a recombinant protein of the present disclosure may be transiently expressed in a plant via viral infection of the plant, or by introducing the recombinant protein-encoding RNA into a plant. Methods of introducing recombinant proteins via viral infection or via the introduction of RNAs into plants are well known in the art. For example, Tobacco rattle virus (TRV) has been successfully used to introduce zinc finger nucleases in plants ("Nontransgenic Genome Modification in Plant Cells", Plant Physiology 154:1079-1087 (2010)).

In some aspects of the disclosure, a plant's endogenous DHAD protein (which is susceptible to inhibition by aspterric acid) may be modified such that it becomes an AstD protein (which has substantially reduced susceptibility to inhibition by aspterric acid).

A recombinant nucleic acid encoding a recombinant protein of the present disclosure can be expressed in a plant with any suitable plant expression vector. Typical vectors useful for expression of recombinant nucleic acids in higher plants are well known in the art and include, for example, vectors derived from the tumor-inducing (Ti) plasmid of Agrobacterium tumefaciens (e.g., see Rogers et al., Meth. in Enzymol. (1987) 153:253-277). These vectors are plant integrating vectors in that on transformation, the vectors integrate a portion of vector DNA into the genome of the host plant. Exemplary A. tumefaciens vectors useful herein are plasmids pKYLX6 and pKYLX7 (e.g., see of Schardl et al., Gene (1987) 61:1-11; and Berger et al., Proc. Natl. Acad. Sci. USA (1989) 86:8402-8406); and plasmid pBI 101.2 that is available from Clontech Laboratories, Inc. (Palo Alto, CA).

In addition to regulatory domains, a recombinant protein of the present disclosure can be expressed as a fusion protein that is coupled to, for example, a maltose binding protein ("MBP"), glutathione S transferase (GST), hexahistidine, c-myc, or the FLAG epitope for ease of purification, monitoring expression, or monitoring cellular and subcellular localization.

Moreover, a recombinant nucleic acid encoding a recombinant protein of the present disclosure can be modified to improve expression of the recombinant protein in plants by using codon preference. When the recombinant nucleic acid is prepared or altered synthetically, advantage can be taken of known codon preferences of the intended plant host where the nucleic acid is to be expressed. For example, recombinant nucleic acids of the present disclosure can be modified to account for the specific codon preferences and GC content preferences of monocotyledons and dicotyledons, as these preferences have been shown to differ (Murray et al., Nucl. Acids Res. (1989) 17: 477-498).

The present disclosure further provides expression vectors encoding recombinant proteins. A nucleic acid sequence coding for the desired recombinant nucleic acid of the present disclosure can be used to construct a recombinant expression vector which can be introduced into the desired host cell. A recombinant expression vector will typically contain a nucleic acid encoding a recombinant protein of the present disclosure, operably linked to transcriptional initiation regulatory sequences which will direct the transcription of the nucleic acid in the intended host cell, such as tissues of a transformed plant.

For example, plant expression vectors may include (1) a cloned gene under the transcriptional control of 5' and 3' regulatory sequences and (2) a dominant selectable marker. Such plant expression vectors may also contain, if desired, a promoter regulatory region (e.g., one conferring inducible or constitutive, environmentally- or developmentally-regulated, or cell- or tissue-specific/selective expression), a transcription initiation start site, a ribosome binding site, an RNA processing signal, a transcription termination site, and/or a polyadenylation signal.

A plant promoter, or functional fragment thereof, can be employed to control the expression of a recombinant nucleic acid of the present disclosure in regenerated plants. The selection of the promoter used in expression vectors will

determine the spatial and temporal expression pattern of the recombinant nucleic acid in the modified plant, e.g., the nucleic acid encoding a recombinant protein of the present disclosure is only expressed in the desired tissue or at a certain time in plant development or growth. Certain promoters will express recombinant nucleic acids in all plant tissues and are active under most environmental conditions and states of development or cell differentiation (i.e., constitutive promoters). Other promoters will express recombinant nucleic acids in specific cell types (such as leaf epidermal cells, mesophyll cells, root cortex cells) or in specific tissues or organs (roots, leaves or flowers, for example) and the selection will reflect the desired location of accumulation of the gene product. Alternatively, the selected promoter may drive expression of the recombinant nucleic acid under various inducing conditions.

Examples of suitable constitutive promoters may include, for example, the core promoter of the Rsyn7, the core CaMV 35S promoter (Odell et al., Nature (1985) 313:810-812), CaMV 19S (Lawton et al., 1987), rice actin (Wang et al., 1992; U.S. Pat. No. 5,641,876; and McElroy et al., Plant Cell (1985) 2:163-171); ubiquitin (Christensen et al., Plant Mol. Biol. (1989)12:619-632; and Christensen et al., Plant Mol. Biol. (1992) 18:675-689), pEMU (Last et al., Theor. Appl. Genet. (1991) 81:581-588), MAS (Velten et al., EMBO J. (1984) 3:2723-2730), nos (Ebert et al., 1987), Adh (Walker et al., 1987), the P- or 2'- promoter derived from T-DNA of Agrobacterium tumefaciens, the Smas promoter, the cinnamyl alcohol dehydrogenase promoter (U.S. Pat. No. 5,683,439), the Nos promoter, the pEmu promoter, the rubisco promoter, the GRP 1 - 8 promoter, and other transcription initiation regions from various plant genes known to those of skilled artisans, and constitutive promoters described in, for example, U.S. Pat. Nos. 5,608,149; 5,608,144; 5,604,121; 5,569,597; 5,466,785; 5,399,680; 5,268,463; and 5, 608,142.

Examples of suitable tissue specific promoters may include, for example, the lectin promoter (Vodkin et al., 1983; Lindstrom et al., 1990), the corn alcohol dehydrogenase 1 promoter (Vogel et al., 1989; Dennis et al., 1984), the corn light harvesting complex promoter (Simpson, 1986; Bansal et al., 1992), the corn heat shock protein promoter (Odell et al., Nature (1985) 313:810-812; Rochester et al., 1986), the pea small subunit RuBP carboxylase promoter (Poulsen et al., 1986; Cashmore et al., 1983), the Ti plasmid mannopine synthase promoter (Langridge et al., 1989), the Ti plasmid nopaline synthase promoter (Langridge et al., 1989), the petunia chalcone isomerase promoter (Van Tunen et al., 1988), the bean glycine rich protein 1 promoter (Keller et al., 1989), the truncated CaMV 35s promoter (Odell et al., Nature (1985) 313:810-812), the potato patatin promoter (Wenzler et al., 1989), the root cell promoter (Conkling et al., 1990), the maize zein promoter (Reina et al., 1990; Kriz et al., 1987; Wandelt and Feix, 1989; Langridge and Feix, 1983; Reina et al., 1990), the globulin-1 promoter (Belanger and Kriz et al., 1991), the $\alpha$-tubulin promoter, the cab promoter (Sullivan et al., 1989), the PEPCase promoter (Hudspeth & Grula, 1989), the R gene complex-associated promoters (Chandler et al., 1989), and the chalcone synthase promoters (Franken et al., 1991).

Alternatively, the plant promoter can direct expression of a recombinant nucleic acid of the present disclosure in a specific tissue or may be otherwise under more precise environmental or developmental control. Such promoters are referred to here as "inducible" promoters. Environmental conditions that may affect transcription by inducible promoters include, for example, pathogen attack, anaerobic conditions, or the presence of light. Examples of inducible promoters include, for example, the AdhI promoter which is inducible by hypoxia or cold stress, the Hsp70 promoter which is inducible by heat stress, and the PPDK promoter which is inducible by light. Examples of promoters under developmental control include, for example, promoters that initiate transcription only, or preferentially, in certain tissues, such as leaves, roots, fruit, seeds, or flowers. An exemplary promoter is the anther specific promoter 5126 (U.S. Pat. Nos. 5,689,049 and 5,689,051). The operation of a promoter may also vary depending on its location in the genome. Thus, an inducible promoter may become fully or partially constitutive in certain locations.

Moreover, any combination of a constitutive or inducible promoter, and a non-tissue specific or tissue specific promoter may be used to control the expression of a recombinant protein of the present disclosure.

The recombinant nucleic acids of the present disclosure and/or a vector housing a recombinant nucleic acid of the present disclosure, may also contain a regulatory sequence that serves as a 3' terminator sequence. One of skill in the art would readily recognize a variety of terminators that may be used in the recombinant nucleic acids of the present disclosure. For example, a recombinant nucleic acid of the present disclosure may contain a 3' NOS terminator. Further, a native terminator from a recombinant protein of the present disclosure may also be used in the recombinant nucleic acids of the present disclosure.

Plant transformation protocols as well as protocols for introducing recombinant nucleic acids of the present disclosure into plants may vary depending on the type of plant or plant cell, e.g., monocot or dicot, targeted for transformation. Suitable methods of introducing recombinant nucleic acids of the present disclosure into plant cells and subsequent insertion into the plant genome include, for example, microinjection (Crossway et al., Biotechniques (1986) 4:320-334), electroporation (Riggs et al., Proc. Natl. Acad Sci. USA (1986) 83:5602-5606), Agrobacterium-mediated transformation (U.S. Pat. No. 5,563,055), direct gene transfer (Paszkowski et al., EMBO J. (1984) 3:2717-2722), and ballistic particle acceleration (U.S. Pat. No. 4,945,050; Tomes et al. (1995). "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); and McCabe et al., Biotechnology (1988) 6:923-926).

Additionally, a recombinant protein of the present disclosure can be targeted to a specific organelle within a plant

cell. Targeting can be achieved by providing the recombinant protein with an appropriate targeting peptide sequence. Examples of such targeting peptides include, for example, secretory signal peptides (for secretion or cell wall or membrane targeting), plastid transit peptides, chloroplast transit peptides, mitochondrial target peptides, vacuole targeting peptides, and nuclear targeting peptides (e.g., see Reiss et al., Mol. Gen. Genet. (1987) 209(1):116-121; Settles and Martienssen, Trends Cell Biol (1998) 12:494-501; Scott et al., J Biol Chem (2000) 10:1074; and Luque and Correas, J Cell Sci (2000) 113:2485-2495).

In some aspects, a recombinant polypeptide of the present disclosure (*e.g.* an AstD polypeptide) may be fused to a chloroplast localization sequence. In some aspects, a chloroplast localization sequence of the present disclosure has an amino acid sequence with at least about 20%, at least about 25%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% amino acid identity to the amino acid sequence of the chloroplast localization sequence of SEQ ID NO: 19.

The modified plant may be grown into plants in accordance with conventional ways (e.g., see McCormick et al., Plant Cell. Reports (1986) 81-84.). These plants may then be grown, and pollinated with either the same transformed strain or different strains, with the resulting hybrid having the desired phenotypic characteristic. Two or more generations may be grown to ensure that the subject phenotypic characteristic is stably maintained and inherited and then seeds harvested to ensure the desired phenotype or other property has been achieved.

Certain aspects of the present disclosure relate to plants (and methods of producing such plants) that have reduced susceptibility to one or more herbicidal symptoms that are induced by aspterric acid as compared to a corresponding control plant. In some aspects of the disclosure, such plants have modified DHAD polypeptides whose activity has reduced susceptibility to inhibition by aspterric acid.

Modified DHAD polypeptides whose activity has reduced susceptibility to inhibition by aspterric acid are discussed above. Methods that could be employed to produce plants having modified DHAD polypeptides whose activity has reduced susceptibility to inhibition by aspterric acid are known in the art. For example, methods involving CRISPR/-Cas9 (with homology template) or base editing approaches to nucleic acid editing may be used to generate such modified DHAD polypeptides in a plant. Other approaches may involve direct delivery of heterologous polypeptides involved in the nucleic acid editing process to the plant. Such editing approaches may be used to generate plants that are 1) not transgenic, and 2) have reduced susceptibility to one or more herbicidal symptoms that are induced by aspterric acid as compared to a corresponding control plant.

Methods of generating new plants from the original plant where a DHAD-encoding nucleic acid was edited to produce a DHAD polypeptide having reduced susceptibility to inhibition by aspterric acid are known in the art. For example, the original edited plant could have any heterologous nucleic acids used during the DHAD editing process crossed away by crossing the original edited plant to the same or another plant, and progeny selected that 1) do not contain the heterologous nucleic acids, and 2) maintain reduced susceptibility to one or more herbicidal symptoms that are induced by aspterric acid as compared to a corresponding control plant. Tissue culture regeneration processes may also be used to generate a new plant from the original edited plant.

In some aspects of the disclosure, a DHAD-encoding nucleic acid in the germ cell line of a plant having a DHAD polypeptide that is susceptible to inhibition by aspterric acid is directly edited in the germ cell line, where the edited DHAD nucleic acid would then encode a DHAD polypeptide that has reduced susceptibility to inhibition by aspterric acid. A progeny plant could then be produced or regenerated from the plant with the edited germ cell line (*e.g.* via crossing the plant with the edited germ cell line to another plant), where the progeny plant has reduced susceptibility to one or more herbicidal symptoms that are induced by aspterric acid as compared to a corresponding control plant.

In some aspects of the disclosure, a plant that has had a DHAD-encoding nucleic acid edited to encode a DHAD polypeptide that has reduced susceptibility to inhibition by aspterric acid may be crossed to a second plant to produce one or more F1 plants that contain a nucleic acid which encodes a DHAD polypeptide that has reduced susceptibility to inhibition by aspterric acid. In some aspects of the disclosure, one or more F1 plants that contain a nucleic acid which encodes a DHAD polypeptide that has reduced susceptibility to inhibition by aspterric acid are selected that 1) do not contain any recombinant nucleic acids involved with the DHAD nucleic acid editing process in the parent plant, and 2) have reduced susceptibility to one or more herbicidal symptoms that are induced by aspterric acid as compared to a corresponding control plant.

**Methods of Cultivating Plants and Herbicidal Activity on Plant Tissues**

Certain aspects of the present disclosure relate to compositions containing aspterric acid or a derivative thereof. In some aspects of the disclosure, plant tissues are contacted with a composition containing aspterric acid or a derivative thereof. These plant tissues may be vegetative tissues or they may be reproductive tissues. Compositions containing aspterric acid or a derivative thereof may have herbicidal activity on plant tissues. Thus, plant tissues that are contacted

with a composition containing aspterric acid or a derivative thereof may have reduced growth or exhibit other herbicidal symptoms as compared to corresponding control plant tissue (*e.g.* a plant tissue not contacted with aspterric acid or a derivative thereof).

Plants and plant tissues contacted with a composition containing aspterric acid or a derivative thereof may exhibit reduced growth as compared to a corresponding control plant. The reduced growth may be reduced vegetative growth and/or reduced reproductive growth. The plant or plant tissue may have its growth rate reduced by at least about 5%, at least about 10%, at least about 15%, at least about 20%, at least about 25%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% as compared to a corresponding control. Corresponding controls will be readily apparent to one of skill in the art. For example, a corresponding control plant or plant tissue may be a plant or plant tissue that is not contacted with aspterric acid or a derivative thereof.

Plants and plant tissues contacted with a composition containing aspterric acid or a derivative thereof may exhibit herbicidal symptoms. Herbicidal symptoms may include, for example, cytotoxicity, cell death, reduced growth, inhibited development, and organism death. The rate of development of herbicidal symptoms in a plant or plant tissue contacted with a composition containing aspterric acid or a derivative thereof may be, for example, at least about 5%, at least about 10%, at least about 15%, at least about 20%, at least about 25%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% or more faster as compared to a corresponding control. Corresponding controls will be readily apparent to one of skill in the art. For example, a corresponding control plant or plant tissue may be a plant or plant tissue that is not contacted with aspterric acid or a derivative thereof, a plant or plant tissue contacted with a different herbicidal agent, etc.

In some aspects of the disclosure, plants containing an AstD protein have increased resistance to the inhibitory growth and/or herbicidal symptoms that are induced by a composition containing aspterric acid or a derivative thereof as compared to a corresponding control. The rate of development of one or more herbicidal symptoms in a plant or plant tissue containing an AstD protein and that is contacted with a composition containing aspterric acid or a derivative thereof may be, for example, at least about 5%, at least about 10%, at least about 15%, at least about 20%, at least about 25%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% or more reduced as compared to a corresponding control. Corresponding controls will be readily apparent to one of skill in the art. For example, a corresponding control plant or plant tissue may be a plant or plant tissue that is contacted with aspterric acid or a derivative thereof, but that does not contain an AstD protein.

In some aspects of the disclosure, the total fresh weight of a plant following a period of time after being contacted with aspterric acid or a derivative thereof may serve as a proxy for a plant's degree of resistance to vegetative growth inhibition induced by aspterric acid. In such aspects of the disclosure, the total fresh weight of a plant exhibiting resistance to aspterric acid may be, for example, at least 2-fold higher, at least 3-fold higher, at least 4-fold higher, at least 5-fold higher, at least 7.5-fold higher, at least 10-fold higher, at least 12.5-fold higher, at least 15-fold higher, at least 17.5-fold higher, at least 20-fold higher, at least 22.5-fold higher, at least 25-fold higher, at least 27.5-fold higher, at least 30-fold higher, at least 35-fold higher, at least 40-fold higher, at least 45-fold higher, at least 50-fold higher, at least 55-fold higher, at least 60-fold higher, at least 70-fold higher, at least 80-fold higher, at least 90-fold higher, at least 100-fold higher, at least 125-fold higher, or at least 150-fold higher as compared to a corresponding control (*e.g.* a plant known to have no resistance to vegetative growth inhibition induced by aspterric acid) following a period of time after being contacted with aspterric acid or a derivative thereof. The period of time may vary, as noted below.

In methods of the present disclosure relating to generating an aspterric acid-resistant plant, further provided are methods of screening a plant or population of plants to identify an aspterric acid-resistant plant. Such screening methods may involve obtaining a plant or population of plants suspected of having increased resistance to aspterric acid (*e.g.* a plant containing an AstD polypeptide) as compared to a corresponding control, and contacting that plant or population of plants with a composition containing aspterric acid or a derivative thereof. The composition containing aspterric acid or derivative thereof should be applied to the plant at a concentration sufficient to induce inhibition of vegetative growth in a plant that is not resistant to aspterric acid. Plants contacted with such compositions may be maintained in a condition or environment such that the aspterric acid or derivative thereof could induce inhibition of vegetative growth in a plant that is not resistant to aspterric acid. Plants may then be scored for their resistance to the inhibition of vegetative growth (or other herbicidal symptom as outlined above) as compared to a corresponding control (*e.g.* a plant that is known to be susceptible to growth inhibition induced by aspterric acid). Plants that are determined to have a degree of resistance to aspterric acid (*e.g.* at

least a 50% reduction in the rate of development of one or more herbicidal symptoms as compared to a corresponding control) may be selected for additional purposes.

Compositions of the present disclosure containing aspterric acid or a derivative thereof may be applied to plants or specific plant tissues with varying frequencies. Plants may be contacted on multiple occasions and/or over a time interval. For example, the compositions may be applied twice per day, once per day, once every day, once every two days, once every three days, once every four days, once every five days, or once per week, or more or less frequently. Suitable application schedules will be readily apparent to one of skill in the art. The total duration of the treatment with a composition containing aspterric acid or a derivative thereof may also vary. Total durations of treatment/application may include, for example, one day, two days, three days, one week, 1.5 weeks, 2 weeks, 2.5 weeks, 3 weeks, 3.5 weeks, or 4 weeks (one month) or longer. Plants may also be grown in a growth media where the compositions containing aspterric acid or a derivative thereof is consistently or continuously present. In other words, plants may be grown in conditions where the exposure of a plant tissue to aspterric acid is continuous.

Concentrations and quantities of aspterric acid or a derivative thereof in compositions of the present disclosure are described above. These compositions may be applied to one or more reproductive or vegetative plant tissues. The quantity of the composition containing aspterric acid or a derivative thereof that is applied to plant tissues may vary. For example, the quantity of the composition may be about 0.1 mL, about 0.2 mL, about 0.3 mL, about 0.4 mL, about 0.5 mL, about 0.6 mL, about 0.7 mL, about 0.8 mL, about 0.9 mL, about 1 mL, about 2.5 mL, about 5 mL, about 7.5 mL, about 10 mL, about 25 mL, about 50 mL, or about 100 mL or more. The application rate of the composition containing aspterric acid or a derivative thereof may also very. For example, the application rate may be at least 0.22417 kg/ha (0.2 lb/acre), at least 0.56043 kg/ha (0.5 lb/acre), at least 0.89668 kg/ha (0.8 lb/acre), at least 1.12085 kg/ha (1 lb/acre), at least 1.34502 kg/ha (1.2 lb/acre), at least 1.56919 kg/ha (1.4 lb/acre), at least 1.79336 kg/ha (1.6 lb/acre), at least 2.01753 kg/ha (1.8 lb/acre), at least 2.2417 kg/ha (2 lb/acre), or at least 2.46587 kg/ha (2.2 lb/acre) or more.

Plants may be grown on various growth media, as will be readily apparent to one of skill in the art. Suitable growth media include, for example, agar and other media plates, soil, turf, etc.

Plants of the present disclosure may be grown in a number of suitable growing conditions depending on the particular desired outcome. Suitable growing conditions may include, for example, ambient environmental conditions, standard greenhouse conditions, growth in long days under standard environmental conditions (e.g. 16 hours of light, 8 hours of dark), growth in 12 hour light : 12 hour dark day/night cycles, etc.

It is to be understood that while the present disclosure has been described in conjunction with the preferred specific aspects thereof, the foregoing description is intended to illustrate the present disclosure.

## EXAMPLES

The following examples are offered to illustrate provided aspects of the disclosure.

### **Example 1: Identification of Biosynthetic Gene Clusters and Self-Resistance Enzymes** (for illustrative purpose only, not part of the claimed invention)

This Example demonstrates the search for and analysis of biosynthetic gene clusters (BGCs) that encode gene products involved in resistance to natural products (NPs). Introduction

Currently, with the large amount of microbial genome information available through next generation sequencing, genome-guided mining of NPs is emerging as a potentially powerful approach to discover new NPs. However, establishing an effective approach to find NPs with new modes of action remains a challenge. NPs discovered with unguided genome mining that relies solely on the activation of gene clusters of unknown function can lead to production of new compounds, but nearly always without any clue regarding potential biological activity.

The approach described in this Example aims to identify biosynthetic gene clusters (BGCs) that encode gene products involved in resistance to natural products (NPs) using a target-guided mining (TGM) strategy. Host organisms that produce NPs must have a method of self-protection, which is frequently achieved through the co-expression of an alternative version of the target enzyme that is insensitive to the NP. The self-resistance enzyme (SRE) is a mutated version of a housekeeping enzyme and is located in the NP BGC. This co-localization of the NP, BGC, and SRE gene is also well conserved during horizontal gene transfer between different host species, because it is essential for the survival of hosts when making a bioactive NP. Accordingly, Applicant proposes a target-guided mining (TGM) approach to analyze genomes that contain biosynthetic gene clusters (BGCs) that encode gene products involved in resistance to natural products (NPs) to bridge the gap between activity-guided NP isolation and genome-guided NP discovery.

A large group of herbicides target the branched-chain amino acid (valine, leucine and isoleucine) biosynthetic pathway, because it only exists in bacteria, archaea, fungi and plants. Animals (including humans), on the other hand, are not able to produce branched-chain amino acids *de novo* and have to obtain them through their diets. Valine and isoleucine are produced by two parallel pathways using a three enzymatic steps: acetolacetate synthase (ALS), Acetohydroxy acid

isomeroreductase (KARI) and DHAD (*See* **FIG. 1**). Among them, ALS has been the target for commercially successful herbicides since 1980, and currently are the second largest class of active herbicidal products in weed control for many non-transgenic crops. Although potent and selective inhibitors of KARI and DHAD have also been identified, these inhibitors showed weak herbicidal activity. Compared to KARI, rationally designing an inhibitor of DHAD is currently not feasible, due to the lack of structural information. To circumvent this structural biological bottleneck for developing herbicides with new modes of action, Applicant searched for potential natural product (NP) gene clusters using DHAD as the self-resistance enzyme (SRE).

Filamentous fungi, which are documented to be prolific producers of NPs, interact with plants ecologically. Thus, the targets of fungal NPs are frequently plant metabolic enzymes and are therefore relevant to weed control. Genome sequencing has shown that many fungal species contain up to 60 BGCs, yet on average, less than 4-5 NPs are reported for each fungus. NP biosynthetic genes are typically clustered in microbial genomes and anchored by one or more core enzymes that are indicative of the product family. These core enzymes include polyketide synthases (PKS), nonribosomal peptide synthetases (NRPS), and terpene synthases (TS), etc. Therefore, candidate BGCs that fit the target-guided mining (TGM) paradigm may contain both a core NP enzyme and a target as SRE.

Materials and Methods

*Target Guided Genome Mining of Biosynthetic Gene Clusters*

An algorithm was developed to search through the ~500 available fungal genomes using the target-guided mining approach described above. The algorithm was based on MultiGeneBlast, with additional restrictions. These restrictions included (1) distances between the SRE and the core NP enzyme should be less than 20kb; (2) a minimal sequence identity of the core NP enzyme to consensus sequences is greater than 20%; (3) the SRE is an additional copy of the housekeeping enzyme elsewhere in the genome; and (4) the gene cluster is conserved and syntenic among multiple species.

Results

Using the search procedure described above, one BGC was found to satisfy the requirements above using pair-wise inputs of core enzymes (TS, terpene synthase) and SRE (DHAD, dihydroxy acid dehydratase). This BGC was a 10kb gene cluster among various fungal species that encodes four enzymes: DHAD (AstD), TS (AstA) and two P450s (AstB and AstC). A second copy of DHAD is present in the genome of fungi carrying this cluster, and is the housekeeping gene that is extremely well conserved in fungal organisms. Indeed, a BLASTP search for homologs of DHAD from *Aspergillus terreus* identified the astD protein from *Aspergillus terreus* as having 70% amino acid identity to the housekeeping DHAD from *Aspergillus terreus.*

From the above, it was reasoned that AstD is likely the self-resistance enzyme, while the TS and the P450s synthesize a natural product that can inhibit the housekeeping copy of DHAD. **FIG. 2A** illustrates the BGC identified above from several organisms, and **FIG. 2B** illustrates the reaction catalyzed by DHAD.

**Example 2: Heterologous Expression and Purification of Aspterric Acid** (for illustrative purpose only, not part of the claimed invention)

This Example demonstrates that expression of the *astABC* gene cluster identified in Example 1 allows for production of aspterric acid in yeast cells. A proposed biosynthetic pathway for aspterric acid is also provided.

Materials and Methods

*Heterologous Expression of AstA, AstB, and AstC*

*astA, astB,* and *astC* from *Aspergillus terreus* NIH2624 were amplified by PCR, cloned into bacterial or yeast expression vectors, and transformed independently or in combination into *Aspergillus nidulans* or *Saccharomyces cerevisiae* cells (**FIG. 3**).

*Identification of Biosynthetic Products*

Compounds that were present in transformed host cells were isolated, purified to homogeneity, and characterized with 1D and 2D NMR spectroscopy.

Results

As an initial step, expression of the *astABC* gene cluster from *A. terreus* that was identified in Example 1 was analyzed in *A. terreus.* However, transcription of this gene cluster in its endogenous organism (*A. terreus*) appeared to be silenced, as indicated by RT-PCR. Analysis of expression of this gene cluster was therefore pursued using heterologous expression in other host cells.

In order to determine the chemical composition of the natural product that AstA, AstB, and AstC were responsible for synthesizing, AstA, AstB, and AstC were expressed either independently or in combination in *Aspergillus nidulans* and *Saccharomyces cerevisiae* cells as outlined in **FIG. 3.** Synthesized compounds were isolated and purified.

Although the *astABC* gene cluster was transcribed in *A. nidulans,* as evidenced by the ability to obtain cDNA, there was no significant production of novel biosynthetic intermediates or final products. Without wishing to be bound by theory, it is thought that failure to obtain these compounds in *A. nidulans* may be the result of a low level of protein expression, deactivation of protein function, or low precursor stability in *A. nidulans.*

In *Saccharomyces cerevisiae,* however, biosynthetic products were readily detectable. Independent expression of AstA in *Saccharomyces cerevisiae* produced a sesquiterpene, which was confirmed to be (-)-daucane (product 1 in **FIG. 3**). Expression of both AstA and AstB in *S. cerevisiae* produced product 2 (see **FIG. 3**). Finally, expression of AstA, AstB, and AstC in *S. cerevisiae* produced product 3 (see **FIG. 3**), which was isolated and purified, characterized with 1D and 2D NMR spectroscopy, and determined to be aspterric acid.

**FIG. 4** outlines a proposed biosynthetic pathway for the production of aspterric acid. Without wishing to be bound by theory, it is thought that farnesyl diphosphate is converted to product 1 by AstA, which is then oxidized four times (once at the C-C double bond between C8 and C9 to form an expoxide, and three times on C14 to form the carboxylic acid) by AstB to form product 2 and, finally, AstC hydroxylates the C15, which is then followed by ring opening of the epoxide to form product 3 (aspterric acid).

**Example 3: Bioactivity of Aspterric Acid on Housekeeping DHADs and AstD** (for illustrative purpose only, not part of the claimed invention)

This Example demonstrates that aspterric acid can effectively inhibit bioactivity of housekeeping DHADs from *A. terreus* and *A. thaliana,* while failing to inhibit AstD from *A. terreus.*

Materials and Methods

*Heterologous Protein Expression and Purification*

The cDNA of DHAD from *A. thaliana* (AT3G23940) was amplified and cloned into with pET28a using NheI and NotI as restriction sites. The resultant DHAD contained an N-terminal 6×His tag with a molecular weight of 65 kD. The resulted plasmid was transformed into E.coli BL21 (DE3) for expression. Expression assays were conducted at 16°C at 220 rpm for 20 h under 100μM IPTG induction (IPTG was added when OD600 = 0.8). Cells of 1 liter culture were then harvested by centrifugation at 4°C. The cell pellet was resuspended in 15 mL buffer A (20 mM Tris-HCl pH 7.5, 100 mM NaCl, 10% glycerol, 5 mM imidazole, 5 mM DTT and 5 mM GSH). The cells were broken by ultra-sonication, and the insoluble material was sedimented by centrifugation at 16000 rpm at 4°C. The protein supernatant was then incubated with 3 mL Ni-NTA sepharose overnight with slow, constant rotation at 4°C. Subsequently the Ni-NTA sepharose was washed with 10 column volume buffer B (buffer A+50mM imidazole). For elution of the target protein, the sepharose was incubated for 10 min with 6 mL buffer C (buffer A + 500 mM imidazole). The supernatant from the elution step was then analyzed by SDS-PAGE together with the supernatants from the other purification steps. The elution fraction containing the recombinant protein was desalted and kept in storage buffer (50 mM Tris-HCl pH 7.2, 50 mM NaCl, 10% glycerol, 5 mM DTT and 5 mM GSH).

DHAD and AstD from *Aspergillus terreus* were also expressed and purified using a similar method.

*Enzymatic Activity and Inhibition Assays*

Enzymatic activity reactions were carried out in 50 mL volumes containing 50 mM storage buffer, 10 mM (±)-Sodium 2,3-dihydroxyisovalerate hydrate (DHAD substrate) and 1μM purified DHAD enzyme. After incubation for 30 minutes at 30°C, the reaction was stopped by adding an equal volume of ethanol. 1/10 volumes of 100mM phenylhydrozine was added at room temperature for 30 minutes to derivatize the DHAD-synthesized molecule (3-methyl-2-oxo-butanoic acid) into a detectable derivatization product (DDP). See **FIG. 5A** for an overview of reactions.

Inhibition assays were carried out according to the reactions described above. First, phenylhydrozine was added to 3-methyl-2-oxo-butanoic acid to validate the derivatization reaction. Next, DHAD substrate was incubated with 1μM

purified DHAD enzyme from *A. thaliana* (with or without DMSO) then derivatized with phenylhydrozine to validate the activity of the purified DHAD enzyme. Finally, DHAD substrate was incubated with 1$\mu$M purified DHAD enzyme from *A. thaliana* and 1mM aspterric acid in DMSO then derivatized with phenylhydrozine to determine if DHAD was inhibited by aspterric acid. 20 $\mu$L of the reaction mixture was subject to LC-MS analysis, and DDP was detected by UV absorption at 350nm. The area of the peak that corresponded to DDP was used to quantify the amount of ($\pm$)-Sodium 2,3-dihydroxyisovalerate hydrate that was converted into DDP.

*$IC_{50}$ and Enzyme Kinetics Measurements*

Inhibition assays were carried out according to the reactions described above by incubating purified DHAD enzymes (either the housekeeping DHAD from *Aspergillus terreus,* the housekeeping DHAD from *Arabidopsis thaliana,* or AstD from *Aspergillus terreus*) with aspterric acid at various concentrations. For AstD, up to 8mM of aspterric acid was tested. Derivatization with phenylhydrozine, LC-MS analysis, and DDP detection by UV absorption at 350nm was carried out. The half maximal inhibitory concentration ($IC_{50}$) and the effect of aspterric acid on the enzyme kinetics of each purified DHAD enzyme was calculated based on the initial reaction rates observed in the inhibition assays.

Results

In order to test if aspterric acid inhibited housekeeping DHADs and/or AstD, inhibition assays were performed. However, a first series of assays tested whether the housekeeping DHAD from *A. thaliana* could enzymatically transform ($\pm$)-Sodium 2,3-dihydroxyisovalerate hydrate (DHAD Substrate) into 3-methyl-2-oxo-butanoic acid in order to confirm enzymatic activity of this DHAD. To create a detectable product, phenylhydrozine was added to probe conversion of 3-methyl-2-oxo-butanoic acid into a detectable derivatization product (DDP). First, the phenylhydrozine derivatization reaction was validated (**FIG. 5B**) and the enzymatic activity of DHAD (**FIG. 5C and 5D**) was confirmed, as determined by the presence of a DDP peak during LC-MS analysis. However, when aspterric acid was added to the *A. thaliana* housekeeping DHAD, no DDP was observed (**FIG. 5E**), indicating that aspterric acid had completely inhibited the enzymatic activity of DHAD.

Inhibition assays of DHADs with increasing concentrations of aspterric acid revealed the inhibition kinetics and $IC_{50}$ of aspterric acid on the housekeeping DHAD from *Aspergillus terreus,* the housekeeping DHAD from *Arabidopsis thaliana,* and AstD from *Aspergillus terreus.* Results are summarized in **FIG. 6A, 6B, 6C, 6D**, and **Table 3A.** The $IC_{50}$ of aspterric acid towards DHADs from *A. terreus* and *A. thaliana* were further determined to be 0.31 $\mu$M and 0.50 $\mu$M respectively at an enzyme concentration of 0.5 $\mu$M. These results reveal that aspterric acid can effectively inhibit bioactivity of housekeeping DHADs from *A. terreus* and *A. thaliana.* Further, the inhibition constant $K_i$ of aspterric acid *against A. thaliana* DHAD was determined to be 0.30 $\mu$M, and kinetic analyses indicate that aspterric acid is a competitive inhibitor.

With respect to AstD, although the $IC_{50}$ of aspterric acid towards AstD was not determined because of its low solubility at high concentrations, no inhibition was observed at the concentration of 8 mM. While AstD is not inhibited by aspterric acid, it is significantly slower in catalyzing the DHAD reaction than the housekeeping DHADs ($k_{cat}$=0.05 $s^{-1}$,$K_m$= 5.4 mM), a phenomenon that has been seen with other self-resistance enzymes that have sequence homology to housekeeping enzymes. Overall, the data supports that AstD is a self-resistance enzyme.

**Table 3A: Inhibition of DHADs by Aspterric Acid**

| Organism | Enzyme | $IC_{50}$ ($\mu$M) | $k_{cat}$ ($s^{-1}$) | $K_m$ (mM) |
|---|---|---|---|---|
| *Aspergillus terreus* NIH2624 | AstD | nd* | 0.05 | 5.4 |
| *Aspergillus terreus* NIH2624 | Housekeeping DHAD | 0.31 | 3.0 | >20 |
| *Arabidopsis thaliana* | Housekeeping DHAD | 0.50 | 1.2 | 5.7 |
| *not determined; no inhibition at 8mM aspterric acid | | | | |

A proposed model for inhibition of the DHAD active site by aspterric acid is presented in **FIG. 7.** Although the structure of DHAD was not determined, the binding mode of aspterric acid to the active site of DHAD can be proposed base on structure-activity relationships on different inhibitors. Without wishing to be bound by theory, the following model is proposed: the binding pocket can be divided into two part based on binding force, which is hydrophobic half and hydrophilic half. The hydrophobic interaction is provided by the hydrophobic multicyclic portions of the inhibitor. The hydrophilic interaction is provided by hydrogen bonding of the 2-hydroxyl group and charge interactions of the carboxylic acid anion. Besides these two interactions, the coordination of the 3-hydroxyl and carboxylic acid group to iron of the iron-sulfur cluster, as well as a magnesium ion, also contribute to the binding. Aspterric acid is able to satisfy all the interactions

outlined, which suggest it is a strong inhibitor: (1) hydrophobic interaction is achieved by side chain and hydrocarbon skeleton; (2) dipole and electrostatic interactions are achieved by charge interaction of carboxylic acid and hydrogen bonding of hydroxyl group; (3) iron coordination is contributed by carboxylic acid and the ether oxygen; (4) the rigid three fused ring structure reduces entropy loss due to configuration adjustment of inhibitor during the binding process.

A proposed model for inhibition of the DHAD active site by derivatives of aspterric acid is presented in **FIG. 8.**

**Example 4: Analysis of Cytotoxicity of Aspterric Acid** (for illustrative purpose only, not part of the claimed invention)

This Example demonstrates that aspterric acid has low toxicity toward human tumor cell lines.

Materials and Methods

*MTT Cytotoxicity Assay*

Standard MTT assays were carried out as follows: two human tumor cell lines (melanoma cell line A375 and SK-MEL-1) were seeded in wells of a 96-well plate. Aspterric acid or glyphosate was added 24 hours post-seeding and incubated with cells for 72 hours. Cell survival was quantified using the CellTiter-GLO assay (Promega). Five replicates per treatment were carried out.

Results

To evaluate cytotoxicity, the cytotoxicity of aspterric acid was compared to glyphosate. MTT assays revealed low toxicity of aspterric acid on both tumor cell lines (**FIG. 9A and FIG. 9B**). Without wishing to be bound by theory, it is thought that aspterric acid may not be toxic to human cell lines because DHAD is not present in human cells.

**Example 5: Growth Inhibition By Aspterric Acid Treatment** (for illustrative purpose only, not part of the claimed invention)

This Example demonstrates the ability of aspterric acid to inhibit normal growth and development in a number of organisms.

Materials and Methods

*Yeast Growth Inhibition Assay*

*Saccharomyces cerevisiae* was plated onto dropout media that lacked isoleucine, leucine, and valine, either with or without 250 μM aspterric acid. *Saccharomyces cerevisiae* was also plated onto rich media that contained all amino acids along with 250 μM aspterric acid. Plates were incubated at 30°C.

*Streptomyces Growth Inhibition Assay*

*Streptomyces sp.* Mg1 was plated onto MS media either with or without 250 μM aspterric acid. Plates were incubated at 28°C.

*Plant Growth Inhibition Assay*

Agar-based growth inhibition assays were carried out on MS media (4.33g Murashige and Skoog basal medium, 20 g sucrose, and 10 g Agar per liter MS media, pH was adjusted to 5.7 using KOH). Aspterric acid at a final concentration of 50 μM was included in the experimental media. DMSO at final concentration of 1% was used to increase the solubility of aspterric acid. The control MS media contained the same amount of DMSO, but no aspterric acid. Sterilized *Arabidopsis thaliana* seeds were plated on MS media. After 2 days of cold treatment at 4°C in the dark, plates were transferred to standard growing condition (16 hour light and 8 hour dark at 22°C) to geminate. After germination on day 4, the seedlings were transferred to MS media containing 50 μM aspterric acid. MS media containing only DMSO was used as a control. Images were taken at day 8 and day 12 to assay growth inhibition activity of aspterric acid on *Arabidopsis thaliana.*

Sterile green bean seedlings were plated on MS media containing 50 μM aspterric acid and 1% DMSO (to increase the solubility of aspterric acid) on day 0. MS media containing only 1% DMSO was used as a control. Growth was assessed on day 3 and day 7.

Sterile tomato seedlings were plated on MS media containing 50 μM aspterric acid and 1% DMSO (to increase the solubility of aspterric acid) or on MS media containing 50 μM glyphosate on day 0. MS media containing only 1% DMSO was used as a control. Growth was assessed on day 3 and day 7.

Results

Growth of various species (*Saccharomyces cerevisiae, Streptomyces, Arabidopsis thaliana,* green bean, and tomato), either with our without the presence of aspterric acid, was assayed.

*Saccharomyces cerevisiae* were plated on media lacking at least three essential amino acids (isoleucine, leucine, and valine) either with or without aspterric acid. Aspterric acid inhibited the growth of *Saccharomyces cerevisiae* when present in media that lacked isoleucine, leucine and valine (**FIG. 10A**, bottom row), while control plates that lacked aspterric acid grew normally (**FIG. 10A**, top row). Yeast on plates containing rich media and aspterric acid also grew normally (data not shown).

Similarly, aspterric acid inhibited the growth of *Streptomyces* when plated on MS media (**FIG. 10B**, bottom row), while control plates containing MS media but without aspterric acid grew normally (**FIG. 10B**, top row).

Growth inhibitory activity of aspterric acid was tested against *Arabidopsis thaliana,* green bean, and tomato. *Arabidopsis thaliana* seedlings that were plated on MS media containing 50 μM aspterric acid had significant vegetative growth inhibition compared to DMSO control plates when observed on day 8 and day 12 (**FIG. 11**).

Green bean seedlings (**FIG. 12**) and tomato seedlings (**FIG. 13**) that were grown on MS media containing 50 μM aspterric acid also showed significant vegetative growth inhibition compared to DMSO control plants when observed on day 3 and day 7. Green bean seedlings grown on aspterric acid-containing media showed attenuated aerial and root tissue development as compared to control DMSO plants (**FIG. 12**). Similar results were observed in tomato seedlings, where development of plants grown on aspterric acid more closely resembled that of plants grown on the herbicide glyphosate than that of control plants grown in the presence of DMSO (**FIG. 13**). Taken together, these data indicate that aspterric acid has herbicidal activity on vegetative plant growth.

**Example 6: Growth Inhibition of Plants *in vivo* Upon Aspterric Acid Treatment**

This Example demonstrates that aspterric acid exhibits herbicidal activity against vegetative growth and development in *Arabidopsis thaliana.*

Materials and Methods

*Herbicidal Spray Experiments*

Aspterric acid was dissolved in the following solvent formulations at a final concentration of 1mM: **(1)** 0.5% silwet L-77 and 1% DMSO (floral dip formulation), (2) 2% EtOH, 1% corn oil, and 0.1% tween 80, and **(3)** 2% EtOH and 0.05% Finale (Finale formulation, contains a final concentration of 200μM glufosinate).

For spray treatments with aspterric acid in the various formulations described above, *Arabidopsis thaliana* Col-0 ecotype plants were grown in soil under long day conditions (16 hours of light followed by 8 hours of dark per day) at 23°C using cool-white fluorescence bulbs as the light source. Ten-day old seedlings were sprayed with 1mM aspterric acid dissolved in formulation (1), (2), or (3) as described above. Spray application with the various respective formulations was repeated every 2 days.

Results

To test the herbicidal activity of aspterric acid in different solvent formulations, 1mM aspterric acid was dissolved into three solvent formulations and each solution was sprayed onto *Arabidopsis thaliana* Col-0 ecotype plants.

18 days after the first spray treatment, plants sprayed with aspterric acid dissolved in formulation (1) showed growth inhibition (**FIG. 14**). This growth inhibition was significant compared to the untreated plants and the plants sprayed with formulation (1) alone, but was weaker than the growth inhibition seen in the glyphosate and glufosinate herbicidal treatments (**FIG**. **14**).

17 days after the first spray treatment, plants sprayed with aspterric acid dissolved in formulation (2) also showed growth inhibition (**FIG. 15**). This growth inhibition was significant compared to the untreated plants and the plants sprayed with formulation (2) alone (**FIG**. **15**). Growth inhibition was stronger than that of plants treated with aspterric acid dissolved in formulation (1), but was still not as strong than the growth inhibition seen in the glyphosate and glufosinate herbicidal treatments (**FIG. 15**).

18 days after the first spray treatment, glufosinate-resistant *Arabidopsis* plants sprayed with aspterric acid

dissolved in formulation (3) showed significant growth inhibition compared to the untreated plants and the plants sprayed with glufosinate (**FIG. 16**). Growth inhibition was stronger than that of plants treated with aspterric acid dissolved in either formulation (1) or formulation (2), exhibiting inhibition of meristem growth and dark green leaves indicating herbicidal injury (**FIG. 16**). However, herbicidal activity was still not as strong as that seen in the glyphosate herbicidal treatment (**FIG. 16**). Taken together, these data reveal that aspterric acid dissolved in various solvent formulations shows herbicidal activity when sprayed onto plants grown in soil.

**Example 7: Use of Aspterric Acid as a Chemical Hybridization Agent** (for illustrative purpose only, not part of the claimed invention)

The Example demonstrates the use of aspterric acid as a chemical hybridization agent in plant breeding.

Materials and Methods

Flowers of *Arabidopsis thaliana* Col-0 plants were treated with aspterric acid. The treated flowers that were missing all six fertile stamens were selected as the female parent in a cross with a male parent of known genetic identity. Non-treated Arabidopsis plants containing a BASTA resistant gene were used as male parent to donate pollen to the plants where the flowers were missing all six fertile stamens. 2-week old F1 progeny resulting from the cross were treated with Finale (11.3% Glufosinate-ammonium) at a 1:2000 dilution.

Results

It was previously reported that aspterric acid was able to inhibit pollen development. Applicant reasoned that it may therefore be possible to use aspterric acid as a chemical hybridization agent in plant breeding. In this sense, flowers may be treated with aspterric acid to inhibit the development of pollen on the stamens of the same flower, eliminating the possibility that this pollen could serve as parent to pollinate the pistil on the same flower. Pollen from a separate donor flower could then be used to pollinate the pistil on the flower treated with aspterric acid, resulting in progeny that all share the same male parent and the same female parent.

To explore whether aspterric acid (AA) could be used as a successful chemical hybridization agent, wild type Arabidopsis flowers were treated with aspterric acid were pollinated with pollen of BASTA resistant Arabidopsis. The results of this cross are outlined in **Table 7A.** The results demonstrate that the siliques were formed and the resulted seeds from the cross grew well with BASTA resistance. This demonstrates that aspterric acid may be used as a chemical hybridization agent in plant breeding.

**Table 7A: Progeny Analysis**

| ♀ (female) | ♂ (male) | Silique and seed | Basta resistance in next generation |
|---|---|---|---|
| Columbia-0 (AA treatment) | Columbia-0 (AA) | - | - |
| Columbia-0 (AA treatment) | Basta resistance | + | + |

**Example 8: Generation of Aspterric Acid-Resistant Plants**

This Example demonstrates a scheme for producing aspterric acid-resistant plants, as well as an exemplary protocol for selecting aspterric-acid resistant plants.

Materials and Methods

*Cloning and Plant Selection*

The coding sequence of *astD* was PCR amplified using primers, and cloned into pENTR/D entry vector. The insert was verified through Sanger sequencing before being mitigated into pEG202 through LR reaction. In pEG202 the expression of *astD* is driven by CaMV 35S promoter. A plasmid containing the desired insert was electro-transformed into Agrobacterium strain Agl0 followed by plant infection using the floral dip method (Clough SJ and Bent AF 1998 Plant J). Wild-type Arabidopsis thaliana of Col-0 ecotype was used as the host plant for astD transgene expression. Positive transgenic plants showing BASTA resistance were selected.

Experimental Outline

Using the method described above, Applicant has developed a construct and cloning procedure for transferring a heterologous *astD* gene into Arabidopsis plants.
The transformation and selection scheme outlined in **FIG. 17** is an exemplary scheme for introducing a heterologous *astD* gene into plants.
Plants suspected of containing the *astD* transgene will be further screened to confirm the existence of the transgene. Plants verified to carry the *astD* transgene will be screened for their resistance to aspterric acid using methods described in previous Examples. Without wishing to be bound by theory, it is thought that plants carrying an *astD* transgene will exhibit resistance to vegetative growth inhibition induced by treatment with aspterric acid.

**Example 9: Genome mining of a natural product herbicide with a new mode of action** (for illustrative purpose only, not part of the claimed invention)

This Example provides additional data and information in conjunction with that provided in the previous Examples. Weeds cause substantial crop losses world-wide and, while effective herbicides are available, weeds continuously evolve herbicide resistance. As a result, there is constant need for herbicides with new modes of action. Dihydroxyacid dehydratase, which is required for branched chain amino acid biosynthesis, is a desired target for herbicide development although no effective inhibitor is available. Applicant performed target-guided genome mining of uncharacterized fungal natural product biosynthetic gene clusters and discovered aspterric acid as a potent herbicide which acts through the submicromolar inhibition of dihydroxyacid dehydratase. A gene cluster-colocalized dihydroxy-acid dehydratase gene that provides self-resistance to aspterric acid was characterized and demonstrated to be useful to confer aspterric acid tolerance in transgenic plants. This powerful herbicide-resistance gene combination complements existing weed control mechanisms.

**Introduction**

Weeds are one of the major causes of worldwide crop loss (*1*, *2*). Effective weed control heavily relies on herbicides (*3, 4*). The constant and often excessive usage of herbicides results in many weeds evolving herbicide resistance (*5, 6*). This is a major issue for crop management leading to an urgent need for herbicides with novel modes of action. The branched-chain amino acids (BCAAs) biosynthetic pathway is essential for plant growth (7). It is not present in animals and is therefore a validated target for highly specific weed control agents (8). The BCAA biosynthetic pathway in plants is carried out by three enzymes: acetolactate synthase (ALS), acetohydroxy acid isomeroreductase (KARI), and dihydroxyacid dehydratase (DHAD) (**FIG. 18A**). ALS is the most targeted enzyme for herbicide development with 56 registered herbicides, including imidazolinone, sulfonylurea and triazolopyrimidine sulfonamide (*7, 9*). Given the success of targeting BCAA synthesis pathway, it is notable that no herbicide that targets either of the other two enzymes has been successfully developed. The last enzyme DHAD in the BCAA pathway, catalyzing $\beta$-dehydration reactions to yield $\alpha$-keto acid precursors to isoleucine, valine and leucine, is an essential and highly conserved enzyme among plant species, showing >80% sequence similarity among even distally related plant species (*10*, *11*) (**FIG. 18B** and **FIG. 19A-19B**). Efforts toward synthetic DHAD inhibitors resulted in compounds with submicromolar $K_i$; however, the compounds do not show herbicidal activities when applied *in planta* (*12*) (**FIG**. **18C**).
Filamentous fungi are prolific producers of natural products (NPs), many of which have biological activities that aid the fungi in competing with, colonizing and killing plants (*13-15*). Therefore, fungal NPs represent a promising source of potential leads for herbicides. The abundance of sequenced fungal genomes, which have revealed vast untapped NP biosynthetic potentials, enables genome mining of new NPs with unprecedented biological activities (*16, 17*). Although no known NP inhibitors of DHAD are known to date, Applicant reasoned that a fungal NP with this property might exist, given the indispensable role of BCAA biosynthesis in plants (*7*).
To identify NP biosynthetic gene clusters that may encode a DHAD inhibitor, Applicant proposed that such cluster must contain an additional copy of DHAD that is insensitive to the inhibitor, thereby providing the required self-resistance for the producing organism to survive. The presence of a gene encoding a self-resistance enzyme is frequently found in NP gene clusters, as highlighted by the presence of an insensitive copy of HMGR or IMPDH in the gene clusters for lovastatin (that targets HMGR) or mycophenolic acid (that targets IMPDH), respectively (**FIG. 20**) (*18, 19*). This phenomenon has been used to predict molecular targets of NPs, as well as to identify gene clusters of NPs of known activities (20).

**Materials and Methods**

*General materials and methods*

Biological reagents, chemicals, media and enzymes were purchased from standard commercial sources unless stated. Plant, fungal, yeast and bacterial strains, plasmids and primers used herein are summarized in **Table 9A, Table 9B,** and **Table 9C.** DNA and RNA manipulations were carried out using Zymo ZR Fungal/Bacterial DNA Microprep™ kit and Invitrogen Ribopure™ kit respectively. DNA sequencing was performed at Laragen, Inc. The primers and codon optimized gblocks were synthesized by IDT, Inc.

**Table 9A: Primers for PCR amplification**

| Primer | Sequences of primer (5'→3') | SEQ ID NO. |
|---|---|---|
| AstD-pYTU-recomb-F | gagagcctgagcttcatccccagcatcattacacctcagcaatgttcgcgtcgaggatcc | 25 |
| AstA-pYTU-recomb-R | gactaaccattaccccgccacatagacacatctaaacaatggacatgaataccttccccg | 26 |
| Gpda-pYTU-F | gtggaggacatacccgtaattttctgggcatttaaatactccggtgaattgatttgggtg | 27 |
| Gpda-R | tgtttagatgtgtctatgtggcggg | 28 |
| AstB-pYTR-recomb-F | aaccattaccccgccacatagacacatctaaacaatgctattccaagacctgtcttttcc | 29 |
| AstB-pYTR-recomb-R | gctaaagggtatcatcgaaagggagtcatccaggtactgcttgtattgaatcctagtttg | 30 |
| AstC-pYTP-recomb-F | cccttctctgaacaataaaccccacagaaggcatttatgggagcttctactttctcccag | 31 |
| AstC-pYTP-recomb-R | caacaaccatgataccaggggatttaaatttaattaaggttggggtttcatgcatatagc | 32 |
| AstA-xw55-recomb-F | tggctagcgattataaggatgatgatgataagactagtatggacatgaataccttccccg | 33 |
| AstA-xw55-recomb-R | atttgtcatttaaattagtgatggtgatggtgatgcacgtgttatgcgttgcctagcggg | 34 |
| AstB-xw06-recomb-F | caactatcaactattaactatatcgtaataccatatgctattccaagacctctcgtttcc | 35 |
| AstB-xw06-recomb-R | tacttgataatggaaactataaatcgtgaaggcatctacttgcagagacccataactcgc | 36 |
| AstC-xw02-recomb-F | atcaactatcaactattaactatatcgtaataccatatgggagcttctactttctccctg | 37 |
| AstC-xw02-recomb-R | ttgataatgaaaactataaatcgtgaaggcatgtttaaacctagcctcgtctctttattc | 38 |
| pDHAD-pET-F | atagctagcatgcaagccaccatcttctctcc | 39 |
| pDHAD-pET-R | atagcggccgcttactcgtcagtcacacatccatctg | 40 |
| fDHAD-pET-F | atacatatgcttctctctcagacccgg | 41 |
| fDHAD-pET-R | atagcggccgettagtcaagagcateggtgatgcag | 42 |

(continued)

| Primer | Sequences of primer (5'→3') | SEQ ID NO. |
|---|---|---|
| AstD-pET-F | atacatatgttcgcgtcgaggatcc | 43 |
| AstD-pET-R | atagcggccgcctagatcggtccgtccgtgac | 44 |
| fDHAD-pXP318-F | gcatagcaatctaatctaagttttaattacaaaactagtatgcttct ctctcagacccgg | 45 |
| fDHAD-pXP318-R | gaatgtaagcgtgacataactaattacatgactcgagttagtca agagcatcggtgatgc | 46 |
| AstD-pXP318-F | tagcaatctaatctaagttttaattacaaaactagtatggactaca aagacgatgacgac | 47 |
| AstD-pXP318-R | gcgtgaatgtaagcgtgacataactaattacatgactcgagcta gatcggtccgtccgtg | 48 |
| DHAD-F | acaggatccgcccaatccgtaaccgc | 49 |
| DHAD-R | cacgtcgacttactcgtcagtcacacatccat | 50 |
| K559AK560A-F | acataggagcagcaagaatagacacacaagtctcacccg | 51 |
| K559AK560A-R | gtctattcttgctgctcctatgtcaatggtgattatgtctc | 52 |

**Table 9B: Plasmids**

| Plasmids | Features |
|---|---|
| pYTU | protein expression vector in *A. nidulans* (*pyrG* marker) |
| pYTR | protein expression vector in *A. nidulans (riboB* marker) |
| pYTP | protein expression in *A. nidulans* (*pyroA* marker) |
| pAstD+AstA-pYTU | pYTU expressing *astA* and *astD* |
| pAstB-pYTR | pYTR expressing *astB* |
| pAstC-pYTP | pYTP expressing *astC* |
| pXW55 | protein expression vector in *S. cerevisiae* (*URA3* marker) |
| pXW06 | protein expression vector in *S. cerevisiae* (*TRP2* marker) |
| pXW02 | protein expression vector in *S. cerevisiae* (*LEU2* marker) |
| pAstA-xw55 | pXW55 expressing *astA* |
| pAstB-xw06 | pXW06 expressing *astB* |
| pAstC-xw02 | pXW02 expressing *astC* |
| pET28a | protein expression vector in *E. coli* BL21 (DE3) |
| pDHAD-pET | pET28a expressing *A. thaliana* DHAD |
| fDHAD-pET | pET28a expressing *A. terreus* housekeeping DHAD |
| AstD-pET | pET28a expressing AstD |
| pXP318 | protein expression vector in *S. cerevisiae* (*URA3* marker) |
| fDHAD-pXP318 | pXP318 expressing *A. terreus* DHAD |
| AstD-pXP318 | pXP318 expressing AstD |
| pEG202 | protein expression vector in *A. thaliana* (*blp*$^R$ marker) |
| pAstDo-pEG | pEG202 expressing codon optimized AstD |

**Table 9C: Microbial Strains**

| Strain | Genotype |
|---|---|
| **Fungi** | |
| *Aspergillus terreus* NIH2624 | |
| *Aspergillus nidulans* A1145 | *ΔpyrG, ΔpvroA, ΔriboB* |
| TY01 | *Aspergillus nidulans* A1145 carrying AstD+AstA-pYTU, AstB-pYTR, AstC-pYTP |
| ***Saccharomyces cerevisiae*** | |
| RC01 | *MATα urα3-52 his3-Δ200 leu2-Δ1 trpl pep4::HIS3 ura3-52::atCPR prb1 Δ1.6R canl GAL* |
| TY02 | RC01 carrying pAstA-xw55 |
| TY03 | TY02 carrying pAstB-xw06 |
| TY04 | TY03 carrying pAstC-xw02 |
| DHY *ΔURA3* | *MATα ura3Δ0* |
| UB01 | DHY *ΔURA3 ilv3::URA3* |
| UB02 | DHY *ΔURA3 AILYV3* |
| TY05 | UB02 carrying pXP318 |
| TY06 | UB02 carrying fDHAD-pXP318 |
| TY07 | UB02 carrying AstD-pXP318 |
| ***Escherichia coli*** | |
| DH10β | |
| BL21 (DE3) | |
| TY08 | BL21 (DE3) carrying AstD-pET28a |
| TY09 | BL21 (DE3) carrying pDHAD-pET28a |
| TY10 | BL21 (DE3) carrying fDHAD-pET28a |

*Expression of ast genes in Aspergillus nidulans for cDNA isolation*

Plasmids pYTU, pYTP, pYTR digested with *Pac*I and *Swa*I were used as vectors to insert genes (*1*). A *gpda* promoter was generated by PCR amplification using primers Gpda-pYTU-F and Gpda-R with pYTR serving as template. Genes to be expressed were amplified through PCR using the genomic DNA of *Aspergillus terreus* NIH2624 as a template. A 4.5 kb fragment obtained using primers AstD-pYTU-recomb-F and AstA-pYTU-recomb-R was cloned into pYTU together with a *gpda* promoter by yeast homologous recombination to obtain pAstD+AstA-pYTU. Yeast transformation was performed using Frozen-EZ Yeast Transformation II Kit™ (Zymo Research). A 2.4 kb fragment obtained using primers AstB-pYTR-recomb-F and AstB-pYTR-recomb-R was cloned into pYTR by yeast homologous recombination to obtain pAstB-pYTR. Similarly, a 2.3 kb fragment obtained using primers AstC-pYTP-recomb-F and AstC-pYTP-recomb-R was cloned into pYTP by yeast homologous recombination to obtain pAstC-pYTP.

All three plasmids (pAstD+AstA-pYTU, pAstB-pYTR and pAstC-pYTP) were transformed into *A. nidulans* following standard protocols to result in the *A. nidulans* strain TY01 (1). TY01 was cultured in liquid CD-ST medium (20 g/L starch, 20 g/L peptone, 50 mL/L nitrate salts and 1 mL/L trace elements) at 28°C for 3 days. Total RNA of TY01 was extracted with the Invitrogen Ribopure™ kit, and total cDNA of TY01 was obtained using the SuperScript III reverse transcriptase kit (Thermo Fisher Scientific). The cDNA fragment of *astA* was PCR amplified using primers AstA-xw55-recomb-F and AstA-xw55-recomb-R. The cDNA fragment of *ast*B was PCR amplified using primers AstB-xw06-recomb-F and AstB-xw06-recomb-R. The cDNA fragment of *astC* was PCR amplified using primers AstC-xw02-recomb-F and AstC-xw02-recomb-R. The cDNA fragment of *ast*D was PCR amplified using primers AstD-pXP318-F and AstD-pXP318-R. All the introns were confirmed to be correctly removed by sequencing.

*Construction of Saccharomyces cerevisiae strains*

**TY02.** Plasmid pXW55 (*URA3* marker) digested with *Nde*I and *Pme*I was used to introduce the *astA* gene (2). A 1.3 kb fragment containing *astA* obtained from PCR using primers AstA-xw55-recomb-F and AstA-xw55-recomb-R was cloned into pXW55 using yeast homologous recombination to afford pAstA-xw55. The plasmid pAstA-xw55 was then transformed into *Saccharomyces cerevisiae* RC01 to generate strain TY02 (3).

**TY03.** Plasmid pXW06 (*TRP1* marker) digested with *Nde*I and *PmeI* was used to introduce the *astB* gene (2). A 1.6 kb fragment containing *astB* obtained from PCR using primers AstB-xw06-recomb-F and AstB-xw06-recomb-R were cloned into pXW06 using yeast homologous recombination to afford pAstB-xw06. The plasmid pAstB-xw06 was then transformed into TY02 to generate strain TY03.

**TY04.** Plasmid pXW06 (*LEU2* marker) digested with *Nde*I and *Pme*I was used to introduce the *astC* gene (2). A 1.6 kb fragment containing *astC* obtained from PCR using primers AstC-xw02-recomb-F and AstC-xw02-recomb-R were cloned into pXW02 using yeast homologous recombination to afford pAstC-xw02. The plasmid pAstC-xw02 was then transformed into TY03 to generate strain TY04.

**UB01.** *URA3* gene was inserted into *ilv3* locus of *Saccharomyces cerevisiae* DHY *ΔURA3* strain to generate UB01. A 879 bp homologous recombination donor fragment with 35-40 bp homologous regions flanking *ilv3* ORF was amplified using primers ILV3p-URA3-F and IL V3t-URA3-R using yeast gDNA as template. The PCR product was gel purified and transformed into *Saccharomyces cerevisiae* DHY *ΔURA3,* and selected on uracil dropout media to give UB01. The resulting strain was subjected to verification by colony PCR with primers IL V3KO-ck-F and ILV3KO-ck-R and the amplified fragment was sequence confirmed.

**UB02.** The URA3 gene inserted into *ilv3* locus of *Saccharomyces cerevisiae* DHY ΔURA3 was deleted from UB01 using homologous recombination to generate UB02. A 150 bp homologous recombination donor fragment with 75 bp homologous regions flanking *ilv3* ORF was amplified using primers ILV3KO-F and ILV3KO-R, gel purified and transformed into UB01, and counterselected on 5-fluoroorotic acid (5-FoA) containing media to give UB02. The resulting strain was subjected to verification by colony PCR with primers ILV3KO-ck-F and ILV3KO-ck-R and the amplified fragment was sequenced confirmed.

**TY05.** The empty plasmid pXP318 (*URA3* marker) was transformed into UB02 to generate TY05 (*4*).

**TY06.** Plasmid pXP318 digested with *Spe*I and *Xho*I was used as vector to introduce gene encoding fDHAD (4). The cDNA of *Aspergillus terreus* NIH 2624 served as template for PCR amplification. A 1.7 kb fragment obtained using primers fDHAD-pXP318-F and fDHAD-pXP318-R were cloned into pXP318 using yeast homologous recombination to afford fDHAD-pXP318. Then, fDHAD-pXP318 was transformed into UB02 to generate TY06. fDHAD was driven by a constitutive promoter *TEF1.*

**TY07.** Plasmid pXP318 digested with *Spe*I and *Xho*I was used as vector to introduce the *astD* gene (*4*). The cDNA isolated from TY01 served as the template for PCR amplification. A 1.8 kb fragment obtained using primers AstD-pXP318-F and AstD-pXP318-R was cloned into pXP318 using yeast homologous recombination to give AstD-pXP318. A FLAG-tag was also added to the *N*-terminal of AstD. Then, AstD-pXP318 was transformed into UB02 to generate TY07. AstD was driven by a constitutive promoter *TEF1.*

*Fermentation, compound isolation and analyses*

*Fermentation of S. cerevisiae strain.* A seed culture of *S. cerevisiae* strain was grown in 40 mL of synthetic dropout medium for 2 days at 28°C, 250 rpm. Fermentation of the yeast was carried out using YPD (yeast extract 10 g/L, peptone 20 g/L) supplement with 2% dextrose for 3 days at 28°C, 250 rpm.

HPLC-MS analyses were performed using a Shimadzu 2020 EVLC-MS (Phenomenex® Luna, 5μ, 2.0 × 100 mm, C-18 column) using positive and negative mode electrospray ionization. The elution method was a linear gradient of 5-95% (v/v) acetonitrile/water in 15 min, followed by 95% (v/v) acetonitrile/water for 3 min with a flow rate of 0.3 mL/min. The HPLC buffers were supplemented with 0.05% formic acid (v/v). HPLC purifications were performed using a Shimadzu Prominence HPLC (Phenomenex® Kinetex, 5μ, 10.0 × 250 mm, C-18 column). The elution method was a linear gradient of 65-100% (v/v) acetonitrile/water in 25 min, with a flow rate of 2.5 mL/min. GC-MS analyses were performed using Agilent Technologies GC-MS 6890/5973 equipped with a DB-FFAP column. An inlet temperature of 240°C and constant pressure of 4.2 psi were used. The oven temperature was initially at 60°C and then ramped at 10°C/min for 20 min, followed by a hold at 240°C for 5 min.

*Isolation of compound **1.*** The fermentation broth of TY02 was centrifuged (5000 rpm, 10 mins), and cell pellet was harvested and soaked in acetone. The organic phase was dried over sodium sulfate, concentrated to oil form, and subjected to silica column purification with hexane. Compound **1,** colorless oil readily dissolved in hexane and chloroform, had a molecular formula $C_{15}H_{24}$, as deduced from EI-MS $[M]^+$ *m/z* 204, and showed $[\alpha]_D^{22} = -30°$ (*n*-hexane; *c* = 0.1). GC-MS 70 eV, *m/z* (relative intensity): 204 $[M]^+$ (42), 189 (5), 161 (35), 136 (100), 133 (10), 121 (70), 119 (25), 107 (20), 105

(27), 93 (21), 91 (26), 79 (13), 77 (15), 69 (20), 55 (12), 43 (12), 41 (13), 38 (21); $^1$H NMR (500 MHz, $CDCl_3$): $\delta$ 5.37 (1H, m), 2.20-2.10 (5H, m), 2.10-2.00 (2H, m), 1.95 (1H, d, 15.3), 1.75 (3H, s), 1.71 (3H, q, 1.7), 1.61 (3H, brs), 1.44 (1H, dd, 11.4, 7.2), 1.36 (1H, m), 1.31 (1H, dd, 11.3, 2.6), 0.73 (3H, s); $^{13}$C NMR (125 MHz, $CDCl_3$): $\delta$ 138.4, 138.3, 122.4, 122.2, 57.4, 42.6, 41.4, 40.3, 34.5, 29.6, 27.3, 25.0, 23.3, 20.6, 19.2. Both of the NMR and MS spectrums are identical to a known compound (+)-daucane, however, the optical rotation is opposite which led to the assignment of **1** to be (-)-daucane (*5*).

*Isolation of compound* ***2.*** The fermentation broth of TY03 was centrifuged (5000 rpm, 10 mins), and supernatant was extracted three times with ethyl acetate. The organic phase was dried over sodium sulfate, concentrated to oil form, and then and subjected to HPLC purification. Compound **2,** colorless oil readily dissolved in ethyl acetate and chloroform, had a molecular formula $C_{15}H_{22}O_3$, as deduced from LC-MS $[M+H]^+$ *m/z* 251, $[M-H]^-$ *m/z* 249. $^1$H NMR (500 MHz, $CDCl_3$): $\delta$ 8.09 (1H, brs), 3.25 (1H, t, 7.4), 2.71 (1H, dd, 14.6, 6.5), 2.48 (1H, dd, 14.8, 6.3), 2.36 (1H, dd, 14.0, 6.6), 2.26 (1H, m), 2.15 (1H, dd, 16.3, 8.9), 2.08 (1H, d, 12.0), 1.84 (1H, q, 13.1), 1.73 (3H, d, 2.3), 1.59 (3H, d, 2.2), 1.48~1.35 (3H, m), 1.31 (1H, td, 11.5, 9.0), 0.86 (3H, s). $^{13}$C NMR (125 MHz, $CDCl_3$): $\delta$ 176.0, 135.8, 123.2, 60.1, 59.8, 59.4, 44.1, 40.5, 38.8, 30.6, 29.3, 24.9, 23.8, 20.6, 17.8.

*Isolation of aspterric acid (**AA**).* The fermentation broth of TY04 was centrifuged (5000 rpm, 10 mins), and supernatant was extracted three times with ethyl acetate. The organic phase was dried over sodium sulfate, concentrated to oil form, and subjected to HPLC purification. **AA** (compound **3**) is a colorless oil readily dissolved in acetone and chloroform, had a molecular formula $C_{15}H_{22}O_4$, as deduced from LC-MS $[M+H]^+$ *m/z* 267, $[M-H]^-$*m/z* 265. $^1$H NMR (500 MHz, $CDCl_3$): $\delta$ 4.29 (1H, d, 8.5), 3.92 (1H, d, 8.3), 3.48 (1H, d, 8.3), 2.42 (1H, dd, 14.9, 7.3), 2.37~2.28 (2H, m), 2.25 (1H, dd, 13.0, 4.4), 2.20~2.17 (1H, m), 2.12 (1H, d, 13.4), 2.01 (1H, m), 1.80~1.65 (2H, m), 1.71 (3H, s), 1.64~1.54 (1H,m), 1.60 (3H, s), 1.50 (1H, m); $^{13}$C NMR (125 MHz, $CDCl_3$): $\delta$ 178.2, 134.5, 125.2, 82.9, 76.3, 75.6, 55.4, 53.0, 36.6, 36.2, 33.8, 32.2, 23.6, 23.4, 20.9. Compound **3** is identical to aspterric acid (**AA**) as reported (Yoshisuke et al., 1978; Shimada et al., 2002).

*Protein expression, purification and biochemical assay*

*A. thaliana DHAD (pDHAD) expression and purification.* Primers pDHAD-pET-F and pDHAD-pET-R were used to amplify a 1.7 kb DNA fragment containing *A. thaliana dhad* (AT3G23940). The PCR product was cloned into pET28a using *Nhe*I and *Not*I restriction sites. The resulting plasmid pDHAD-pET was transformed into *E.coli* BL21 (DE3) to give TY08. pDHAD fused a 6×His-tag with a molecular weight of ~63 kD was expressed at 16°C 220 rpm for 20 h after 100 μM IPTG induction (IPTG was added when $OD_{600}$ = 0.8). Cells of 1 L culture were then harvested by centrifugation at 5000 rpm at 4°C. Cell pellet was resuspended in 15 mL Buffer A10 (20 mM Tris-HCl pH 7.5, 50 mM NaCl, 8% glycerol, 10 mM imidazole). The cells were lysed by sonication, and the insoluble material was sedimented by centrifugation at 16000 rpm at 4°C. The protein supernatant was then incubated with 3 mL Ni-NTA for 4 hours with slow, constant rotation at 4°C. Subsequently the Ni-NTA resin was washed with 10 column volumes of Buffer A50 (Buffer A + 50 mM imidazole). For elution of the target protein, the Ni-NTA resin was incubated for 10 min with 6 mL Buffer A300 (Buffer A + 300 mM imidazole). The supernatant from the elution step was then analyzed by SDS-PAGE together with the supernatants from the other purification steps. The elution fraction containing the recombinant protein was buffer exchanged into storage buffer (50 mM Tris-HCl pH 7.2, 50mM NaCl, 10mM $MgCl_2$, 10% glycerol, 5 mM DTT, 5 mM GSH).

*Aspergillus terreus DHAD (fDHAD)(XP_001208445.1) expression and purification.* Primers fDHAD-pET-F and fDHAD-pET-R were used to amplify a 1.6 kb DNA fragment containing *fdhad*. The PCR product was cloned into pET28a using *Nde*I and *Not*I restriction sites. The resulted plasmid fDHAD-pET was transformed into *E.coli* BL21 (DE3) to obtain TY09. fDHAD fused a 6×His tag with a molecular weight of ~62 kD was expressed at 16°C 220 rpm for 20 h under 100μM IPTG induction (IPTG was added when $OD_{600}$ = 0.8). Cells of 1 liter culture were then harvested by centrifugation at 5000 rpm at 4°C. The cell pellet was resuspended in 15 mL Buffer A10 (20 mM Tris-HCl pH 7.5, 50 mM NaCl, 8% glycerol, 10 mM imidazole). The cells were broken by ultra-sonication, and the insoluble material was sedimented by centrifugation at 16000 rpm at 4°C. The protein supernatant was then incubated with 3 mL Ni-NTA sepharose for 4 hours with slow, constant rotation at 4°C. Subsequently the Ni-NTA sepharose was washed with 10 column volume Buffer A50 (Buffer A + 50mM imidazole). For elution of the target protein, the sepharose was incubated for 10 min with 6 mL Buffer A300 (Buffer A + 300 mM imidazole). The supernatant from the elution step was then analyzed by SDS-PAGE together with the supernatants from the other purification steps. The elution fraction containing the recombinant protein was buffer exchanged into storage buffer (50 mM Tris-HCl pH 7.2, 50mM NaCl, 10mM $MgCl_2$, 10% glycerol, 5 mM DTT, 5 mM GSH).

*AstD (XP_001213593.1) expression and purification.* Primers AstD-pET-F and AstD-pET-R were used to amplify a 1.6 kb DNA fragment containing *astD.* The PCR product was cloned into pET28a using *Nde*I and *Not*I restriction sites. The resulted plasmid AstD-pET was transformed into *E.coli* BL21 (DE3) to obtain TY10. AstD fused to a 6×His-tag with a molecular weight of ~62 kD was expressed at 16°C 220 rpm for 20 h under 100mM IPTG induction (IPTG was added when $OD_{600}$ = 0.8). Cells of 1 liter culture were then harvested by centrifugation at 5000 rpm at 4°C. The cell pellet was resuspended in 15 mL Buffer A10 (20 mM Tris-HCl pH 7.5, 50 mM NaCl, 8% glycerol, 10 mM imidazole). The cells were broken by ultra-sonication, and the insoluble material was sedimented by centrifugation at 16000 rpm at 4°C. The protein supernatant was then incubated with 3 mL Ni-NTA sepharose for 4 hours with slow, constant rotation at 4°C. Subsequently

the Ni-NTA sepharose was washed with 10 column volume Buffer A50 (Buffer A + 50mM imidazole). For elution of the target protein, the sepharose was incubated for 10 min with 6 mL Buffer A300 (Buffer A + 300 mM imidazole). The supernatant from the elution step was then analyzed by SDS-PAGE together with the supernatants from the other purification steps. The elution fraction containing the recombinant protein was buffer exchanged into storage buffer (50 mM Tris-HCl pH 7.2, 50mM NaCl, 10mM $MgCl_2$, 10% glycerol, 5 mM DTT, 5 mM GSH).

*Biochemical assay of DHADs.* In vitro activity assays were carried out in 50 μL reaction mixture containing storage buffer, 10 mM (±)-sodium α,β-dihydroxyisovalerate hydrate (**4**) and 0.5 μM of purified DHAD enzyme. The reaction was initiated by adding the enzyme. After 0.5 h incubation at 30°C, the reactions were stopped by adding equal volume of ethanol. Approximately 0.1 volume of 100 mM phenylhydrozine (PHH) was added to derivatize the product 3-methyl-2-oxo-butanoic acid (**5**) into **6** at room temperature for 30 min. 20 μL of the reaction mixture was subject to LC-MS analysis. The area of the HPLC peak with UV absorption at 350 nm was used to quantify the amount of **6.** (**FIG. 26A-26B**).

*Growth inhibition assay*

*Growth inhibition assay of S. cerevisiae on plates or in the tubes. S. cerevisiae* was grown in isoleucine, leucine and valine (ILV) dropout media (20 g/L glucose, 0.67 g/L Difco™ Yeast Nitrogen Base w/o amino acids, 18 mg/L adenine, arginine 76 mg/L, asparagine 76 mg/L, aspartic acid 76 mg/L, glutamic acid 76 mg/L, histidine 76 mg/L, lysine 76 mg/L, methionine 76 mg/L, phenylalanine 76 mg/L, serine 76 mg/L, threonine 76 mg/L, tryptophan 76 mg/L, tyrosine 76 mg/L) to test growth inhibition of **AA** on *S. cerevisiae. S. cerevisiae* was incubated at 28°C until $OD_{600}$ of the control strain without **AA** treatment reached about 0.8. The ratio of yeast $OD_{600}$ in media with **AA** treatment to yeast $OD_{600}$ in media without **AA** was calculated as the percentage of growth inhibition. The inhibition curve was plotted as percentage of inhibition versus **AA** concentrations. To further prove **AA** affects BCAA biosynthesis, isoleucine, leucine and valine was also complemented to the media with or without treatment of **AA.** The growth curves of TY05, TY06 and TY07 were also plotted (**FIG. 29A-29D**). The $OD_{600}$ was recorded for every 20 minutes over a total of 50 h. The inhibition percentage can be calculated by following equation:

$$\text{inhibition percentage} = 1 - \frac{\text{initial reaction rate with } \mathbf{AA}}{\text{initial reaction rate without } \mathbf{AA}}$$

*Growth inhibition assay of plants on plates or in the tubes.* MS (2.16 g/L Murashige and Skoog basal medium, 8 g/L sucrose, 8 g/L agar) media was used to test the growth inhibition of **AA** on *A. thaliana, Solanum lycopersicum,* and *Zea mays. A. thaliana, S. lycopersicum, G. max* and *Z. mays* were grown under long day condition (16/8 h light/dark) using cool-white fluorescence bulbs as the light resource at 23°C. **AA** was dissolved in ethanol and added to the media before inoculating strains or growing plants. The media of control treatment contains the same amount of ethanol, but without **AA.**

*Plant growth inhibition assay by spraying.* **AA** was firstly dissolved in ethanol and then added to solvent (0.06 g/L Finale® Bayer Inc. + 20 g/L EtOH). The control plants were treated with solvent containing ethanol only. *A. thaliana* that are resistant to glufosinate (containing the *bar* gene) were grown under long day conditions (16/8 h light/dark) using cool-white fluorescence bulbs as the light resource at 23°C. Spraying treatments began when the seeds germinated, and was repeated once every two days with approximately 0.4 mL AA solution per time per pot.

*Structure determination of apo-pDHAD*

The gene encoding pDHAD (residues 35-608) was cloned into pET21a derivative vector pSJ2 with an eight histidine (8×His) tag and a TEV protease cleavage site at the *N*-terminus. The following primers were used for cloning: the forward primer DHAD-F and the reverse primer DHAD-R. The double mutant K559A/K560A for efficient crystallization was designed using the surface entropy reduction prediction (SERp) server (*6*). Mutations were generated by PCR using the forward primer K559AK560A-F and reverse primer K559AK560A-R. All constructed plasmids were verified by DNA sequencing.

pDHAD purified under aerobic conditions was found to contain no iron-sulfur cluster (*apo* form). Hence, [2Fe-2S] Cluster reconstitution was performed under the atmosphere of nitrogen in an anaerobic box. The protein was incubated with $FeCl_3$ at the ratio of 1:10 for 1 h on ice and then 10 equivalents of $Na_2S$ per protein was added drop-wise every 30 min for 3 h. The reaction mixture was then incubated overnight. Excess $FeCl_3$ and $Na_2S$ were removed using a SephadexTM G-25 Fine column (GE Healthcare)(Rahman et al, 2017).

The reconstituted *holo*-pDHAD was crystallized in an anaerobic box. The proteins (at 10 mg/mL) were mixed in a 1:1 ratio with the reservoir solution in a 50 μL volume of 2 μL and equilibrated against the reservoir solution, using the sitting-drop vapor diffusion method at 16°C. Crystals for diffraction were observed in 0.1 M sodium acetate pH 5.0, 1.5 M ammonium sulfate after 5 d.

All crystals were flash-cooled in liquid nitrogen after cryo-protected with solution containing 25% glycerol, 1.5 M ammonium sulfate, 0.1 M sodium acetate pH 5.0. The data were collected at the Beam Line 19U1 in Shanghai Synchrotron Radiation Facility (SSRF). Diffraction data of *holo*-pDHAD was collected at the wavelength of 0.97774 Å. The best crystals diffracted to a resolution of 2.11 Å. All data sets were indexed, integrated, and scaled using the HKL3000 package (Otwinowski et al., 1997). The crystals belonged to space group $P4_22_12$. The statistics of the data collection are summarized in **Table 9D.**

The *holo*-pDHAD structure was solved by the molecular replacement method Phaser embedded in the CCP4i suite and the L-arabinonate dehydratase crystal structure (PDB_ID: 5J83) as the search model. All the side chains were removed during the molecular replacement process (McCoy et al., 2007; Winn et al., 2011). The resulting model was refined against the diffraction data using the REFMAC5 program of CCP4i (Murshudov et al., 2011). Based on the improved electron density, the side chains of *holo*-pDHAD protein, iron sulfur cluster, water molecule, acetate ion, sulfate ions, and magnesium ion were manually built using the program WinCoot (Emsley et al., 2010). The $R_{work}$ and $R_{free}$ values of the structure are 17.67% and 22.15%, respectively. The detailed refinement statistics are summarized in **Table 9D.** The geometry of the model was validated by WinCoot. Structural factor and coordinate of *holo*-pDHAD have been deposited in the Protein Bank (PDB code: 5ZE4).

**Table 9D: X-ray data collection and refinement statistics**

| Name | pDHAD |
|---|---|
| PDB ID | 5ZE4 |
| **Data collection** | |
| Beamline | SSRF-BL19U1 |
| Wavelength (Å) | 0.97774 |
| Space group | $P4_22_12$ |
| Unit cell parameters (Å) | a = 135.5 |
| | b = 135.5 |
| | c = 66.0 |
| No of measured reflecions | 50-2.11 (2.15-2.11) |
| Resolution (Å)[a] | 907124 |
| No of unique reflections[a] | 36139 |
| Redundancy[a] | 25.1 (23.1) |
| Completeness (%)[a] | 100 (100.0) |
| Average (I/σ)[a] | 17.86 (2.33) |
| $R_{merge}$ (%)[a,b] | 0.189 (1.240) |
| **Refinement** | |
| Resolution (Å)[a] | 95.79-2.11 |
| No of reflections (work/free) | 33235 (1714) |
| $R_{work}/R_{free}$[c] | 0.1767/0.2216 |
| No of non-H atom | 4366 |
| protein | 4208 |
| waters | 142 |
| Average B factor [$A^2$] | 28.39 |
| Bond lengths (Å) | 0.007 |
| Bond angles (°) | 1.195 |
| Ramachandran plot favored (%) | 98.05 |
| Ramachandran plot allowed (%) | 1.60 |
| Ramachandran plot outlier (%) | 0.36 |

[a]Numbers in parentheses are values for the highest-resolution shell.
[b]$R_{merge} = \Sigma_{hkl}\Sigma_i|I_i - \langle I\rangle|/\Sigma_{hkl}\Sigma_i|\langle I\rangle|$, where $I_i$ is the intensity for the ith measurement of an equivalent reflection with indices h, k, and l.
[c]$R_{free}$ was calculated with the 5% of reflections set aside randomly throughout the refinement.

*Homology modelling of AstD and docking of substrate or AA into active site of holo-pDHAD*

The structure of *holo*-pDHAD was prepared in Schrodinger suite software under OPLS3 force field (Harder et al., 2016). Hydrogen atoms were added to reconstituted crystal structures according to the physiological pH (7.0) with the PROPKA tool in Protein Preparation tool in Maestro to optimize the hydrogen bond network (Rahman et al., 2017; Søndergaard et al., 2011). Constrained energy minimizations were conducted on the full-atomic models, with heavy atom coverage to 0.5 Å. The homology model was performed in Modeller 9.18 (Eswar et al., 2006), using the crystal structure of *holo*-pDHAD solved in this work as a template. Sequence alignment in Modeller indicated that AstD and pDHAD shared 56.8% sequence identity and 75.0% sequence similarity (**FIG. 36**). All the highly conserved residues and motifs were properly aligned. A total of 2000 models were generated for each target in Modeller with the fully annealed protocol. The optimal models were chosen for docking studies according to DOPE (Discrete Optimized Protein Energy) score.

All ligand structures were built in Schrodinger Maestro software (Rahman et al., 2017). The LigPrep module in Schrodinger software was introduced for geometric optimization by using OPLS3 force field (Harder et al., 2016). The ionization states of ligands were calculated with Epik tool employing Hammett and Taft methods in conjunction with ionization and tautomerization tools (Greenwood et al., 2010). The docking of a ligand to the receptor was performed using Glide (Friesner et al., 2004). Cofactors observed in crystal structure during the docking were included. Since both water and $SO_4^{2-}$ occupied the catalytic site, they were excluded prior to docking. Cubic boxes centered on the ligand mass center with a radius 8 Å for all ligands defined the docking binding regions. Flexible ligand docking was executed for all structures. Ten poses per ligand out of 20,000 were included in the post-docking energy minimization. The best scored pose for the ligand was chosen as the initial structure for further study. The MM/GBSA method was introduced to evaluate the ligand binding affinity based on the best scored docking pose in Schrodinger software. Figures are prepared in PyMOL and Inkscape (Yuan et al., 2016; Yuan et al., 2017). Both of native substrate α,β-dihydroxyisovalerate and **AA** were docked into the catalytic site of pDHAD. The cross-section electrostatic surface map shows this unique catalytic pocket has a positively charged internal and a hydrophobic entrance, which binds to negatively charged "head" and hydrophobic "tail" of substrate or **AA** respectively. Thus the negatively charged "head" can lead both of the substrate and **AA** into the catalytic chamber. The bulky hydrophobic tricyclic moiety of **AA,** however, provides stronger hydrophobic interactions to the entrance and blocks the entrance of active site due to the hydrophobic residues at the entrance, including G68, A71, I72, I134, A133, M141, V212, F215, M498 and P501. In contrast, the smaller "tail" of native substrate provides less interactions to entrance because the smaller size limits efficient hydrophobic contact to nearby residues. This implies that once **AA** binds to pDHAD, it can prevent substrate approaching the active site. Molecular mechanics generalized Born and surface area (MM/GBSA) continuum solvation method was also introduced, which is a widely used approach for relative binding energy calculation, to evaluate the relative binding affinity for both ligands (Genheden et al., 2015). The MM/GBSA calculations had been done in Prime (Sirin et al., 2014) (Schrödinger 2015 suite). The MM/GBSA energy was calculated using following equation:

$$\Delta G_{bind} = E_{complex} - E_{protein} - E_{ligand}$$

E denotes energy and includes terms such as protein-ligand van der Waals contacts, electrostatic interactions, ligand desolvation, and internal strain (ligand and protein) energies, using VSGB2.0 implicit solvent model with the OPLS2005 force field. The solvent entropy is also included in the VSGB2.0 energy model, as it is for other Generalized Born (GB) and Poison-Boltzmann (PB) continuum solvent models.

MM/GBSA calculation shows that the relative binding energy for **AA** and α,β-dihydroxyisovalerate is -18.6 ± 0.3 kcal/mol and -13.3 ± 0.2 kcal/mol respectively, which shows the binding constant of **AA** to active site is about 6000 times greater than α,β-dihydroxyisovalerate. This further confirms that **AA** is a competitive inhibitor of pDHAD.

*Cytotoxicity assay of **AA***

Cell proliferation experiments were performed in a 96-well format (five replicates per sample) using melanoma cell line A375 and SK-MEL-1. **AA** treatments were initiated 24 h postseeding for 72 h, and cell survival was quantified using CellTiter-GLO assay (Promega).

*Cross experiment of Arabidopsis thaliana*

To make male sterile *A. thaliana,* **AA** was added to chemical hybridization agent (CHA) formulation (250 μM **AA,** 2% ethanol, 0.1% Tween-80, 1% corn oil in water), which has less inhibition effect on the growth of *A. thaliana.* Flowers of the **AA** treated Col-0 were selected as the female parent. The non-treated *A. thaliana* containing a glufosinate resistant gene was used as male parent to donate pollen. 2-week old F1 progeny resulting from the cross were treated by Finale

(11.3% glufosinate-ammonium) at 1:2000 dilution. The results are summarized in **Table 9H**.

*Construction of the transgenic plants*

The coding sequence of AstD was codon optimized for *A. thaliana.* A chloroplast localization signal (CLS) of 35-amino acid residues derived from the *N*-terminal of*A. thaliana* DHAD (SEQ ID NO: 19) was fused to N-terminus of the codon optimized AstD. A 3×FLAG-tag was inserted between the CLS and the codon optimized AstD. The gene block containing CLS, FLAG-tag and AstD was synthesized and then cloned into pEG202 vector using Gateway LR Clonase II Enzyme Mix (ThermoFisher scientific). The original CaMV 35S promoter of pEG202 was substituted by Ubiquitin-10 promoter to drive the expression of AstD. The construct was electro-transformed into *Agrobacterium tumefaciens* strain Agl0 followed by *A. thaliana* transformation using the standard floral dip method (16). The *A. thaliana* Col-0 ecotype was transformed. Positive transgenic plants were selected through the glufosinate resistance marker, and were tested for survival in presence of **AA.**

The codon-optimized nucleotide sequence of astD for expression in *A. thaliana,* including the chloroplast localization signal and FLAG-tag, is shown in SEQ ID NO: 17. The nucleotide sequence of the chloroplast localization signal is shown in SEQ ID NO: 18. The nucleotide sequence of the FLAG tag is shown in SEQ ID NO: 20. The codon-optimized nucleotide sequence of astD is shown in SEQ ID NO: 21.

*Protein expression verification with western blot*

Approximately 0.5 gram of leaf tissue of transgenic *A. thaliana* was ground in liquid nitrogen. Proteins were homogenized in 2× SDS buffer followed by 5-minutes of centrifugation at 21,000 g to remove undissolved debris. The supernatant containing resolved proteins was loaded onto a 4-12% Bis-Tris gel, and separated using MOPS running buffer. Transfer was conducted using iBlot2 dry transfer device and PVDF membrane. The total proteins were stained with Ponceau to demonstrate equal loading. Western blotting was performed using Sigma monoclonal anti-FLAG M2-Peroxidase antibody, followed by detection using Amersham ECL Prime detection reagent.

*Additional NMR Information*

Additional information regarding the NMR analyses described herein is found in **Table 9E** and **Table 9F.**

**Table 9E: NMR data and structure: $^{1}H$ (500 MHz, $CDCl_3$) and $^{13}C$ NMR (125 MHz, $CDCl_3$) of compound below:**

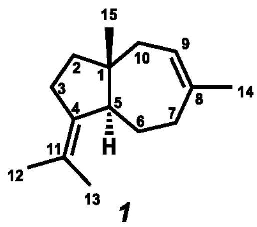

***1***

| no. | $\delta_H$ (mult., *J* in Hz) | $\delta_C$ | mult. | HMBC |
|---|---|---|---|---|
| 1 | - | 42.6 | C | - |
| 2 | 1.44 (1H, dd, 11.4, 7.2) | 40.3 | $CH_2$ | 138.4, 57.4, 42.6, 29.6, 19.2 |
| 2' | 1.31 (1H, dd, 11.3, 2.6) | | | 42.6, 41.4, 29.6, 19.2 |
| 3 | 2.20 (1H, m) | 29.6 | $CH_2$ | 138.4, 42.6, 34.5 |
| 3' | 2.15 (1H, m) | | | 138.4, 122.2, 57.4, 42.6, 40.3 |
| 4 | - | 138.4 | C | - |
| 5 | 2.16 (1H, m) | 57.4 | CH | 138.4, 42.6, 40.3, 34.5, 25.0 |
| 6 | 2.19 (1H, m) | 25.0 | $CH_2$ | 138.4,138.3, 57.4, 42.6, 34.5 |
| 6' | 1.36 (1H, m) | | | 34.5 |
| 7 | 2.15 (1H, m) | 34.5 | $CH_2$ | 138.3, 122.4, 57.4 |
| 7' | 2.07 (1H, m) | | | 138.3, 122.4, 57.4, 27.3, 25.0 |
| 8 | - | 138.3 | C | - |

(continued)

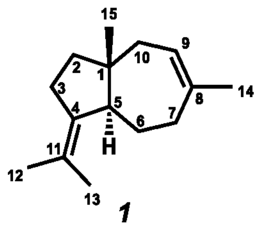

*1*

| no. | $\delta_H$ (mult., *J* in Hz) | $\delta_C$ | mult. | HMBC |
|---|---|---|---|---|
| 9 | 5.37 (1H, m) | 122.4 | CH | |
| 10 | 2.00 (1H, m) | 41.4 | $CH_2$ | 138.3, 122.4, 57.4, 42.6, 40.3, 19.2 |
| 10' | 1.95 (1H, d, 15.3) | | | 138.3, 122.4, 57.4, 42.6 |
| 11 | - | 122.2 | C | - |
| 12 | 1.61 (3H, brs) | 23.3 | $CH_3$ | 138.4, 122.2, 20.6 |
| 13 | 1.71 (3H, q, 1.7) | 20.6 | $CH_3$ | 138.4, 122.2, 23.3 |
| 14 | 1.75 (3H, s) | 27.3 | $CH_3$ | 138.3, 122.4, 34.5 |
| 15 | 0.73 (3H, s) | 19.2 | $CH_3$ | 57.4, 42.6, 41.4, 40.3 |

**Table 9F: NMR data and structure: $^1$H (500 MHz, $CDCl_3$) and $^{13}$C NMR (125 MHz, $CDCl_3$) of compound below:**

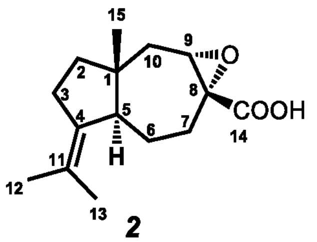

*2*

| no. | $\delta_H$ (mult., *J* in Hz) | $\delta_C$ | mult. | HMBC |
|---|---|---|---|---|
| 1 | - | 44.1 | C | - |
| 2 | 1.41 (1H, m) | 38.8 | $CH_2$ | 135.8, 60.1, 44.1, 29.3, 17.8 |
| 2' | 1.31 (1H, td, 11.5, 9.0) | | | 44.1, 40.5, 29.3, 17.8 |
| 3 | 2.26 (1H, m) | 29.3 | $CH_2$ | 135.8 |
| 3' | 2.15 (1H, dd, 16.3, 8.9) | | | 135.8, 123.2, 60.1, 44.1, 38.8 |
| 4 | - | 135.8 | C | - |
| 5 | 2.08 (1H, d, 12.0) | 60.1 | CH | |
| 6 | 2.48 (1H, dd, 14.8, 6.3) | 24.9 | $CH_2$ | 59.4, 44.1, 30.6 |
| 6' | 1.84 (1H, q, 13.1) | | | 59.4, 30.6 |
| 7 | 2.71 (1H, dd, 14.6, 6.5) | 30.6 | $CH_2$ | 176.0, 60.1, 59.8, 59.4, 24.9 |
| 7' | 1.39 (1H, m) | | | 176.0, 60.1, 59.8, 59.4, 24.9 |
| 8 | - | 59.4 | C | - |
| 9 | 3.25 (1H, t, 7.4) | 59.8 | CH | 176.0, 59.4, 40.5 |
| 10 | 2.36 (1H, dd, 14.0, 6.6) | 40.5 | $CH_2$ | 60.1, 59.8, 59.4, 44.1, 38.8 |
| 10' | 1.44 (1H, m) | | | 60.1, 59.8, 59.4, 44.1, 17.8 |
| 11 | - | 123.2 | C | - |
| 12 | 1.59 (3H, d, 2.2) | 23.8 | $CH_3$ | 135.8, 123.2, 20.6 |

(continued)

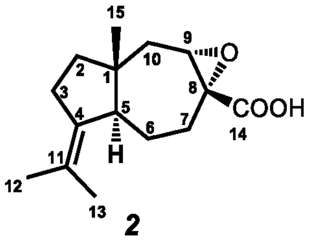

2

| no. | $\delta_H$ (mult., $J$ in Hz) | $\delta_C$ | mult. | HMBC |
|---|---|---|---|---|
| 13 | 1.73 (3H, d, 2.3) | 20.6 | $CH_3$ | 135.8, 123.2, 23.8 |
| 14 | - | 176.0 | C | - |
| 15 | 0.86 (3H, s) | 17.8 | $CH_3$ | 59.8, 44.1, 40.5, 38.8 |
| 14-COOH | 8.09 (1H, brs) | - | COOH | |

**Table 9I: NMR data and structure: $^1H$ (500 MHz, $CDCl_3$) and $^{13}C$ NMR (125 MHz, $CDCl_3$) of compound 3 (AA):**

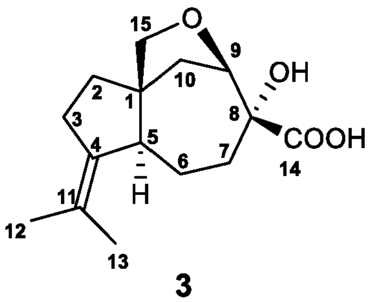

3

| no. | $\delta_H$ (mult., $J$ in Hz) | $\delta_C$ | mult. | HMBC |
|---|---|---|---|---|
| 1 | - | 53.0 | C | - |
| 2 | 1.73 (1H, m) | 33.8 | $CH_2$ | 134.5, 76.3, 53.0, 23.6 |
| 2' | 1.50 (1H, m) | | | 134.5, 76.3, 55.4, 53.0, 23.6 |
| 3 | 2.42 (1H, dd, 14.9, 7.3) | 23.6 | $CH_2$ | 76.3, 55.4, 53.0, 33.8 |
| 3' | 1.61 (1H, m) | | | 134.5, 55.4, 53.0, 33.8 |
| 4 | - | 134.5 | C | - |
| 5 | 2.34 (1H, m) | 55.4 | CH | 134.5, 125.2, 76.3, 53.0, 33.8, 23.6 |
| 6 | 2.20 (1H, m) | 36.6 | $CH_2$ | 75.6, 55.4, 53.0 |
| 6' | 1.70 (1H, m) | | | 75.6, 53.0 |
| 7 | 2.32 (1H, m) | 32.2 | $CH_2$ | 178.2, 82.9, 75.6, 55.4 |
| 7' | 2.01 (1H, m) | | | 75.6, 55.4 |
| 8 | - | 75.6 | C | - |
| 9 | 4.29 (1H, d, 8.5) | 82.9 | CH | 76.3, 75.6, 53.0, 36.2 |
| 10 | 2.26 (1H, m) | 36.2 | $CH_2$ | 82.9, 76.3, 75.6, 55.4 |
| 10' | 2.12 (1H, d, 13.4) | | | 76.3, 75.6, 55.4, 53.0 |
| 11 | - | 125.2 | C | - |
| 12 | 1.71 (3H, s) | 20.9 | $CH_3$ | 134.5, 125.2, 23.4 |
| 13 | 1.60 (3H, s) | 23.4 | $CH_3$ | 134.5, 125.2, 20.9 |
| 14 | - | 178.2 | C | - |

(continued)

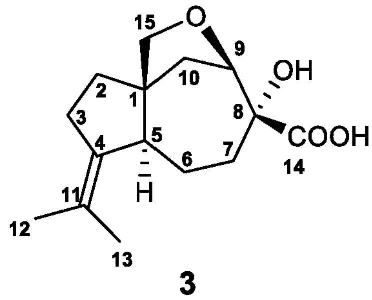

**3**

| no. | $\delta_H$ (mult., *J* in Hz) | $\delta_C$ | mult. | HMBC |
|---|---|---|---|---|
| 15 | 3.92 (1H, d, 8.3) | 76.3 | $CH_2$ | 82.9, 55.4, 53.0, 36.2 |
| 15' | 3.48 (1H, d, 8.3) | | | 55.4, 53.0, 33.8 |

*References for Materials and Methods*

1. N. Liu et al., Identification and Heterologous Production of a Benzoyl-Primed Tricarboxylic Acid Polyketide Intermediate from the Zaragozic Acid A Biosynthetic Pathway. Org. Lett. 19, 3560-3563 (2017).

2. W. Xu, X. Cai, M. E. Jung, Y. Tang, Analysis of Intact and Dissected Fungal Polyketide Synthase-Nonribosomal Peptide Synthetase in Vitro and in Saccharomyces cerevisiae. J. Am. Chem. Soc. 132, 13604-13607 (2010).

3. M.-C. Tang et al., Discovery of unclustered fungal indole diterpene biosynthetic pathways through combinatorial pathway reassembly in engineered yeast. J. Am. Chem. Soc. 137, 13724-13727 (2015).

4. F. Fang et al., A vector set for systematic metabolic engineering in Saccharomyces cerevisiae. Yeast 28, 123-136 (2011).

5. L. G. Cool, ent-Daucane and acorane sesquiterpenes from Cupressocyparis leylandii foliage. Phytochemistry 58, 969-972 (2001).

6. L. Goldschmidt, D. R. Cooper, Z. S. Derewenda, D. Eisenberg, Toward rational protein crystallization: a web server for the design of crystallizable protein variants. Protein Sci. 16, 1569-1576 (2007).

7. N. Eswar et al., in Curr. Protoc. Protein Sci. (John Wiley & Sons, Inc., 2007).

8. E. Harder et al., OPLS3: a force field providing broad coverage of drug-like small molecules and proteins. J. Chem. Theory Comput. 12, 281-296 (2016).

9. C. R. Søndergaard, M. H. M. Olsson, M. Rostkowski, J. H. Jensen, Improved treatment of ligands and coupling effects in empirical calculation and rationalization of pKa values. J. Chem. Theory Comput. 7, 2284-2295 (2011).

10. L. Schrodinger. (LLC, New York, NY, 2017).

11. J. R. Greenwood, D. Calkins, A. P. Sullivan, J. C. Shelley, Towards the comprehensive, rapid, and accurate prediction of the favorable tautomeric states of drug-like molecules in aqueous solution. J. Comput. Aided Mol. Des. 24, 591-604 (2010).

12. R. A. Friesner et al., Glide: a new approach for rapid, accurate docking and scoring. 1. method and assessment of docking Accuracy. J. Med. Chem. 47, 1739-1749 (2004).

13. S. Yuan, H. C. S. Chan, S. Filipek, H. Vogel, PyMOL and Inkscape bridge the data and the data visualization. Structure 24, 2041-2042 (2016).

14. S. Yuan, H. C. S. Chan, Z. Hu, Using PyMOL as a platform for computational drug design. Wiley Interdiscip. Rev. Comput. Mol. Sci. 7, e1298-n/a (2017).

15. S. Genheden, U. Ryde, The MM/PBSA and MM/GBSA methods to estimate ligand-binding affinities. Expert Opin. Drug Discov. 10, 449-461 (2015).

16. S. J. Clough, A. F. Bent, Floral dip: a simplified method forAgrobacterium-mediated transformation of Arabidopsis thaliana. Plant J. 16, 735-743 (1998).

17. J. Kennedy et al., Modulation of Polyketide Synthase Activity by Accessory Proteins During Lovastatin Biosynthesis. Science 284, 1368-1372 (1999).

18. T. B. Regueira et al., Molecular basis for mycophenolic acid biosynthesis in Penicillium brevicompactum. Appl. Environ. Microbiol. 77, 3035-3043 (2011).

Otwinowski, Z., Minor, W. & W Jr, C. C. Processing of X-ray diffraction data collected in oscillation mode. Methods Enzymol 276, 307-326 (1997).

McCoy, A. J. et al. Phaser crystallographic software. Journal of applied crystallography 40, 658-674 (2007).

Winn, M. D. et al. Overview of the CCP4 suite and current developments. Acta crystallographica. Section D, Biological crystallography 67, 235-242 (2011).

Murshudov, G. N. et al. REFMAC5 for the refinement of macromolecular crystal structures. Acta crystallographica. Section D, Biological crystallography 67, 355-367 (2011).

Emsley, P., Lohkamp, B., Scott, W. G. & Cowtan, K. Features and development of Coot. Acta crystallographica. Section D, Biological crystallography 66, 486-501 (2010).

Eswar, N. et al. Comparative protein structure modeling using Modeller. Current protocols in bioinformatics / editoral board, Andreas D. Baxevanis ... [et al.] Chapter 5, Unit 5 6 (2006).

Sirin, S. et al. A computational approach to enzyme design: predicting ω-aminotransferase catalytic activity using docking and MM-GBSA scoring. J. Chem. Inf. Model. 54, 2334-2346 (2014).

**Results**

To identify possible self-resistance enzymes, sequenced fungal genomes in public databases were scanned to search for colocalizations of genes encoding DHAD with core biosynthetic enzymes, such as terpene cyclases, polyketide synthases, etc (*21, 22*). A well-conserved set of four genes across multiple fungal genomes was identified (**FIG. 21A**), including the common soil fungus *Aspergillus terreus* that is best known to produce the cholesterol lowering drug lovastatin. The conserved gene clusters include genes that encode a sesquiterpene cyclase homolog (*astA*), two cytochrome P450s (*astB* and *astC*), and a homolog of DHAD (*astD*). Genes outside of this cluster are not conserved across the identified genomes and are hence unlikely to be involved. AstD represents the second copy of DHAD encoded in the genome, and is ~70% similar to the housekeeping copy that is well-conserved across all fungi (**FIG. 22A-22B**). Therefore, it was reasoned that AstD is potentially a self-resistance enzyme that confers resistance the encoded NP. Like a majority of biosynthetic gene clusters in sequenced fungal genomes, the *ast* cluster was not associated with the production of a known NP (*16, 17*). The proposed functions of genes within the *ast* cluster of *A. terreus* (**FIG. 21A**) is shown in **Table 9G.**

**Table 9G: Proposed functions of genes within the *ast* cluster in *A. terreus***

| *A. terreus* NIH 2624, scaffold 6 (NT 165929.1, 469,00-486,00), 17 kbp | | | | | |
|---|---|---|---|---|---|
| **Gene** | **Accession number** | **Size (gene/protein)** | **BLASTP homologs** | **Identitiy/similarity (%)** | **Putative function** |
| *astA* | XP_001213594.1 | 1230/409 | XP_001266526.1 | 94/97 | Terpene synthase |

(continued)

| *A. terreus* NIH 2624, scaffold 6 (NT 165929.1, 469,00-486,00), 17 kbp | | | | | |
|---|---|---|---|---|---|
| **Gene** | **Accession number** | **Size (gene/protein)** | **BLASTP homologs** | **Identitiy/similarity (%)** | **Putative function** |
| *astB* | XP_001213595.1 | 1760/512 | XP_001266527.1 | 94/96 | Cytochrome P450 |
| *astC* | XP_001213596.1 | 1716/538 | CEJ61176.1 | 84/89 | Cytochrome P450 |
| *astD* | XP_001213593.1 | 1874/598 | OJJ72940.1 | 98/98 | Dihydroxy-acid dehydratase |

To identify the NP encoded by the *ast* cluster, the *astA, astB,* and *astC* genes were heterologously expressed in the host *Saccharomyces cerevisiae* RC01, which has been engineered to contain a chromosomal copy of the *A. terreus* cytochrome P450 reductase (CPR) that is required for transferring electrons from NADPH to the P450 heme (23). New compounds that emerged were purified and their structures were elucidated with NMR spectroscopy (**FIG. 23A-23L**). RC01 expressing only *astA* produced a new sesquierpene (**1**), which was confirmed to be (-)-daucane (**FIG. 21B**). RC01 expressing both *ast*A and *ast*B led to the biosynthesis of a new product that was structurally determined to be the $\alpha$-epoxy carboxylate (2) (**FIG. 21B**). When *ast*A, *ast*B and *astC* were expressed together, a new compound (3) became the dominant product (~ 20 mg/L). Full structural and absolute stereochemical determination revealed the compound to be the tricylic aspterric acid (**AA**), which is a previously isolated compound (**FIG. 21B**) (*24*). The biosynthetic pathway for **AA** is therefore concise: following cyclization of farnesyl diphosphate by AstA to create the carbon skeleton in **1**, AstB catalyzes the 8 $e^-$ oxidation of **1** to yield the epoxide **2**. Further oxidation by AstC at carbon 15 yields an alcohol, which can undergo intramolecular epoxide opening to create **AA**.

Upon its initial discovery, **AA** was shown to have inhibitory activity towards pollen development in *Arabidopsis thaliana,* however, the mode of action was not known (*25*). The genome mining approach described herein led to rediscovery of this compound with DHAD as a potential target. It was first demonstrated that **AA** is able to potently inhibit *A. thaliana* growth in an agar-based assay (**FIG. 24A**). **AA** was also an effective inhibitor of root development and plant growth when applied to a representative monocot (*Zea mays*) and dicot (*Solanum lycopersicum*) (**FIG. 24B**). To test if **AA** indeed targets DHAD, housekeeping DHAD from both *A. terreus* (XP_001208445.1, fDHAD) and *A. thaliana* (AT3G23940, pDHAD), as well as the putative self-resistance enzyme AstD using *Escherichia coli,* were expressed and purified (**FIG. 25A-25C**). Both housekeeping DHAD enzymes converted dihydroxyisovalerate to ketoisovalerate (pDHAD $k_{cat}$ =1.2 $sec^{-1}$, $K_m$ = 5.7 mM) as expected. The enzyme activities, however, were inhibited in the presence of **AA** (**FIG. 26A-26B**). The $IC_{50}$ values of **AA** towards fDHAD and pDHAD were 0.31 $\mu$M and 0.50 $\mu$M at an enzyme concentration of 0.50 $\mu$M, respectively (**FIG. 27A-27B**). **AA** was further determined to be a competitive inhibitor of pDHAD with a $K_i$ = 0.30 $\mu$M (**FIG. 27C**). In contrast to the potent inhibitory properties towards plant growth, **AA** displayed no significant cytotoxicity towards human cell lines up to 500 $\mu$M concentration, consistent with the lack of DHAD in mammalian cells (**FIG. 28**).

AstD was also shown to catalyze the identical $\beta$-dehydration reaction as DHAD, albeit with a significantly more sluggish turnover rate ($k_{cat}$ =0.03 $sec^{-1}$, $K_m$ = 5.4 mM). However, the enzyme was not inhibited by **AA,** even at the solubility limit of 8 mM (**FIG. 27D**). To determine if AstD can confer resistance to **AA**-sensitive strains, a yeast based assay was developed. The genome copy of DHAD encoded by *ILV3* was first deleted from *Saccharomyces cerevisiae* strain DHY *ΔURA3,* which resulted in an auxotroph that requires exogenous addition of Ile, Leu and Val to grow. The deletion was then complemented by introducing either *fDHAD* or *astD* episomally, both of which allowed the strain to grow in the absence of the three BCAAs (**FIG. 29A-29D**). However, yeast expressing fDHAD was approximately 100 times more sensitive to **AA** ($IC_{50}$ of 2 $\mu$M) compared to yeast expressing AstD ($IC_{50}$ of 200 $\mu$M) (**FIG. 24C**). Collectively, the biochemical and genetic assays validated the target-guided genome mining premise described herein, and showed that **AA** is the first natural product inhibitor of fungal and plant DHAD; and AstD serves as the self-resistance enzyme in the *ast* biosynthetic gene cluster.

Comparison of structures of **AA** and DHAD substrates revealed how **AA** may be ideally suited to be a DHAD inhibitor. The *(R)*-$\alpha$-hydroxyacid and (*R*)-configured $\beta$-ether oxygen moieties formed from nucleophilic epoxide opening mimic closely the (*2R, 3R*)-dihydroxy groups present in natural substrates such as dihydroxyisovalerate. The $\beta$-ether oxygen in **AA** is in position to coordinate to the 2Fe-2S cluster that is present in both fungal and plant DHAD (*11*, *12*). In addition, the hydrophobic tricyclic ring system not only mimics the hydrophobic side-chain of the native substrate, but also should reduce configurational entropy loss during ligand-protein binding. To shed further light on the potential **AA** mechanism of action, the crystal structure (2.11 Å) of the pDHAD complexed with 2Fe-2S cluster (*holo*-pDHAD) was determined (**FIG. 30A-30E** and **Table 9D**). A binding chamber was identified at the homodimer interface, similar to that

found in the *holo* bacterial l-arabinonate dehydratase (26). The interior of the chamber is positively charged (2Fe-2S and $Mg^{2+}$) while the entrance is lined with hydrophobic residues. The best modeled binding mode of $\alpha,\beta$-dihydroxyisovalerate and **AA** predicted by computational docking are shown in **FIG. 31A** and **FIG. 31B.** The pocket is sufficiently spacious to accommodate the bulkier **AA,** and provide stronger hydrophobic interactions than the native substrate with a $5.3 \pm 0.3$ kcal/mol gain in binding energy (**FIG. 31A - FIG. 31B**). Based on the *holo*-pDHAD structure, a homology model of AstD was constructed to determine potential mechanism of resistance (**FIG. 30A - FIG. 30E**). Comparison of pDHAD and the modeled AstD structures shows that while most the residues in the catalytic chamber are conserved, the hydrophobic region at the entrance to the reactive chamber in AstD is more constricted as a result of two amino acid substitutions (V496L and 1177L). Narrowing of the entrance could therefore sterically exclude the bulkier **AA** from binding in the active site, while the smaller, natural substrates are still able to enter the chamber.

To explore the potential of **AA** as an herbicide, spray treatment of *A. thaliana* with **AA** was performed. Because formulation optimization of herbicides to enhance wetting, deposition and penetration is a time-consuming process, **AA** was instead added into a commercial glufosinate formulation known as Finale® at a final **AA** concentration of 250 $\mu$M *(27, 28).* This **AA** solution was then sprayed onto glufosinate resistant *A. thaliana.* Finale® alone had no observable inhibitory effects on plant growth, but adding **AA** severely inhibited plant growth (**FIG. 32**). In addition, *A. thaliana* plants treated with **AA** before flowering failed to form normal pollen, which was also observed previously (Shimada et al., 2002). It was also found that the pistil of treated plants could still be successfully pollinated using healthy pollen from untreated *A. thaliana,* indicating that **AA** preferentially affects pollen but not egg formation (**FIG**. **33A-33C**). This affect was also observed with a lower concentration of **AA** (100 $\mu$M). These results are summarized in **Table 9H.** Thus, in addition to its herbicidal properties, **AA** could be used as a chemical hybridization agent for hybrid seed production (29). Results of AA treatment of wheat inflorescences are shown in **FIG. 33D.**

**Table 9H: Results of Cross Experiment with *A. thaliana***

| female parent | male parent | offspring obtained | inherit resistance |
|---|---|---|---|
| **AA** treated wild type | un-treated Glufosinate resistant plant | Yes | Yes |
| **AA** treated wild type | **AA** treated glufosinate resistant plant | No | N/A |

It was next investigated whether plants expressing *astD* can be resistant to **AA.** This was motivated by the successful combination of glyphosate and genetically modified crops that are selectively resistant to glyphosate (Roundup Ready®) (30). The *A. terreus astD* gene was codon optimized and the *N*-terminus was fused to a chloroplast localization signal derived from pDHAD. Wild type or *astD* transgene-expressing *A. thaliana* was then grown on media that contained 100 $\mu$M **AA.** In the presence of **AA,** the growth of wild-type plants was strongly inhibited, and arrested at the cotyledon stage (**FIG. 34A**). In contrast, the growth of *astD* transgenic plants was relatively unaffected by **AA,** as indicated by the normally expanded rosette leaves, elongated roots, and whole plant fresh weight (**FIG. 34A** and **FIG. 34B**). The expression of AstD was verified by western blot (**FIG. 35**). A spray assay was also performed using T2 *astD* transgenic *A. thaliana* plants, which showed no observable growth defects under such treatment (**FIG. 34C**). In contrast, the control plants carrying the empty vector showed a strong growth inhibitory phenotype when treated with **AA** (**FIG**. **34C**). Quantitative measurements of plant height showed AstD effectively confers **AA** resistance to *A. thaliana* (**FIG. 34D**).

**Conclusion**

In summary, genome mining the fungus *A. terreus* led to the rediscovery of a natural herbicide **AA**, and has allowed the determination its mode of action. **AA** has the potential to become an additional class of herbicide that targets DHAD and inhibits plant BCAA synthesis. **AA**-resistant crops can be developed by introducing *astD* into crop plants. Given its low cytotoxicity in mammalian cell lines, high phytotoxicity toward plants, and new mode of action, it is suggested that **AA** shows promise for its development as a broad spectrum commercial herbicide. This work further underscores that NPs mined from sequenced genomes of microorganisms will continue to be an important source of bioactive compounds.

**REFERENCES**

Shimada A, Yamane H, Kimura Y. Z Naturforsch C. 2008 Jul-Aug;63(7-8):554-6.

Shimada A, Yamane H, Kimura Y. Z Naturforsch C. 2005 Jul-Aug;60(7-8):572-6.

Shimada A, Kusano M, Takeuchi S, Fujioka S, Inokuchi T, Kimura Y. Z Naturforsch C. 2002 May-Jun;57(5-6):459-64.

Plant Physiol. 1984 Jul;75(3):827-31.

Plant Physiol. 1993 Dec;103(4):1221-1226.

J. Chem. Soc., Chem. Commun., 1978, 160-161

J. Antibiot. 2016, 69, 57-61.

1978, J. Chem. Soc., Chem. Commun., 160-161

1. N. Soltani et al., Potential corn yield losses from weeds in north America. Weed Technol. 30, 979-984 (2016).

2. C. J. Swanton, K. N. Harker, R. L. Anderson, Crop losses due to weeds in Canada. Weed Technol. 7, 537-542 (1993).

3. L. P. Gianessi, The increasing importance of herbicides in worldwide crop production. Pest Manag. Sci. 69, 1099-1105 (2013).

4. H. Kraehmer, B. Laber, C. Rosinger, A. Schulz, Herbicides as weed control agents: state of the art: I. weed control research and safener technology: the path to modern agriculture. Plant Physiol. 166, 1119-1131 (2014).

5. I. Heap, Global perspective of herbicide-resistant weeds. Pest Manag. Sci. 70, 1306-1315 (2014).

6. Q. Yu, S. Powles, Metabolism-based herbicide resistance and cross-resistance in crop weeds: a threat to herbicide sustainability and global crop production. Plant Physiol. 166, 1106-1118 (2014).

7. B. K. Singh, D. L. Shaner, Biosynthesis of branched chain amino acids: from test tube to field. Plant Cell 7, 935-944 (1995).

8. G. M. Kishore, D. M. Shah, Amino acid biosynthesis inhibitors as herbicides. Annu. Rev. Biochem. 57, 627-663 (1988).

9. P. J. Tranel, T. R. Wright, Resistance of weeds to ALS-inhibiting herbicides: what have we learned? Weed Sci. 50, 700-712 (2002).

10. D. H. Flint, M. H. Emptage, Dihydroxy acid dehydratase from spinach contains a [2Fe-2S] cluster. J. Biol. Chem. 263, 3558-3564 (1988).

11. D. H. Flint, M. H. Emptage, M. G. Finnegan, W. Fu, M. K. Johnson, The role and properties of the iron-sulfur cluster in Escherichia coli dihydroxy-acid dehydratase. J. Biol. Chem. 268, 14732-14742 (1993).

12. D. H. Flint, A. Nudelman, Studies on the active site of dihydroxy-acid dehydratase. Bioorg. Chem. 21, 367-385 (1993).

13. D. J. Newman, G. M. Cragg, Natural products as sources of new drugs from 1981 to 2014. J. Nat. Prod. 79, 629-661 (2016).

14. F. E. Dayan, S. O. Duke, Natural compounds as next generation herbicides. Plant Physiol. 166, 1090-1105 (2014).

15. T. Pusztahelyi, I. Holb, I. Pócsi, Secondary metabolites in fungus-plant interactions. Front. Plant Sci. 6, 573 (2015).

16. Peter J. Rutledge, Gregory L. Challis, Discovery of microbial natural products by activation of silent biosynthetic gene clusters. Nat. Rev. Microbiol. 13, 509-523 (2015).

17. N. Ziemert, M. Alanjary, T. Weber, The evolution of genome mining in microbes - a review. Nat. Prod. Rep. 33, 988-1005 (2016).

18. Jonathan Kennedy et al., Modulation of polyketide synthase activity by accessory proteins during lovastatin biosynthesis. Science 284, 1368-1372 (1999).

19. T. B. Regueira et al., Molecular basis for mycophenolic acid biosynthesis in Penicillium brevicompactum. Appl. Environ. Microbiol. 77, 3035-3043 (2011).

20. X. Tang et al., Identification of thiotetronic acid antibiotic biosynthetic pathways by target-directed genome mining. ACS Chem. Biol. 10, 2841-2849 (2015).

21. M. A. Fischbach, C. T. Walsh, Assembly-line enzymology for polyketide and nonribosomal peptide antibiotics: logic, machinery, and mechanisms. Chem. Rev. 106, 3468-3496 (2006).

22. D. W. Christianson, Structural biology and chemistry of the terpenoid cyclases. Chem. Rev. 106, 3412-3442 (2006).

23. M.-C. Tang et al., Discovery of unclustered fungal indole diterpene biosynthetic pathways through combinatorial pathway reassembly in engineered yeast. J. Am. Chem. Soc. 137, 13724-13727 (2015).

24. Yoshisuke Tsuda et al., Aspterric acid, a new sesquiterpenoid of the carotane group, a metabolite from Aspergillus terreus IFO-6123. X-Ray crystal and molecular structure of its p-bromobenzoate J. Chem. Soc., Chem. Commun. 0, 160-161 (1978).

25. A. Shimada et al., Aspterric acid and 6-hydroxymellein, inhibitors of pollen development in Arabidopsis thaliana, produced by Aspergillus terreus. Z. Naturforsch. C 57, 459-464 (2002).

26. M. M. Rahman et al., The crystal structure of a bacterial L-arabinonate dehydratase contains a [2Fe-2S] cluster. ACS Chem. Biol. 12, 1919-1927 (2017).

27. R. C. Kirkwood, Use and mode of action of adjuvants for herbicides: a review of some current work. Pestic. Sci. 38, 93-102 (1993).

28. G. Hoerlein, Glufosinate (phosphinothricin), a natural amino acid with unexpected herbicidal properties. Rev. Environ. Contam. Toxicol. 138, 73-145 (1994).

29. D. H. McRae, in Plant Breed. Rev. (John Wiley & Sons, Inc., 1985), chap. Advances in chemical hybridization, pp. 169-191.

30. C. M. Benbrook, Trends in glyphosate herbicide use in the United States and globally. Environ. Sci. Eur. 28, 3-19 (2016).

Jon, C. & Christopher, W. Lessons from natural molecules. Nature 432, 829-837 (2004).

Clevenger, K. D. et al. A scalable platform to identify fungal secondary metabolites and their gene clusters. Nat. Chem. Biol. 13, 895 (2017).

Alanjary, M. et al. The antibiotic resistant target seeker (ARTS), an exploration engine for antibiotic cluster prioritization and novel drug target discovery. Nucleic Acids Res. 45, W42-W48 (2017).

Yeh, H.-H. et al. Resistance Gene-Guided Genome Mining: Serial Promoter Exchanges in Aspergillus nidulans Reveal the Biosynthetic Pathway for Fellutamide B, a Proteasome Inhibitor. ACS Chem. Biol. 11, 2275-2284 (2016).

Amorim Franco, T. M. & Blanchard, J. S. Bacterial branched-chain amino acid biosynthesis: structures, mechanisms, and drugability. Biochemistry 56, 5849-5865 (2017).

Swanton, C. J., Harker, K. N. & Anderson, R. L. Crop losses due to weeds in Canada. Weed Technol. 7, 537-542 (1993).

**Claims**

1. A method of reducing growth of a vegetative tissue in a plant, the method comprising:

   spraying a vegetative tissue in the plant one or more times with a herbicidally active amount of a composition comprising (a) aspterric acid and (b) glufosinate;
   wherein growth of the vegetative tissue in the plant is reduced as compared to a corresponding control plant not receiving the composition.

2. The method of claim 1, wherein the composition further comprises an ingredient selected from the group consisting of dimethyl sulfoxide (DMSO), ethanol, and corn oil.

3. The method of claim 1, wherein the concentration of aspterric acid in the composition is in the range of:

   i) about 25 μM to about 75 μM;
   ii) about 50 μM to about 300 μM; or
   iii) about 0.5 mM to about 1.5 mM.

4. The method of claim 1, wherein the plant is grown in a growth medium comprising soil or agar.

5. The method of claim 1, wherein the spraying occurs on multiple occasions over a time interval.

6. The method of claim 5, wherein the spraying occurs for a total duration of about one week to about one month.

7. The method of claim 1, wherein the plant is glufosinate-resistant.

8. The method of claim 1, wherein the plant is a seedling.

**Patentansprüche**

1. Verfahren zum Reduzieren des Wachstums eines vegetativen Gewebes in einer Pflanze, wobei das Verfahren umfasst:

   einmaliges oder mehrmaliges Besprühen eines vegetativen Gewebes in der Pflanze mit einer herbizid wirksamen Menge einer Zusammensetzung, die (a) Aspterrinsäure und (b) Glufosinat umfasst;
   wobei das Wachstum des vegetativen Gewebes in der Pflanze im Vergleich zu einer entsprechenden Kontrollpflanze, die die Zusammensetzung nicht erhält, reduziert ist.

2. Verfahren nach Anspruch 1, wobei die Zusammensetzung ferner einen Inhaltsstoff umfasst, der aus der Gruppe ausgewählt ist, bestehend aus Dimethylsulfoxid (DMSO), Ethanol und Maisöl.

3. Verfahren nach Anspruch 1, wobei die Konzentration von Aspterrinsäure in der Zusammensetzung in dem Bereich liegt von:

   i) etwa 25 μM bis etwa 75 μM;
   ii) etwa 50 μM bis etwa 300 μM; oder
   iii) etwa 0,5 mM bis etwa 1,5 mM.

4. Verfahren nach Anspruch 1, wobei die Pflanze in einem Wachstumsmedium gezüchtet wird, das Erde oder Agar umfasst.

5. Verfahren nach Anspruch 1, wobei das Besprühen mehrfach über ein Zeitintervall erfolgt.

6. Verfahren nach Anspruch 5, wobei das Besprühen über eine Gesamtdauer von etwa einer Woche bis etwa einem Monat erfolgt.

7. Verfahren nach Anspruch 1, wobei die Pflanze glufosinatresistent ist.

8. Verfahren nach Anspruch 1, wobei die Pflanze ein Keimling ist.

## Revendications

1. Procédé de réduction de la croissance d'un tissu végétatif chez une plante, le procédé comprenant l'étape consistant à :

   pulvériser un tissu végétatif de la plante une ou plusieurs fois avec une quantité active du point de vue herbicide d'une composition comprenant (a) de l'acide aspterrique et (b) du glufosinate ;
   ladite croissance du tissu végétatif chez la plante étant réduite par rapport à une plante témoin correspondante n'ayant pas reçu la composition.

2. Procédé selon la revendication 1, dans lequel la composition comprend en outre un ingrédient choisi dans le groupe constitué par le diméthylsulfoxyde (DMSO), l'éthanol et l'huile de maïs.

3. Procédé selon la revendication 1, dans lequel la concentration en acide aspterrique de la composition se situe dans la plage allant de :

   i ) environ 25 $\mu$M à environ 75 $\mu$M,
   ii) environ 50 $\mu$M à environ 300 $\mu$M, ou
   iii) environ 0,5 mM à environ 1,5 mM.

4. Procédé selon la revendication 1, dans lequel la plante est cultivée dans un milieu de croissance comprenant de la terre ou de la gélose.

5. Procédé selon la revendication 1, dans lequel la pulvérisation se produit à plusieurs reprises sur un certain intervalle de temps.

6. Procédé selon la revendication 5, dans lequel la pulvérisation se produit sur une durée totale d'environ une semaine à environ un mois.

7. Procédé selon la revendication 1, dans lequel la plante est résistante au glufosinate .

8. Procédé selon la revendication 1, dans lequel la plante est une plantule.

# FIG. 1

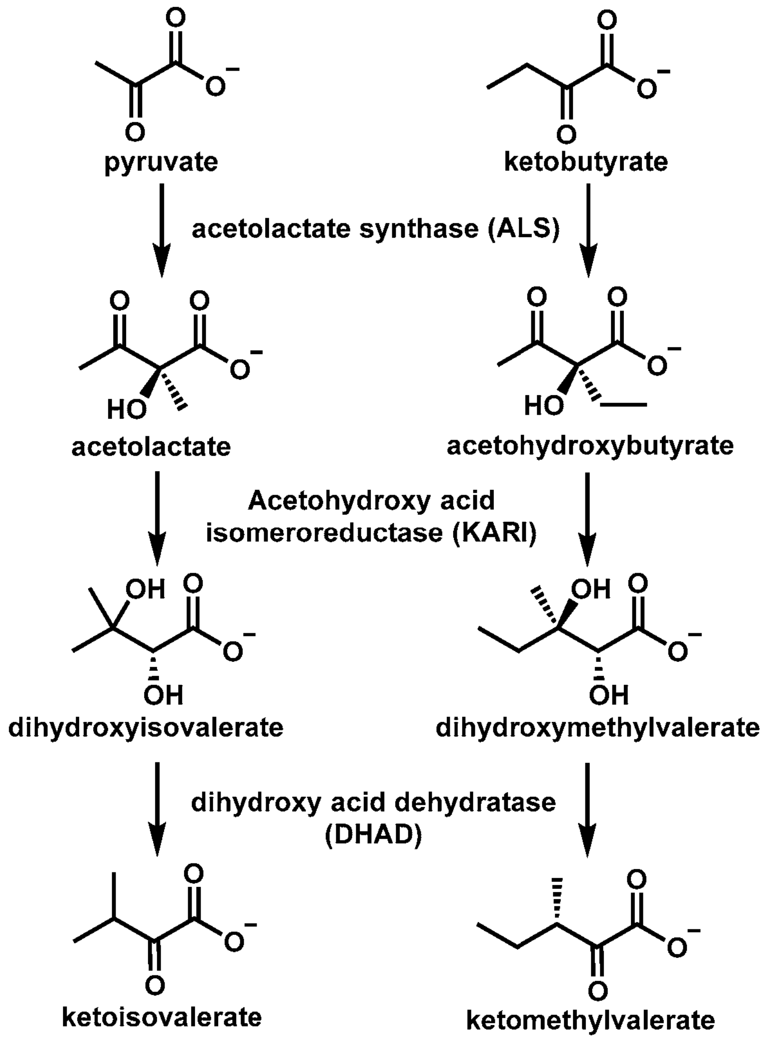


pyruvate

ketobutyrate

acetolactate

acetohydroxybutyrate

dihydroxyisovalerate

dihydroxymethylvalerate

ketoisovalerate

ketomethylvalerate

# FIG. 2A

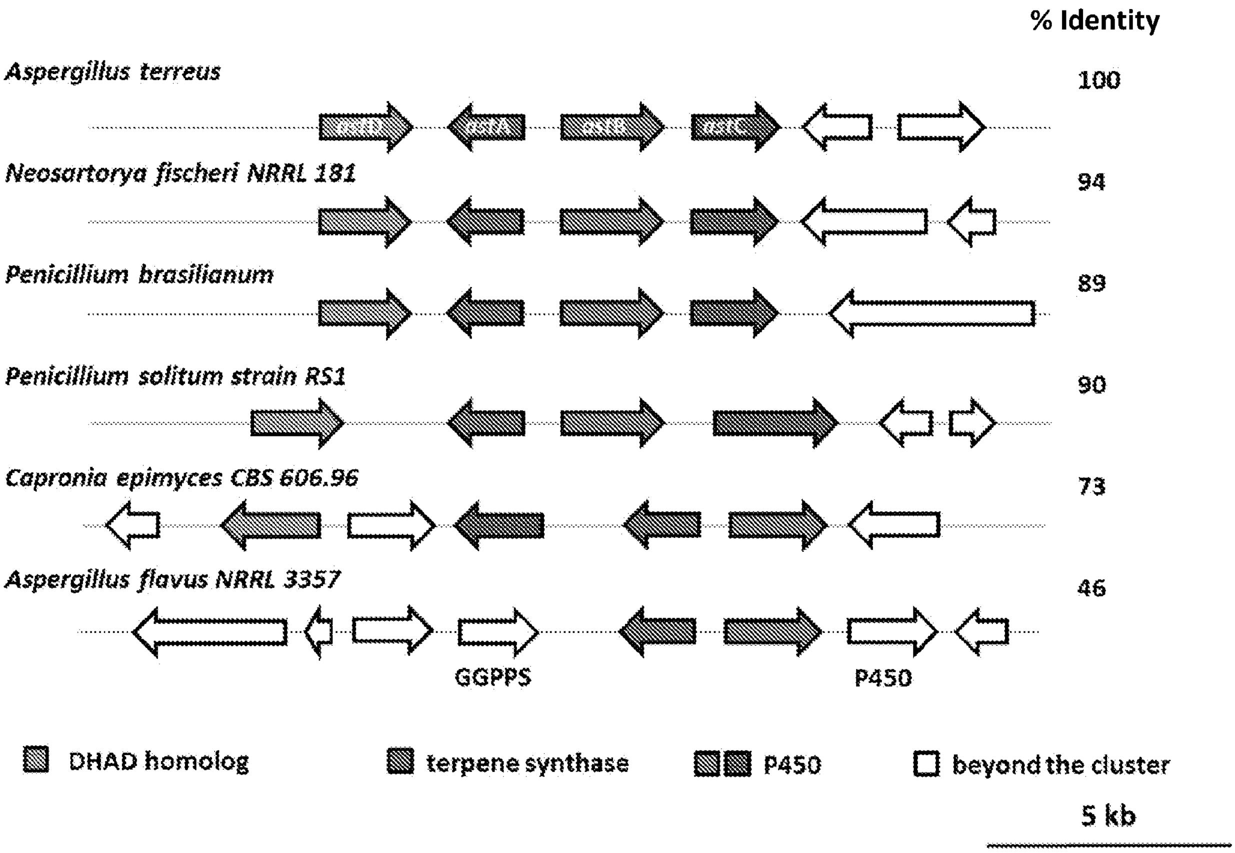


# FIG. 2B

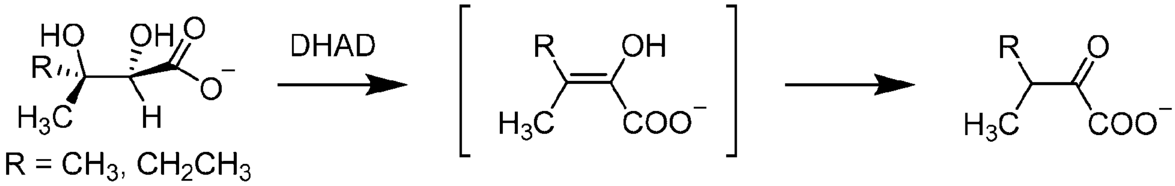


R = $CH_3$, $CH_2CH_3$

# FIG. 3

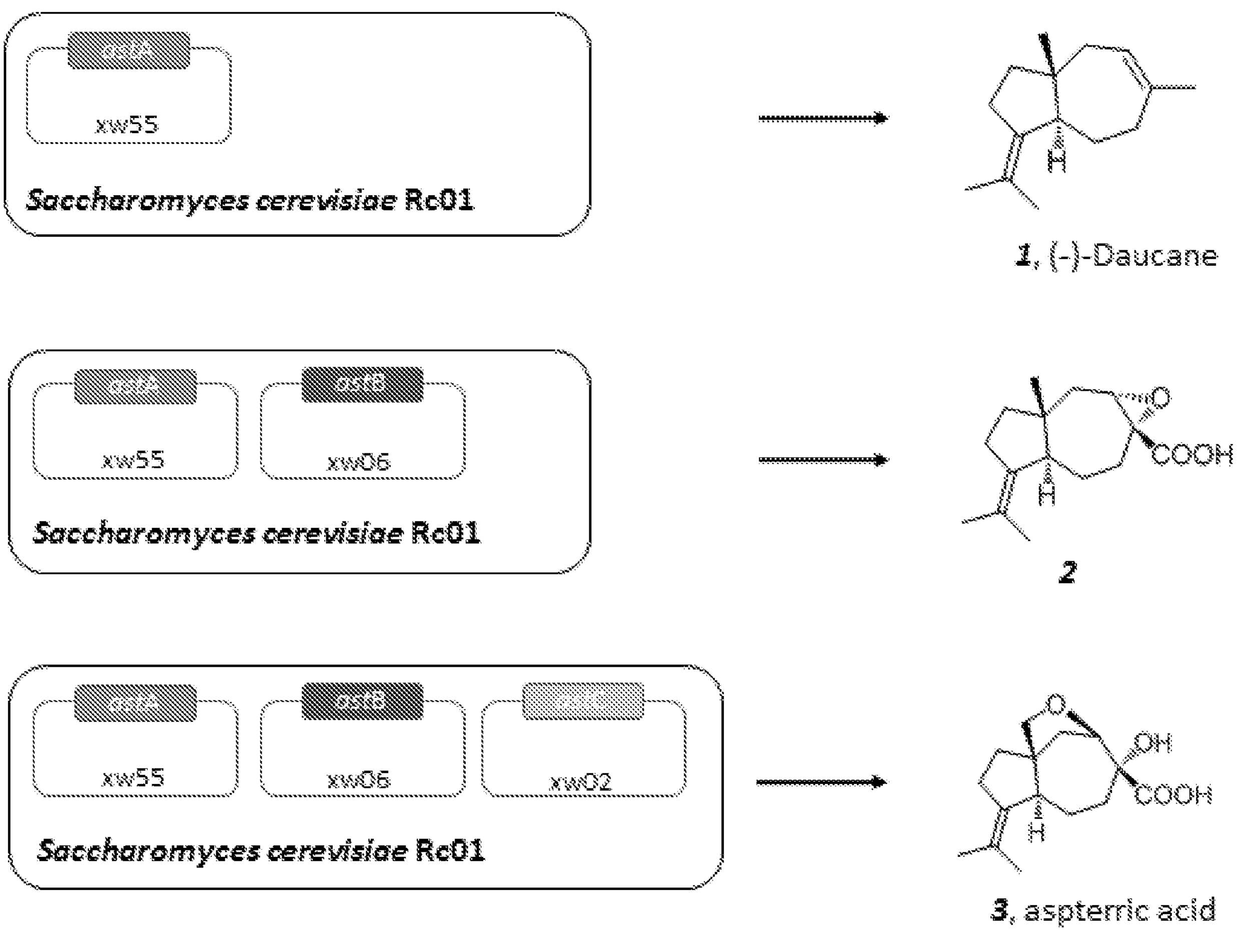


**1**, (-)-Daucane

**2**

**3**, aspterric acid

# FIG. 4

Precursor formation

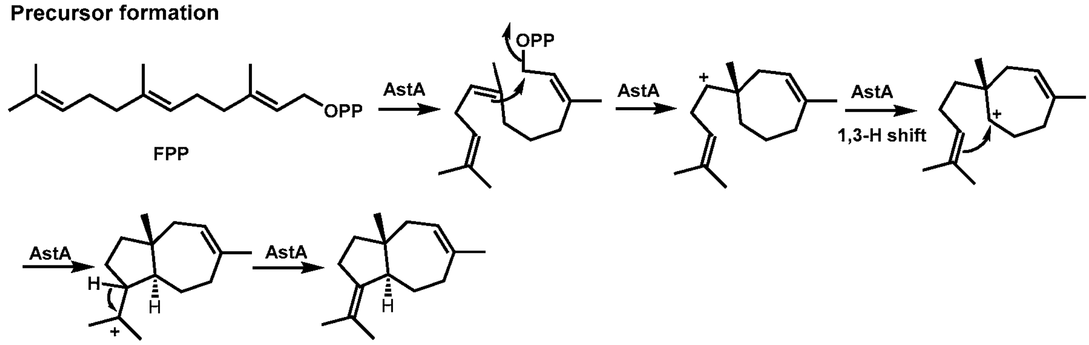


1

Tailoring steps

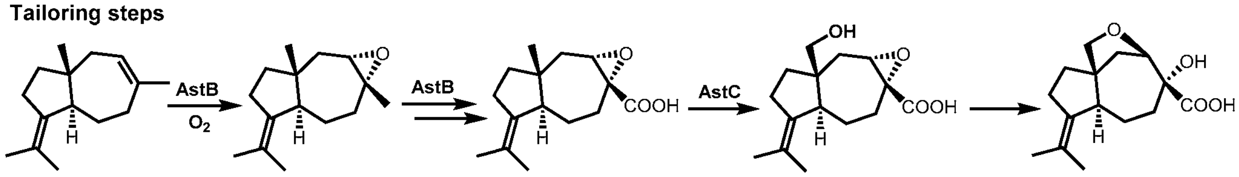


1 2 3

FIG. 5A

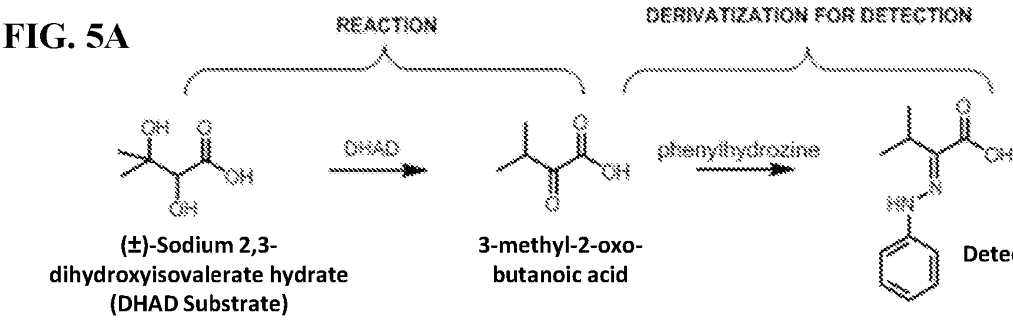


(±)-Sodium 2,3-dihydroxyisovalerate hydrate (DHAD Substrate)

**1**

3-methyl-2-oxo-butanoic acid

**2**

Detectable derivatization product (DDP)

**3**

FIG. 5B

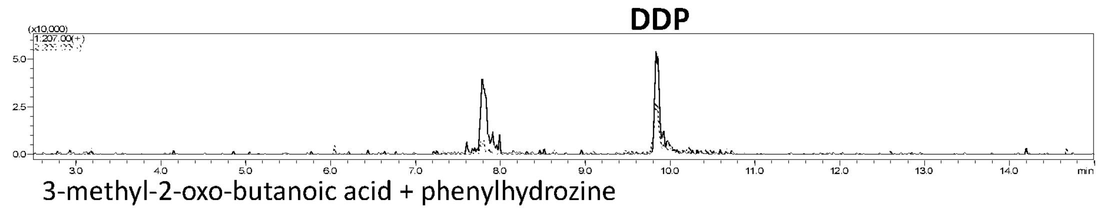


3-methyl-2-oxo-butanoic acid + phenylhydrozine

FIG. 5C

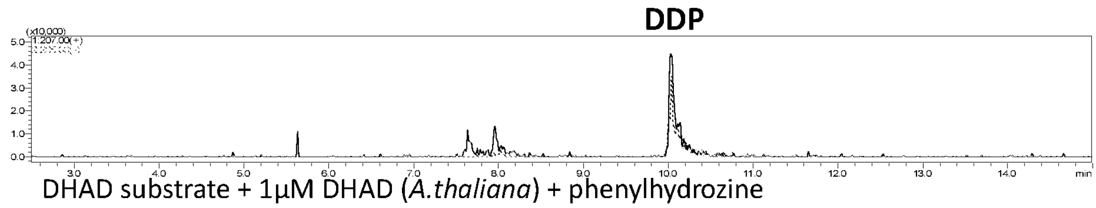


DHAD substrate + 1µM DHAD (*A.thaliana*) + phenylhydrozine

FIG. 5D

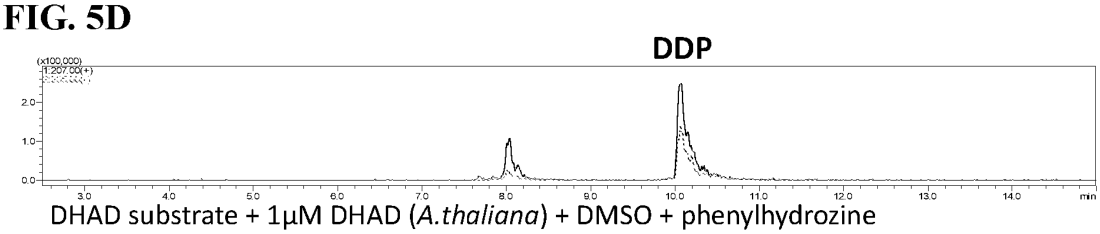


DHAD substrate + 1µM DHAD (*A.thaliana*) + DMSO + phenylhydrozine

FIG. 5E

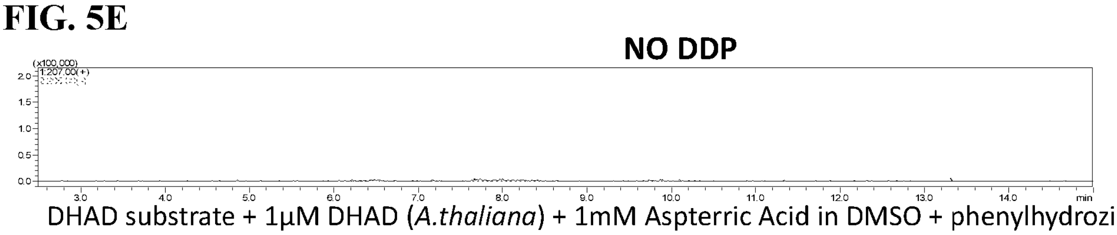


DHAD substrate + 1µM DHAD (*A.thaliana*) + 1mM Aspterric Acid in DMSO + phenylhydrozine

**FIG. 6A**

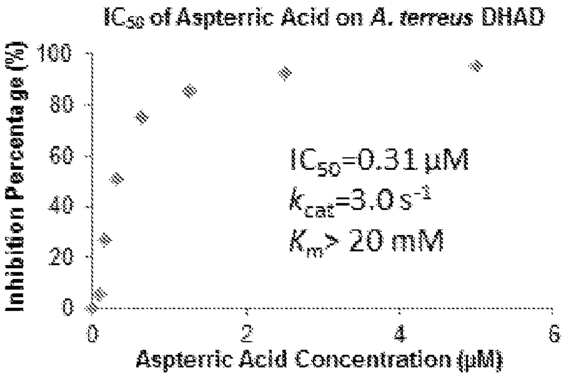


**FIG. 6B**

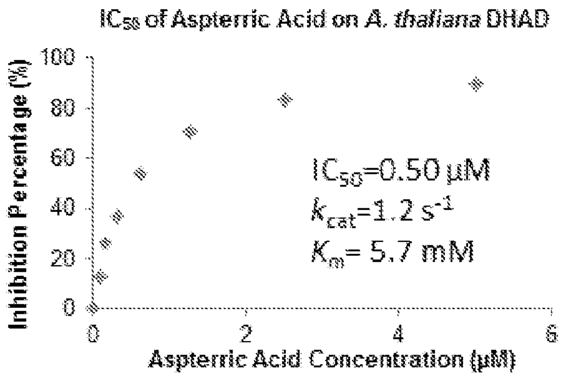


**FIG. 6C**

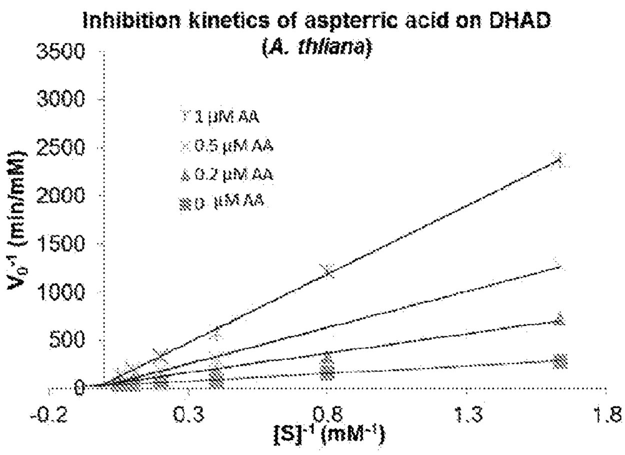


**FIG. 6D**

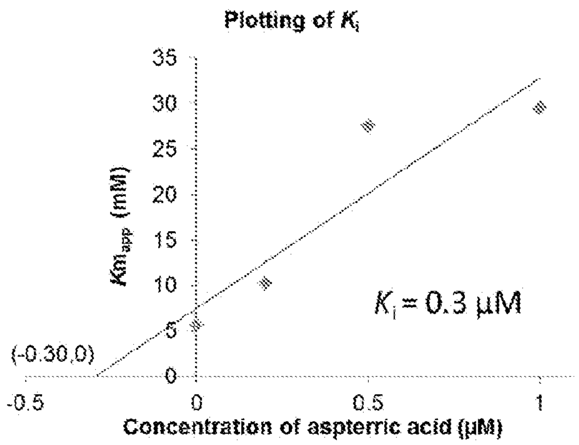

# FIG. 7

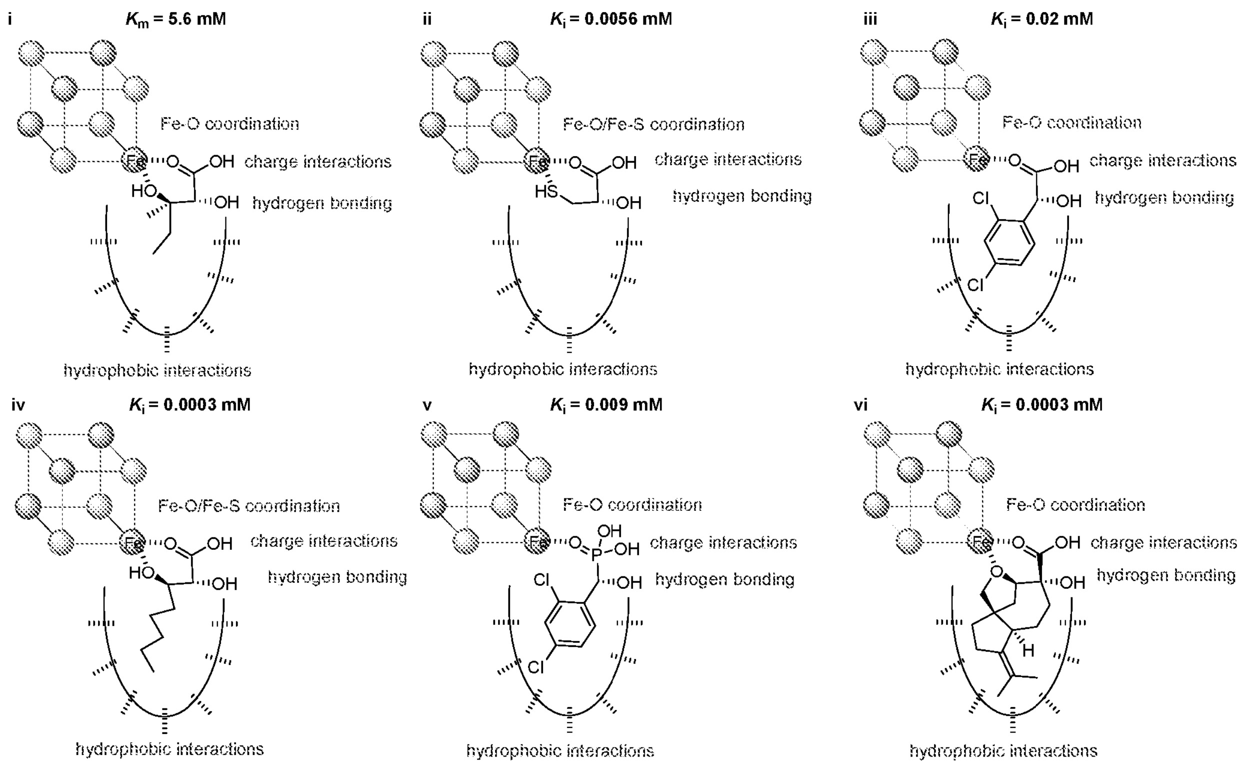
i
$K_m$ = 5.6 mM
Fe-O coordination
charge interactions
hydrogen bonding
hydrophobic interactions
ii
$K_i$ = 0.0056 mM
Fe-O/Fe-S coordination
charge interactions
hydrogen bonding
hydrophobic interactions
iii
$K_i$ = 0.02 mM
Fe-O coordination
charge interactions
hydrogen bonding
hydrophobic interactions
iv
$K_i$ = 0.0003 mM
Fe-O/Fe-S coordination
charge interactions
hydrogen bonding
hydrophobic interactions
v
$K_i$ = 0.009 mM
Fe-O coordination
charge interactions
hydrogen bonding
hydrophobic interactions
vi
$K_i$ = 0.0003 mM
Fe-O coordination
charge interactions
hydrogen bonding
hydrophobic interactions

# FIG. 8

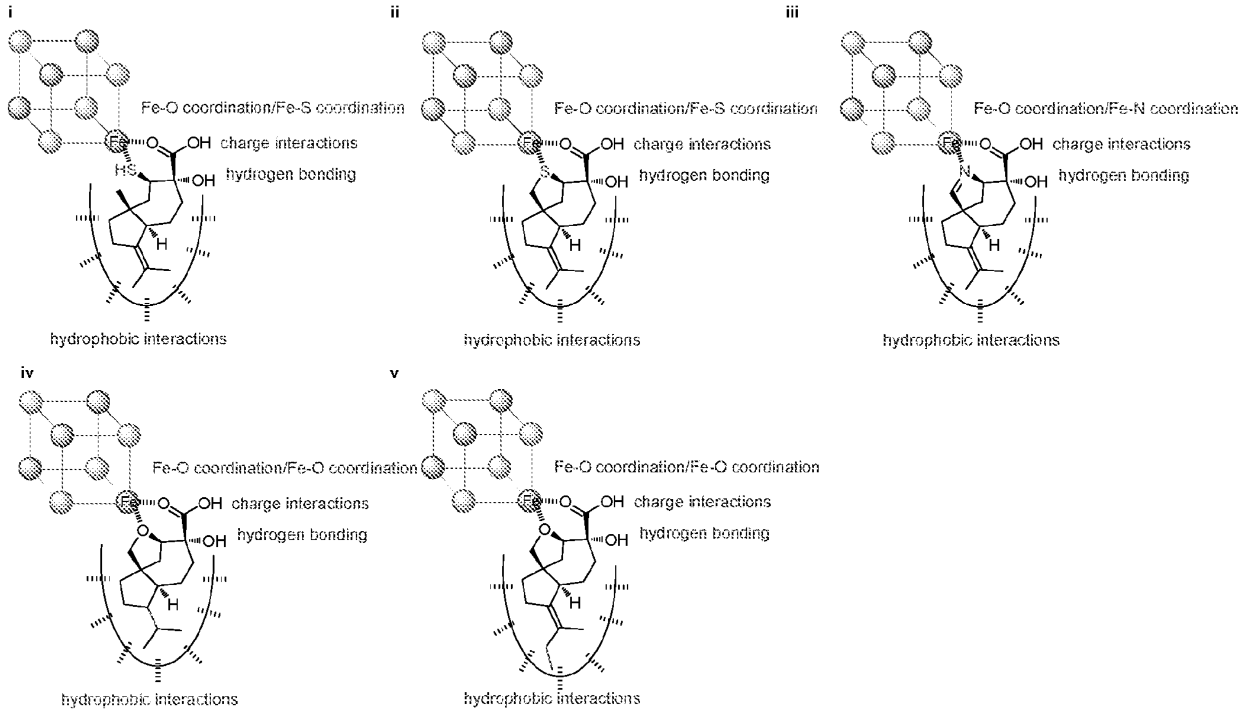
i
Fe-O coordination/Fe-S coordination
charge interactions
hydrogen bonding
hydrophobic interactions
ii
Fe-O coordination/Fe-S coordination
charge interactions
hydrogen bonding
hydrophobic interactions
iii
Fe-O coordination/Fe-N coordination
charge interactions
hydrogen bonding
hydrophobic interactions
iv
Fe-O coordination/Fe-O coordination
charge interactions
hydrogen bonding
hydrophobic interactions
v
Fe-O coordination/Fe-O coordination
charge interactions
hydrogen bonding
hydrophobic interactions

FIG. 9A

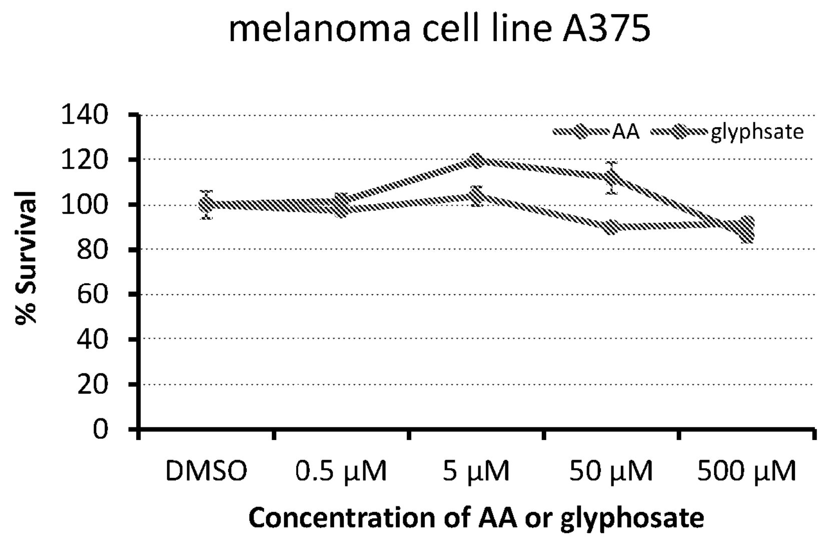
melanoma cell line A375
140
120
100
80
60
40
20
0
% Survival
AA
glyphsate
DMSO
0.5 µM
5 µM
50 µM
500 µM
Concentration of AA or glyphosate

FIG. 9B

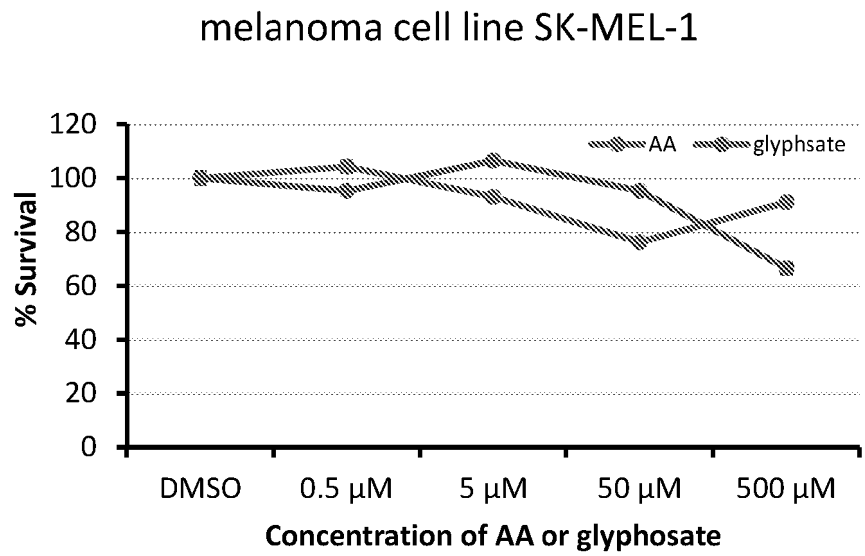
melanoma cell line SK-MEL-1
120
100
80
60
40
20
0
% Survival
AA
glyphsate
DMSO
0.5 µM
5 µM
50 µM
500 µM
Concentration of AA or glyphosate

FIG. 10A

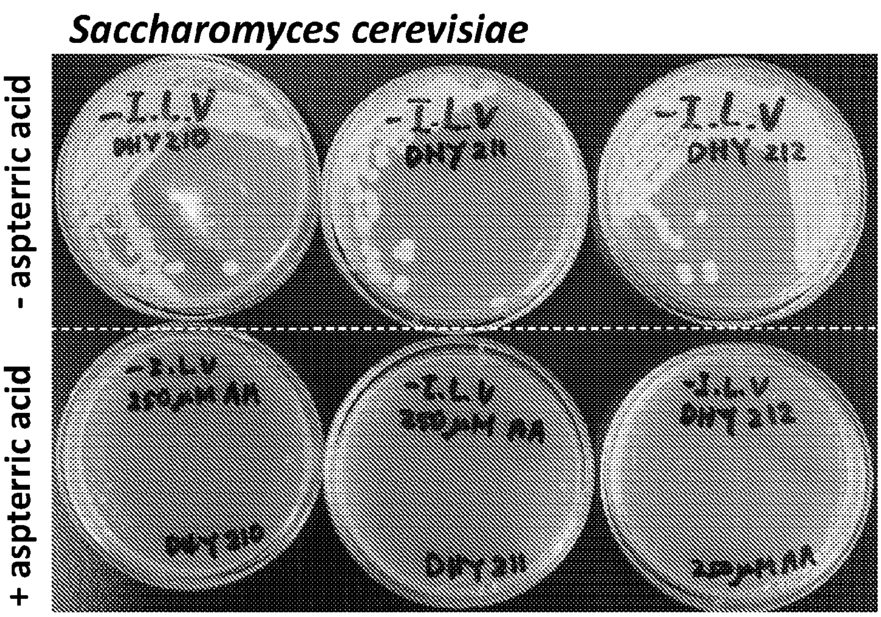


FIG. 10B

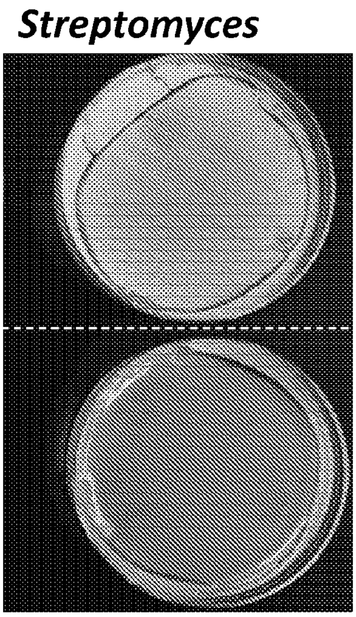

# FIG. 11

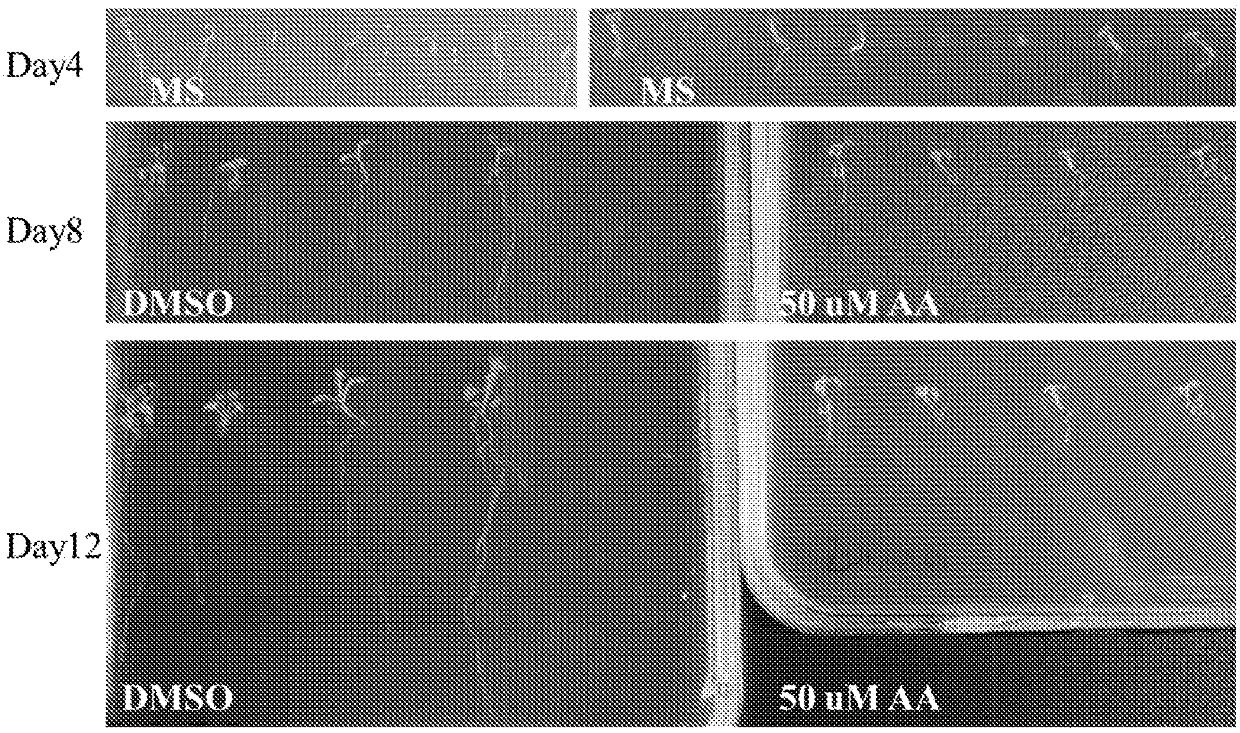

FIG. 12

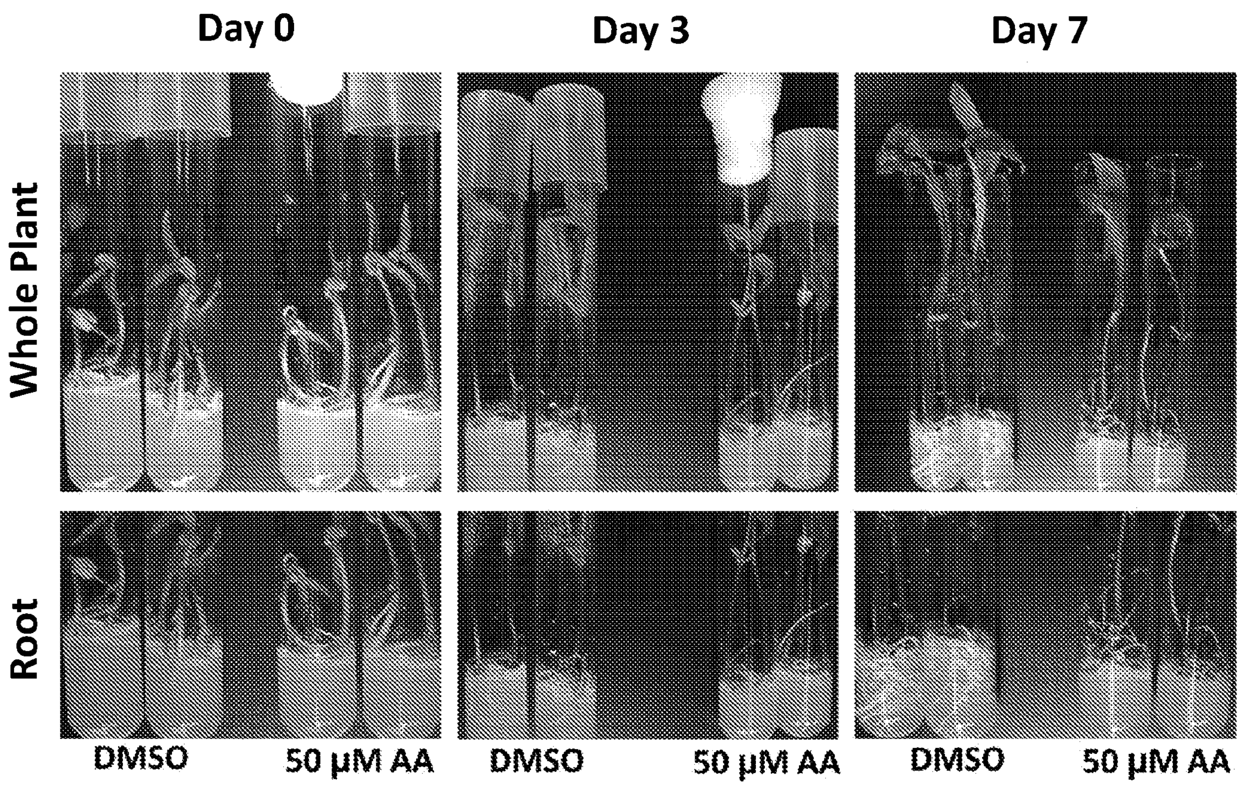
Day 0
Day 3
Day 7
Whole Plant
Root
DMSO
50 μM AA
DMSO
50 μM AA
DMSO
50 μM AA

FIG. 13

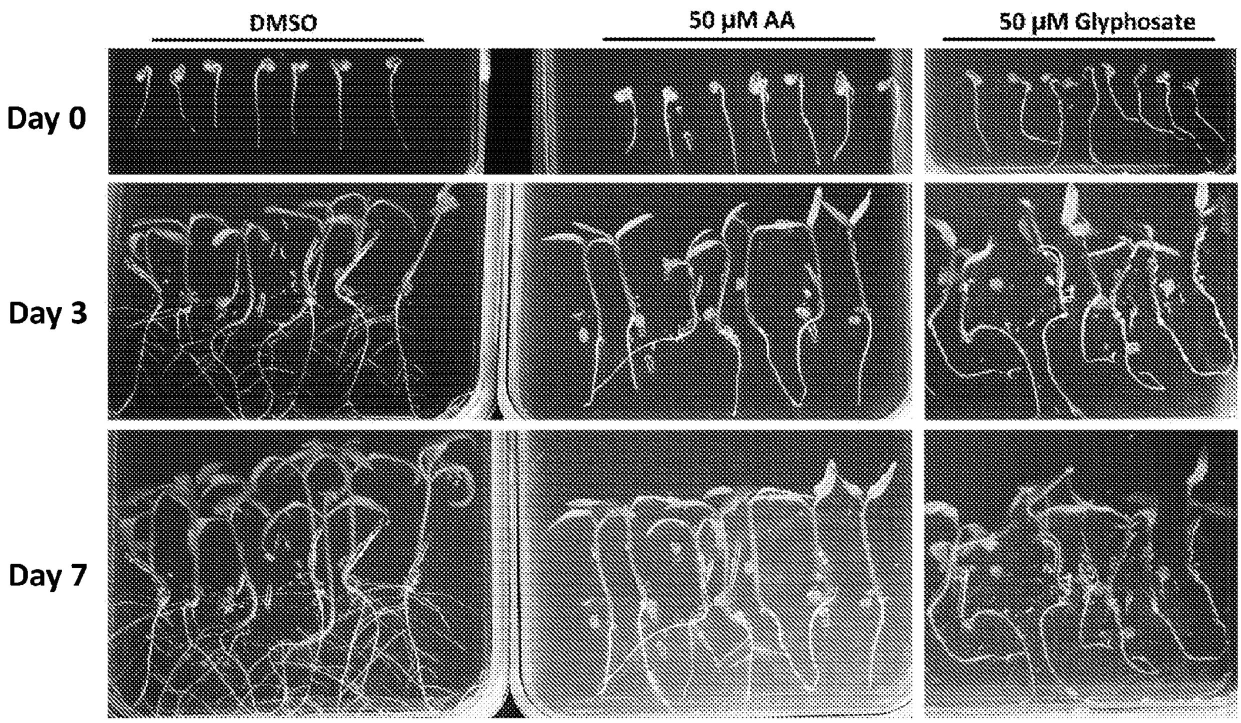

# FIG. 14

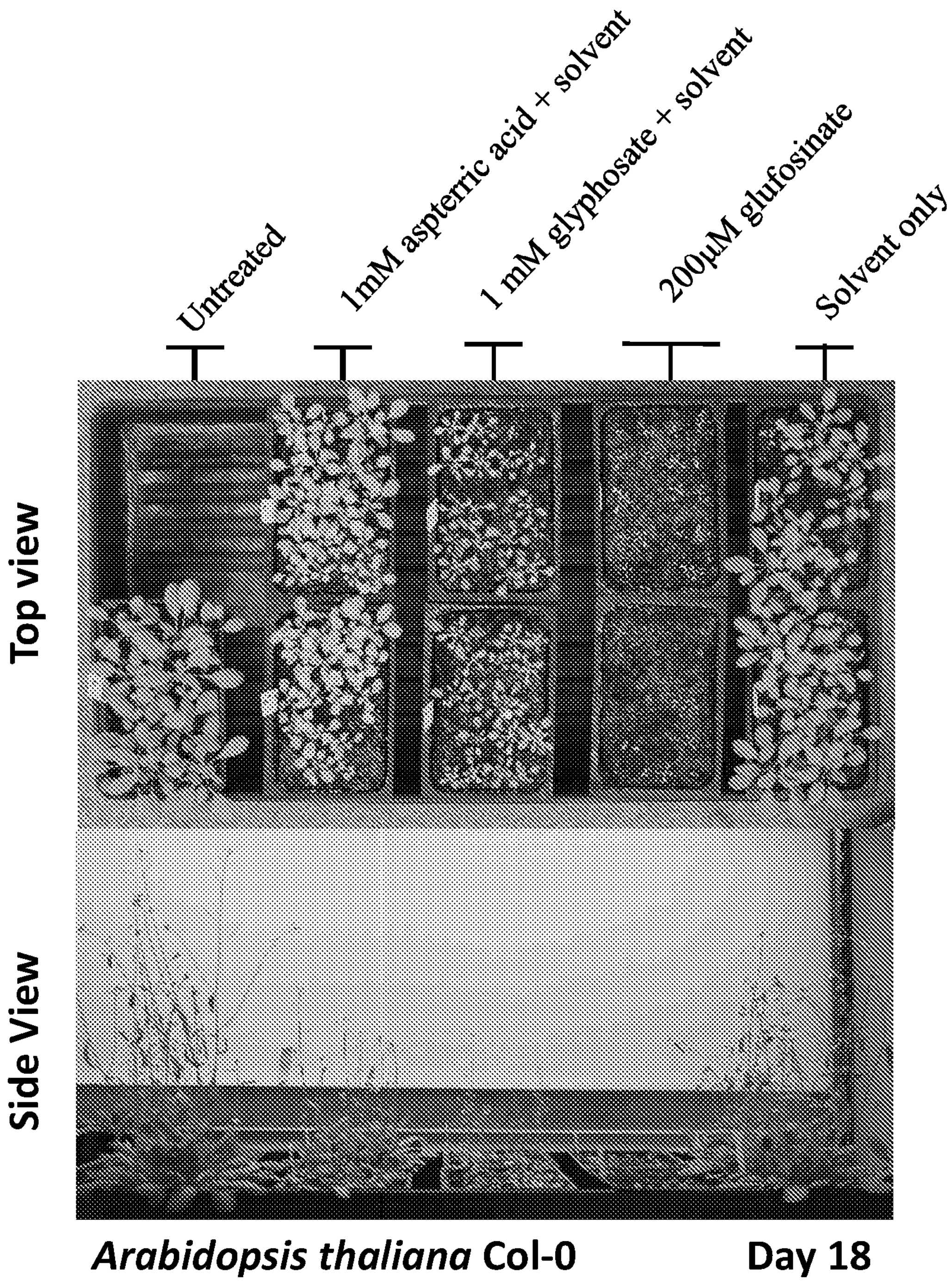


Solvent: 0.5% silwet L-77 + ,1% DMSO

FIG. 15

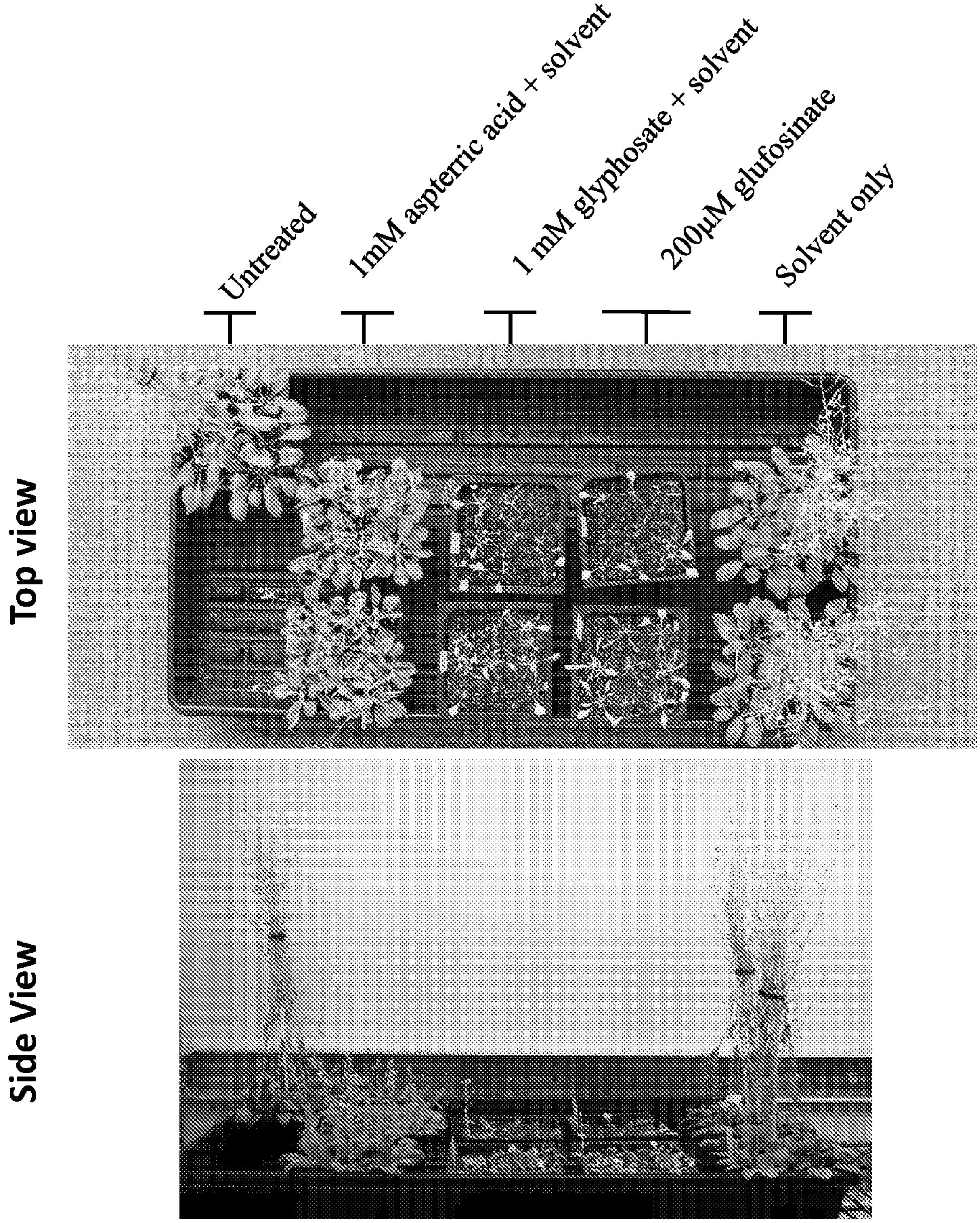


*Arabidopsis thaliana* Col-0 Day 17

(Solvent: 2% EtOH, 1% corn oil and 0.1% tween 80)

FIG. 16

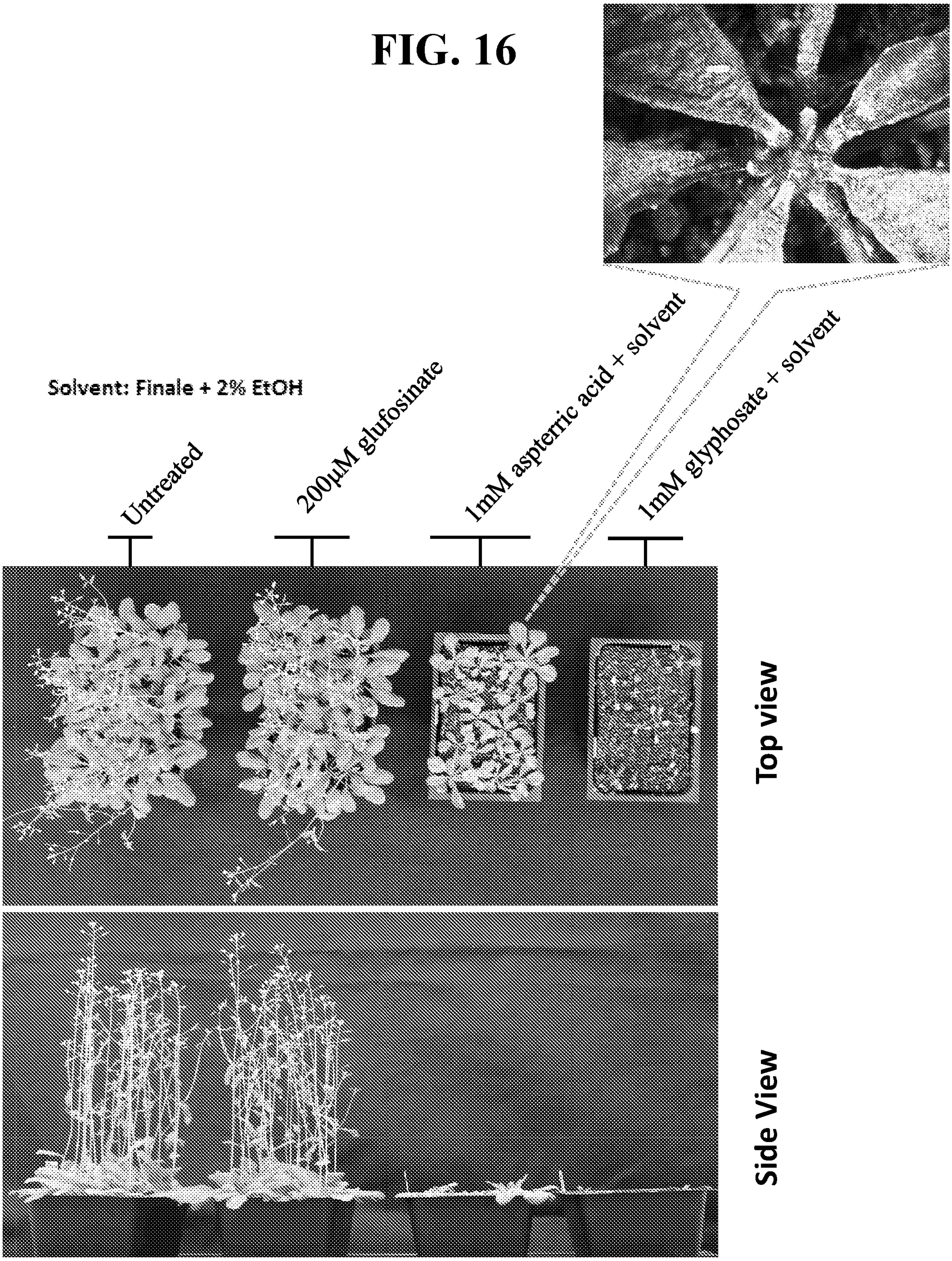


*Arabidopsis thaliana* Col-0 glufosinate$^R$ Day 18

# FIG. 17

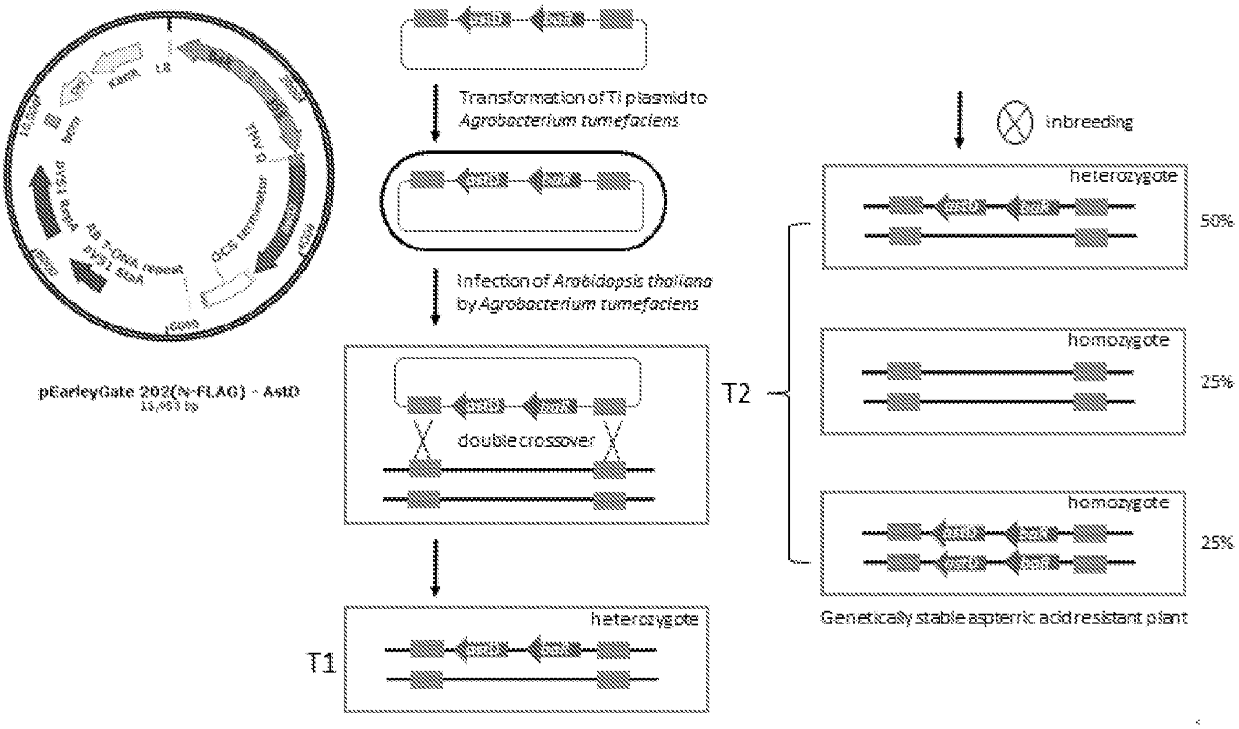
Transformation of Ti plasmid to
Agrobacterium tumefaciens
Infection of Arabidopsis thaliana
by Agrobacterium tumefaciens
double crossover
heterozygote
T1
inbreeding
heterozygote
50%
T2
homozygote
25%
homozygote
25%
Genetically stable aspterric acid resistant plant
pEarleyGate 202(N-FLAG) - AstD

# FIG. 18A

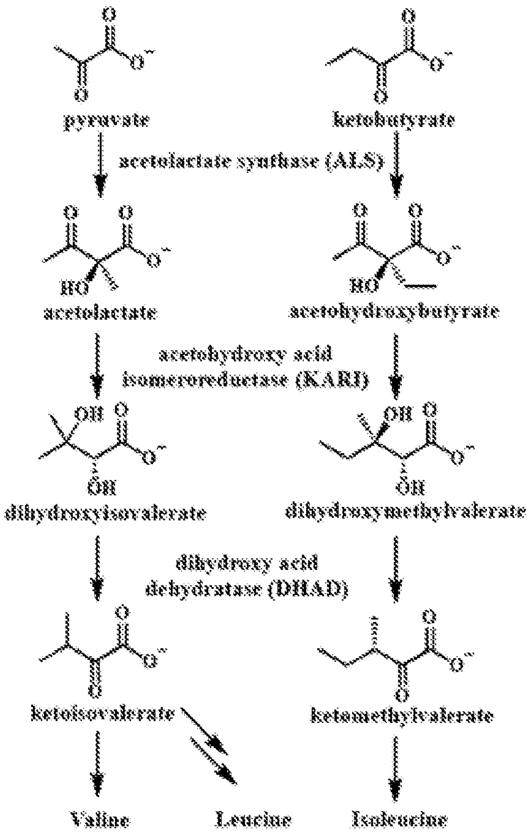


# FIG. 18B

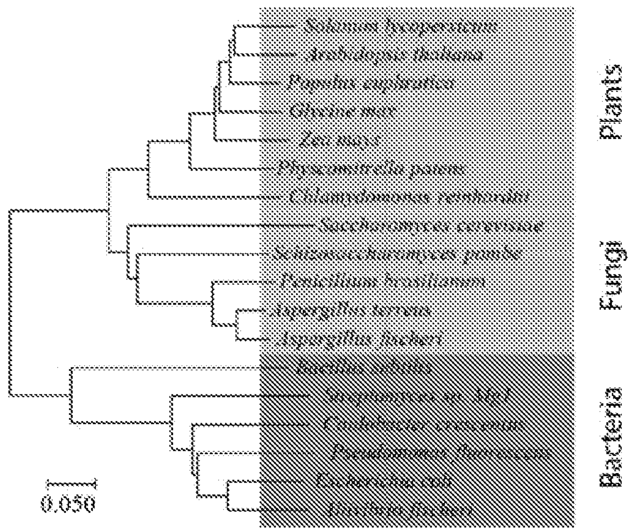


# FIG. 18C

| | 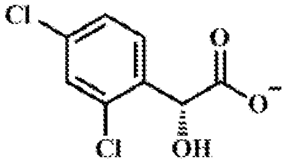 | 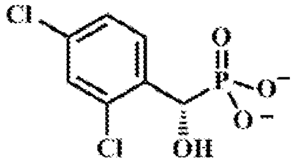 | 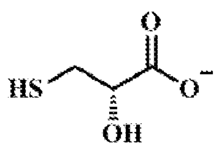 | 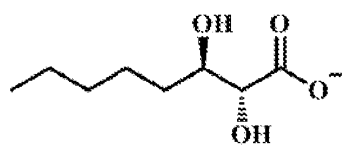 |
|---|---|---|---|---|
| $K_i$ (μM) | 20 | 9 | 5 | 0.3 |

# FIG. 19A

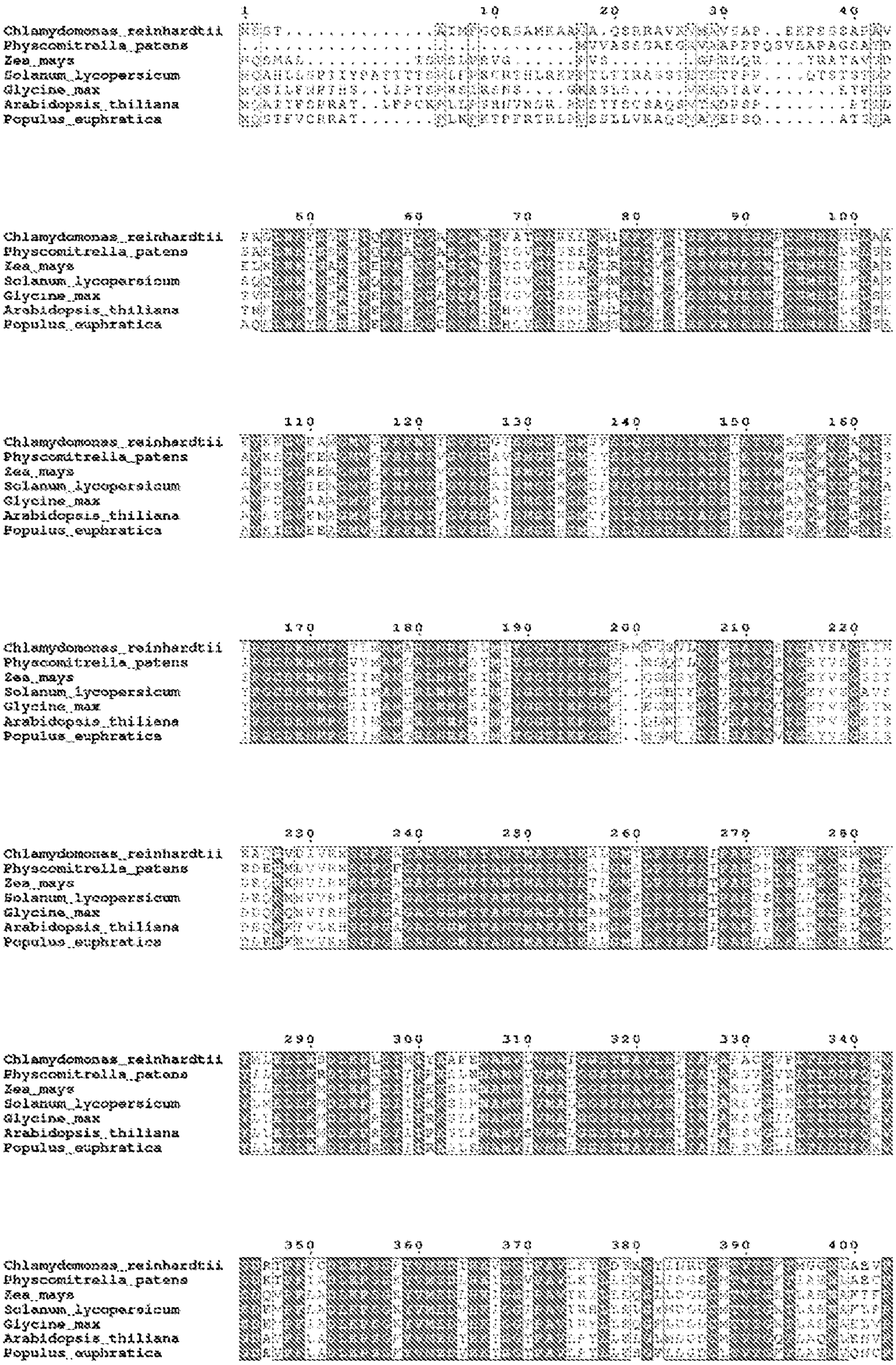
Chlamydomonas_reinhardtii
Physcomitrella_patens
Zea_mays
Solanum_lycopersicum
Glycine_max
Arabidopsis_thiliana
Populus_euphratica

# FIG. 19B

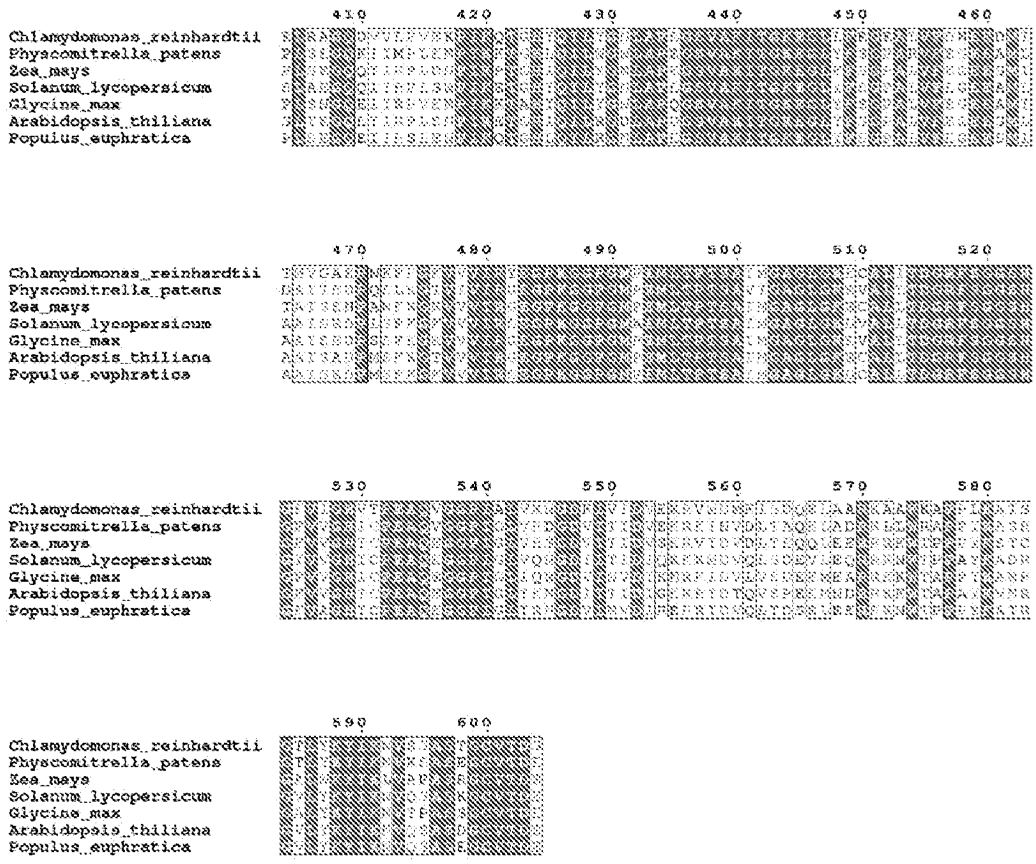
410
420
430
440
450
460
470
480
490
500
510
520
530
540
550
560
570
580
590
600
Chlamydomonas_reinhardtii
Physcomitrella_patens
Zea_mays
Solanum_lycopersicum
Glycine_max
Arabidopsis_thiliana
Populus_euphratica

# FIG. 20

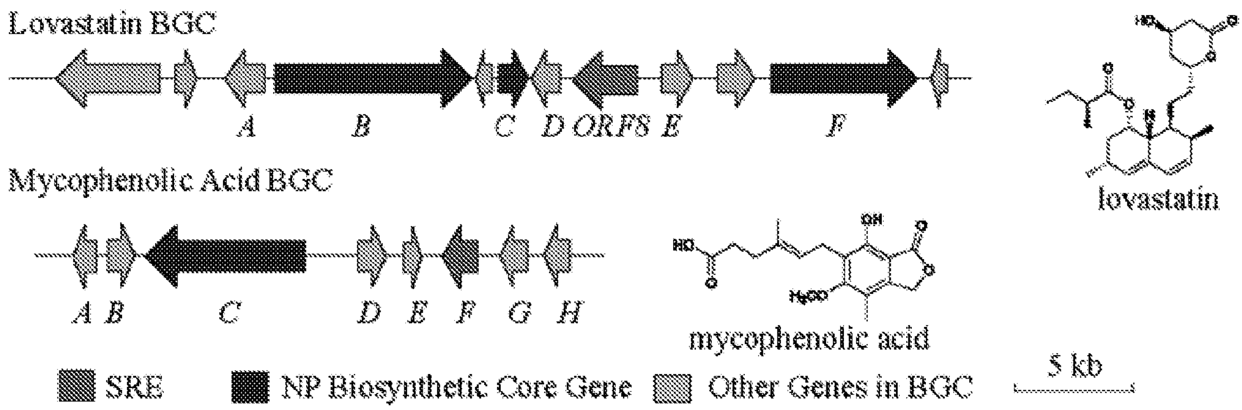
Lovastatin BGC
A
B
C
D
ORF8
E
F
lovastatin
Mycophenolic Acid BGC
A
B
C
D
E
F
G
H
mycophenolic acid
SRE
NP Biosynthetic Core Gene
Other Genes in BGC
5 kb

# FIG. 21A

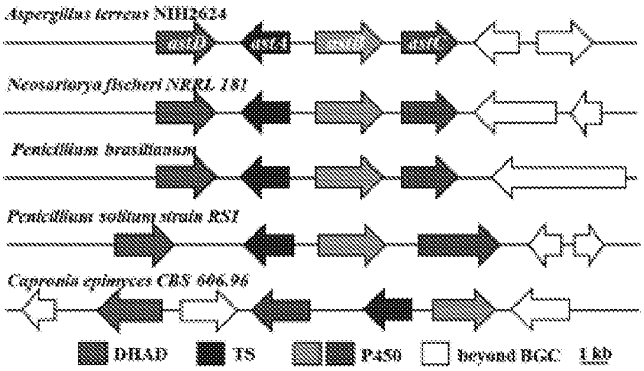


# FIG. 21B

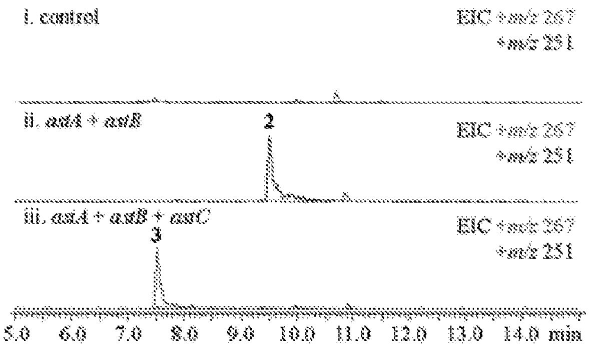


# FIG. 21C

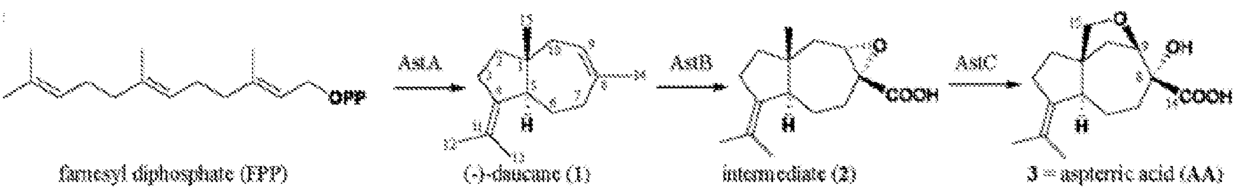


farnesyl diphosphate (FPP)

(-)-daucane (1)

intermediate (2)
MW=250 [M+H]+=251

3 = aspterric acid (AA)
MW=266 [M+H]+=267

# FIG. 22A

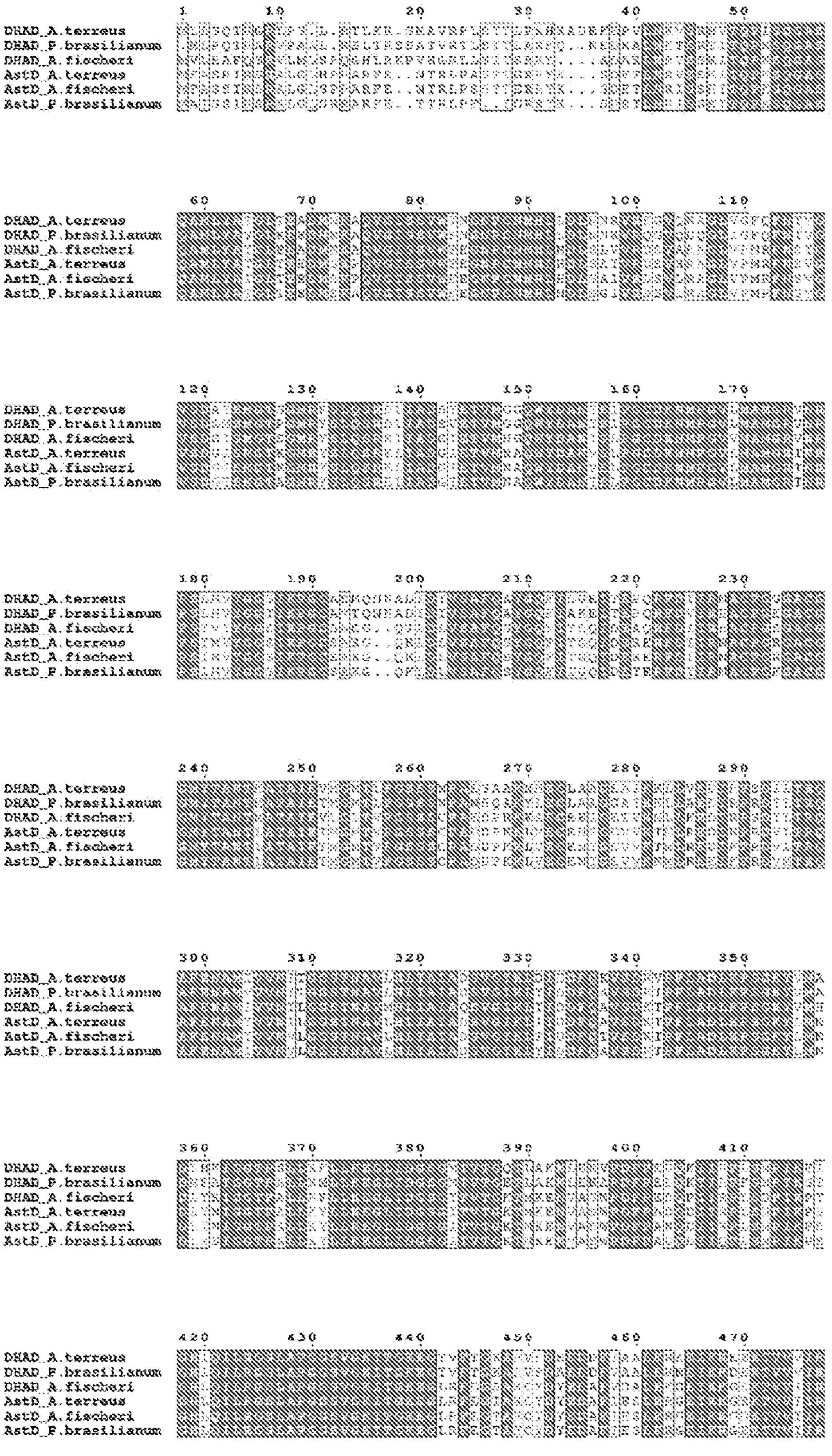
1 10 20 30 40 50
60 70 80 90 100 110
120 130 140 150 160 170
180 190 200 210 220 230
240 250 260 270 280 290
300 310 320 330 340 350
360 370 380 390 400 410
420 430 440 450 460 470
DHAD_A.terreus
DHAD_P.brasilianum
DHAD_A.fischeri
AstD_A.terreus
AstD_A.fischeri
AstD_P.brasilianum

# FIG. 22B

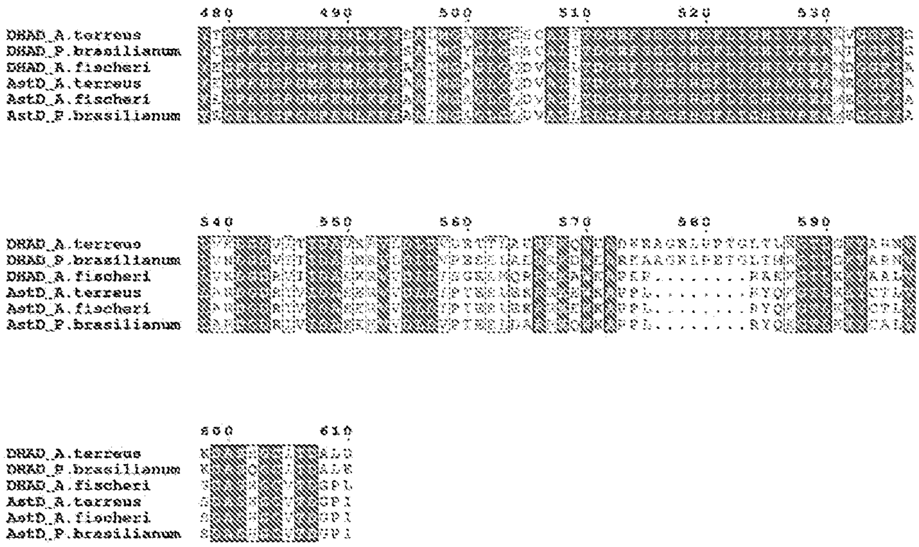
480
490
500
510
520
530
DHAD_A.terreus
DHAD_P.brasilianum
DHAD_A.fischeri
AstD_A.terreus
AstD_A.fischeri
AstD_P.brasilianum
540
550
560
570
580
590
DHAD_A.terreus
DHAD_P.brasilianum
DHAD_A.fischeri
AstD_A.terreus
AstD_A.fischeri
AstD_P.brasilianum
600
610
DHAD_A.terreus
DHAD_P.brasilianum
DHAD_A.fischeri
AstD_A.terreus
AstD_A.fischeri
AstD_P.brasilianum

# FIG. 23A

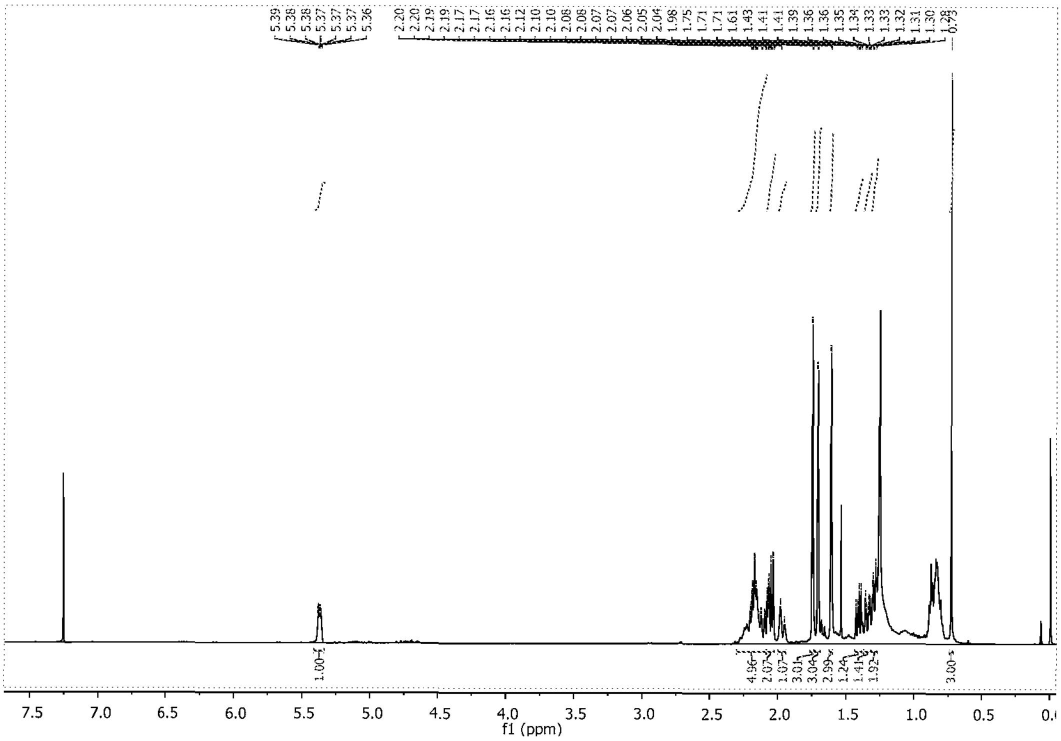

# FIG. 23B

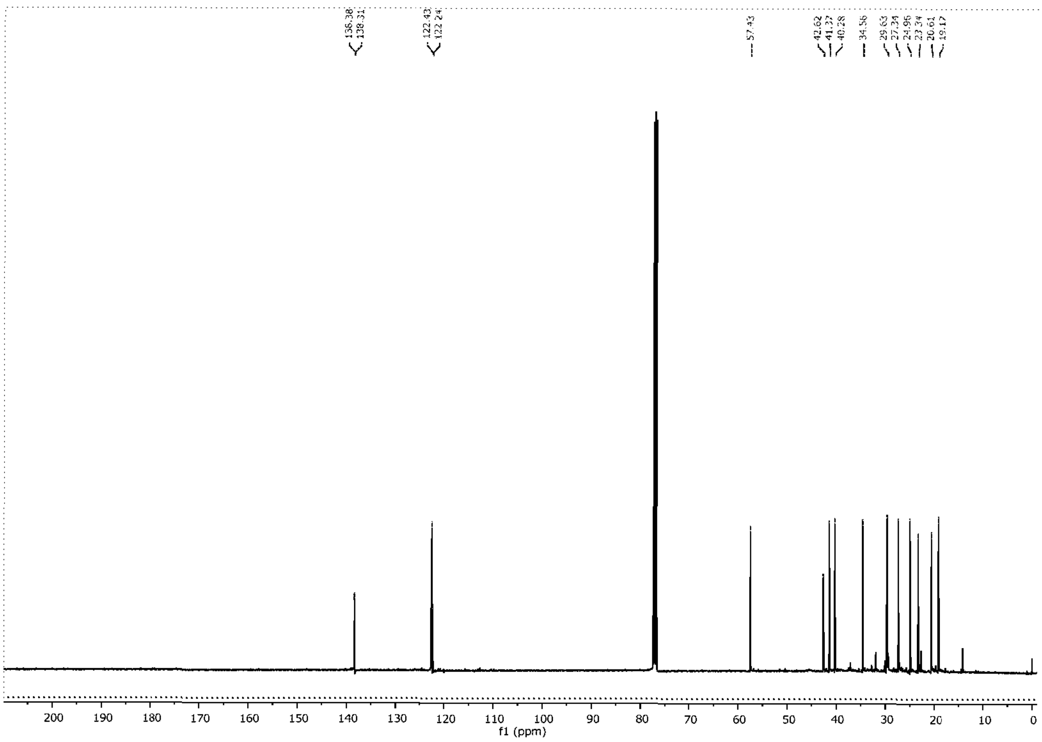

# FIG. 23C

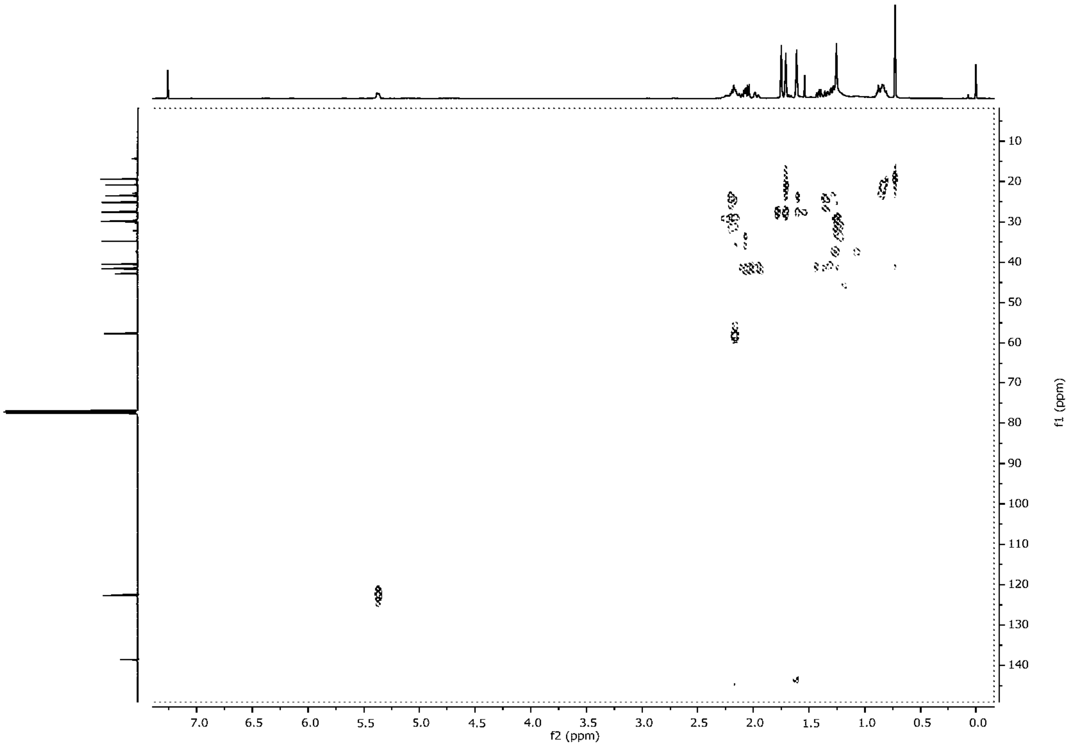


# FIG. 23D

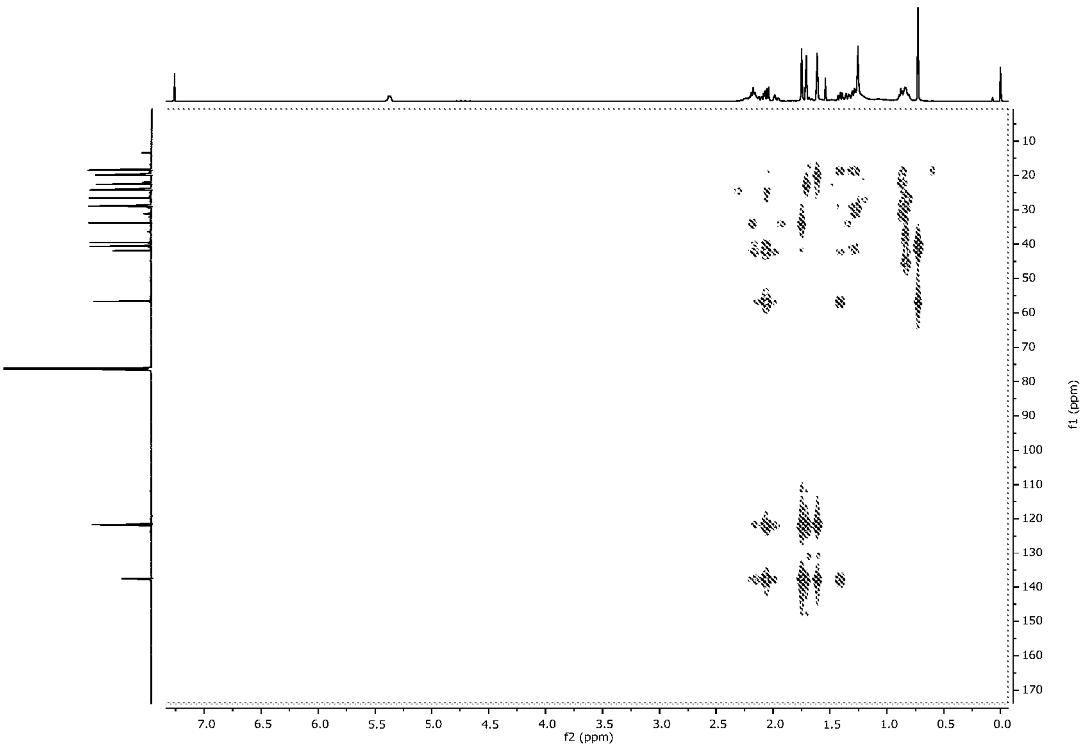

# FIG. 23E

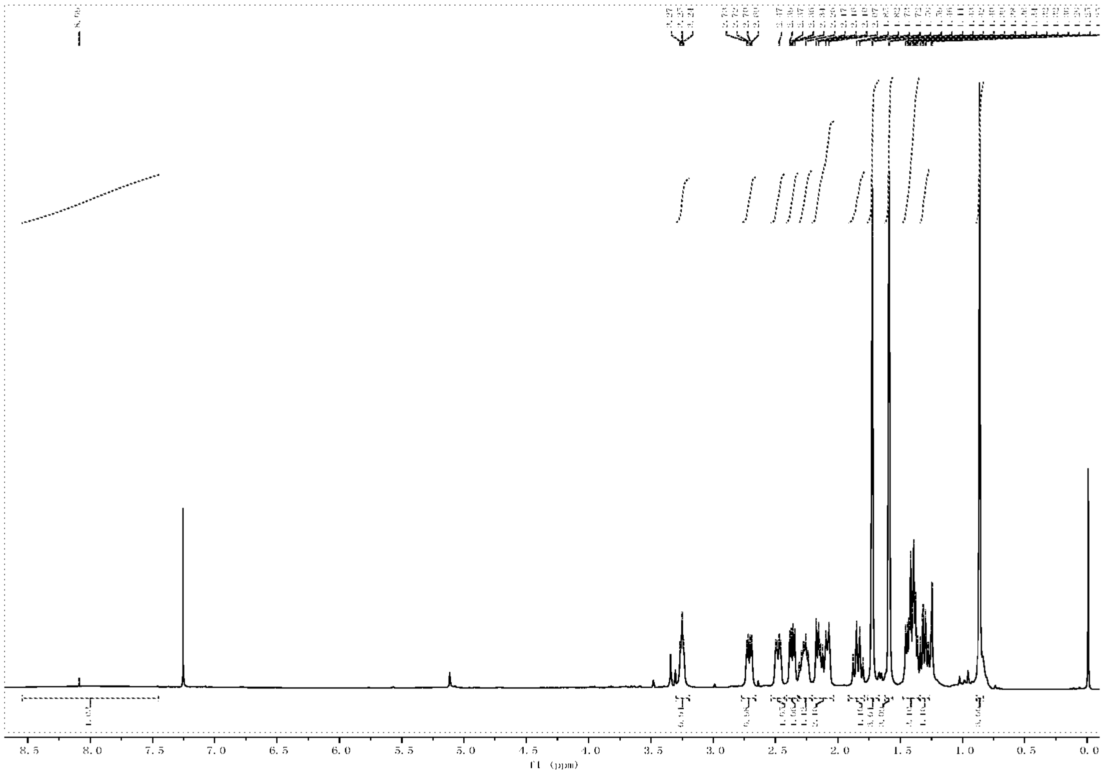

# FIG. 23F

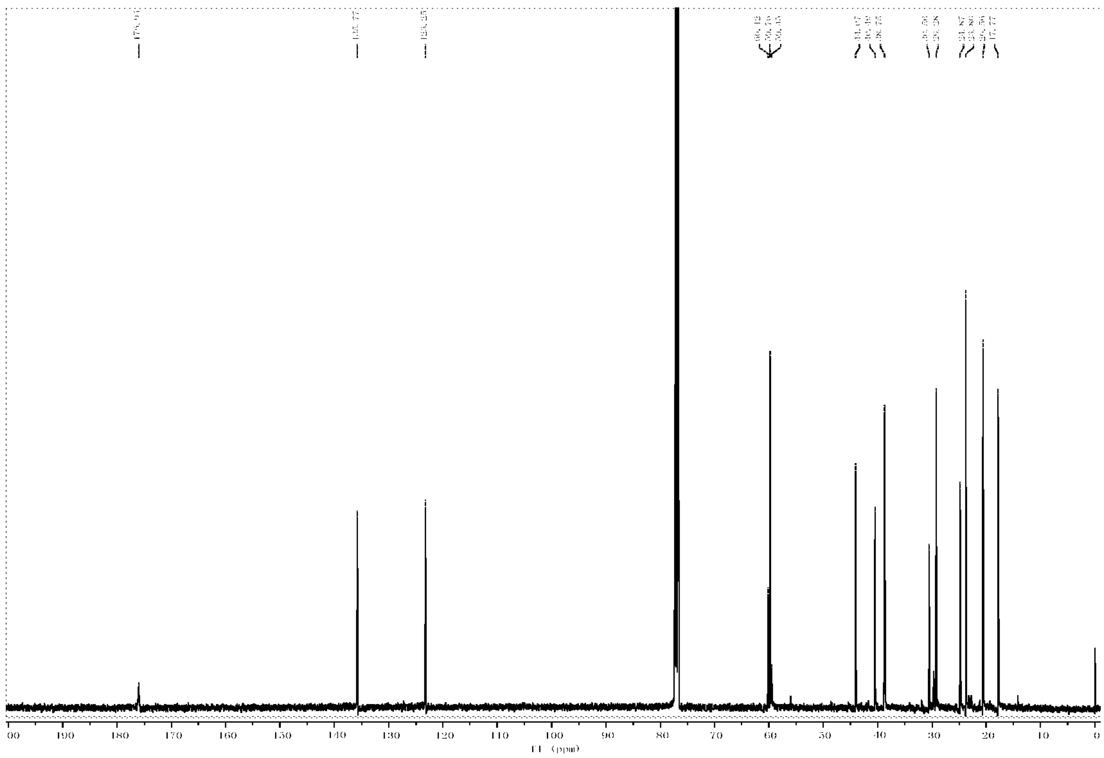

# FIG. 23G

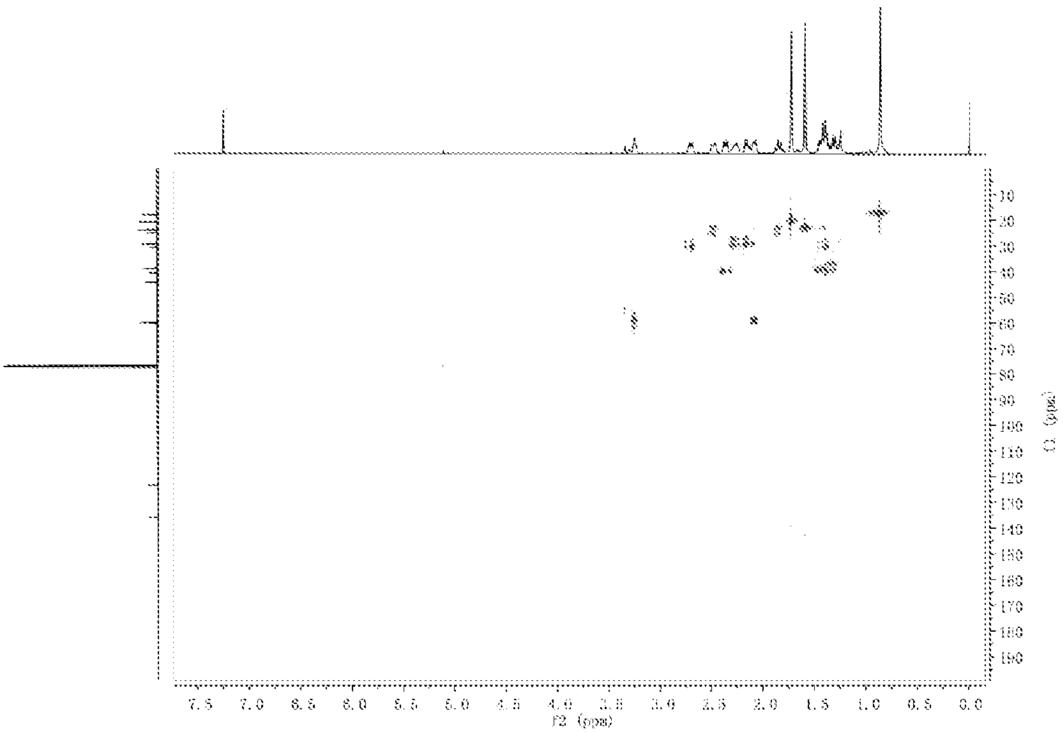


# FIG. 23H

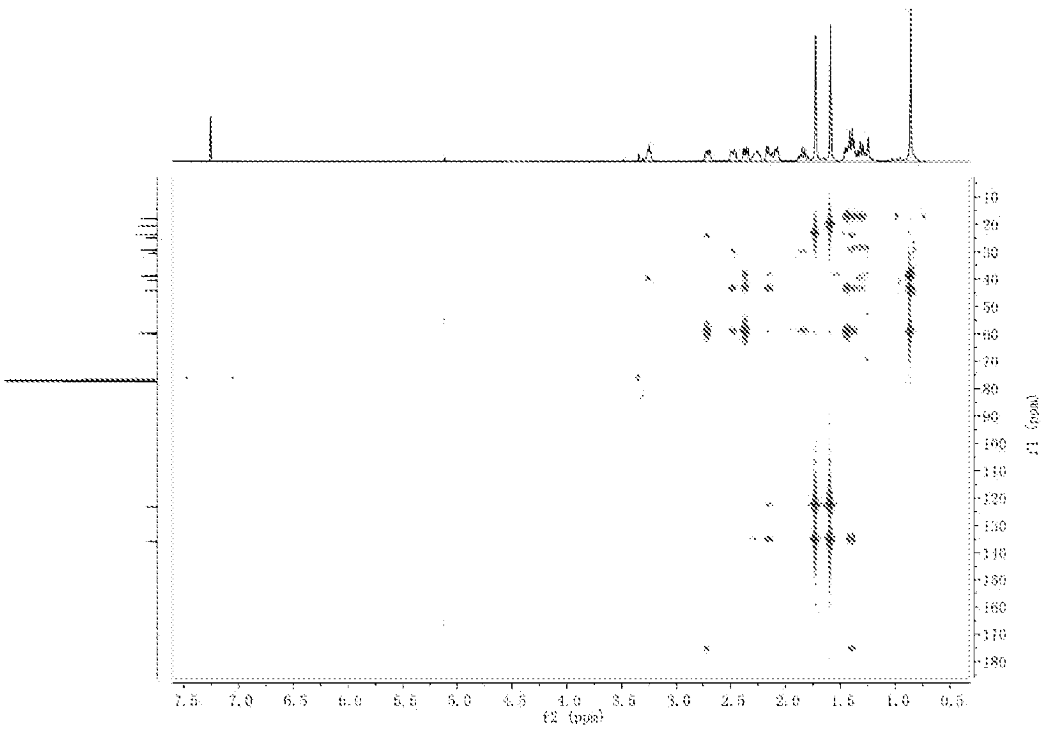

# FIG. 23I

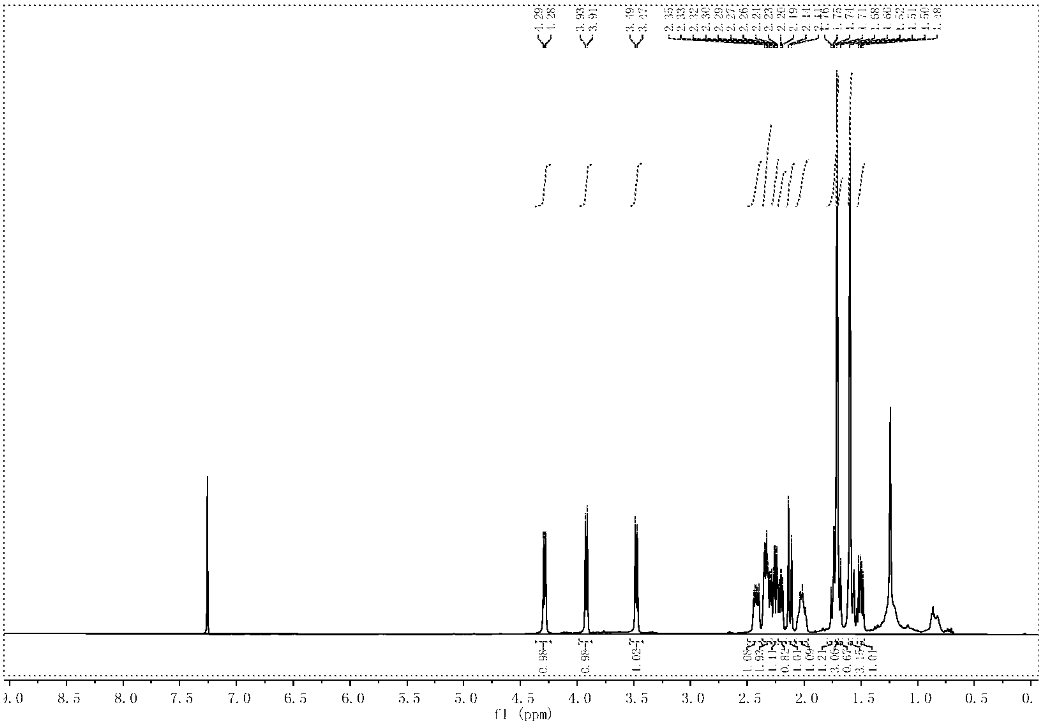

# FIG. 23J

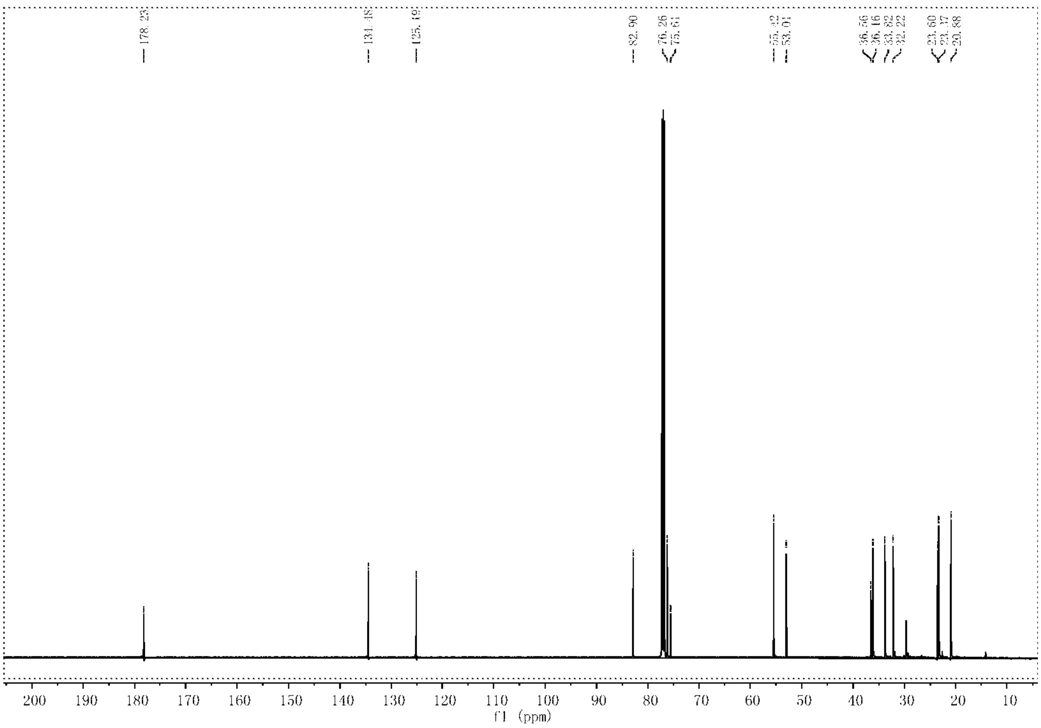

# FIG. 23K

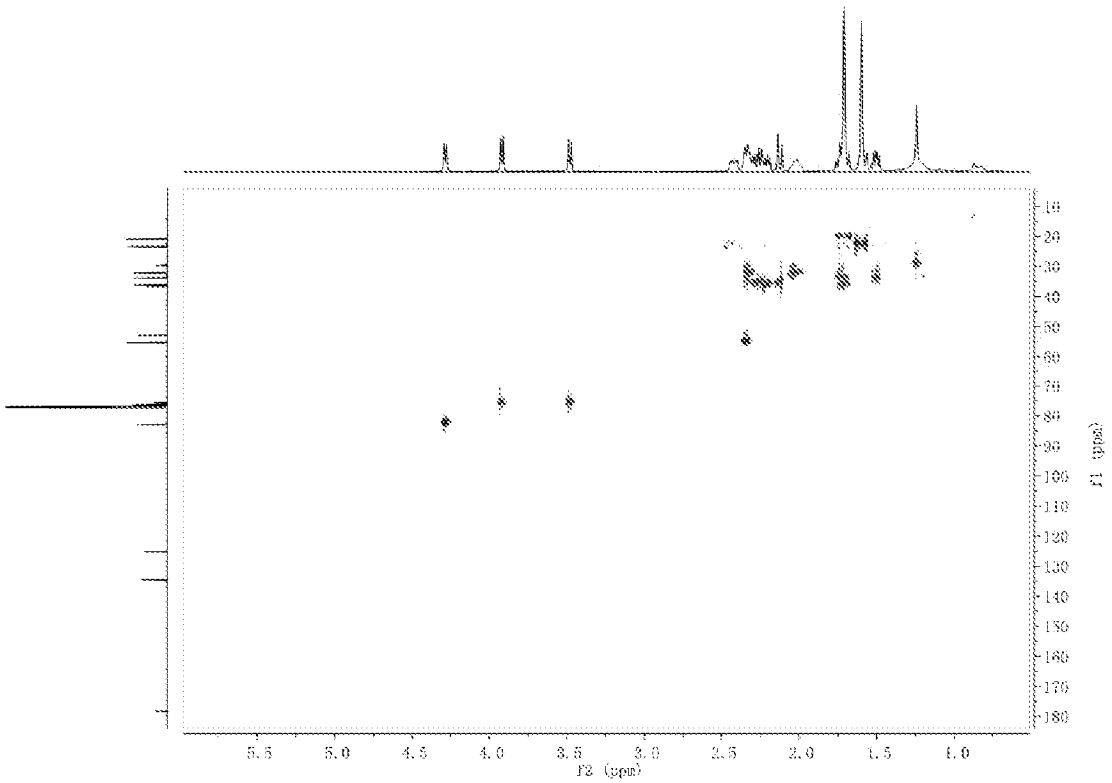


# FIG. 23L

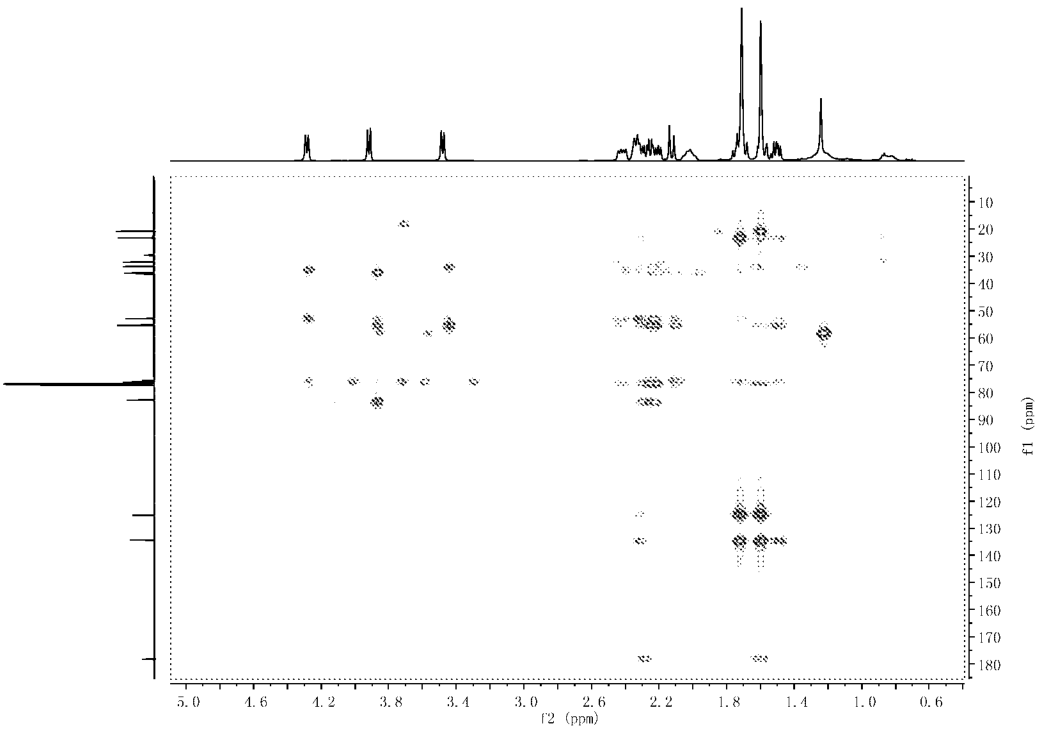

# FIG. 23M

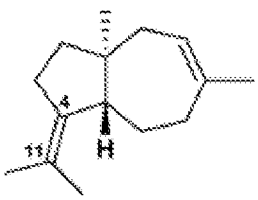

(+)-dauca-4(11),8-diene

$[\alpha]_D^{22} > +40°$ (*n*-hexane; $c < 0.1$)

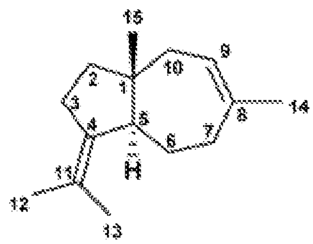

1, (-)-dauca-4(11),8-diene

$[\alpha]_D^{22} = -30°$ (*n*-hexane; $c = 0.1$)

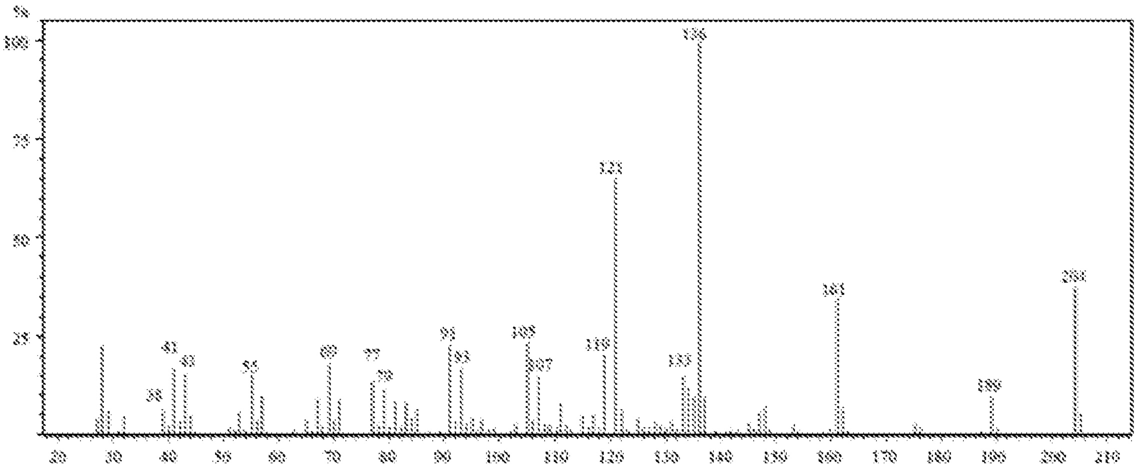

# FIG. 24A

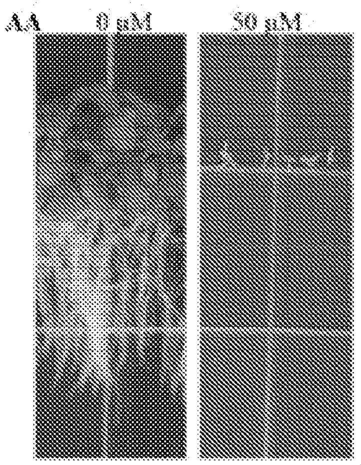


# FIG. 24B

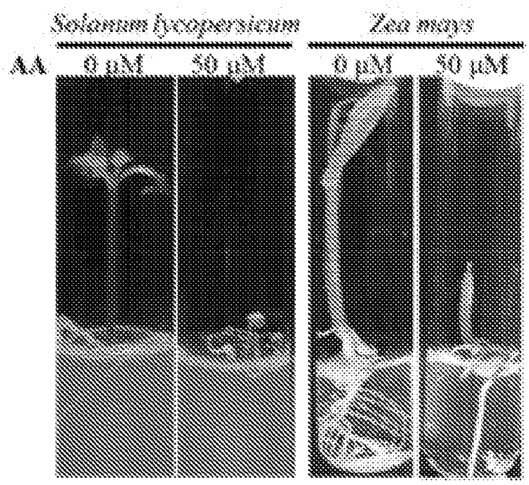


# FIG. 24C

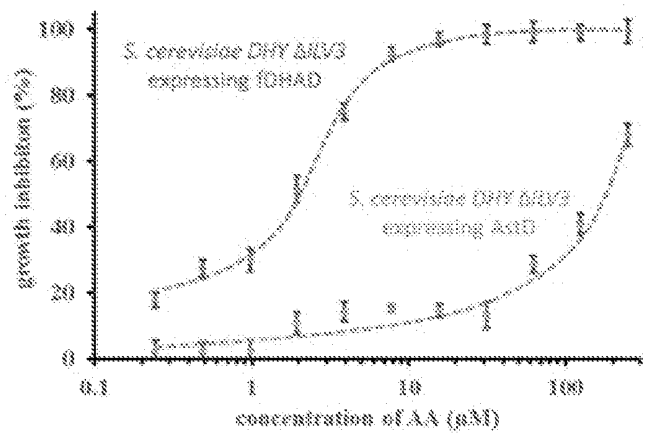


# FIG. 24D

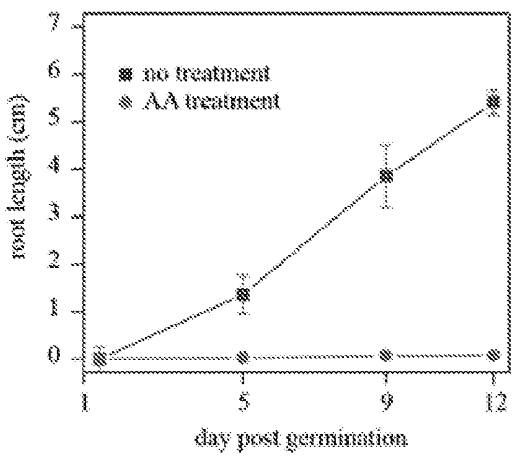

FIG. 25A

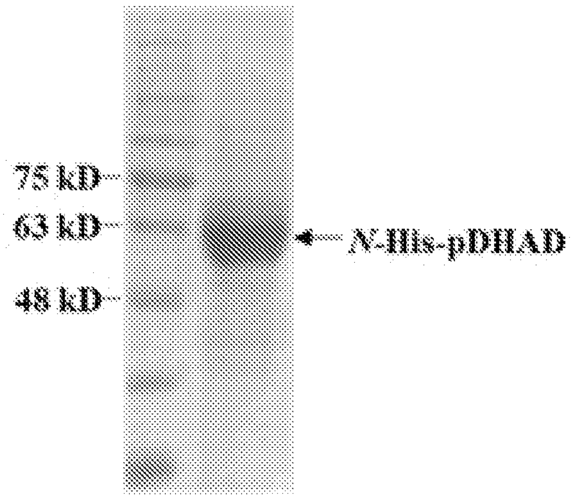


FIG. 25B

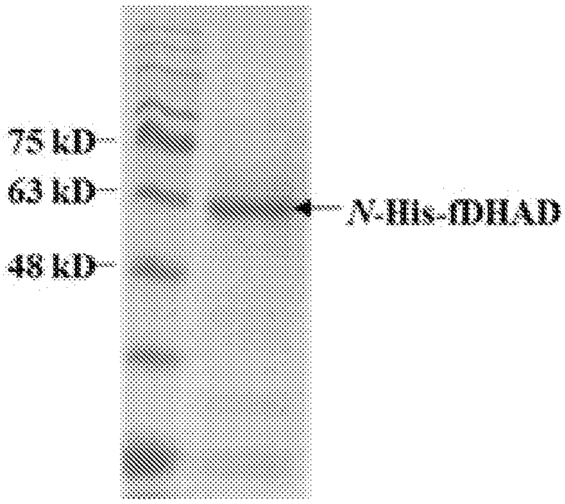


FIG. 25C

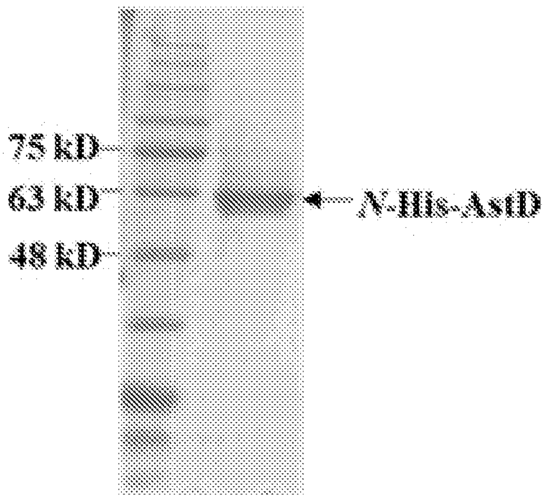

# FIG. 26A

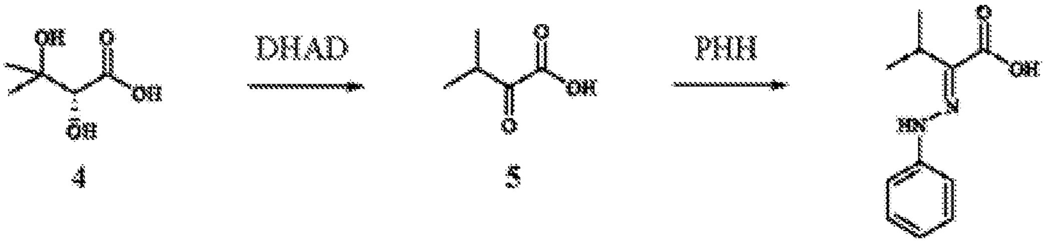


4

5

6

MW=206 [M+H]+=207

# FIG. 26B

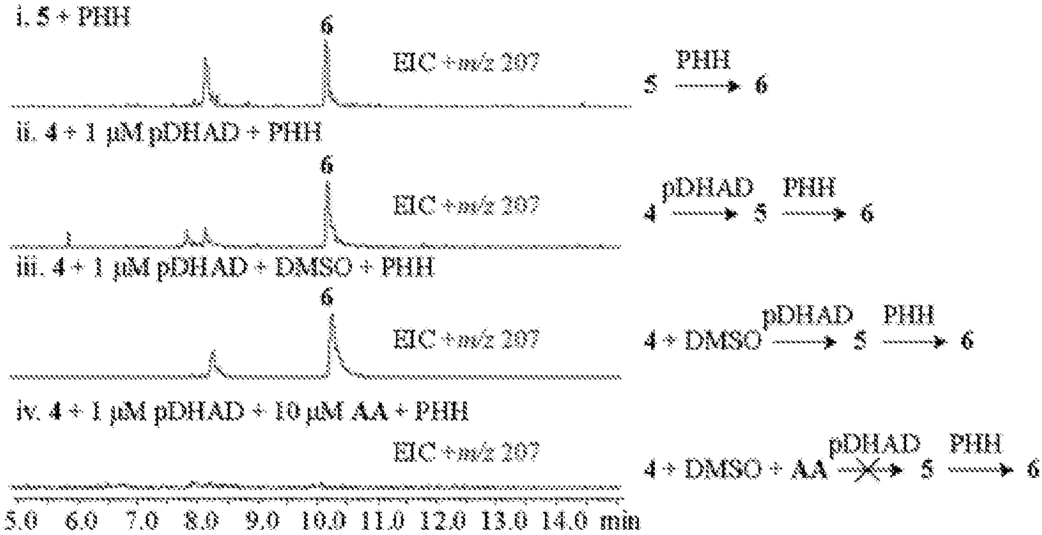

FIG. 27A

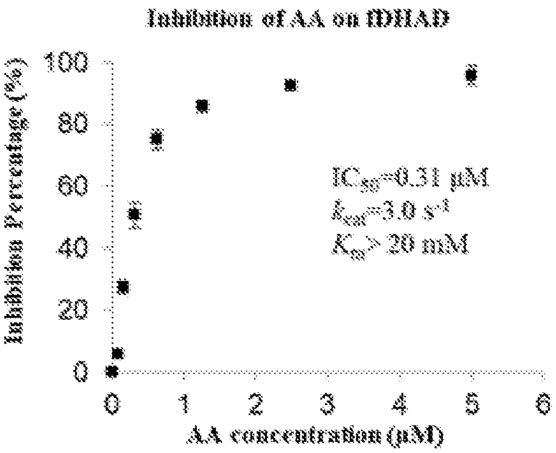


FIG. 27B

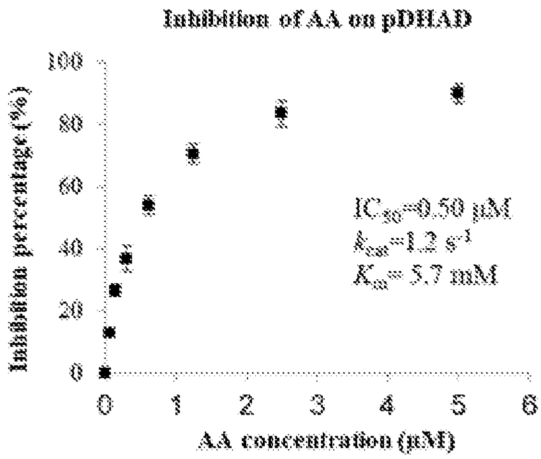


FIG. 27C

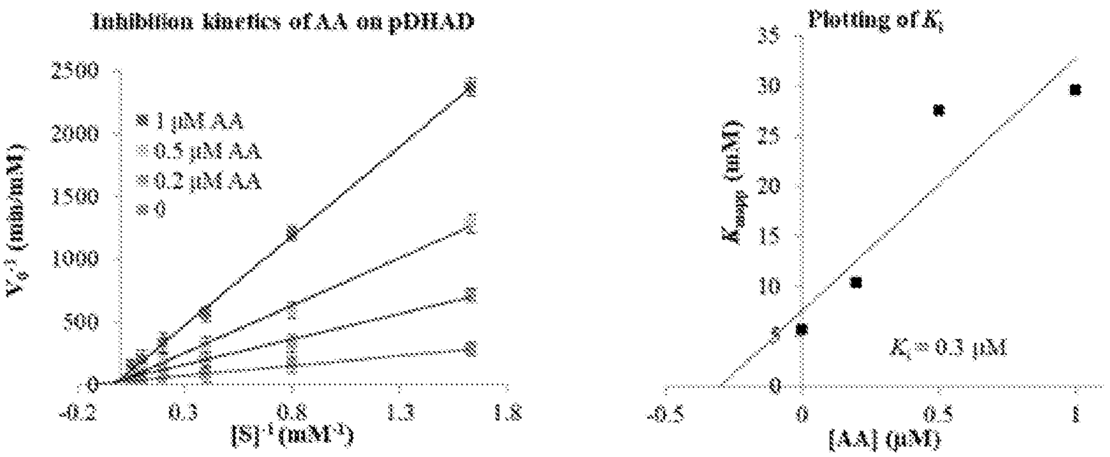


FIG. 27D

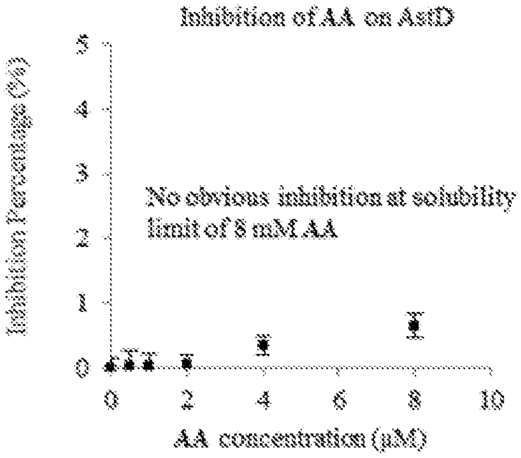

# FIG. 28

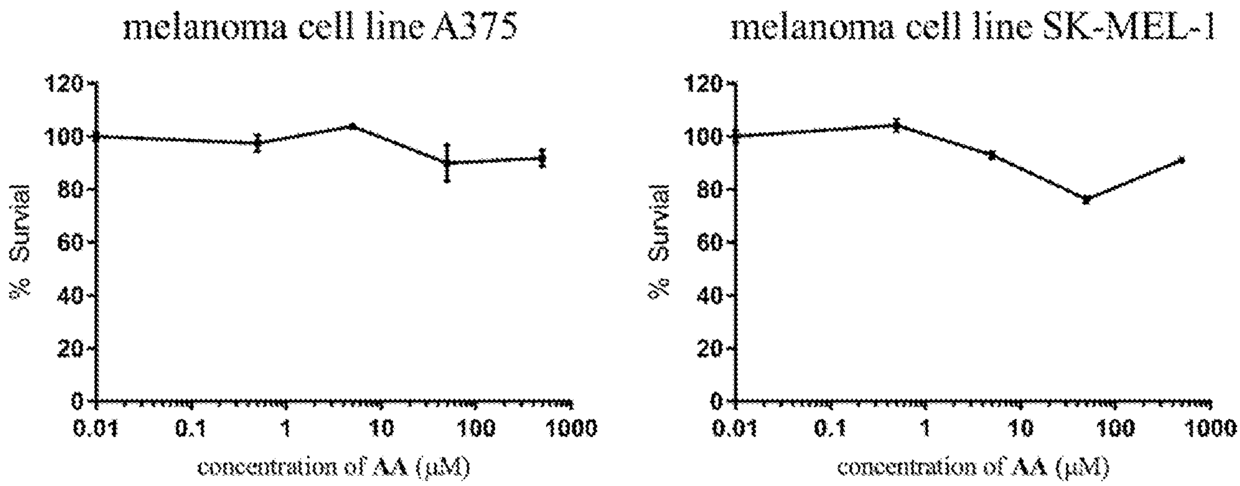
melanoma cell line A375
melanoma cell line SK-MEL-1
120
100
80
60
40
20
0
% Survial
0.01
0.1
1
10
100
1000
concentration of AA (μM)

FIG. 29A

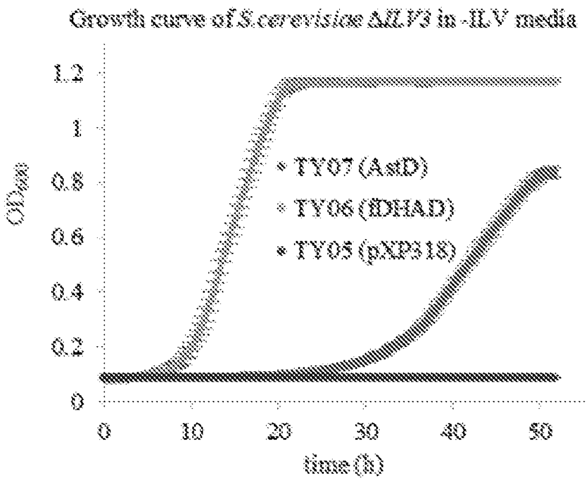


FIG. 29B

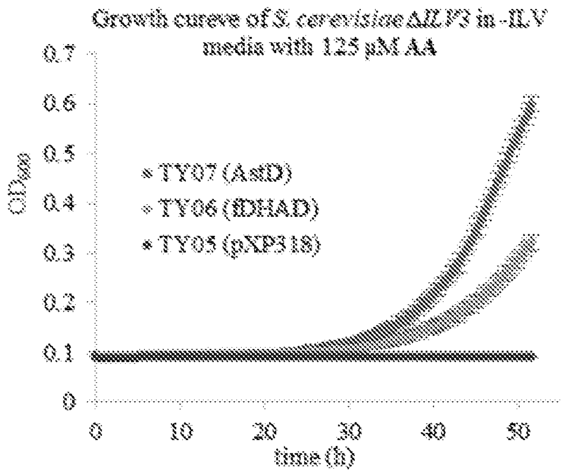


FIG. 29C

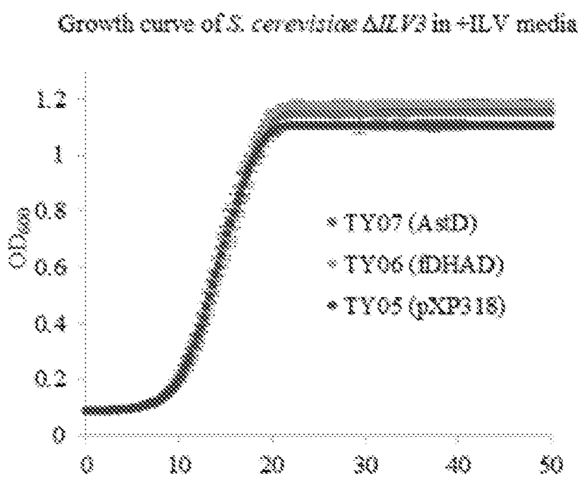


FIG. 29D

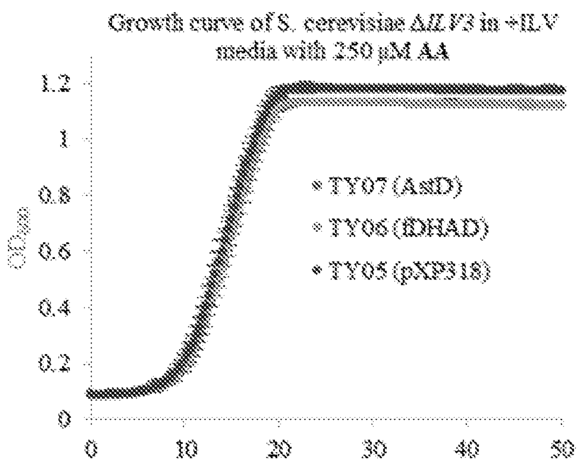

FIG. 30A

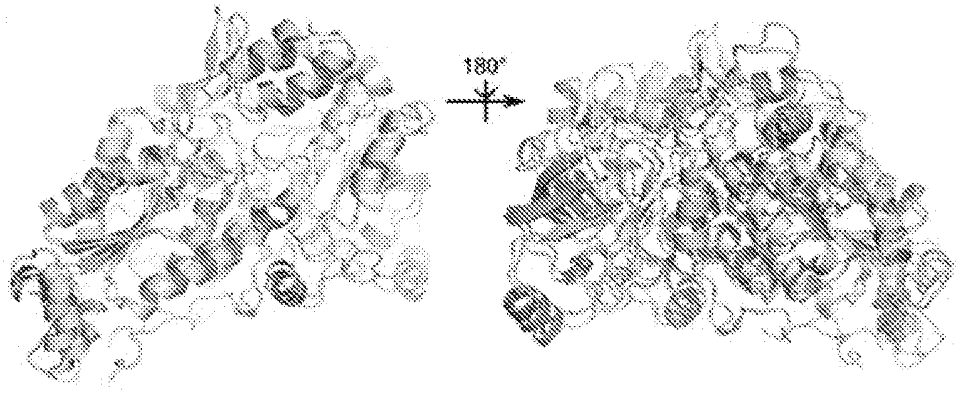


FIG. 30B

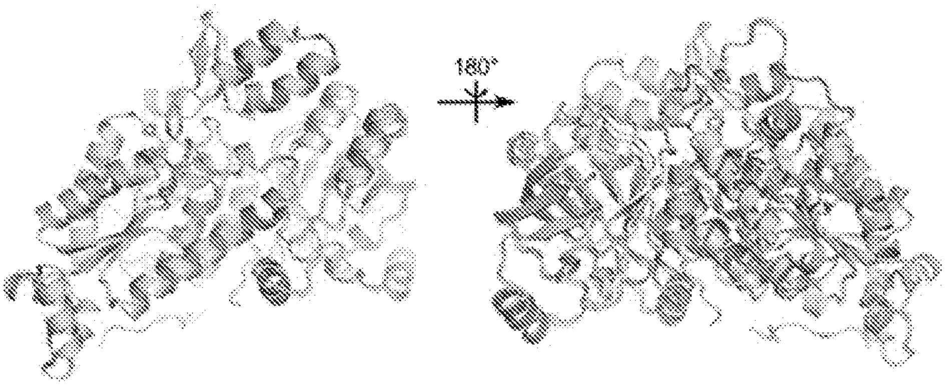


FIG. 30C

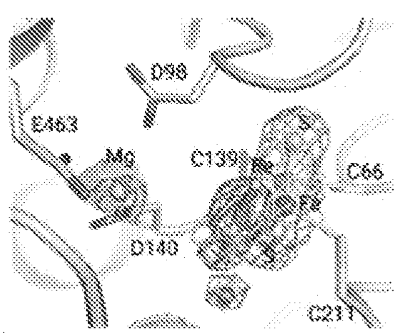


FIG. 30D

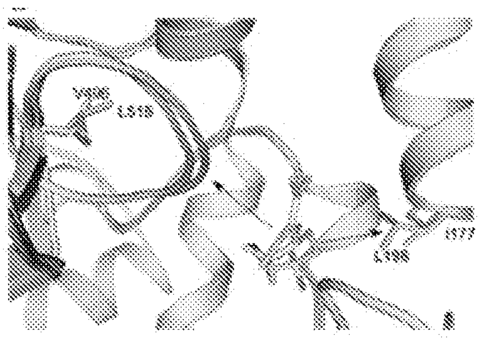

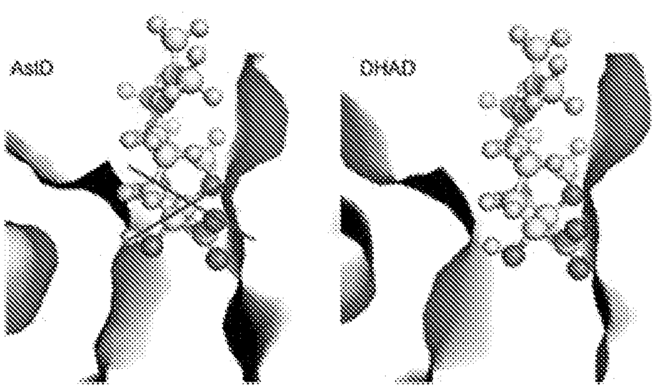


FIG. 30E

# FIG. 31A

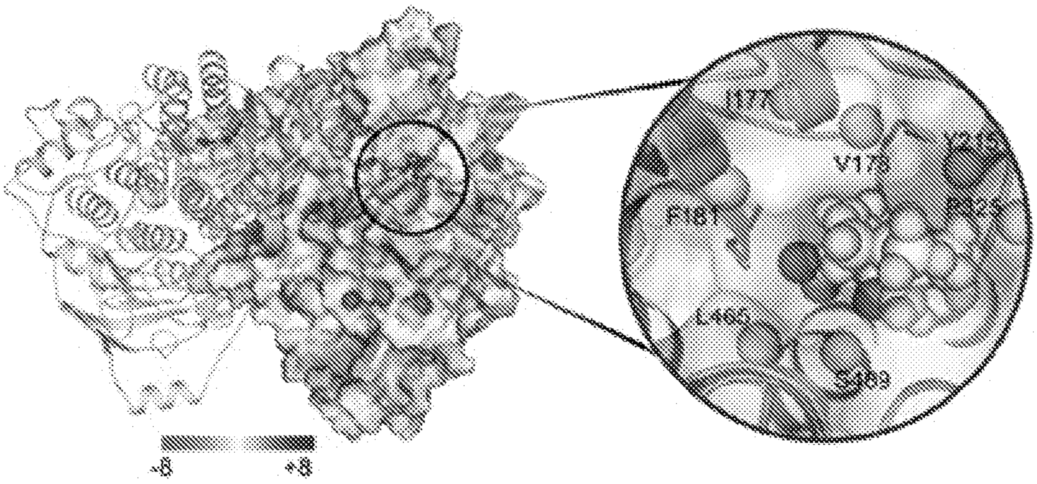


# FIG. 31B

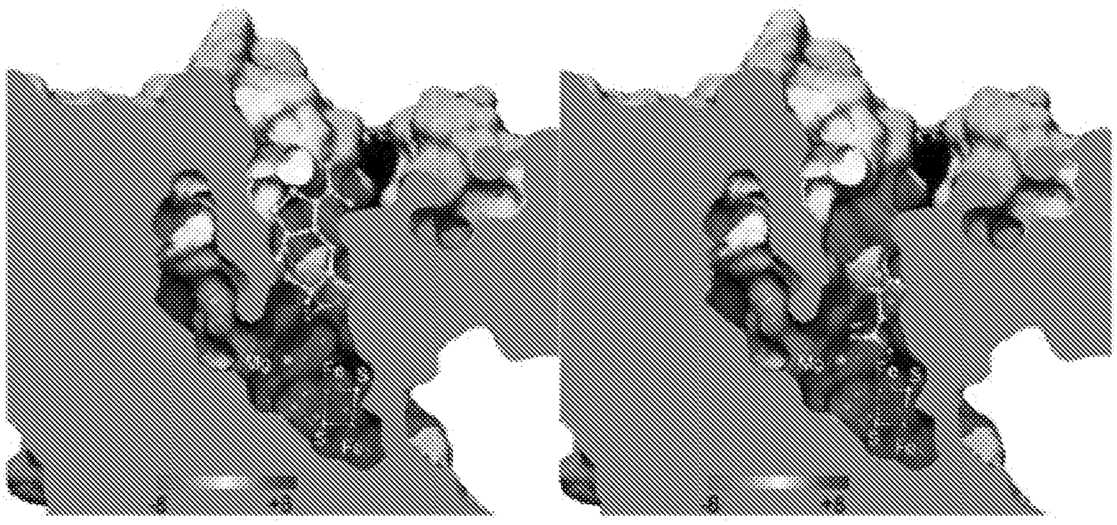

# FIG. 32

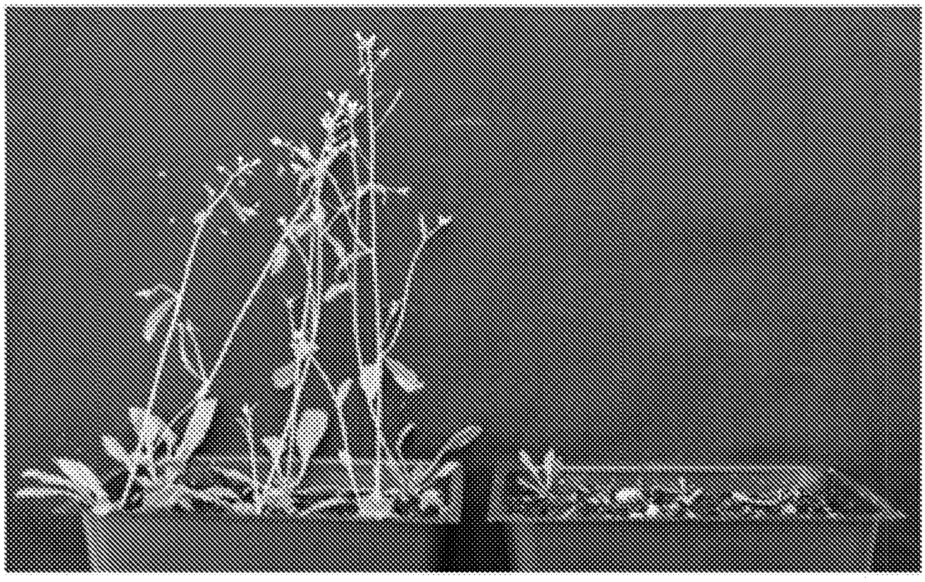

Solvent 250 µM AA in solvent

FIG. 33A

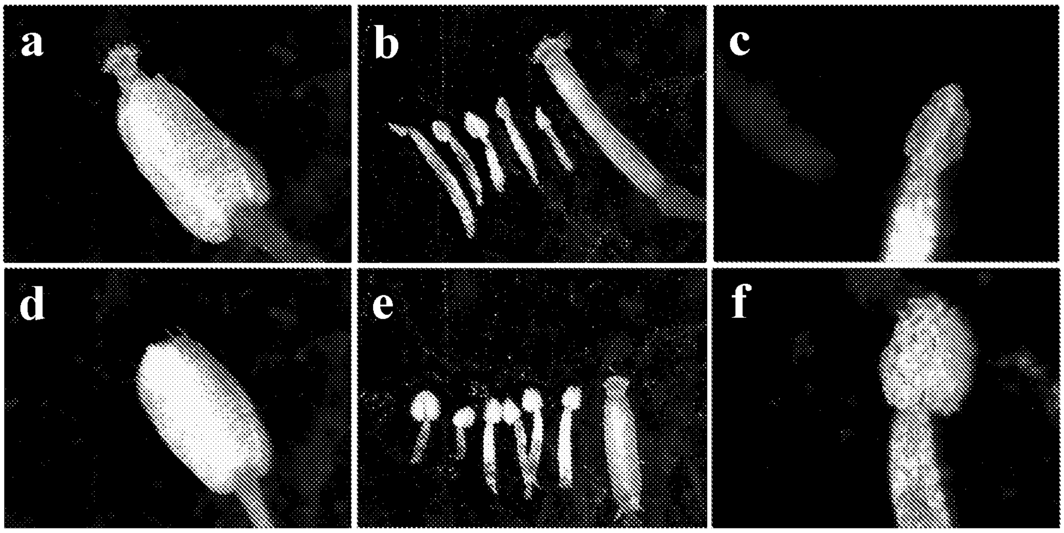


FIG. 33B

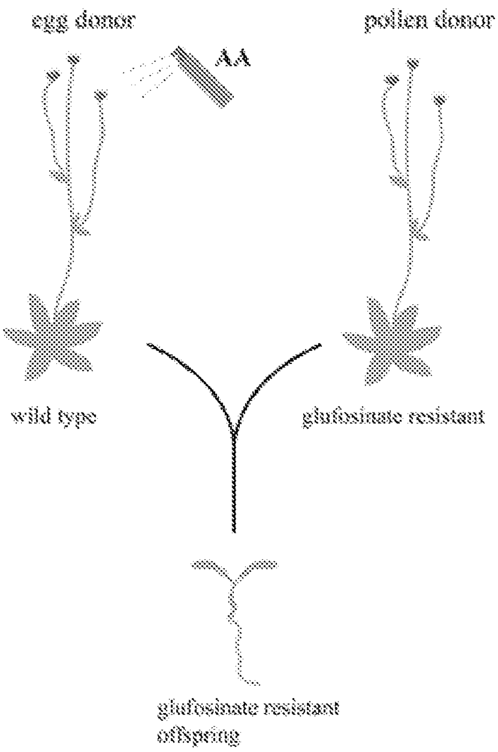


FIG. 33C

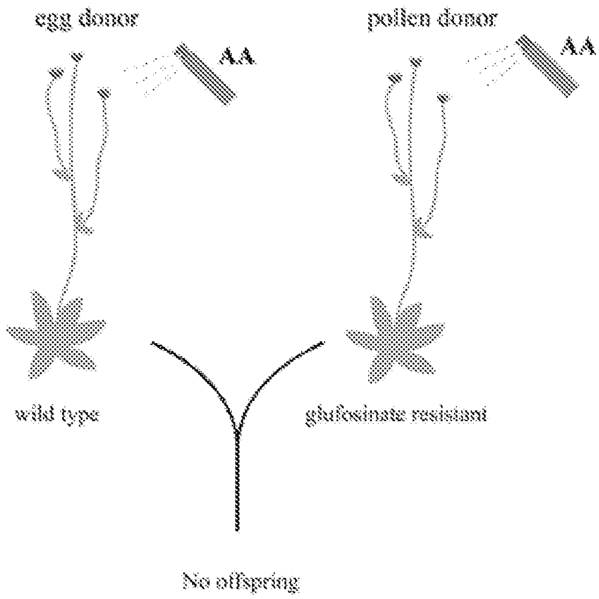

# FIG. 33D

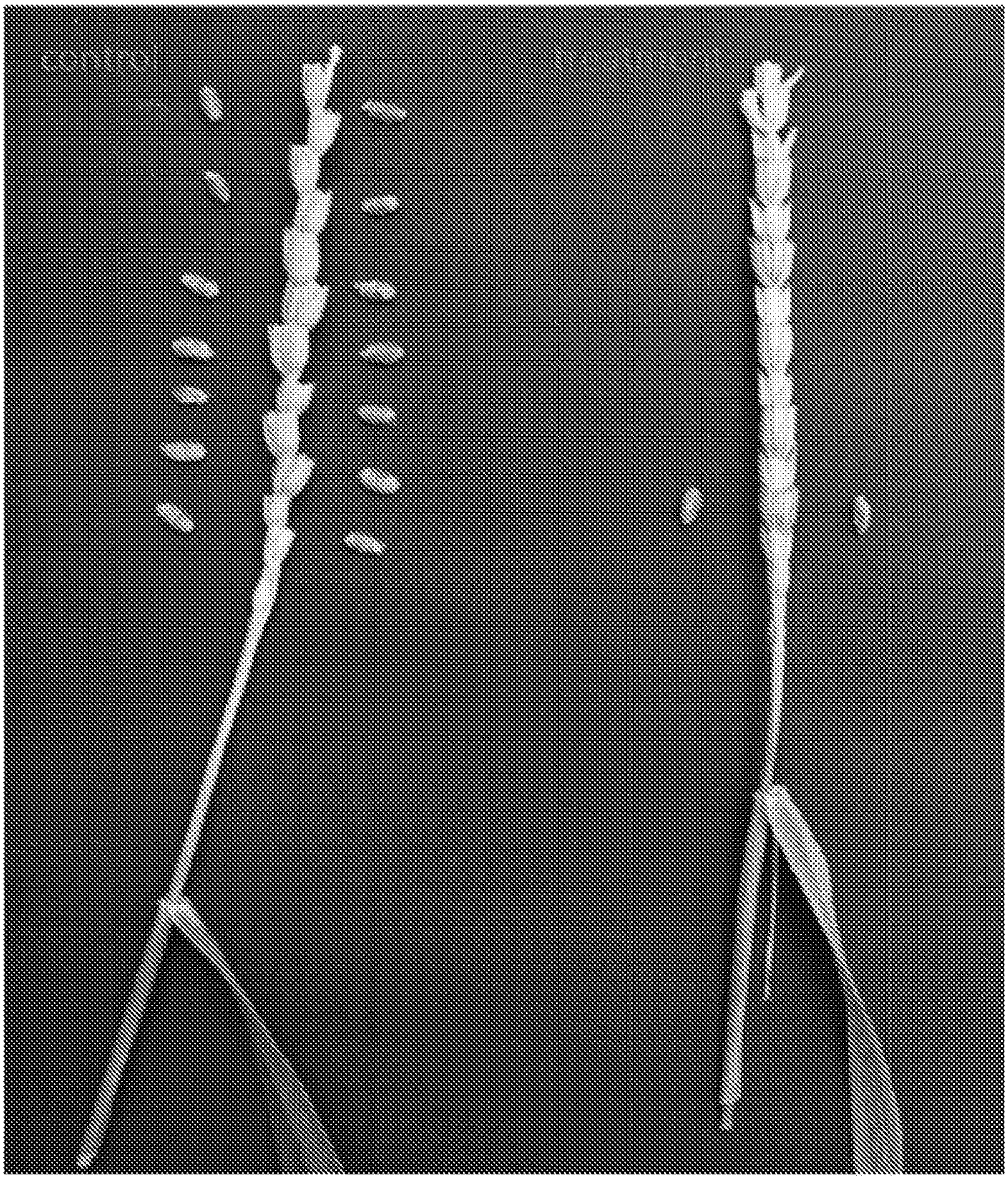

FIG. 34A

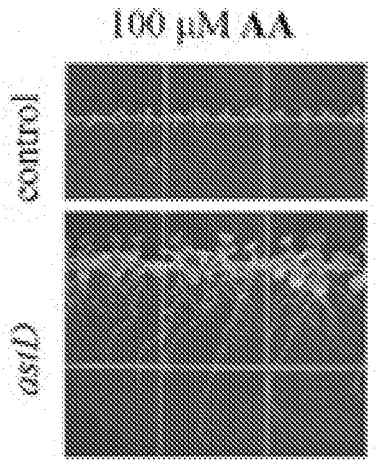


FIG. 34B

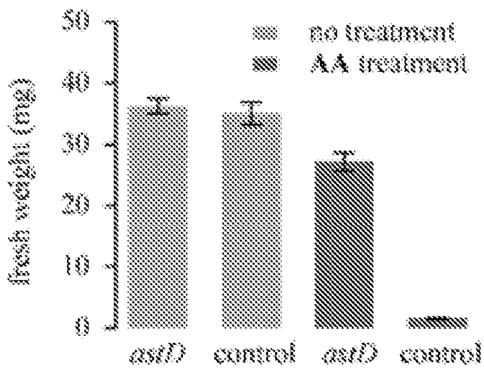


FIG. 34C

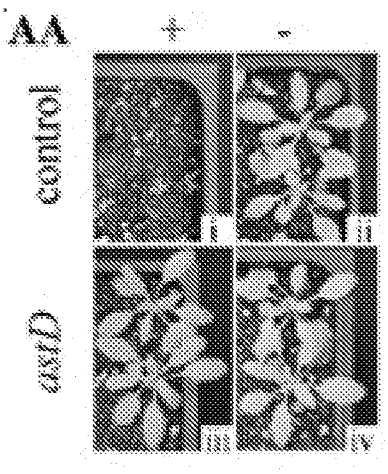


FIG. 34D

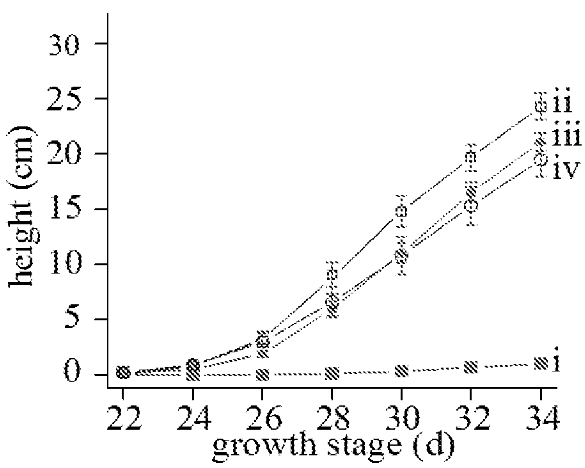

# FIG. 35

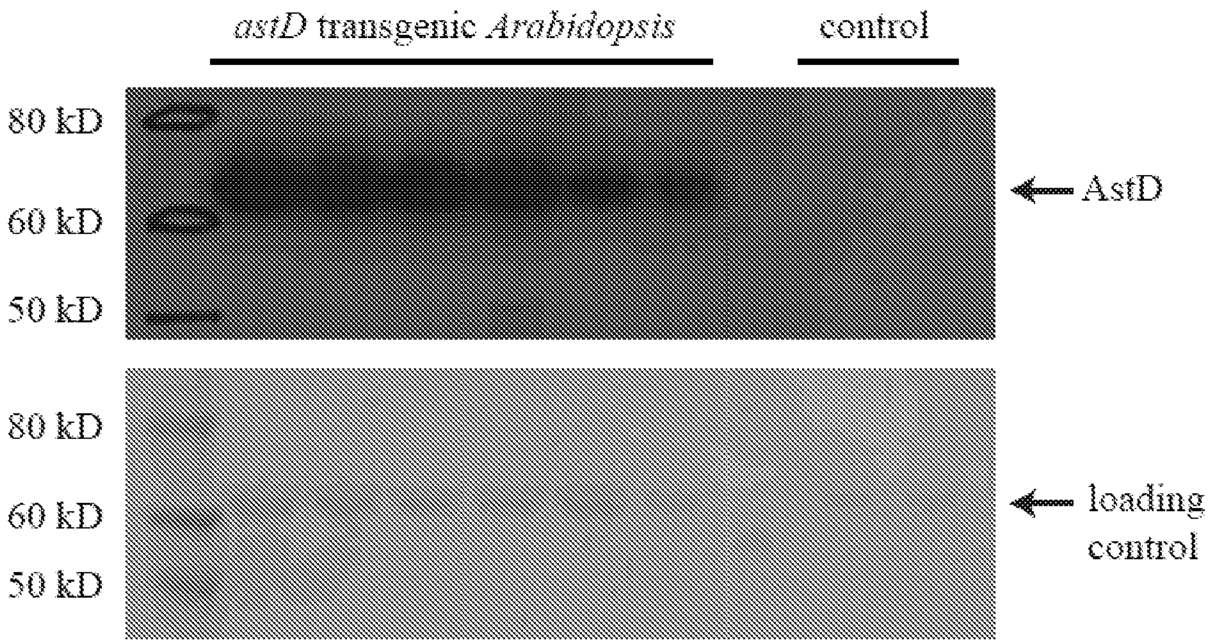

astD transgenic Arabidopsis
control
80 kD
60 kD
50 kD
AstD
80 kD
60 kD
50 kD
loading control

FIG. 36

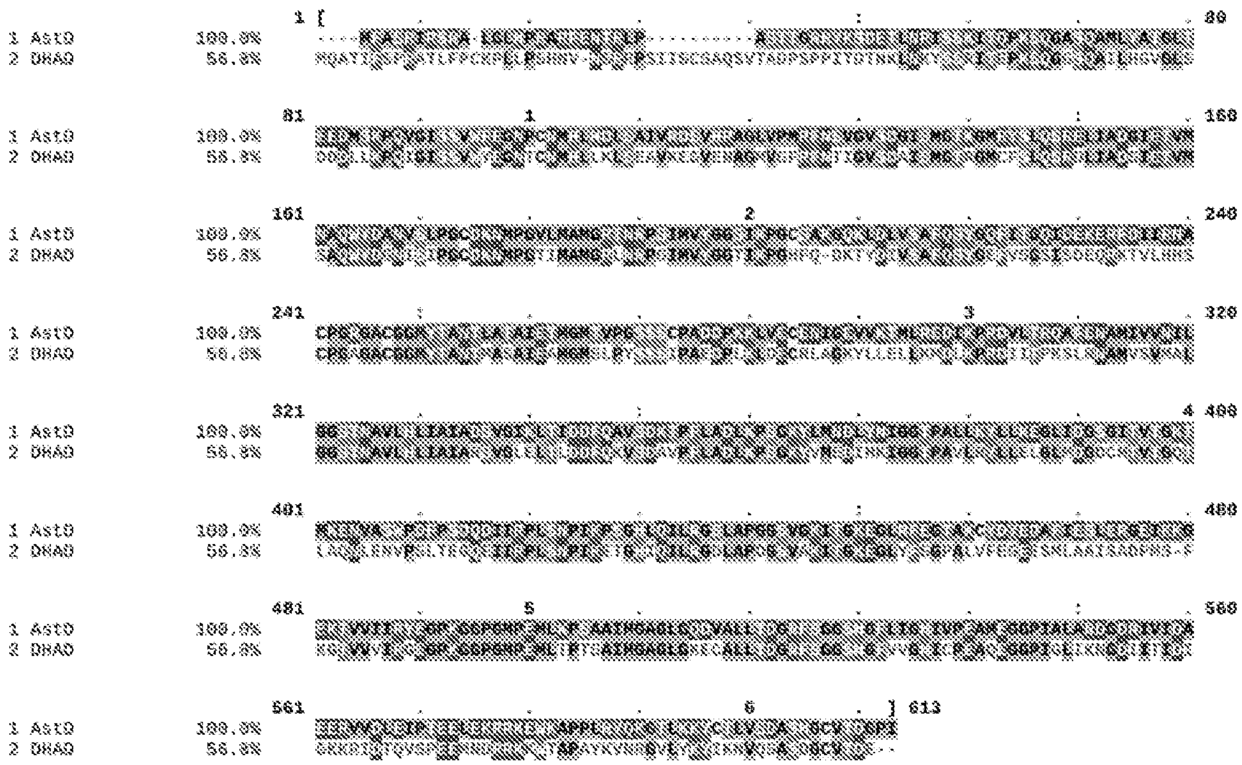

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4500707 A
- US 5436327 A
- US 5700637 A
- US 4683195 A
- US 20010010913 A
- US 5641876 A
- US 5683439 A
- US 5608149 A
- US 5608144 A
- US 5604121 A
- US 5569597 A
- US 5466785 A
- US 5399680 A
- US 5268463 A
- US 5608142 A
- US 5689049 A
- US 5689051 A
- US 5563055 A
- US 4945050 A


### Non-patent literature cited in the description


- **SHIMADA, A. et al.** *Zeitschrift für Naturforschung*, 2005, vol. 60 (7-8), 572-576
- **CREIGHTON**. *Proteins*, 1984
- **MATTEUCCI et al.** *Tetrahedron Lett*, 1980, vol. 21, 719-722
- **THOMPSON et al.** *Nucleic Acids Res.*, 1994, vol. 22, 4673-4680
- **HIGGINS et al.** *Methods Enzymol*, 1996, vol. 266, 383-402
- **TAMURA et al.** *Mol. Biol. & Evo.*, 2007, vol. 24, 1596-1599
- **SAITOU** ; **NEI**. *Mol. Biol. & Evo.*, 1987, vol. 4, 406-425
- **ZUCKERKANDL** ; **PAULING**. Evolving Genes and Proteins. Academic Press, 1965, 97-166
- **EISEN**. *Genome Res*, 1998, vol. 8, 163-167
- **EISEN**. *Genome Res.*, 1998, vol. 8, 163-167
- **FENG** ; **DOOLITTLE**. *J. Mol. Evol.*, 1987, vol. 25, 351-360
- **MOUNT**. Bioinformatics: Sequence and Genome Analysis. Cold Spring Harbor Laboratory Press, 2001, 543
- **ALTSCHUL et al.** *Nucleic Acids Res.*, 1997, vol. 25, 3389
- **MYERS** ; **MILLER**. *CABIOS*, 1988, vol. 4, 11-17
- **SMITH et al.** *Adv. Appl. Math.*, 1981, vol. 2, 482
- **NEEDLEMAN** ; **WUNSCH**. *J. Mol. Biol.*, 1970, vol. 48, 443-453
- **PEARSON** ; **LIPMAN**. *Proc. Natl. Acad. Sci.*, 1988, vol. 85, 2444-2448
- **KARLIN** ; **ALTSCHUL**. *Proc. Natl. Acad. Sci. USA*, 1990, vol. 87, 2264-2268
- **KARLIN** ; **ALTSCHUL**. *Proc. Natl. Acad. Sci. USA*, 1993, vol. 90, 5873-5877
- **HIGGINS et al.** *Gene*, 1988, vol. 73, 237-244
- **HIGGINS et al.** *CABIOS*, 1989, vol. 5, 151-153
- **CORPET et al.** *Nucleic Acids Res.*, 1988, vol. 16, 10881-90
- **HUANG et al.** *CABIOS*, 1992, vol. 8, 155-65
- **PEARSON et al.** *Meth. Mol. Biol.*, 1994, vol. 24, 307-331
- **ALTSCHUL et al.** *J. Mol. Biol.*, 1990, vol. 215, 403-410
- **SAMBROOK et al.** Molecular Cloning: A Laboratory Manual. Cold Spring Harbor Laboratory, 1989, vol. 1-3
- **BERGER** ; **KIMMEL**. Guide to Molecular Cloning Techniques, Methods in Enzymology. Academic Press, Inc., 1987, vol. 152
- Quantitative Filter Hybridisation. **ANDERSON** ; **YOUNG**. Nucleic Acid Hybridisation, A Practical Approach.. TRL Press, 1985, 73-111
- **WAHL** ; **BERGER**. *Methods Enzymol.*, 1987, vol. 152, 399-407
- **KIMMEL**. *Methods Enzymo.*, 1987, vol. 152, 507-511
- Nontransgenic Genome Modification in Plant Cells. *Plant Physiology*, 2010, vol. 154, 1079-1087
- **ROGERS et al.** *Meth. in Enzymol.*, 1987, vol. 153, 253-277
- **SCHARDL et al.** *Gene*, 1987, vol. 61, 1-11
- **BERGER et al.** *Proc. Natl. Acad. Sci. USA*, 1989, vol. 86, 8402-8406
- **MURRAY et al.** *Nucl. Acids Res*, 1989, vol. 17, 477-498
- **ODELL et al.** *Nature*, 1985, vol. 313

- **MCELROY et al.** *Plant Cell*, 1985, vol. 2, 163-171
- **CHRISTENSEN et al.** *Plant Mol. Biol.*, 1989, vol. 12, 619-632
- **CHRISTENSEN et al.** *Plant Mol. Biol.*, 1992, vol. 18, 675-689
- **LAST et al.** *Theor. Appl. Genet*, 1991, vol. 81, 581-588
- **VELTEN et al.** *EMBO J.*, 1984, vol. 3, 2723-2730
- **ODELL et al.** *Nature*, 1985, vol. 313, 810-812
- **CROSSWAY et al.** *Biotechniques*, 1986, vol. 4, 320-334
- **RIGGS et al.** *Proc. Natl. Acad Sci. USA*, 1986, vol. 83, 5602-5606
- **PASZKOWSKI et al.** *EMBO J.*, 1984, vol. 3, 2717-2722
- Direct DNA Transfer into Intact Plant Cells via Microprojectile Bombardment. **TOMES et al.** Plant Cell, Tissue, and Organ Culture: Fundamental Methods. Springer-Verlag, 1995
- **MCCABE et al.** *Biotechnology*, 1988, vol. 6, 923-926
- **REISS et al.** *Mol. Gen. Genet.*, 1987, vol. 209 (1), 116-121
- **SETTLES** ; **MARTIENSSEN**. *Trends Cell Biol*, 1998, vol. 12, 494-501
- **SCOTT et al.** *J Biol Chem*, 2000, vol. 10, 1074
- **LUQUE** ; **CORREAS**. *J Cell Sci*, 2000, vol. 113, 2485-2495
- **MCCORMICK et al.** *Plant Cell. Reports*, 1986, 81-84
- **CLOUGH SJ** ; **BENT AF**. *Plant J*, 1998
- **N. LIU et al.** Identification and Heterologous Production of a Benzoyl-Primed Tricarboxylic Acid Polyketide Intermediate from the Zaragozic Acid A Biosynthetic Pathway.. *Org. Lett.*, 2017, vol. 19, 3560-3563
- **W. XU** ; **X. CAI** ; **M. E. JUNG** ; **Y. TANG**. Analysis of Intact and Dissected Fungal Polyketide Synthase-Nonribosomal Peptide Synthetase in Vitro and in Saccharomyces cerevisiae.. *J. Am. Chem. Soc.*, 2010, vol. 132, 13604-13607
- **M.-C. TANG et al.** Discovery of unclustered fungal indole diterpene biosynthetic pathways through combinatorial pathway reassembly in engineered yeast. *J. Am. Chem. Soc.*, 2015, vol. 137, 13724-13727
- **F. FANG et al.** , A vector set for systematic metabolic engineering in Saccharomyces cerevisiae.. *Yeast*, 2011, vol. 28, 123-136
- **L. G. COOL**. ent-Daucane and acorane sesquiterpenes from Cupressocyparis leylandii foliage.. *Phytochemistry*, 2001, vol. 58, 969-972
- **L. GOLDSCHMIDT** ; **D. R. COOPER** ; **Z. S. DEREWENDA** ; **D. EISENBERG**. Toward rational protein crystallization: a web server for the design of crystallizable protein variants.. *Protein Sci*, 2007, vol. 16, 1569-1576
- **N. ESWAR et al.** Curr. Protoc. Protein Sci.. John Wiley & Sons, Inc., 2007
- **E. HARDER et al.** OPLS3: a force field providing broad coverage of drug-like small molecules and proteins.. *J. Chem. Theory Comput*, 2016, vol. 12, 281-296
- **C. R. SØNDERGAARD** ; **M. H. M. OLSSON** ; **M. ROSTKOWSKI** ; **J. H. JENSEN**. Improved treatment of ligands and coupling effects in empirical calculation and rationalization of pKa values.. *J. Chem. Theory Comput.*, 2011, vol. 7, 2284-2295
- **J. R. GREENWOOD** ; **D. CALKINS** ; **A. P. SULLIVAN** ; **J. C. SHELLEY**. Towards the comprehensive, rapid, and accurate prediction of the favorable tautomeric states of drug-like molecules in aqueous solution.. *J. Comput. Aided Mol. Des.*, 2010, vol. 24, 591-604
- **R. A. FRIESNER et al.** Glide: a new approach for rapid, accurate docking and scoring. 1. method and assessment of docking Accuracy. *J. Med. Chem.*, 2004, vol. 47, 1739-1749
- **S. YUAN** ; **H. C. S. CHAN** ; **S. FILIPEK** ; **H. VOGEL**. PyMOL and Inkscape bridge the data and the data visualization.. *Structure*, 2016, vol. 24, 2041-2042
- **S. YUAN** ; **H. C. S. CHAN** ; **Z. HU**. Using PyMOL as a platform for computational drug design.. *Wiley Interdiscip. Rev. Comput. Mol. Sci.*, 2017, vol. 7, e1298
- **S. GENHEDEN** ; **U. RYDE**. The MM/PBSA and MM/GBSA methods to estimate ligand-binding affinities.. *Expert Opin. Drug Discov.*, 2015, vol. 10, 449-461
- **S. J. CLOUGH** ; **A. F. BENT**. Floral dip: a simplified method forAgrobacterium-mediated transformation of Arabidopsis thaliana.. *Plant J.*, 1998, vol. 16, 735-743
- **J. KENNEDY et al.** Modulation of Polyketide Synthase Activity by Accessory Proteins During Lovastatin Biosynthesis.. *Science*, 1999, vol. 284, 1368-1372
- **T. B. REGUEIRA et al.** Molecular basis for mycophenolic acid biosynthesis in Penicillium brevicompactum.. *Appl. Environ. Microbiol*, 2011, vol. 77, 3035-3043
- **OTWINOWSKI, Z.** ; **MINOR, W.** ; **W JR, C. C.** Processing of X-ray diffraction data collected in oscillation mode.. *Methods Enzymol*, 1997, vol. 276, 307-326
- **MCCOY, A. J. et al.** Phaser crystallographic software.. *Journal of applied crystallography*, 2007, vol. 40, 658-674

- **WINN, M. D. et al.** Overview of the CCP4 suite and current developments.. *Acta crystallographica. Section D, Biological crystallography*, 2011, vol. 67, 235-242
- **MURSHUDOV, G. N. et al.** REFMAC5 for the refinement of macromolecular crystal structures. Acta crystallographica.. *Section D, Biological crystallography*, 2011, vol. 67, 355-367
- **EMSLEY, P.** ; **LOHKAMP, B.** ; **SCOTT, W. G.** ; **COWTAN, K.** Features and development of Coot. Acta crystallographica.. *Section D, Biological crystallography*, 2010, vol. 66, 486-501
- Comparative protein structure modeling using Modeller.. **ESWAR, N.** ; **ANDREAS D. BAXEVANIS . et al.** Current protocols in bioinformatics / editoral board. 2006
- **SIRIN, S. et al.** A computational approach to enzyme design: predicting ω-aminotransferase catalytic activity using docking and MM-GBSA scoring.. *J. Chem. Inf. Model*, 2014, vol. 54, 2334-2346
- **SHIMADA A** ; **YAMANE H** ; **KIMURA Y. Z**. *Naturforsch C.*, July 2008, vol. 63 (7-8), 554-6
- **SHIMADA A** ; **YAMANE H** ; **KIMURA Y. Z**. *Naturforsch C.*, July 2005, vol. 60 (7-8), 572-6
- **SHIMADA A** ; **KUSANO M** ; **TAKEUCHI S** ; **FUJIOKA S** ; **INOKUCHI T** ; **KIMURA Y. Z**. *Naturforsch C.*, May 2002, 459-64
- *Plant Physiol.*, July 1984, vol. 75 (3), 827-31
- *Plant Physiol.*, December 1993, vol. 103 (4), 1221-1226
- *J. Chem. Soc., Chem. Commun.*, 1978, 160-161
- *J. Antibiot*, 2016, vol. 69, 57-61
- **N. SOLTANI et al.** Potential corn yield losses from weeds in north America.. *Weed Technol.*, 2016, vol. 30, 979-984
- **C. J. SWANTON** ; **K. N. HARKER** ; **R. L. ANDERSON**. Crop losses due to weeds in Canada.. *Weed Technol.*, 1993, vol. 7, 537-542
- **L. P. GIANESSI**. The increasing importance of herbicides in worldwide crop production.. *Pest Manag. Sci.*, 2013, vol. 69, 1099-1105
- **H. KRAEHMER** ; **B. LABER** ; **C. ROSINGER** ; **A. SCHULZ**. Herbicides as weed control agents: state of the art: I. weed control research and safener technology: the path to modern agriculture.. *Plant Physiol.*, 2014, vol. 166, 1119-1131
- **I. HEAP**. Global perspective of herbicide-resistant weeds. *Pest Manag. Sci.*, 2014, vol. 70, 1306-1315
- **Q. YU** ; **S. POWLES**. Metabolism-based herbicide resistance and cross-resistance in crop weeds: a threat to herbicide sustainability and global crop production.. *Plant Physiol.*, 2014, vol. 166, 1106-1118
- **B. K. SINGH** ; **D. L. SHANER**. Biosynthesis of branched chain amino acids: from test tube to field.. *Plant Cell*, 1995, vol. 7, 935-944
- **G. M. KISHORE** ; **D. M. SHAH**. Amino acid biosynthesis inhibitors as herbicides.. *Annu. Rev. Biochem.*, 1988, vol. 57, 627-663
- **P. J. TRANEL** ; **T. R. WRIGHT**. Resistance of weeds to ALS-inhibiting herbicides: what have we learned?. *Weed Sci.*, 2002, vol. 50, 700-712
- **D. H. FLINT** ; **M. H. EMPTAGE**. Dihydroxy acid dehydratase from spinach contains a [2Fe-2S] cluster.. *J. Biol. Chem.*, 1988, vol. 263, 3558-3564
- **D. H. FLINT** ; **M. H. EMPTAGE** ; **M. G. FINNEGAN** ; **W. FU** ; **M. K. JOHNSON**. The role and properties of the iron-sulfur cluster in Escherichia coli dihydroxy-acid dehydratase.. *J. Biol. Chem.*, 1993, vol. 268, 14732-14742
- **D. H. FLINT** ; **A. NUDELMAN**. Studies on the active site of dihydroxy-acid dehydratase.. *Bioorg. Chem.*, 1993, vol. 21, 367-385
- **D. J. NEWMAN** ; **G. M. CRAGG**. Natural products as sources of new drugs from 1981 to 2014.. *J. Nat. Prod.*, 2016, vol. 79, 629-661
- **F. E. DAYAN** ; **S. O. DUKE**. Natural compounds as next generation herbicides.. *Plant Physiol.*, 2014, vol. 166, 1090-1105
- **T. PUSZTAHELYI** ; **I. HOLB** ; **I. PÓCSI**. Secondary metabolites in fungus-plant interactions.. *Front. Plant Sci.*, 2015, vol. 6, 573
- **PETER J. RUTLEDGE** ; **GREGORY L. CHALLIS**. Discovery of microbial natural products by activation of silent biosynthetic gene clusters.. *Nat. Rev. Microbiol.*, 2015, vol. 13, 509-523
- **N. ZIEMERT** ; **M. ALANJARY** ; **T. WEBER**. The evolution of genome mining in microbes - a review.. *Nat. Prod. Rep.*, 2016, vol. 33, 988-1005
- **JONATHAN KENNEDY et al.** Modulation of polyketide synthase activity by accessory proteins during lovastatin biosynthesis.. *Science*, 1999, vol. 284, 1368-1372
- **T. B. REGUEIRA et al.** Molecular basis for mycophenolic acid biosynthesis in Penicillium brevicompactum.. *Appl. Environ. Microbiol.*, 2011, vol. 77, 3035-3043
- **X. TANG et al.** Identification of thiotetronic acid antibiotic biosynthetic pathways by target-directed genome mining.. *ACS Chem. Biol.*, 2015, vol. 10, 2841-2849
- **M. A. FISCHBACH** ; **C. T. WALSH**. Assembly-line enzymology for polyketide and nonribosomal peptide antibiotics: logic, machinery, and mechanisms. *Chem. Rev.*, 2006, vol. 106, 3468-3496
- **D. W. CHRISTIANSON**. Structural biology and chemistry of the terpenoid cyclases.. *Chem. Rev.*, 2006, vol. 106, 4312-4332
- **M.-C. TANG et al.** , Discovery of unclustered fungal indole diterpene biosynthetic pathways through combinatorial pathway reassembly in engineered yeast.. *J. Am. Chem. Soc.*, 2015, vol. 137, 13724-13727

- **YOSHISUKE TSUDA et al.** , Aspterric acid, a new sesquiterpenoid of the carotane group, a metabolite from Aspergillus terreus IFO-6123. X-Ray crystal and molecular structure of its p-bromobenzoate. *J. Chem. Soc., Chem. Commun.*, 1978, vol. 0, 160-161
- **A. SHIMADA et al.** Aspterric acid and 6-hydroxymellein, inhibitors of pollen development in Arabidopsis thaliana, produced by Aspergillus terreus.. *Z. Naturforsch. C*, 2002, vol. 57, 459-464
- **M. M. RAHMAN et al.** The crystal structure of a bacterial L-arabinonate dehydratase contains a [2Fe-2S] cluster.. *ACS Chem. Biol.*, 2017, vol. 12, 1919-1927
- **R. C. KIRKWOOD**. Use and mode of action of adjuvants for herbicides: a review of some current work.. *Pestic. Sci.*, 1993, vol. 38, 93-102
- **G. HOERLEIN**. Glufosinate (phosphinothricin), a natural amino acid with unexpected herbicidal properties.. *Rev. Environ. Contam. Toxicol.*, 1994, vol. 138, 73-145
- Advances in chemical hybridization. **D. H. MCRAE**. Plant Breed. Rev.. John Wiley & Sons, Inc., 1985, 169-191
- **C. M. BENBROOK**. Trends in glyphosate herbicide use in the United States and globally.. *Environ. Sci. Eur.*, 2016, vol. 28, 3-19
- **JON, C.** ; **CHRISTOPHER, W.** Lessons from natural molecules.. *Nature*, 2004, vol. 432, 829-837
- **CLEVENGER, K. D. et al.** A scalable platform to identify fungal secondary metabolites and their gene clusters.. *Nat. Chem. Biol.*, 2017, vol. 13, 895
- **ALANJARY, M. et al.** The antibiotic resistant target seeker (ARTS), an exploration engine for antibiotic cluster prioritization and novel drug target discovery.. *Nucleic Acids Res.*, 2017, vol. 45, W42-W48
- **YEH, H.-H. et al.** Resistance Gene-Guided Genome Mining: Serial Promoter Exchanges in Aspergillus nidulans Reveal the Biosynthetic Pathway for Fellutamide B, a Proteasome Inhibitor.. *ACS Chem. Biol*, 2016, vol. 11, 2275-2284
- **AMORIM FRANCO, T. M.** ; **BLANCHARD, J. S.** Bacterial branched-chain amino acid biosynthesis: structures, mechanisms, and drugability.. *Biochemistry*, 2017, vol. 56, 5849-5865
- **SWANTON, C. J.** ; **HARKER, K. N.** ; **ANDERSON, R. L.** Crop losses due to weeds in Canada.. *Weed Technol*, 1993, vol. 7, 537-542